# EUROPÄISCHE PATENTSCHRIFT

(11) **EP 1 869 053 B1**
(45) Veröffentlichungstag und Bekanntmachung des Hinweises auf die Patenterteilung: **09.10.2013**
(21) Anmeldenummer: 06724034.1
(22) Anmeldetag: 05.04.2006
(51) Int. Cl.: C07D 498/04, A61K 31/4353, A61P 29/02

(54) **SUBSTITUIERTE 4,5,6,7-TETRAHYDRO-ISOXAZOLO[4,5-C]PYRIDIN-VERBINDUNGEN UND DEREN VERWENDUNG ZUR HERSTELLUNG VON ARZNEIMITTELN**
SUBSTITUTED 4,5,6,7-TETRAHYDRO-ISOXAZOLO[4,5-C]PYRIDINE COMPOUNDS AND THEIR USE IN THE PRODUCTION OF MEDICAMENTS
COMPOSES 4,5,6,7-TETRAHYDRO-ISOXAZOLO[4,5-C]PYRIDINE SUBSTITUES ET LEUR UTILISATION POUR PRODUIRE DES MEDICAMENTS

(30) Priorität: 07.04.2005 DE 102005016170
(43) Veröffentlichungstag der Anmeldung: 26.12.2007
(73) Patentinhaber: Grünenthal GmbH, 52078 Aachen (DE)
(72) Erfinder: SUNDERMANN, Corinna, 61381 Friedrichsdorf (DE); SUNDERMANN, Bernd, 61381 Friedrichsdorf (DE); HOLENZ, Jörg, Boston 02127 MA (US); HENNIES, Hagen-Heinrich, 52152 Simmerath (DE)
(74) Vertreter: Brosch, Oliver
(86) Internationale Anmeldenummer: PCT/EP2006/003084
(87) Internationale Veröffentlichungsnummer: WO 2006/105945

(56) Entgegenhaltungen:
- WO-A-2005/110971
- US-A- 4 309 433
- US-A1- 2004 192 916
- NITU A N ET AL: "EMERGING TRENDS IN THE PHARMACOTHERAPHY OF CHRONIC PAIN" EXPERT OPINION ON INVESTIGATIONAL DRUGS, ASHLEY PUBLICATIONS LTD., LONDON, GB, Bd. 12, Nr. 4, 1. April 2003 (2003-04-01), Seiten 545-559, XP009025745 ISSN: 1354-3784

## Beschreibung

Die vorliegende Erfindung betrifft substituierte 4,5,6,7-Tetrahydro-isoxazolo[4,5-c]pyridin-Verbindungen, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des weiteren sind sie bei neuropathischen Schmerzen, unter denen insbesondere Tumorpatienten leiden, häufig nicht ausreichend wirksam.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Prophylaxe und/oder Behandlung von Schmerzen, insbesondere akuten Schmerzen, chronischen Schmerzen und/oder neuropathischen Schmerzen.

Es wurde nun überraschenderweise gefunden, daß sich substituierte 4,5,6,7-Tetrahydro-isoxazolo[4,5-c]pyridin-Verbindungen der nachstehend angegebenen allgemeinen Formel I zur Noradrenalin-Rezeptor-Regulation, insbesondere zur Hemmung der Noradrenalin-Wiederaufnahme (NA-Uptake), zur 5-HT-Rezeptor-Regulation, insbesondere zur Hemmung der 5-Hydroxy-Tryptophan-Wiederaufnahme (5-HT-Uptake) und/oder zur Batrachotoxin-(BTX)-Rezeptor-Regulation eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher substituierte 4,5,6,7-Tetrahydro-isoxazolo[4,5-c]pyridin-Verbindungen der allgemeinen Formel I, 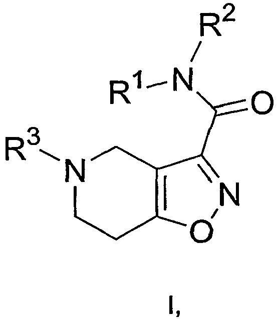 worin
- R¹: für -(CHR⁴)-(CH₂)_{c}-(CH₂)_{d}-C(=O)-OR⁵ mit c = 0 oder 1 und d = 0 oder 1;
für -(CHR⁶)-(CHR⁷)ₑ-V_{f}(CH₂)_{g}-Wₕ-(CH₂)ᵢ-R⁸ mit e = 0 oder 1, f = 0 oder 1, g = 0 oder 1, h = 0 oder 1 und i = 0 oder 1, worin V und W unabhängig voneinander jeweils für O, S, NH, N(CH₃) oder N(C₂H₅) stehen;
für einen linearen oder verzweigten, gesättigten oder ungesättigten, ggf. substituierten C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. aromatischen, ggf. substituierten mono- oder polyzyklischen Ringsystem kondensiert und/oder mit einer linearen oder verzweigten, ggf. substituierten C₁₋₅-Alkylen-Gruppe überbrückt sein kann,
wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NO₂, -O-CF₃, -S-CF₃, -SH, -S-C₁₋₅-Alkyl, - C₁₋₅-Alkyl, -C(=O)-H, -C(=O)-C₁₋₅-Alkyl, -C(=O)-OH, -C(=O)-O-C₁₋₅-Alkyl, - (CH₂)-C(=O)-OH, -(CH₂)-C(=O)-O-C₁₋₅-Alkyl, -(CH₂)-Benzo[b]furanyl, -O-Phenyl, -O-Benzyl), Phenyl, Benzyl, Naphthyl und -(CH₂)-Naphthyl substituiert sein können, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl,-C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
- R²: für einen Wasserstoff-Rest;
für -(CH₂)-R⁹
oder für einen linearen oder verzweigten, gesättigten oder ungesättigten, ggf. substituierten C₁₋₁₀ aliphatischen Rest steht;
oder
R¹ und R² zusammen mit dem sie verbindenden Stickstoffatom als Ringglied einen gesättigten oder ungesättigten, ggf, substituierten 4-, 5-, 6-, 7-, 8- oder 9-gliedrigen heterocycloaliphatischen Rest bilden,
wobei der heterocycloaliphatische Rest jeweils mit 1, 2, 3, 4 oder 5 Substituenten unabhängig voneinander ausgewählt aus der Gruppe bestehend aus R¹⁰, -C(=O)-R¹¹ und -(CH₂)-NH-C(=O)-R¹² substituiert und/oder jeweils weitere 1, 2, 3, 4 oder 5 Heteroatom(e) unabhängig voneinander ausgewählt aus der Gruppe bestehend aus Sauerstoff, Stickstoff und Schwefel als Ringglied(er) aufweisen kann;
- R³: für -C(=O)NR¹³R¹⁴;
für -C(=O)-R¹⁵;
für -C(=O)-(CH₂)ⱼ-Xₖ-(CH₂)ₘ-(CH₂)ₙ-C(=O)-OR¹⁶ mit j = 0 oder 1, k = 0 oder 1, m = 0 oder 1 und n = 0 oder 1, worin X für O, S, NH, N(CH₃), N(C₂H₅) oder CR¹⁷R¹⁸ steht;
für -C(=O)-(CHR¹⁹)-NH-C(=O)-OR²⁰;
für -C(=O)-(CH₂)-(CH₂)ₚ-(CH₂)_{q}-Yᵣ-(CH₂)s-R²¹ mit p = 0 oder 1, q = 0 oder 1, r = 0 oder 1 und s = 0 oder 1, worin Y für 0, S, NH, N(CH₃) oder N(C₂H₅) steht;
für -C(=O)-(CH=CH)-R²²;
für -S(=O)₂-R²³;
für -S(=O)₂-NR²⁴R²⁵;
für -C(=S)-NR²⁶-(CH₂)ₜ(CH₂)ᵤ-Zᵥ-R²⁷ mit t=0 oder 1, u = 0 oder 1 und v = 0 oder 1, worin Z für O, S, NH, N(CH₃) oder N(C₂H₅) steht;
für -C(=S)-NR²⁶-(CHR²⁸)-R²⁹;
für -C(=S)-NR²⁶-C(O)-R³⁰;
oder für -(CH₂)-R³¹ steht;
- R⁴: für -C(=O)-NH₂;
für einen Wasserstoff-Rest
oder für einen linearen oder verzweigten, gesättigten oder ungesättigten, ggf. substituierten C₁₋₁₀ aliphatischen Rest steht;
- R⁵, R¹⁷, R¹⁸, R²⁴ und R²⁵,: unabhängig voneinander, jeweils
für einen linearen oder verzweigten, gesättigten oder ungesättigten, ggf. s ubstituierten C₁₋₁₀ aliphatischen Rest stehen;
- R⁶, R⁷, R¹³, R¹⁶ und R²⁶,: unabhängig voneinander, jeweils
für einen Wasserstoff-Rest
oder für einen linearen oder verzweigten, gesättigten oder ungesättigten, ggf. substituierten C₁₋₁₀ aliphatischen Rest stehen;
- R⁸, R⁹, R²¹, R²², R²³, R³⁰ und R³¹,: unabhängig voneinander, jeweils
für einen ungesättigten oder gesättigten, ggf. substituierten 3-, 4-, 5-, 6-, 7-, 8-oder 9-gliedrigen cycloaliphatischen Rest, der mit einem gesättigten oder ungesättigten, ggf. aromatischen, ggf. substituierten mono- oder polyzyklischen Ringsystem kondensiert sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH, -C(=O)-O-C₁₋₅-Alkyl, -(CH₂)-C(=O)-OH, - (CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste - O-Phenyl, -O-Benzyl, Phenyl, -(CH₂)-Benzo[b]furanyl, Benzyl, Naphthyl und - (CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, - NO_{2,} -O-C₁₋₅-Alkyl, -C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der mit einem gesättigten oder ungesättigten, ggf. substituierten mono-oder polyzyklischen Ringsystem kondensiert sein kann, stehen;
- R¹⁰, R¹¹, R¹², R¹⁴ und R²⁰,: unabhängig voneinander, jeweils
für einen linearen oder verzweigten, gesättigten oder ungesättigten, ggf. substituierten C₁₋₁₀ aliphatischen Rest,
für einen ungesättigten oder gesättigten, ggf. substituierten 3-, 4-, 5-, 6-, 7-, 8-oder 9-gliedrigen cycloaliphatischen Rest, der mit einem gesättigten oder ungesättigten, ggf. aromatischen, ggf. substituierten mono- oder polyzyklischen Ringsystem kondensiert und/oder über eine lineare oder verzweigte, ggf. substituierte C₁₋₅-Alkylen-, C₂₋₅-Alkenylen- oder C₂₋₅-Alkinylen-Gruppe gebunden sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH, -C(=O)-O-C₁₋₅-Alkyl, -(CH₂)-C(=O)-OH,-(CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste - O-Phenyl, -O-Benzyl, Phenyl, -(CH₂)-Benzo[b]furanyl, Benzoyl, Naphthyl und - (CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -O-C₁₋₅-Alkyl, -C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der über eine lineare oder verzweigte, ggf. subsituierte C₁₋₅-Alkylen-, C₂-₅-Alkenylen- oder C₂₋₅-Alkinylen-Gruppe gebunden sein kann, stehen;
- R¹⁵ und R²⁷,: unabhängig voneinander, jeweils
für einen linearen oder verzweigten, gesättigten oder ungesättigten, ggf. substituierten C₁₋₁₀aliphatischen Rest,
für einen ungesättigten oder gesättigten, ggf. substituierten 3-, 4-, 5-, 6-, 7-, 8-oder 9-gliedrigen cycloaliphatischen Rest, der mit einer linearen oder verzweigten, ggf. substituierten C₁₋₅-Alkylen-Gruppe überbrückt sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)C₁₋₅-Alkyl, -C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
- R¹⁹: für einen ggf. substituierten 5- bis 14-gliedrigen Aryl- oder Heteroaryl-Rest, der über eine lineare oder verzweigte, ggf. substituierte, ggf. 1 oder 2 Heteroatom(e) als Kettenglied(er) aufweisende C₁₋₅-Alkylen-, C₂₋₅-Alkenylen-oder C₂₋₆-Alkinylen-Gruppe gebunden sein kann, steht;
- R²⁸ und R²⁹,: unabhängig voneinander, jeweils
für einen linearen oder verzweigten, gesättigten oder ungesättigten, ggf. substituierten C₁₋₁₀ aliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
wobei
die vorstehend genannten ggf. substituierten C₁₋₁₀ aliphatischen Reste jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH_{2,} - NH-C₁₋₅-Alkyl und -N(C₁₋₅-Alkyl)(C₁₋₅-Alkyl) substituiert sein können;
die vorstehend genannten cycloaliphatischen Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten ggf. substituierten C₁₋₅-Alkylen-, C₂₋₅-Alkenylen- oder C₂₋₅-Alkinylen-Gruppen jeweils mit 1, 2, 3, 4 oder 5 Substituenten unabhängig voneinander ausgewählt aus der Gruppe bestehend aus F, Cl, Br, -OH, -SH, -NH₂, - CN, NO₂ und Phenyl substituiert sein können,
und die vorstehend genannten C₁₋₅-Alkylen-, C₂₋₅-Alkenylen- oder C₂₋₅-Alkinylen-Gruppen ggf. jeweils 1 oder 2 Heteroatom(e) ausgewählt aus der Gruppe bestehend aus Sauerstoff, Stickstoff und Schwefel als Kettenglied(er) aufweisen können;
die Ringe der vorstehend genannten ggf. substituierten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, - CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-OH, -C(=O)-O-C₁-₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(-O)-NH-C₁₋₅-Alkyl, -C(=O)-N-(C_{1- 5}-Alkyl)₂, -S(=O)₂-NH₂, -S(=O)₂-NH-C₁₋₅-Alkyl, -(CH₂)-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten ggf. substituierten Aryl- oder Heteroaryl-Reste jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₆-Alkyl, -C₁₋₅-Alkyl, -C(=O)-O-C₁₋₅-Alkyl, -NH-C₁₋₅-Alkyl, -N(C₁₋₅-Alkyl)₂, -NH-C(=O) O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -(CH₂)-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -O-C₁₋₅-Alkyl, -C₁₋₅-Alkyl, -O-CF₃, -S-CF₃, Phenyl und -O-Benzyl substituiert sein kann,
und
die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Unter einem mono-oder polyzyklischen Ringsystem werden im Sinne der vorliegenden Erfindung mono- oder polyzyklische Kohlenwasserstoffreste verstanden, die gesättigt, ungesättigt oder aromatisch sein und ggf. eines oder mehrere Heteroatome als Ringglieder aufweisen können. Ein solches mono- bzw. polyzyklisches Ringsystem kann beispielsweise mit einem cycloaliphatischen Rest, einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein. Bevorzugt ist ein polyzyklisches Ringsystem ein bizyklisches Ringsystem.

Der Fachmann versteht, daß einige der erfindungsgemäßen substituierten 4,5,6,7-Tetrahydro-isoxazolo[4,5-c]pyridin-Verbindungen der allgemeinen Formel I in Form von Rotameren vorliegen können, die somit ebenfalls Gegenstand der vorliegenden Erfindung sind und jeweils auch als Wirkstoffe in den untenstehend beschriebenen Arzneimitteln vorliegen können.

Als geeignete Aryl-Reste seien beispielsweise Phenyl-, 1-Naphthyl, und 2-Naphthyl genannt.

Als geeignete Heteroaryl-Reste seien beispielhaft Pyridinyl, Thiophenyl (Thienyl), Furanyl (Furyl), Pyrazolinyl, Pyrimidinyl, Pyridinyl, 2-Pyridinyl, 3-Pyridinyl, 4-Pyridinyl, 2-Pyrimidinyl, 4-Pyrimidinyl, 5-Pyrimidinyl, Pyridazinyl, 3-Pyridazinyl, 4-Pyridazinyl, Pyrazinyl, 3-Pyrazinyl, Imidazolyl, 2-Imidazolyl, 4-Imidazolyl, Isoxazolyl, 3-Isoxazolyl, 4-Isoxazolyl, 5-Isoxazolyl, Pyrazolyl, 3-Pyrazolyl, 4-Pyrazolyl, 5-Pyrazolyl, Oxazolyl, 2-Oxazolyl, 4-Oxazolyl, 5-Oxazolyl, 1,2,3-Oxathiazolyl, 1,2,3-Oxadiazolyl, 1,2,4-Oxadiazolyl, 1,2,5-Oxadiazolyl, 1,3,4-Oxadiazolyl, Thiazolyl, 2-Thiazolyl, 4-Thiazolyl, 5-Thiazolyl, 3-Isothiazolyl, 4-Isothiazolyl, 5-Isothiazolyl, 2-Thiophenyl, 3-Thiophenyl, Pyrrolyl, 2-Pyrrolyl, 3-Pyrrolyl, 3-Isopyrrolyl, 4-Isopyrrolyl, 5-Isopyrrolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Thiadiazol-5-yl, Triazolyl, 1,2,4-Triazol-3-yl, 1,2,4-Triazol-5-yl, 1,2,3,4-Tetrazol-5-yl, 1,2,3,4-Thiatriazolyl, Chinolinyl, Triazinyl, Chinoxalinyl, Pyranyl, Indolyl, Isoindolyl, Benzo[b]furanyl, Benzo[b]thiophenyl, Indazolyl und Isochinolinyl genannt.

Dem Fachmann ist bekannt, daß die erfindungsgemäßen 4,5,6,7-Tetrahydro-isoxazolo[4,5-c]pyridin-Verbindungen der allgemeinen Formel I auch als 6,7-Dihydro-4H-isoxazolo[4,5-c]pyridin-Verbindungen bezeichnet werden können.

Bevorzugt sind substituierte 4,5,6,7-Tetrahydro-isoxazolo[4,5-c]pyridin-Verbindungen der vorstehend angegebenen allgemeinen Formel I, worin
- R¹: für -(CHR⁴)-(CH₂)_{c}(CH₂)_{d}-C(=O)-OR⁵ mit c = 0 oder 1 und d = 0 oder 1 steht;
für -(CHR⁶)-(CHR⁷)ₑ-V_{f}-(CH₂)_{g}-Wₕ-(CH₂)ᵢ-R⁸ mit e = 0 oder 1, f = 0 oder 1, g = 0 oder 1, h = 0 oder 1 und i = 0 oder 1 steht, worin V und W jeweils unabhängig voneinander für O, S, NH, N(CH₃) oder N(C₂H₅) stehen;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Penty),-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
für einen Alkenyl-Rest ausgewählt aus der Gruppe bestehend aus Vinyl, 1-Propenyl, 2-Propenyl, 1-Butenyl, 2-Butenyl und 3-Butenyl steht, wobei der Alkenyl-Rest jeweils mit 1, 2, 3, 4 oder 5 Substituenten unabhängig voneinander ausgewählt aus der Gruppe bestehend aus F, Cl, Br, I, -CN,-NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
für einen Alkinyl-Rest ausgewählt aus der Gruppe bestehend aus Ethinyl, 1-Propinyl, 2-Propinyl, 1-Butinyl, 2-Butinyl und 3-Butinyl steht, wobei der Alkinyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, (6,6)-Dimethyl-[3,1.1]-bicycloheptyl, Cyclopentenyl, Cyclohexenyl, Cycloheptenyl, Imidazolidinyl, Tetrahydrofuranyl, Tetrahydrothiophenyl, Pyrroildinyl, Piperidinyl, Morpholinyl, Piperazinyl, Thiomorpholinyl, Tetrahydropyrenyl, Azepanyl, Diazepanyl, Dithiolanyl, Indanyl, Indenyl, (1,4)-Benzodioxanyl, (1,2,3,4)-Tetrahydronaphthyl, (1,2,3,4)Tetrahydrochinolinyl und (1,2,3,4)-Tetrahydrochinazolinyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NO₂, -O-CF_{3,} -S-CF₃, -SH, -S-C₁₋₅-Alkyl, -C₁₋₅-Alkyl, -C(=O)-H, -C(=O)-C₁₋₅-Alkyl, -C(=O)-OH, -C(=O)-O-C₁₋₅-Alkyl, -(CH₂)-C(=O)-OH, -(CH₂)-C(=O)-O-C₁₋₅-Alkyl, -(CH₂)-Benzo[b]furanyl, -O-Phenyl, -O-Benzyl, Phenyl, Benzyl, Naphthyl und -(CH₂)-Naphthyl substituiert sein kann, wobei jewells der zyklische Teil der Substituenten -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isolndotyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₆-Alkyl, -O-C₂₋₅-Alkenyl, -NH₂, NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, - C(=O)-O-C₁₋₅-Alkyl, -NH-C₁₋₅-Alkyl, -N(C₁₋₅-Alkyl)₂, -NH-C(=O)-O-C₁₋₆-Alkyl, - NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -(CH₂)-Benzo[b]furanyl, -O-Phenyl, -O-Benzyl, Phenyl und Benzyl substituiert sein kann, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
- R²: für einen Wasserstoff-Rest;
für -(CH₂)-R⁹
oder für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann;
oder
- R¹ und R²: zusammen mit dem sie verbindenden Stickstoffatom als Ringglied einen heterocycloaliphatischen Rest bilden ausgewählt aus der Gruppe bestehend aus Imidazolidinyl, (1,3)-Thiazolidinyl, Piperazinyl, Morpholinyl, Thiomorpholinyl, Pyrrolidinyl; Piperidinyl; (1,2,3,6)-Tetrahydropyridinyl und (1,2,3,4)-Tetrahydropyridinyl, wobei der heterocycloaliphatische Rest jeweils mit 1, 2 oder 3 Substituenten unabhängig voneinander ausgewählt aus der Gruppe bestehend aus R¹⁰, -C(=O)-R¹¹ und -(CH₂)-NH-C(=O)-R¹² substituiert sein kann;
- R³: für -C(=O)-NR¹³R¹⁴;
für -C(=O)-R¹⁵;
für -C(=O)-(CH₂)X-(CH₂)-C(=O)-OR¹⁶ oder -C(=O)-X-(CH₂)-C(=O)-OR¹⁶, worin X jeweils für O, S, NH, N(CH₃), N(C₂H₅) oder CR¹⁷R¹⁸ steht; oder für -C(=O)-(CH₂)-(CH₂)-(CH₂)-C(=O)-OR¹⁶
für -C(=O)-(CHR¹⁹)-NH-C(=O)-OR²⁰;
für -C(=O)-(CH₂)-R²¹, -C(=O)-(CH₂)-Y-R²¹, -C(=O)-(CH₂)-Y-(CH₂)-R²¹ oder - C(=O)-(CH₂)-(CH₂)-(CH₂)-Y-R²¹, worin Y jeweils für O, S, NH, N(CH₃) oder N(C₂H₅) steht;
für -C(=O)-(CH=CH)-R²²;
für -S(=O)₂-R²³;
für -S(=O)₂-NR²⁴R²⁵;
für -C(=S)-NR²⁶-R²⁷, -C(=S)-NR²⁶-(CH₂)-R²⁷, -C(=S)-NR²⁶-(CH₂)-(CH₂)-R²⁷ oder -C(=S)-NR²⁶-(CH₂)-(CH₂)-Z-R²⁷, worin Z für O, S, NH, N(CH₃) oder N(C₂H₅) steht;
für -C(=S)-NR²⁶-(CHR²⁸)-R²⁹;
für -C(=S)-NR²⁶-C(=O)-R³⁰;
oder für -(CH₂)-R³¹ steht;
- R⁴: für -C(=O)-NH₂;
für einen Wasserstoff-Rest;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
oder für einen Alkenyl-Rest ausgewählt aus der Gruppe bestehend aus Vinyl, 1-Propenyl, 2-Propenyl, 1-Butenyl, 2-Butenyl und 3-Butenyl steht, wobei der Alkenyl-Rest jeweils mit 1, 2, 3, 4 oder 5 Substituenten unabhängig voneinander ausgewählt aus der Gruppe bestehend aus F, Cl, Br, I, -CN, - NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
- R⁵, R¹⁷, R¹⁸ , R²⁴ und R²⁵: unabhängig voneinander jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann,
oder für einen Alkenyl-Rest ausgewählt aus der Gruppe bestehend aus Vinyl, 1-Propenyl, 2-Propenyl, 1-Butenyl, 2-Butenyl und 3-Butenyl stehen, wobei der Alkenyl-Rest jeweils mit 1, 2, 3, 4 oder 5 Substituenten unabhängig voneinander ausgewählt aus der Gruppe bestehend aus F, Cl, Br, I, -CN, - NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
- R⁶, R⁷, R¹³, R¹⁶ und R²⁶,: unabhängig voneinander, jeweils
für einen Wasserstoff-Rest
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann,
oder für einen Alkenyl-Rest ausgewählt aus der Gruppe bestehend aus Vinyl, 1-Propenyl, 2-Propenyl, 1-Butenyl, 2-Butenyl und 3-Butenyl stehen, wobei der Alkenyl-Rest jeweils mit 1, 2, 3, 4 oder 5 Substituenten unabhängig voneinander ausgewählt aus der Gruppe bestehend aus F, Cl, Br, I, -CN, - NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
- R⁸, R⁹, R²¹, R²², R²³, R³⁰ und R³¹,: unabhängig voneinander, jeweils
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, - C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, - C(=O)-O-C₁₋₅-Alkyl, -NH-C₁₋₅-Alkyl, -N(C₁₋₅-Alkyl)₂, -NH-C(=O)-O-C₁₋₅-Alkyl, - NH-C(=O)-C₁₋₅-Alkyl, -C(=O)-H, -C(=O)-C₁₋₅-Alkyl, -C(=O)-NH₂, -C(=O)-NH-C₁₅-Alkyl, -C(=O)-N-(C₁₋₅-Alkyl)₂, -S(=O)₂-NH₂, -S(=O)₂-NH-C₁₋₅-Alkyl, -(CH₂)-Benzo[b]furanyl, -O-Phenyl, -O-Benzyl, Phenyl und Benzyl substituiert sein kann, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
- R¹⁰_{,} R¹¹, R¹², R¹⁴ und R²⁰,: unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₆-Alkyl und -N(C₁₋₅-Alkyl)(C₁₋₅-Alkyl) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, - OH, -O-C₁₋₅-Alkyl, -NH₂, -NO₂, -O-CF₃, -S-CF₃, -SH, -S-C₁₋₅-Alkyl, -C₁₋₅-Alkyl, - C(=O)-H, -C(=O)-C₁₋₅-Alkyl, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Substituenten -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -O-C₁₋₅-Alkyl, -C₁₋₅-Alkyl, -O-CF₃, -C₁₋₅-Alkyl, -S-CF₃, Phenyl und -O-Benzyl substituiert und ggf. der (hetero)cycloaliphatische Rest über eine - (CH₂)-, -CH(CH₃)-, -(CH₂)-(CH₂)-, -(CH₂)-(CH₂)-(CH₂)- oder -(CH₂)-(CH=CH)-Gruppe gebunden sein kann;
oder für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl und Thieno[2,3-d]pyrimidinyl stehen, wobei der Rest jeweils mit 1, 2, 3, 4 oder 5 Substituenten unabhängig voneinander ausgewählt aus der Gruppe bestehend aus F, Cl, Br, I, -CN, - CF₃, -SF₅, -OH, -O-C₁₋₅-Alkyl, -O-C₂₋₅-Alkenyl, -NH₂, -NO₂, -O-CF₃, -O-CHF₂, - O-CH₂F, -S-CF₃, -S-CHF₂, -S-CH₂F, -SH, -S-C₁₋₅-Alkyl, -C₁₋₅-Alkyl, -C(=O)-O-C₁₋₅-Alkyl, -NH-C₁-₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -S(=O)₂-NH₂, -S(=O)2-NH-C₁₋₅-Alkyl, -(CH₂)-Benzo[b]furanyl, -O-Phenyl, -O-Benzyl, Phenyl und Benzyl substituiert und/oder über eine -(CH₂)-, -CH(CH₃)-, -(CH₂)-(CH₂)-, -(CH₂)-(CH₂)-(CH₂)- oder -(CH₂)-(CH=CH)-Gruppe gebunden sein kann, wobei jeweils der zyklische Teil der Substituenten -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -O-C₁₋₅-Alkyl, -C₁₋₅-Alkyl, -O-CF₃, -S-CF₃, Phenyl und -O-Benzyl substituiert sein kann;
- R¹⁵ und R²⁷,: unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann;
für einen Alkenyl-Rest ausgewählt aus der Gruppe bestehend aus Vinyl, 1-Propenyl, 2-Propenyl, 1-Butenyl, 2-Butenyl und 3-Butenyl stehen, wobei der Alkenyl-Rest jeweils mit 1, 2, 3, 4 oder 5 Substituenten unabhängig voneinander ausgewählt aus der Gruppe bestehend aus F, Cl, Br, I, -CN, - NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl und 7,7-Dimethyl-2-oxa-bicyclo[2.2:1]heptyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, - C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, -N(C₁₋₅-Alkyl)₂, -O-Phenyl, -O-Benzyl, Phenyl und Benzyl, substituiert sein kann, wobei jeweils der zyklische Teil der Reste -O-Phenyl, - O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, - C(=O)-O-C₁₋₅-Alkyl, -NH-C₁₋₅-Alkyl, -N(C₁₋₅-Alkyl)₂, -NH-C(=O)-O-C₁₋₅-Alkyl, - NH-C(=O)-C₁₋₅-Alkyl, -C(=O)-H, -C(=O)-C₁₋₅-Alkyl, -C(=O)-NH₂, -C(=O)-NH-C₁-₅-Alkyl, -C(=O)-N-(C₁₋₅-Alkyl)₂, -S(=O)₂-NH₂, -S(=O)₂-NH-C₁₋₅-Alkyl, -O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
- R¹⁹: für einen Rest ausgewählt aus der Gruppe bestehend aus Phenyl, Naphthyl, Thiophenyl, Furanyl und Pyridinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, Phenyl und Benzyl substituiert und/oder über eine - (CH₂)-, -(CH₂)-(CH₂)- oder-(CH₂)-O-(CH₂)-Gruppe gebunden sein kann;
und
- R²⁸ und R²⁹,: unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann;
oder für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, -S(=O)₂-CH₃, Phenyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 4,5,6,7-Tetrahydro-isoxazolo[4,5-c]pyridin-Verbindungen der vorstehend angegebenen allgemeinen Formel I, worin
- R¹: für -(CHR⁴)-C(=O)-OR⁵, -(CHR⁴)-(CH₂)-C(=O)-OR⁵ oder -(CHR⁴)-(CH₂)-(CH₂)-C(=O)-OR⁵ steht;
für -(CHR⁶)-R⁸, -(CHR⁶)-(CHR⁷)-R⁸, -(CHR⁶)-(CHR⁷)-V-R⁸, -(CHR⁶)-(CHR⁷)-(CH₂)-R⁸, -(CHR⁶)-(CHR⁷)-(CH₂)-W-R⁸ oder -(CHR⁶)-(CHR⁷)-(CH₂)-W-(CH₂)-R⁸ steht, worin V und W unabhängig voneinander jeweils für O, S, NH, N(CH₃) oder N(C₂H₆) stehen;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H_{5,} -N(CH₃)₂,-N(C₂H₅)₂ und - N(CH₃)(C₂H₅) substituiert sein kann;
für einen Alkinyl-Rest ausgewählt aus der Gruppe bestehend aus Ethinyl, 1-Propinyl, 2-Propinyl, 1-Butinyl, 2-Butinyl und 3-Butinyl steht;
für einen (hetero)cycloaliphatischen Rest ausgewählt aus der Gruppe bestehend aus Cyclopropyl, Cyclobutyl, Cyclopentyl, Cyclohexyl, Cycloheptyl, (6,6)-Dimethyl-[3.1.1]-bicycloheptyl, Pyrrolidinyl, Piperidinyl, Morpholinyl, Thiomorpholinyl, Indanyl und Indenyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅. -OH, -O-CH₃, -O-C₂H_{5,} NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H, -C(=O)-CH₃, -C(=O)-C₂H_{5,} -C(=O)-C(CH₃)₃, -C(=(O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, (CH₂)-Benzo[b]furanyl, -O-Phenyl, -O-Benzyl, Phenyl, Benzyl, Naphthyl und -(CH₂)-Naphthyl substituiert sein kann, wobei der zyklische Teil der Benzyl-Reste mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅ und -O-CF₃ substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyridinyl, Imidazolyl und Indolyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, - NO₂, -O-CF₃, -O-CHF₂, -O-CH₂F, -S-CF₃, -S-CHF₂, -S-CH₂F, -SH, Methyl, Ethyl, n-Propyl, Isopropyl, n-Butyl, sec-Butyl, Isobutyl und tert-Butyl substituiert sein kann;
- R²: für einen Wasserstoff-Rest oder
für -(CH₂)-R⁹ steht.
oder
- R¹ und R²: zusammen mit dem sie verbindenden Stickstoffatom als Ringglied einen Reste bilden, der ausgewählt ist aus der Gruppe bestehend aus 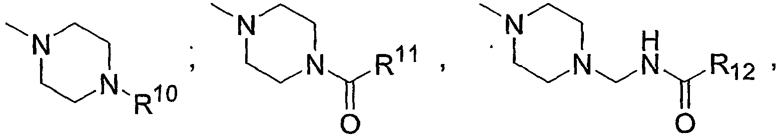 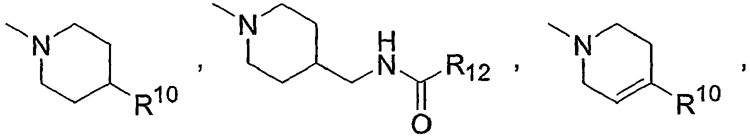 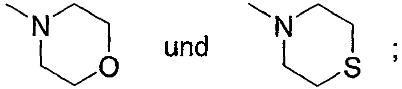
- R³: für -C(=O)-NR¹³R¹⁴;
für -C(=O)-R¹⁵;
für -C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-CR¹⁷R¹⁸-(CH₂)-C(=O)-OR¹⁶ oder -C(=O)-(CH₂)-(CH₂)-(CH₂)-C(=O)-OR¹⁶;
für -C(=O)-(CHR¹⁹)-NH-C(=O)-OR²⁰;
für -C(=O)-(CH₂)-R²¹, -C(=O)-(CH₂)-O-R²¹, -C(=O)-(CH₂)-O-(CH₂)-R²¹ oder - C(=O)-(CH₂)-(CH₂)-(CH₂)-O-R²¹;
für -C(=O)-(CH=CH)-R²²;
für -S(=O)₂-R²³;
für -S(=O)₂-NR²⁴R²⁵;
für -C(=S)-NR²⁶-R²⁷, -C(=S)-NR²⁶-(CH₂)-R²⁷, -C(=S)-NR²⁶-(CH₂)-(CH₂)-R²⁷ oder -C(=S)-NR²⁶-(CH₂)-(CH₂)-O-R²⁷;
für -C(=S)-NR²⁶-(CHR²⁸)-R²⁹;
für -C(=S)-NR²⁶-C(=O)-R³⁰;
oder für -(CH₂)-R³¹ steht;
- R⁴: für -C(=O)-NH₂;
für einen Wasserstoff-Rest oder
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann;
- R⁵: für einen Alkyl-Rest ausgewählt aus der Gruppe bestehend aus Methyl, Ethyl, n-Propyl, Isopropyl, n-Butyl, sec-Butyl, Isobutyl und tert-Butyl steht;
- R⁶, R⁷ und R¹⁶,: unabhängig voneinander, jeweils für
einen Wasserstoff-Rest oder für einen Alkyl-Rest ausgewählt aus der Gruppe bestehend aus Methyl, Ethyl, n-Propyl und Isopropyl stehen;
- R⁸, R⁹, R²¹, R²², R²³, R³⁰ und R³¹,: unabhängig voneinander, jeweils
für einen (hetero)cycloaliphatischen Rest ausgewählt aus der Gruppe bestehend aus Cyclopropyl, Cyclobutyl, Cyclopentyl, Cyclohexyl, Cycloheptyl, Pyrrolidinyl, Piperidinyl, Morpholinyl, Thiomorpholinyl, Indanyl und Indenyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H, - C(=O)-CH₃, -C(=O)-C₂H₅, -C(=O)-C(CH₃)₃, -C(=O)-OH, -C(=O)-O-CH₃, - C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), Phenyl und Benzyl substituiert sein kann;
oder für einen Rest ausgewählt aus der Gruppe bestehend aus Phenyl, Naphthyl, (1;3)-Benzodioxolyl, (1,4)-Benzodioxanyl, Thiophenyl, Furanyl, Pyridinyl, Imidazolyl, Indolyl und Isoindolyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -(CH₂)-C(=O)-O-CH₃, -(CH₂)-C(=O)-O-C₂H₅, -N(CH₃)₂, -N(C₂H₅)₂, -N(H)(CH₃), - N(H)(C₂H₅), -C(=O)-H, -C(=O)-CH₃, -C(=O)-C₂H₅, -C(=O)-NH₂, -S(=O)₂-NH₂, - S(=O)₂-NH-CH₃, -O-Phenyl, -O-Benzyl, Phenyl und Benzyl substituiert sein kann;
- R¹⁰, R¹¹, R¹², R¹⁴ und R²⁰,: unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl und n-Octyl stehen, wobei der Alkyl-Rest jeweils mit 1, 2, 3, 4 oder 5 Substituenten unabhängig voneinander ausgewählt aus der Gruppe bestehend aus -N(CH₃)₂, -N(C₂H₅)₂, F, Cl und Br substituiert sein kann;
für einen cycloaliphatischen Rest ausgewählt aus der Gruppe bestehend aus Morpholinyl, Piperidinyl, Piperazinyl, Pyrrolidinyl, Cyclopropyl, Cyclobutyl, Cyclopentyl und Cyclohexyl stehen, wobei der cycloaliphatische Rest jeweils mit 1, 2 oder 3 Substituenten unabhängig voneinander ausgewählt aus der Gruppe bestehend aus F, Cl, Br, I, -CN, -CF₃, -SF₅, -OH, -O-CH₃, -O-C₂H₅, - NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H, -C(=O)-CH₃, - C(=O)-C₂H₅, -C(=O)-C(CH₃)₃, -C(=O)-OH, -C(=O)-O-CH₃, -C(=O)-O-C₂H₅, - (CH₂)-C(=O)-OH, -(CH₂)-C(=O)-O-CH₃, -(CH₂)-C(=O)-O-C₂H₅, -N(CH₃)₂, - N(C₂H₅)₂, -N(H)(CH₃), -N(H)(C₂H₅), Phenyl und Benzyl substituiert und/oder über eine -(CH₂)- oder -(CH₂)-(CH₂)-Gruppe gebunden sein kann;
oder für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl und Thieno[2,3-d]pyrimidinyl stehen, wobei der Rest jeweils mit 1, 2, 3, 4 oder 5 Substituenten unabhängig voneinander ausgewählt aus der Gruppe bestehend aus F, Cl, Br, I, -CN, - CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F,-S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -N(CH₃)₂, -N(C₂H₅)₂, -N(H)(CH₃), -N(H)(C₂H₅), -C(=O)-H, -C(=O)-CH₃, -C(=O)-C₂H₅, -C(=O)-NH₂, - C(=O)-NH-CH₃, -C(=O)-N-(CH₃)₂, -S(=O)₂-NH₂, -S(=O)₂-CH₃, Phenyl und Benzyl substituiert und/oder über eine -(CH₂)-, -CH(CH₃)-, -(CH₂)-(CH₂)-, - (CH₂)-(CH₂)-(CH₂)- oder -(CH₂)-(CH=CH)-Gruppe gebunden sein kann;
- R¹³ und R²⁶: für einen Wasserstoff-Rest stehen;
- R¹⁵ und R²⁷,: unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, Vinyl, 1-Propenyl, 2-Propenyl, 1-Butenyl, 2-Butenyl und 3-Butenyl stehen;
für einen (hetero)cycloaliphatischen Rest ausgewählt aus der Gruppe bestehend aus Cyclopropyl, Cyclobutyl, Cyclopentyl, Cyclohexyl und 7,7-Dimethyl-2-oxa-bicyclo[2.2.1]heptyl stehen, wobei der (hetero)cycloaliphatische Rest jeweils mit 1, 2 oder 3 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, - NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, - C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, - N(H)(CH₃), -N(H)(C₂H₅), Phenyl und Benzyl substituiert sein kann;
oder für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH_{3,} -C(=O)-O-C₂H_{5,} -N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, -S(=O)₂-CH₃, Phenyl und Benzyl substituiert sein kann;
- R¹⁷ und R¹⁸,: unabhängig voneinander, jeweils für einen Methyl- oder Ethyl-Rest stehen;
- R¹⁹: für einen Phenyl-Rest steht, der über eine -(CH₂)-, -(CH₂)-(CH₂)- oder -(CH₂)-O-(CH₂)-Gruppe gebunden sein kann;
- R²⁴ und R²⁵,: unabhängig voneinander, jeweils für einen Methyl- oder Ethyl-Rest stehen;
- R²⁸: für einen Phenyl-Rest steht, der mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl substituiert sein kann;
und
- R²⁹: für einen Alkyl-Rest ausgewählt aus der Gruppe bestehend aus Methyl, Ethyl, n-Propyl, Isopropyl, n-Butyl, Isobutyl und tert-Butyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte 4,5,6,7-Tetrahydro-isoxazolo[4,5-c]pyridin-Verbindungen der vorstehend angegebenen allgemeinen Formel I, worin
- R¹: für -(CHR⁴)-C(=O)-OR⁵, -(CHR⁴)-(CH₂)-C(=O)-OR⁵ oder -(CHR⁴)-(CH₂)-(CH₂)-C(=O)-OR⁵ steht;
für-(CHR⁶)-R⁸, -(CHR⁶)-(CHR⁷)-R⁸, -(CHR⁶)-(CHR⁷)-O-R⁸, -(CHR⁶)-(CHR⁷)-(CH₂)-R⁸ oder -(CHR⁶)-(CHR⁷)-(CH₂)-N(CH₃)-R⁸ steht;
für einen ggf. substituierten Alkyl-Rest ausgewählt aus der Gruppe bestehend aus Methyl, Ethyl, n-Propyl, Isopropyl, -(CH₂)-(CH₂)-CN, -(CH₂)-(CH₂)-N(CH₃)₂, -(CH₂)-(CH₂)-O-CH₃, -(CH₂)-(CH₂)-S-C₂H₅, -n-Butyl, sec-Butyl, Isobutyl, tert-Butyl, -(CH₂)-(CH₂)-(CH₂)-O-CH₃, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht;
für einen Alkinyl-Rest ausgewählt aus der Gruppe bestehend aus 1-Propinyl und 2-Propinyl steht;
für einen cycloaliphatischen Rest ausgewählt aus der Gruppe bestehend aus Cyclopropyl, Cyclobutyl, Cyclopentyl, Cyclohexyl, Cycloheptyl, (6,6)-Dimethyl-[3.1.1]-bicycloheptyl, Indanyl und Indenyl, wobei der cycloaliphatische Rest jeweils mit einem -O-Benzyl-Rest oder einem Methyl-Rest substituiert sein kann,
für einen Pyrrolidinyl- oder Piperidinyl-Rest steht, der jeweils am Stickstoffatom mit einem Substituenten ausgewählt aus der Gruppe bestehend aus -C(=O)-CH₃, -C(=O)-C₂H₅, -C(=O)-C(CH₃)₃, Benzyl, -C(=O)-OC₂H₅ und-(CH₂)-Naphthyl substituiert sein kann, wobei der zyklische Teil des Benzyl-Rests mit 1, 2 oder 3 Substituenten unabhängig voneinander ausgewählt aus der Gruppe bestehend aus F, Cl, Br, -CF₃ und -O-CF₃ substituiert sein kann,
oder für einen Phenyl-Rest steht, der mit 1, 2 oder 3 Substituenten unabhängig voneinander ausgewählt aus der Gruppe bestehend aus -O-CH₃, -O-C₂H₅, Methyl, Ethyl, n-Propyl, Isopropyl, n-Butyl, sec-Butyl, Isobutyl und tert-Butyl substituiert sein kann;
- R²: für einen Wasserstoff-Rest oder
für -(CH₂)-R⁹ steht;

oder
- R¹ und R²: zusammen mit dem sie verbindenden Stickstoffatom als Ringglied einen Rest bilden, der ausgewählt ist aus der Gruppe bestehend aus 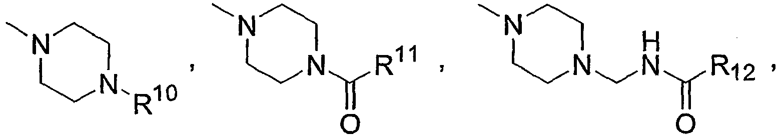 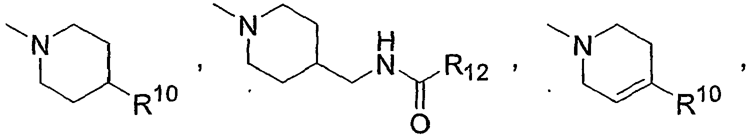 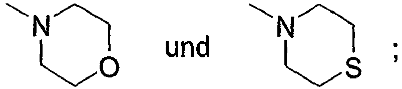
- R³: für -C(=O)-NR¹³R¹⁴;
für -C(=O)-R¹⁵;
für -C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-CR¹⁷R¹⁸-(CH₂)-C(=O)-OR¹⁶ oder -C(=O)-(CH₂)-(CH₂)-(CH₂)-C(=O)-OR¹⁶;
für -C(=O)-(CHR¹⁹)-NH-C(=O)-OR²⁰;
für -C(=O)-(CH₂)-R²¹, -C(=O)-(CH₂)-O-R²¹, -C(=O)-(CH₂)-O-(CH₂)-R²¹ oder-C(=O)-(CH₂)-(CH₂)-(CH₂)-O-R²¹;
für -C(=O)-(CH=CH)-R²²;
für -S(=O)₂-R²³;
für -S(=O)₂-NR²⁴R²⁵;
für -C(=S)-NR²⁶-R²⁷, -C(=S)-NR²⁶-(CH₂)-R²⁷, -C(=S)-NR²⁶-(CH₂)-(CH₂)-R²⁷ oder -C(=S)-NR²⁶-(CH₂)-(CH₂)-O-R²⁷;
für -C(=S)-NR²⁶-(CHR²⁸)-R²⁹_{;}
für -C(=S)-NR²⁶-C(=O)-R³⁰;R⁶, R⁷ und R¹⁶, oder für -(CH₂)-R³¹ steht;
- R⁴: für -C(=O)-NH₂;
für einen Wasserstoff-Rest oder
für einen Alkyl-Rest ausgewählt aus der Gruppe bestehend aus Methyl, Ethyl, n-Propyl, Isopropyl und n-Butyl steht;
- R⁵: für einen Alkyl-Rest ausgewählt aus der Gruppe bestehend aus Methyl, Ethyl, n-Propyl, Isopropyl, n-Butyl, sec-Butyl, Isobutyl und tert-Butyl steht;
- R⁶, R⁷ und R¹⁶,: unabhängig voneinander, jeweils für einen Wasserstoff-Rest oder für einen Alkyl-Rest ausgewählt aus der Gruppe bestehend aus Methyl, Ethyl, n-Propyl und Isopropyl stehen;
- R⁸: für einen (hetero)cycloaliphatischen Rest ausgewählt aus der Gruppe bestehend aus Cyclopropyl, Cyclobutyl, Cyclopentyl, Cyclohexyl, Pyrrolidinyl, Piperidinyl, Morpholinyl und Thiomorpholinyl steht
oder für einen Rest ausgewählt aus der Gruppe bestehend aus Phenyl, Naphthyl, Thiophenyl, Furanyl, Pyridinyl und Indolyl steht, wobei der Rest jeweils mit 1, 2 oder 3 Substituenten unabhängig voneinander ausgewählt aus der Gruppe bestehend aus F, Cl, Br, -CF₃, -O-CH₃, -O-C₂H₅, -O-CH₂-CH=CH₂, Methyl, Ethyl, n-Propyl, Isopropyl, n-Butyl, tert-Butyl, -S-CH₃, -S-C₂H₅,-S(=O)₂-NH₂ und -S(=O)₂-NH-CH₃ substituiert sein kann;
- R⁹: für einen Rest ausgewählt aus der Gruppe bestehend aus Phenyl, Naphthyl, Thiophenyl, Furanyl und Pyridinyl steht;
- R¹⁰: für einen ggf. substituierten Alkyl-Rest ausgewählt aus der Gruppe bestehend aus -(CH₂)-N(CH₃)₂, -(CH₂)-(CH₂)-N(CH₃)₂, -(CH₂)-(CH₂)-(CH₂)-N(CH₃)₂,-(CH₂)-N(C₂H₅)₂, -(CH₂)-(CH₂)-N(C₂H₅)₂ und-(CH₂)-(CH₂)-(CH₂)-N(C₂H₅)₂;
für einen cycloaliphatischen Rest ausgewählt aus der Gruppe bestehend aus Piperazinyl, Pyrrolidinyl, Piperidinyl, Morpholinyl, Cyclopropyl, Cyclobutyl, Cyclopentyl und Cyclohexyl steht, wobei der cycloaliphatische Rest über eine -(CH₂)- oder -(CH₂)-(CH₂)- oder -(CH₂)-(CH₂)-(CH₂)-Gruppe gebunden sein kann;
oder für einen Rest ausgewählt aus der Gruppe bestehend aus Phenyl, Thiazolyl, Naphthyl und Thieno[2,3-d]pyrimidinyl steht, wobei der Rest jeweils mit 1, 2 oder 3 Substituenten unabhängig voneinander ausgewählt aus der Gruppe bestehend aus F, Cl, Br, Methyl, Ethyl, n-Propyl, -C(=O)-CH₃ und-C(=O)-C₂H₅ substituiert und/oder über eine -(CH₂)-, -CH(CH₃)-, -(CH₂)-(CH₂)-, -(CH₂)-(CH₂)-(CH₂)- oder-(CH₂)-(CH=CH)-Gruppe gebunden sein kann;
- R¹¹: für einen ggf. substituierten Alkyl-Rest ausgewählt aus der Gruppe bestehend aus Methyl, Ethyl, n-Propyl, -CF₃ und -CF₂-CF₃ steht,
oder für einen Rest ausgewählt aus der Gruppe bestehend aus Phenyl, und Naphthyl steht, wobei der Rest jeweils mit 1, 2 oder 3 Substituenten unabhängig voneinander ausgewählt aus der Gruppe bestehend aus F, Cl und Br substituiert und/oder über eine -(CH₂)- oder -(CH₂)-(CH₂)-Gruppe gebunden sein kann;
- R¹²: für einen ggf. substituierten Alkyl-Rest ausgewählt aus der Gruppe bestehend aus Methyl, Ethyl, n-Propyl, -CF₃ und -CF₂-CF₃ steht;
- R¹³: für einen Wasserstoff-Rest steht;
- R¹⁴: für einen Rest ausgewählt aus der Gruppe bestehend aus Phenyl und Naphthyl steht, wobei der Rest jeweils mit 1, 2 oder 3 Substituenten unabhängig voneinander ausgewählt aus der Gruppe bestehend aus F, Cl, Br, -CN, -CF₃, -O-CH₃, -O-C₂H₅, -O-CH(CH₃)₂, -O-CH₂-CH₂-CH₂-CH₃, -NO₂, -O-CF₃, -O-CHF₂, -O-CH₂F, -S-CF₃, -S-CHF₂, -S-CH₂F, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-CH₃ und -C(=O)-C₂H₅ substituiert und/oder über eine -(CH₂)-,-CH(CH₃)- oder -(CH₂)-(CH₂)-Gruppe gebunden sein kann;
- R¹⁵: für einen Rest ausgewählt aus der Gruppe bestehend aus 1-Butenyl, 2-Butenyl und 3-Butenyl steht;
für einen 4,7,7-Trimethyl-3-oxo-2-oxa-bicyclo[2.2.1]heptyl-Rest steht
oder für einen Rest ausgewählt aus der Gruppe bestehend aus Phenyl, Naphthyl, Thiophenyl, Furanyl, Triazolyl, Pyridinyl, Chinolinyl, Pyrazolyl und Isochinolinyl steht, wobei der Rest jeweils mit 1, 2 oder 3 Substituenten unabhängig voneinander ausgewählt aus der Gruppe bestehend aus F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, -S-CF₃, -S-CHF₂, -S-CH₂F, Methyl, Ethyl, n-Propyl, Isopropyl, n-Butyl, sec-Butyl, Isobutyl, tert-Butyl, Phenyl, -NH-C(=O)-CH₃ und -NH-C(=O)-C₂H₅ substituiert sein kann;
- R¹⁷ und R¹⁸: unabhängig voneinander jeweils für einen Methyl- oder Ethyl-Rest stehen;
- R¹⁹: für einen Phenyl-Rest steht, der über eine -(CH₂)-, -(CH₂)-(CH₂)- oder -(CH₂)-O-(CH₂)-Gruppe gebunden sein kann;
- R²⁰: für einen Alkyl-Rest ausgewählt aus der Gruppe bestehend aus Methyl, Ethyl, n-Propyl, Isopropyl, n-Butyl, sec-Butyl, Isobutyl und tert-Butyl steht
oder für einen Benzyl-Rest steht;
- R²¹: für einen cycloaliphatischen Rest ausgewählt aus der Gruppe bestehend aus Cyclopropyl, Cyclobutyl, Cyclopentyl, Cyclohexyl, Cycloheptyl steht, wobei der cycloaliphatische Rest jeweils mit 1, 2 oder 3 Substituenten unabhängig voneinander ausgewählt aus der Gruppe bestehend aus -C(=O)-OH und -(CH₂)-C(=O)-OH substituiert sein kann,
oder für einen Rest ausgewählt aus der Gruppe bestehend aus Phenyl, Naphthyl, (1,3)-Benzodioxolyl und (1,4)-Benzodioxanyl steht, wobei der Rest jeweils mit 1, 2 oder 3 Substituenten unabhängig voneinander ausgewählt aus der Gruppe bestehend aus F, Cl, Br, -O-CH₃ und -O-C₂H₅ substituiert sein kann;
- R²²: für einen Phenyl-Rest steht;
- R²³: für einen Rest ausgewählt aus der Gruppe bestehend aus Phenyl, Naphthyl, Thiophenyl, Furanyl und Pyridinyl steht, wobei der Rest jeweils mit 1, 2 oder 3 Substituenten unabhängig voneinander ausgewählt aus der Gruppe bestehend aus F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, Methyl, Ethyl, n-Propyl, Isopropyl, n-Butyl, sec-Butyl, Isobutyl und tert-Butyl substituiert sein kann;
- R²⁴ und R²⁵,: unabhängig voneinander, jeweils für einen Methyl- oder Ethyl-Rest stehen;
- R²⁶: für einen Wasserstoff-Rest steht;
- R²⁷: für einen Alkyl-Rest ausgewählt aus der Gruppe bestehend aus Methyl, Ethyl, n-Propyl, Isopropyl und n-Butyl steht;
für einen Cyclohexyl-Rest steht
oder für einen Phenyl-Rest steht, der mit 1, 2, 3, 4 oder 5 Substituenten unabhängig voneinander ausgewählt aus der Gruppe bestehend aus F, Cl, Br, -O-CH₃ und -O-C₂H₅ substituiert sein kann;
- R²⁸: für einen Phenyl-Rest steht, der mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl substituiert sein kann;
- R²⁹: für einen Alkyl-Rest ausgewählt aus der Gruppe bestehend aus Methyl, Ethyl, n-Propyl, Isopropyl, n-Butyl, Isobutyl und tert-Butyl steht;
- R³⁰: für einen Phenyl-Rest steht
und
- R³¹: für einen Rest ausgewählt aus der Gruppe bestehend aus Phenyl und Naphthyl steht, wobei der Rest jeweils mit 1, 2 oder 3 Substituenten unabhängig voneinander ausgewählt aus der Gruppe bestehend aus F, Cl und Br substituiert sein kann;
jeweils ggf. in Form eines ihrer reinen Stereoisomeren, insbesondere Enantiomeren oder Diastereomeren; ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Insbesondere bevorzugt sind substituierte 4,5,6,7-Tetrahydro-isoxazolo[4,5-c]pyridin-Verbindungen der vorstehend angegebenen allgemeinen Formel I ausgewählt aus der Gruppe bestehend aus
[1] 6,7-Dihydro-4H-isoxazolo[4,5-c]pyridin-3,5-dicarbonsäure 3-[(2-methoxy-ethyl)-amid] 5-[(3-methoxy-phenyl)-amid],
[2] 6,7-Dihydro-4H-isoxazolo[4,5-c]pyridin-3,5-dicarbonsäure 3-cyclopentylamid 5-(4-fluor-benzylamid),
[3] 6,7-Dihydro-4H-isoxazolo[4,5-c]pyridin-3,5-dicarbonsäure 3-phenylamid 5-[(4-trifluormethoxy-phenyl)-amid],
[4] 6,7-Dihydro-4H-isoxazolo[4,5-c]pyridin-3,5-dicarbonsäure 5-[(4-methyl-3-nitro-phenyl)-amid] 3-(phenethyl-amid),
[5] 6,7-Dihydro-4H-isoxazolo[4,5-c]pyridin-3,5-dicarbonsäure 3-[(2-methoxy-ethyl)-amid] 5-[(4-methyl-3-nitro-phenyl)-amid],
[6] 3-{[5-(2,5-Difluor-phenylcarbamoyl)-4,5,6,7-tetrahydro-isoxazolo[4,5-c]pyridin-3-carbonyl]-amino}-propionsäure ethyl ester,
[7] 6,7-Dihydro-4H-isoxazolo[4,5-c]pyridin-3,5-dicarbonsäure 5-[(4-butoxy-phenyl)-amid] 3-[(2-methoxy-ethyl)-amid],
[8] 6,7-Dihydro-4H-isoxazolo[4,5-c]pyridin-3,5-dicarbonsäure 5-[(3-fluor-phenyl)-amid] 3-(phenethyl-amid),
[9] 6,7-Dihydro-4H-isoxazolo[4,5-c]pyridin-3,5-dicarbonsäure 5-[(4-methyl-3-nitrophenyl)-amid] 3-[(thiophen-2-ylmethyl)-amid],
[10] 6,7-Dihydro-4H-isoxazolo[4,5-c]pyridin-3,5-dicarbonsäure 3-benzylamid 5-(phenethyl-amid),
[11] 6,7-Dihydro-4H-isoxazolo[4,5-c]pyridin-3,5-dicarbonsäure 3-[(2-ethylsulfanyl-ethyl)-amid] 5-[(3-methoxy-phenyl)-amid],
[12] 6,7-Dihydro-4H-isoxazolo[4,5-c]pyridin-3,5-dicarbonsäure 5-[(3-cyano-phenyl)-amid] 3-[(thiophen-2-ylmethyl)-amid],
[13] 6,7-Dihydro-4H-isoxazolo[4,5-c]pyridin-3,5-dicarbonsäure 5-[(4-ethoxy-phenyl)-amid] 3-phenylamid,
[14] 6,7-Dihydro-4H-isoxazolo[4,5-c]pyridin-3,5-dicarbonsäure 3-(4-fluor-benzylamid) 5-[(4-methyl-3-nitro-phenyl)-amid],
[15] 6,7-Dihydro-4H-isoxazolo[4,5-c]pyridin-3,5-dicarbonsäure 5-[(3-acetyl-phenyl)-amid] 3-[(5-methyl-furan-2-ylmethyl)-amid],
[16] 6,7-Dihydro-4H-isoxazolo[4,5-c]pyridin-3,5-dicarbonsäure 5-[(4-methyl-3-nitrophenyl)-amid] 3-prop-2-ynylamid,
[17] 6,7-Dihydro-4H-isoxazolo[4,5-c]pyridin-3,5-dicarbonsäure 3-benzylamid 5-phenylamid,
[18] 6,7-Dihydro-4H-isoxazolo[4,5-c]pyridin-3,5-dicarbonsäure 5-[(1-naphthalen-1-yl-ethyl)-amid] 3-(phenethyl-amid),
[19] 6,7-Dihydro-4H-isoxazolo[4,5-c]pyridin-3,5-dicarbonsäure 5-[(3-fluor-phenyl)-amid] 3-prop-2-ynylamid,
[20] 3-{[5-(2,5-Dimethoxy-phenylcarbamoyl)-4,5,6,7-tetrahydro-isoxazolo[4,5-c]pyridin-3-carbonyl]-amino}-propionsäure ethyl ester,
[21] 6,7-Dihydro-4H-isoxazolo[4,5-c]pyridin-3,5-dicarbonsäure 5-[(4-ethoxy-phenyl)-amid] 3-(4-fluor-benzylamid),
[22] 6,7-Dihydro-4H-isoxazolo[4,5-c]pyridin-3,5-dicarbonsäure 5-[(5-chlor-2-methoxy-phenyl)-amid] 3-(phenethyl-amid),
[23] 3-({3-[(5-Methyl-furan-2-ylmethyl)-carbamoyl]-6,7-dihydro-4H-isoxazolo[4,5-c]pyridin-5-carbonyl}-amino)-benzoesäure ethyl ester,
[24] 6,7-Dihydro-4H-isoxazolo[4,5-c]pyridin-3,5-dicarbonsäure 5-[(5-chlor-2-methoxy-phenyl)-amid] 3-(isobutyl-amid),
[25] [2-Oxo-2-(3-prop-2-ynylcarbamoyl-6,7-dihydro-4H-isoxazolo[4,5-c]pyridin-5-yl)-ethoxy]-essigsäure,
[26] 3-Ethyl-5-[3-(2-methoxy-ethylcarbamoyl)-6,7-dihydro-4H-isoxazolo[4,5-c]pyridin-5-yl]-3-methyl-5-oxo-pentansäure,
[27] (1-Benzyloxymethyl-2-oxo-2-{3-[(pyridin-3-ylmethyl)-carbamoyl]-6,7-dihydro-4H-isoxazolo[4,5-c]pyridin-5-yl}-ethyl)-carbaminsäure tert-butyl ester,
[28] {1-[2-Oxo-2-(3-phenylcarbamoyl-6,7-dihydro-4H-isoxazolo[4,5-c]pyridin-5-yl)-ethyl]-cyclopentyl}-essigsäure,
[29] 3-Ethyl-3-methyl-5-oxo-5-(3-phenylcarbamoyl-6,7-dihydro-4H-isoxazolo[4,5-c]pyridin-5-yl)-pentansäure,
[30] (2-Oxo-2-{3-[(thiophen-2-ylmethyl)-carbamoyl]-6,7-dihydro-4H-isoxazolo[4,5-c]pyridin-5-yl}-ethoxy)-essigsäure,
[31] 3,3-Dimethyl-4-{3-[(5-methyl-furan-2-ylmethyl)-carbamoyl]-6,7-dihydro-4H-isoxazolo[4,5-c]pyridin-5-yl}-4-oxo-butansäure,
[32] 5-(4-Trifluormethoxy-benzolsulfonyl)-4,5,6,7-tetrahydro-isoxazolo[4,5-c]pyridin-3-carbonsäure benzyl-phenethyl-amid,
[33] 5-Benzolsulfonyl-4,5,6,7-tetrahydro-isoxazolo[4,5-c]pyridin-3-carbonsäure (1-naphthalen-2-ylmethyl-pyrrolidin-3-yl)-amid,
[34] [5-(4-Fluor-benzolsulfonyl)-4,5,6,7-tetrahydro-isoxazolo[4,5-c]pyridin-3-yl]-(4-thieno[2,3-d]pyrimidin-4-yl-piperazin-1-yl)-methanon,
[35] 5-(4-Fluor-benzolsulfonyl)-4,5,6,7-tetrahydro-isoxazolo[4,5-c]pyridin-3-carbonsäure (3-phenyl-propyl)-amid,
[36] 5-(3-Phenyl-acryloyl)-4,5,6,7-tetrahydro-isoxazolo[4,5-c]pyridin-3-carbonsäure (2-dimethylamino-ethyl)-amid,
[37] 5-(2-Phenoxy-acetyl)-4,5,6,7-tetrahydro-isoxazolo[4,5-c]pyridin-3-carbonsäure (2-dimethylamino-ethyl)-amid,
[38] 5-(4-Fluor-benzolsulfonyl)-4,5,6,7-tetrahydro-isoxazolo[4,5-c]pyridin-3-carbonsäure (1-methyl-3-phenyl-propyl)-amid,
[39] 5-(4-Fluor-benzolsulfonyl)-4,5,6,7-tetrahydro-isoxazolo[4,5-c]pyridin-3-carbonsäure [1-(4-trifluormethyl-benzyl)-pyrrolidin-3-yl]-amid,
[40] 5-(2,5-Dichlor-benzolsulfonyl)-4,5,6,7-tetrahydro-isoxazolo[4,5-c]pyridin-3-carbonsäure (2-benzyloxy-cyclopentyl)-amid,
[41] 5-(5-tert-Butyl-2-methyl-2H-pyrazole-3-carbonyl)-4,5,6,7-tetrahydro-isoxazolo[4,5-c]pyridin-3-carbonsäure (2-dimethylamino-ethyl)-amid,
[42] [5-(4-Fluor-benzolsulfonyl)-4,5,6,7-tetrahydro-isoxazolo[4,5-c]pyridin-3-yl]-[4-(4-fluor-phenyl)-3,6-dihydro-2H-pyridin-1-yl]-methanon,
[43] 4-{[5-(4-Fluor-benzolsulfonyl)-4,5,6,7-tetrahydro-isoxazolo[4,5-c]pyridin-3-carbonyl]-amino}-piperidin-1-carbonsäure ethyl ester,
[44] 5-(3-Phenyl-acryloyl)-4,5,6,7-tetrahydro-isoxazolo[4,5-c]pyridin-3-carbonsäure cyclopentylamid,
[45] 5-(4-Acetylamino-benzoyl)-4,5,6,7-tetrahydro-isoxazolo[4,5-c]pyridin-3-carbonsäure cyclopropylmethyl-amid,
[46] 5-(5-Methyl-2-phenyl-2H-[1,2,3]triazol-4-carbonyl)-4,5,6,7-tetrahydro-isoxazolo[4,5-c]pyridin-3-carbonsäure (pyridin-3-ylmethyl)-amid,
[47] 5-Benzolsulfonyl-4,5,6,7-tetrahydro-isoxazolo[4,5-c]pyridin-3-carbonsäure 3,4-dimethoxy-benzylamid,
[48] 2-(3,4-Difluor-phenyl)-1-{4-[5-(4-fluor-benzolsulfonyl)-4,5,6,7-tetrahydro-isoxazolo[4,5-c]pyridin-3-carbonyl]-piperazin-1-yl}-ethanon,
[49] 3-(Morpholin-4-carbonyl)-6,7-dihydro-4H-isoxazolo[4,5-c]pyridin-5-sulfonsäure dimethylamid,
[50] 5-(Furan-2-carbonyl)-4,5,6,7-tetrahydro-isoxazolo[4,5-c]pyridin-3-carbonsäure (thiophen-2-ylmethyl)-amid,
[51] 5-(4,7,7-Trimethyl-3-oxo-2-oxa-bicyclo[2.2.1]heptan-1-carbonyl)-4,5,6,7-tetrahydro-isoxazolo[4,5-c]pyridin-3-carbonsäure (2-pyrrolidin-1-yl-ethyl)-amid,
[52] 5-(2,5-Dichlor-benzolsulfonyl)-4,5,6,7-tetrahydro-isoxazolo[4,5-c]pyridin-3-carbonsäure [2-(2-chlor-phenyl)-ethyl]-amid,
[53] 5-(4-Methoxy-benzolsulfonyl)-4,5,6,7-tetrahydro-isoxazolo[4,5-c]pyridin-3-carbonsäure p-tolyl-amid,
[54] 5-(2-Phenoxy-acetyl)-4,5,6,7-tetrahydro-isoxazolo[4,5-c]pyridin-3-carbonsäure 4-fluor-benzylamid,
[55] 1-{4-[5-(4-Methoxy-benzolsulfonyl)-4,5,6,7-tetrahydro-isoxazolo[4,5-c]pyridin-3-carbonyl]-piperazin-1-yl}-ethanon,
[56] 5-(Toluol-4-sulfonyl)-4,5,6,7-tetrahydro-isoxazolo[4,5-c]pyridin-3-carbonsäure 4-sulfamoyl-benzylamid,
[57] 5-(Thiophen-2-sulfonyl)-4,5,6,7-tetrahydro-isoxazolo[4,5-c]pyridin-3-carbonsäure (2-morpholin-4-yl-ethyl)-amid,
[58] 5-(4-Methoxy-benzoyl)-4,5,6,7-tetrahydro-isoxazolo[4,5-c]pyridin-3-carbonsäure (2-dimethylamino-ethyl)-amid,
[59] 5-(2,5-Dichlor-benzolsulfonyl)-4,5,6,7-tetrahydro-isoxazolo[4,5-c]pyridin-3-carbonsäure benzyl-phenethyl-amid,
[60] 5-Dimethylsulfamoyl-4,5,6,7-tetrahydro-isoxazolo[4,5-c]pyridin-3-carbonsäure (2,6,6-trimethyl-bicyclo[3.1.1]hept-3-yl)-amid,
[61] 5-Benzolsulfonyl-4,5,6,7-tetrahydro-isoxazolo[4,5-c]pyridin-3-carbonsäure indan-1-ylamid,
[62] 5-(4-Methoxy-benzolsulfonyl)-4,5,6,7-tetrahydro-isoxazolo[4,5-c]pyridin-3-carbonsäure (3-methoxy-propyl)-amid,
[63] 5-(4,5-Dichlor-thiophen-2-sulfonyl)-4,5,6,7-tetrahydro-isoxazolo[4,5-c]pyridin-3-carbonsäure (2-morpholin-4-yl-ethyl)-amid,
[64] 5-(2-Benzyloxy-acetyl)-4,5,6,7-tetrahydro-isoxazolo[4,5-c]pyridin-3-carbonsäure (2-pyrrolidin-1-yl-ethyl)-amid,
[65] [5-(2,5-Dichlor-benzolsulfonyl)-4,5,6,7-tetrahydro-isoxazolo[4,5-c]pyridin-3-yl]-[4-(2,4-dimethyl-phenyl)-piperazin-1-yl]-methanon,
[66] 5-Pent-4-enoyl-4,5,6,7-tetrahydro-isoxazolo[4,5-c]pyridin-3-carbonsäure cyclopropylmethyl-amid,
[67] 3-Methyl-2-{[5-(3-trifluormethyl-benzolsulfonyl)-4,5,6,7-tetrahydro-isoxazolo[4,5-c]pyridin-3-carbonyl]-amino)-butansäure tert-butyl ester,
[68] 5-(3-Trifluormethyl-benzolsulfonyl)-4,5,6,7-tetrahydro-isoxazolo[4,5-c]pyridin-3-carbonsäure benzyl-phenethyl-amid,
[69] 5-(3-Trifluormethyl-benzolsulfonyl)-4,5,6,7-tetrahydro-isoxazolo[4,5-c]pyridin-3-carbonsäure 2,4-dichlor-benzylamid,
[70] 5-Dimethylsulfamoyl-4,5,6,7-tetrahydro-isoxazolo[4,5-c]pyridin-3-carbonsäure (2-cyano-ethyl)-pyridin-3-ylmethyl-amid,
[71] 5-(2-Cyclopentyl-acetyl)-4,5,6,7-tetrahydro-isoxazolo[4,5-c]pyridin-3-carbonsäure (2-pyrrolidin-1-yl-ethyl)-amid,
[72] 5-(5-Methyl-2-phenyl-2H-[1,2,3]triazol-4-carbonyl)-4,5,6,7-tetrahydro-isoxazolo[4,5-c]pyridin-3-carbonsäure 4-fluor-benzylamid,
[73] 5-(3-Trifluormethyl-benzolsulfonyl)-4,5,6,7-tetrahydro-isoxazolo[4,5-c]pyridin-3-carbonsäure (2-ethyl-hexyl)-amid,
[74] 5-(2,3-Difluor-4-methyl-benzoyl)-4,5,6,7-tetrahydro-isoxazolo[4,5-c]pyridin-3-carbonsäure (2-dimethylamino-ethyl)-amid,
[75] 5-(4-Trifluormethoxy-benzolsulfonyl)-4,5,6,7-tetrahydro-isoxazolo[4,5-c]pyridin-3-carbonsäure 2,3-dichlor-benzylamid,
[76] 5-Dimethylsulfamoyl-4,5,6,7-tetrahydro-isoxazolo[4,5-c]pyridin-3-carbonsäure [2-(4-chlor-phenyl)-ethyl]-amid,
[77] 5-(6-Chlor-pyridin-3-carbonyl)-4,5,6,7-tetrahydro-isoxazolo[4,5-c]pyridin-3-carbonsäure (2-pyrrolidin-1-yl-ethyl)-amid,
[78] 5-[3-(4-Fluor-benzylcarbamoyl)-6,7-dihydro-4H-isoxazolo[4,5-c]pyridin-5-yl]-5-oxo-pentansäure methyl ester,
[79] 5-[2-(4-Methoxy-phenyl)-acetyl]-4,5,6,7-tetrahydro-isoxazolo[4,5-c]pyridin-3-carbonsäure benzylamid,
[80] 5-(4-Trifluormethoxy-benzolsulfonyl)-4,5,6,7-tetrahydro-isoxazolo[4,5-c]pyridin-3-carbonsäure [3-(methyl-phenyl-amino)-propyl]-amid,
[81] 5-[2-(4-Chlor-phenoxy)-acetyl]-4,5,6,7-tetrahydro-isoxazolo[4,5-c]pyridin-3-carbonsäure cyclopentylamid,
[82] [4-(2-Cyclohexyl-ethyl)-piperazin-1-yl]-[5-(toluol-4-sulfonyl)-4,5,6,7-tetrahydro-isoxazolo[4,5-c]pyridin-3-yl]-methanon,
[83] N-{1-[5-(2,5-Dichlor-benzolsulfonyl)-4,5,6,7-tetrahydro-isoxazolo[4,5-c]pyridin-3-carbonyl]-piperidin-4-ylmethyl}-2,2,2-trifluor-acetamid,
[84] 5-(4-Methoxy-benzolsulfonyl)-4,5,6,7-tetrahydro-isoxazolo[4,5-c]pyridin-3-carbonsäure [1-(4-methoxy-phenyl)-ethyl]-amid,
[85] 2-{[5-(Thiophen-2-sulfonyl)-4,5,6,7-tetrahydro-isoxazolo[4,5-c]pyridin-3-carbonyl]-amino}-propionsäure ethyl ester,
[86] 1-(4-{4-[5-(Toluol-4-sulfonyl)-4,5,6,7-tetrahydro-isoxazolo[4,5-c]pyridin-3-carbonyl]-piperazin-1-yl}-phenyl)-ethanon,
[87] 5-(3-Trifluormethyl-benzolsulfonyl)-4,5,6,7-tetrahydro-isoxazolo[4,5-c]pyridin-3-carbonsäure (4-allyloxy-benzyl)-furan-2-ylmethyl-amid,
[88] 4-Carbamoyl-4-{[5-(4-methoxy-benzolsulfonyl)-4,5,6,7-tetrahydro-isoxazolo[4,5-c]pyridin-3-carbonyl]-amino}-butansäure tert-butyl ester,
[89] 5-Dimethylsulfamoyl-4,5,6,7-tetrahydro-isoxazolo[4,5-c]pyridin-3-carbonsäure furan-2-ylmethyl-(4-methylsulfanyl-benzyl)-amid,
[90] 5-(4-Bromo-3-methyl-benzoyl)-4,5,6,7-tetrahydro-isoxazolo[4,5-c]pyridin-3-carbonsäure (2-morpholin-4-yl-ethyl)-amid,
[91] 5-(Toluol-4-sulfonyl)-4,5,6,7-tetrahydro-isoxazolo[4,5-c]pyridin-3-carbonsäure [1-(2,6-dichlor-benzyl)-pyrrolidin-3-yl]-amid,
[92] 5-Benzolsulfonyl-4,5,6,7-tetrahydro-isoxazolo[4,5-c]pyridin-3-carbonsäure [2-(3,4-dichlor-phenyl)-ethyl]-amid,
[93] 5-(3-Difluormethylsulfanyl-benzoyl)-4,5,6,7-tetrahydro-isoxazolo[4,5-c]pyridin-3-carbonsäure (2-dimethylamino-ethyl)-amid,
[94] 5-[2-(3-Chlor-phenoxy)-acetyl]-4,5,6,7-tetrahydro-isoxazolo[4,5-c]pyridin-3-carbonsäure (2-pyrrolidin-1-yl-ethyl)-amid,
[95] 5-(4-Methoxy-benzolsulfonyl)-4,5,6,7-tetrahydro-isoxazolo[4,5-c]pyridin-3-carbonsäure 2,3-dimethoxy-benzylamid,
[96] 5-(4-Trifluormethoxy-benzolsulfonyl)-4,5,6,7-tetrahydro-isoxazolo[4,5-c]pyridin-3-carbonsäure (1-methyl-3-phenyl-propyl)-amid,
[97] (5-Benzolsulfonyl-4,5,6,7-tetrahydro-isoxazolo[4,5-c]pyridin-3-yl)-[4-(3-phenyl-allyl)-piperazin-1-yl]-methanon,
[98] 3-{[5-(4-Methoxy-benzoyl)-4,5,6,7-tetrahydro-isoxazolo[4,5-c]pyridin-3-carbonyl]-amino}-propionsäure ethyl ester,
[99] 5-(4-Phenoxy-butyryl)-4,5,6,7-tetrahydro-isoxazolo[4,5-c]pyridin-3-carbonsäure (2-dimethylamino-ethyl)-amid,
[100] 5-(4-Trifluormethoxy-benzolsulfonyl)-4,5,6,7-tetrahydro-isoxazolo[4,5-c]pyridin-3-carbonsäure [2-(7-methyl-1H-indol-3-yl)-ethyl]-amid,
[101] [4-(3-Phenyl-allyl)-piperazin-1-yl]-[5-(thiophen-2-sulfonyl)-4,5,6,7-tetrahydro-isoxazolo[4,5-c]pyridin-3-yl]-methanon,
[102] 5-(4-Bromo-3-methyl-benzoyl)-4,5,6,7-tetrahydro-isoxazolo[4,5-c]pyridin-3-carbonsäure (2-pyrrolidin-1-yl-ethyl)-amid,
[103] 5-Benzolsulfonyl-4,5,6,7-tetrahydro-isoxazolo[4,5-c]pyridin-3-carbonsäure [1-(4-trifluormethyl-benzyl)-pyrrolidin-3-yl]-amid,
[104] 5-(Thiophen-2-sulfonyl)-4,5,6,7-tetrahydro-isoxazolo[4,5-c]pyridin-3-carbonsäure (2-phenyl-propyl)-amid,
[105] 5-(3-Chlor-4-fluor-benzoyl)-4,5,6,7-tetrahydro-isoxazolo[4,5-c]pyridin-3-carbonsäure phenethyl-amid,
[106] 5-Benzolsulfonyl-4,5,6,7-tetrahydro-isoxazolo[4,5-c]pyridin-3-carbonsäure [2-(4-methoxy-phenoxy)-ethyl]-amid,
[107] 5-Dimethylsulfamoyl-4,5,6,7-tetrahydro-isoxazolo[4,5-c]pyridin-3-carbonsäure 3-fluor-4-trifluormethyl-benzylamid,
[108] 5-(3-Methyl-benzoyl)-4,5,6,7-tetrahydro-isoxazolo[4,5-c]pyridin-3-carbonsäure prop-2-ynylamid,
[109] 5-(Toluol-4-sulfonyl)-4,5,6,7-tetrahydro-isoxazolo[4,5-c]pyridin-3-carbonsäure [2-(4-methoxy-phenoxy)-ethyl]-amid,
[110] [4-(2-Chlor-phenyl)-piperazin-1-yl]-[5-(2,5-dichlor-benzolsulfonyl)-4,5,6,7-tetrahydro-isoxazolo[4,5-c]pyridin-3-yl]-methanon,
[111] 3-{[5-(2,6-Difluor-3-methyl-benzoyl)-4,5,6,7-tetrahydro-isoxazolo[4,5-c]pyridin-3-carbonyl]-amino}-propionsäure ethyl ester,
[112] 5-(2,5-Dichlor-benzolsulfonyl)-4,5,6,7-tetrahydro-isoxazolo[4,5-c]pyridin-3-carbonsäure 2,4-difluor-benzylamid,
[113] 5-Benzolsulfonyl-4,5,6,7-tetrahydro-isoxazolo[4,5-c]pyridin-3-carbonsäure 3-fluor-4-trifluormethyl-benzylamid,
[114] 5-(4-Butoxy-benzolsulfonyl)-4,5,6,7-tetrahydro-isoxazolo[4,5-c]pyridin-3-carbonsäure isobutyl-amid,
[115] 5-(3-Fluor-4-methyl-benzoyl)-4,5,6,7-tetrahydro-isoxazolo[4,5-c]pyridin-3-carbonsäure (2-pyrrolidin-1-yl-ethyl)-amid,
[116] 5-(2-Chlor-5-trifluormethyl-benzoyl)-4,5,6,7-tetrahydro-isoxazolo[4,5-c]pyridin-3-carbonsäure isobutyl-amid,
[117] 5-(4-Trifluormethoxy-benzolsulfonyl)-4,5,6,7-tetrahydro-isoxazolo[4,5-c]pyridin-3-carbonsäure (1-benzyl-pyrrolidin-3-yl)-amid,
[118] 5-(3-Chlor-benzolsulfonyl)-4,5,6,7-tetrahydro-isoxazolo[4,5-c]pyridin-3-carbonsäure (2-pyrrolidin-1-yl-ethyl)-amid,
[119] 5-(3-Trifluormethoxy-benzoyl)-4,5,6,7-tetrahydro-isoxazolo[4,5-c]pyridin-3-carbonsäure (2-dimethylamino-ethyl)-amid,
[120] 2-{[5-(Toluol-4-sulfonyl)-4,5,6,7-tetrahydro-isoxazolo[4,5-c]pyridin-3-carbonyl]-amino}-propionsäure tert-butyl ester,
[121] 3-{[5-(4-Chlor-benzolsulfonyl)-4,5,6,7-tetrahydro-isoxazolo[4,5-c]pyridin-3-carbonyl]-amino}-propionsäure ethyl ester,
[122] 5-Dimethylsulfamoyl-4,5,6,7-tetrahydro-isoxazolo[4,5-c]pyridin-3-carbonsäure [2-(3,4-dichlor-phenyl)-ethyl]-amid,
[123] 5-(Thiophen-2-sulfonyl)-4,5,6,7-tetrahydro-isoxazolo[4,5-c]pyridin-3-carbonsäure cyclopropylmethyl-amid,
[124] 5-(Chinolin-6-carbonyl)-4,5,6,7-tetrahydro-isoxazolo[4,5-c]pyridin-3-carbonsäure 4-fluor-benzylamid,
[125] 3-{[5-(2-Naphthalen-2-yl-acetyl)-4,5,6,7-tetrahydro-isoxazolo[4,5-c]pyridin-3-carbonyl]-amino}-propionsäure ethyl ester,
[126] [2-(3-Isobutylcarbamoyl-6,7-dihydro-4H-isoxazolo[4,5-c]pyridin-5-yl)-2-oxo-1-phenyl-ethyl]-carbaminsäure benzyl ester,
[127] 5-(4-Methoxy-benzylthiocarbamoyl)-4,5,6,7-tetrahydro-isoxazolo[4,5-c]pyridin-3-carbonsäure cyclopentylamid,
[128] 5-Benzoylaminocarbothloyl-4,5,6,7-tetrahydro-isoxazolo[4,5-c]pyridin-3-carbonsäure (2-pyrrolidin-1-yl-ethyl)-amid,
[129] 5-(4-Chlor-benzylthiocarbamoyl)-4,5,6,7-tetrahydro-Isoxazolo[4,5-c]pyridin-3-carbonsäure-cyclopropylmethyl-amid,
[130] 5-(2-Methoxy-ethylthiocarbamoyl)-4,5,6,7-tetrahydro-isoxazolo[4,5-c]pyridin-3-carbonsäure phenethyl-amid,
[131] 5-pentafluorphenylthiocarbamoyl-4,5,6,7-tetrahydro-isoxazolo[4,5-c]pyridin-3-carbonsäure phenethyt-amid,
[132] 5-(1-Phenyl-ethylthiocarbamoyl)-4,5,6,7-tetrahydro-isoxazolo[4,5-c]pyridin-3-carbonsäure phenethyl-amid,
[133] 5-Pentafluorphenylthiocarbamoyl-4,5,6,7-tetrahydro-isoxazolo[4,5-c]pyridin-3-carbonsäure (5-methyl-furan-2-ylmethyl)-amid,
[134] 3-{[5-(Cyclohexylmethyl-thiocarbamoyl)-4,5,6,7-tetrahydro-isoxazolo[4,5-c]pyridin-3-carbonyl]-amino}-propionsäure ethyl ester,
[135] 5-(1-Brom-naphthalen-2-ylmethyl)-4,5,6,7-tetrahydro-isoxazolo[4,5-c]pyridin-3-carbonsäurephenylamid.
jeweils ggf. In Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem bellebigen Mischungsverhältnis, oder jeweils in Form entsprechender Salze, oder jeweils in Form entsprechender Solvate.

Ein weiterer Gegenstand der vorliegenden Erfindung ist ein Verfahren zur Herstellung der erfindungsgemäßen Verbindungen der vorstehend angegebenen allgemeinen Formel I gemäß dem wenigstens einer Verbindung der allgemeinen Formel II, 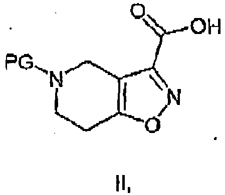 worin PG für eine Schutzgruppe, bevorzugt für eine tert-Butyloxy-carbonyl- oder Benzyloxycarbonyl-Gruppe steht, in einem Reaktionsmedium, in Gegenwart wenigstens eines Kupplungsreagenzes, ggf. in Gegenwart wenigstens einer Base, bevorzugt in Gegenwart wenigstens einer Base ausgewählt aus der Gruppe bestehend aus Pyridin, N-Methylmorpholin, Diisopropylethylamin, Triethylamin und 4,4-Dimethylaminopyridin, mit wenigstens einem Amin der allgemeinen Formel HNR¹R², worin die Reste R¹ und R² die vorstehend angegebene Bedeutung haben, zu wenigstens einer Verbindung der allgemeinen Formel III, 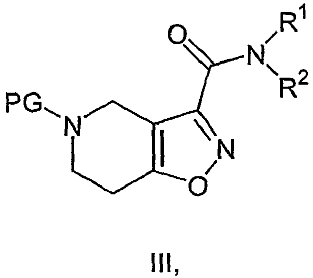 worin R¹, R² und PG die vorstehend genannte Bedeutung haben, umgesetzt wird, und diese ggf. gereinigt und/oder isoliert wird,
und wenigstens eine Verbindung der allgemeinen Formel III, durch Abspaltung der Schutzgruppe PG in einem Reaktionsmedium, vorzugsweise durch Abspaltung in Gegenwart wenigstens einer Säure oder in Gegenwart wenigstens einer Base für die tert-Butyloxy-carbonyl-Gruppe oder in Gegenwart von Wasserstoff und Katalysator, bevorzugt Palladium auf Kohle für die Benzyloxycarbonyl-Gruppe, zu wenigstens einer Verbindung der allgemeinen Formel IV, ggf. in Form eines entsprechenden Salzes, bevorzugt in Form des entsprechenden Hydrochlorids, 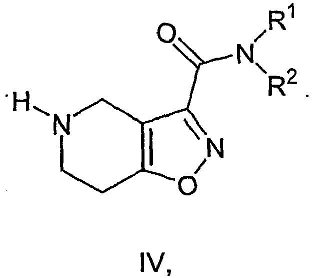 worin R¹ und R² die vorstehend genannte Bedeutung haben, umgesetzt wird, und diese ggf. gereinigt und/oder isoliert wird,
und
ggf. wenigstens eine Verbindung der allgemeinen Formel IV in Form eines entsprechenden Salzes, bevorzugt in Form eines entsprechenden Hydrochlorids, in einem Reaktionsmedium, in Gegenwart wenigstens einer Base, bevorzugt in Gegenwart wenigstens eines Metallhydroxids, besonders bevorzugt in Gegenwart von Kalium- und/oder Natriumhydroxid, zu wenigstens einer Verbindung der allgemeinen Formel IV umgesetzt wird, und diese ggf. gereinigt und/oder isoliert wird,
und
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Pyridin, Triethylamin, 4,4-Dimethylaminopyridin, Diisopropylethylamin und Diisopropylamin, mit wenigstens einem Carbonsäure-Derivat der allgemeinen Formel LG-C(=O)-R¹⁵, LG-C(=O)-(CH₂)ⱼ-Xₖ-(CH₂)ₘ-(CH₂)ₙ-C(=O)-OR¹⁶, LG-C(=O)-(CHR¹⁹)-NH-C(=O)-OR²⁰, LG-C(=O)-(CH₂)-(CH₂)ₚ-(CH₂)_{q}-Yᵣ-(CH₂)ₛ-R²¹ oder LG-C(=O)-(CH=CH)-R²², worin R¹⁵, R¹⁶, R¹⁹, R²⁰, R²¹, R²², j, k, m, n, p, q, r, s, X und Y die vorstehend angegebene Bedeutung haben und LG für eine Abgangsgruppe, bevorzugt für ein Halogen-Atom, besonders bevorzugt für ein Chloratom steht, zu wenigstens einer Verbindung der allgemeinen Formel umgesetzt, wird, worin R¹ und R² die vorstehend genannte Bedeutung haben und R³ für -C(=O)-R¹⁵, -C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂)-(CH₂)ₚ-(CH₂)_{q}-Yᵣ-(CH₂)ₛ-R²¹ oder -C(=O)-(CH=CH)-R²² steht, wobei R¹⁵, R¹⁶, R¹⁹, R²⁰, R²¹, R²², j, k, m, n, p, q, r, s, X und Y die vorstehend genannte Bedeutung haben, und diese ggf. gereinigt und/oder isoliert wird
oder
wenigstens eine Verbindung der allgemeinen Formel IV in einem Reaktionsmedium, in Gegenwart eines Kupplungsreagenzes, ggf. in Gegenwart wenigstens einer Base, bevorzugt in Gegenwart wenigstens einer Base ausgewählt aus der Gruppe bestehend aus Pyridin, N-Methylmorpholin, Diisopropylethylamin, Triethylamin und 4,4-Dimethylaminopyridin, mit wenigstens einer Carbonsäure der allgemeinen Formel HO-C(=O)-R¹⁵, HO-C(=O)-(CH₂)ⱼ-Xₖ-(CH₂)ₘ-(CH₂)ₙ-C(=O)-OR¹⁶, HO-C(=O)-(CHR¹⁹)-NH-C(=O)-OR²⁰, HO-C(=O)-(CH₂)-(CH₂)ₚ-(CH₂)_{q}-Yᵣ-(CH₂)ₛ-R²¹ oder HO-C(=O)-(CH=CH)-R²², worin R¹⁵, R¹⁶, R¹⁹, R²⁰, R²¹, R²², j, k, m, n, p, q, r, s, X und Y die vorstehend angegebene Bedeutung haben, zu wenigstens einer Verbindung der allgemeinen Formel I umgesetzt wird, worin R¹ und R² die vorstehend genannte Bedeutung haben und R³ für -C(=O)-R¹⁵, -C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂)-(CH₂)ₚ-(CH₂)_{q}-Yᵣ-(CH₂)ₛ-R²¹ oder -C(=O)-(CH=CH)-R²² steht, wobei R¹⁵, R¹⁶, R¹⁹, R²⁰, R²¹, R²², j, k, m, n, p, q, r, s, X und Y die vorstehend genannte Bedeutung haben, und diese ggf. gereinigt und/oder isoliert wird
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Pyridin, Triethylamin, 4,4-Dimethylaminopyridin, Diisopropylethylamin und Diisopropylamin, mit wenigstens einem Sulfonsäure-Derivat der allgemeinen Formel LG-S(=O)₂-R²³ oder LG-S(=O)₂-NR²⁴R²⁵, worin R²³, R²⁴ und R²⁵ die vorstehend angegebene Bedeutung haben und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ und R² die vorstehend genannte Bedeutung haben und R³ für -S(=O)₂-R²³ oder -S(=O)₂-NR²⁴R²⁵ steht, wobei R²³, R²⁴ und R²⁵ die vorstehend genannte Bedeutung haben, und diese ggf. gereinigt und/oder isoliert wird
oder
wenigstens eine Verbindung der allgemeinen Formel IV in einem Reaktionsmedium mit wenigstens einem Isocyanat der allgemeinen Formel R¹⁴-N=C=O, worin R¹⁴ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt, wird, worin R¹ und R² die vorstehend genannte Bedeutung haben und R³ für -C(=O)-NR¹³R¹⁴ steht, wobei R¹⁴ die vorstehend genannte Bedeutung hat und R¹³ für Wasserstoff steht, und diese ggf. gereinigt und/oder isoliert wird
oder
wenigstens eine Verbindung der allgemeinen Formel IV in einem Reaktionsmedium mit wenigstens einem Isothiocyanat der allgemeinen Formel S=C=N-(CH₂)ₜ-(CH₂)ᵤ-Zᵥ-R²⁷, S=C=N-(CHR²⁸)-R²⁹ oder S=C=N-C(=O)-R³⁰, worin R²⁷, R²⁸, R²⁹, R³⁰, Z, t, u und v die vorstehend angegebene Bedeutung haben, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹ und R² die vorstehend genannte Bedeutung haben und R³ für -C(=S)-NR²⁶-(CH₂)ₜ-(CH₂)ᵤ-Zᵥ-R²⁷, -C(=S)-NR²⁶-(CHR²⁸)-R²⁹ oder -C(=S)-NR²⁶-C(=O)-R³⁰ steht, wobei R²⁷, R²⁸, R²⁹, R³⁰, Z, t, u und v die vorstehend genannte Bedeutung haben und R²⁶ für Wasserstoff steht, und diese ggf. gereinigt und/oder isoliert wird
und ggf. wenigstens eine Verbindung der allgemeinen Formel I, worin R¹ und R² die vorstehend genannte Bedeutung haben und R³ für -C(=S)-NR²⁶-(CH₂)ₜ-(CH₂)ᵤ-Zᵥ-R²⁷, -C(=S)-NR²⁶-(CHR²⁸)-R²⁹, -C(=S)-NR²⁶-C(=O)-R³⁰ oder -C(=O)-NR¹³R¹⁴ steht, wobei R¹⁴, R²⁷, R²⁸, R²⁹, R³⁰, Z, t, u und v die vorstehend genannte Bedeutung haben und R¹³ und R²⁶ für Wasserstoff stehen, in einem Reaktionsmedium, ggf. in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-R¹³ oder LG-R²⁶, worin R¹³ und R²⁶ die vorstehend genannte Bedeutung mit Ausnahme eines Wasserstoff-Restes haben und LG für eine Abgangsgruppe, bevorzugt für ein Halogen-Atom, besonders bevorzugt für ein Chloratom steht, zu wenigstens einer Verbindung der allgemeinen Formel I umgesetzt, wird, worin R¹ und R² die vorstehend genannte Bedeutung haben und R³ für -C(=S)-NR²⁶-(CH₂)ₜ-(CH₂)ᵤ-Zᵥ-R²⁷, -C(=S)-NR²⁶-(CHR²⁸)-R²⁹, -C(=S)-NR²⁶-C(=O)-R³⁰ oder -C(=O)-NR¹³R¹⁴ steht, wobei R¹³, R²⁶, R¹⁴, R²⁷, R²⁸, R²⁹, R³⁰, Z, t, u und v die vorstehend genannte Bedeutung haben, und diese ggf. gereinigt und/oder isoliert wird
oder
wenigstens eine Verbindung der allgemeinen Formel IV in einem Reaktionsmedium, ggf. in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-(CH₂)-R³¹, worin R³¹ die vorstehend angegebene Bedeutung hat und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ und R² die vorstehend genannte Bedeutung haben und R³ für -(CH₂)-R³¹ steht, wobei R³¹ die vorstehend genannte Bedeutung hat, und diese ggf. gereinigt und/oder isoliert wird
oder wenigstens eine Verbindung der allgemeinen Formel IV in einem Reaktionsmedium, in Gegenwart wenigstens eines Reduktionsmittels, mit wenigstens einer Verbindung der allgemeinen Formel R³¹-C(=O)-H, worin R³¹ die vorstehend genannte Bedeutung hat, zu wenigstens einer Verbindung der allgemeinen Formel I umgesetzt wird, worin R¹ und R² die vorstehend genannte Bedeutung haben und R³ für -(CH₂)-R³¹ steht, wobei R³¹ die vorstehend genannte Bedeutung hat, und diese ggf. gereinigt und/oder isoliert wird.

Das erfindungsgemäße Verfahren zur Herstellung substituierter 4,5,6,7-Tetrahydro-isoxazolo[4,5-c]pyridin-Verbindungen der vorstehend angegebenen allgemeinen Formel I ist auch in dem folgenden Schema 1 wiedergegeben.

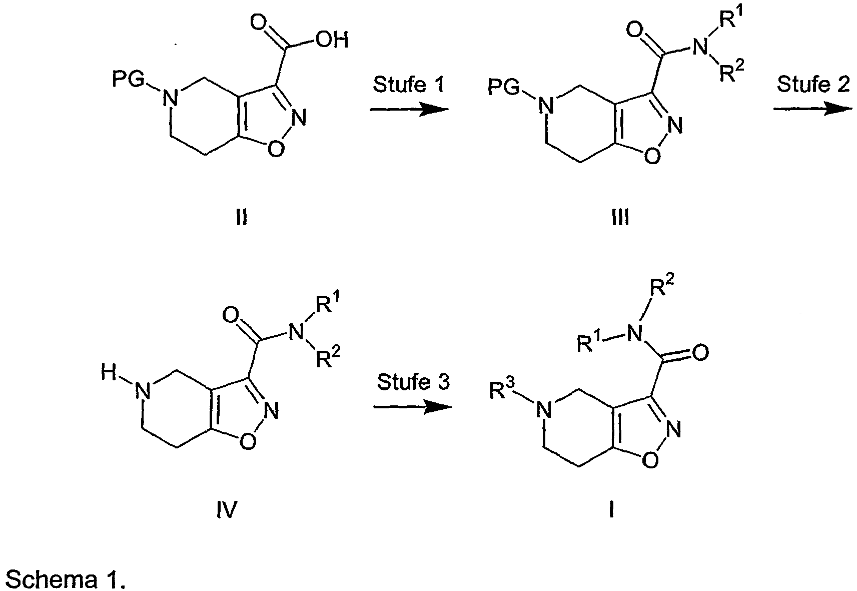

In Stufe 1. werden Verbindungen der vorstehend angegeben allgemeinen Formel II mit Aminen der allgemeinen Formel HNR¹R², worin R¹ und R² die vorstehend genannte Bedeutung haben, in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan bzw. entsprechenden Mischungen, ggf. in Gegenwart wenigstens eines Kupplungsreagenzes vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1 H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, N-Methylmorpholin, 4,4-Dimethylaminopyridin und Diisopropylethylamin vorzugsweise bei Temperaturen von vorzugsweise -70°C bis 100°C zu Verbindungen der allgemeinen Formel III umgesetzt.

In Stufe 2. werden Verbindungen der allgemeinen Formel III, worin PG für eine tert-Butyloxy-carbonyl-Gruppe steht, in einem Reaktionsmedium vorzugsweise ausgewählt aus der Gruppe bestehend aus Methanol, Ethanol, Isopropanol, Wasser, Diethylether, Tetrahydrofuran sowie entsprechenden Mischungen in Gegenwart wenigstens einer Säure vorzugsweise ausgewählt aus der Gruppe bestehend aus Salzsäure, Schwefelsäure, Trifluoressigsäure und Essigsäure bei Temperaturen von vorzugsweise 20 bis 30 °C zu Verbindungen der allgemeinen Formel IV umgesetzt. Besonders bevorzugt erfolgt die Umsetzung der Verbindung der allgemeinen Formel III in einer 4 M Salzsäure-Lösung in Methanol bei einer Temperatur von vorzugsweise 20 bis 30 °C zu einer Verbindung der allgemeinen Formel IV in Form eines entsprechenden Hydrochlorids.

Alternativ werden Verbindungen der allgemeinen Formel III, worin PG für eine Benzyloxycarbonyl-Gruppe steht, in einem Reaktionsmedium vorzugsweise ausgewählt aus der Gruppe bestehend aus Diethylether, Tetrahydrofuran, Dioxan, Acetonitril, Toluol und entsprechenden Mischungen in Gegenwart von Wasserstoff und Palladium auf Kohle bei einer Temperatur von vorzugsweise 20 bis 80 °C zu einer Verbindung der allgemeinen Formel IV umgesetzt.
Sofern Verbindungen der allgemeinen Formel IV in Form eines entsprechenden Hydrochlorids vorliegen, werden diese in einem Reaktionsmedium, vorzugsweise ausgewählt aus der Gruppe bestehend aus Dioxan, Tetrahydrofuran, Diethylether, Methanol, Ethanol, Isopropanol, Wasser und entsprechenden Mischungen, in Gegenwart einer anorganischen Base, vorzugsweise unter Zusatz eines Metallhydroxids, beispielsweise Natriumhydroxid, Kaliumhydroxid oder Lithiumhydroxid, bei Temperaturen von vorzugsweise 0°C bis 30°C in die entsprechende Basen der allgemeinen Formel IV umgesetzt.

In Stufe 3. werden Verbindungen der allgemeinen Formel IV mit Carbonsäure-Derivaten der allgemeinen Formel LG-C(=O)-R¹⁵, LG-C(=O)-(CH₂)ⱼ-Xₖ-(CH₂)ₘ-(CH₂)ₙ-C(=O)-OR¹⁶, LG-C(=O)-(CHR¹⁹)-NH-C(=O)-OR²⁰, LG-C(=O)-(CH₂)-(CH₂)ₚ-(CH₂)_{q}-Yᵣ-(CH₂)ₛ-R²¹ oder LG-C(=O)-(CH=CH)-R²², worin R¹⁵, R¹⁶ , R¹⁹, R²⁰, R²¹ , R²², j, k, m, n, p, q, r, s, X und Y die vorstehend angegebene Bedeutung haben und LG für eine Abgangsgruppe, bevorzugt für ein Halogen-Atom, besonders bevorzugt für ein Chloratom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimethylaminopyridin, Pyridin und Diisopropylethylamin, oder einer anorganischen Base, bei Temperaturen von vorzugsweise -70°C bis 100°C zu Verbindungen der allgemeinen Formel I, worin R³ für -C(=O)-R¹⁵, -C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂) -(CH₂)ₚ-(CH₂)_{q}-Yᵣ-(CH₂)ₛ-R²¹ oder -C(=O)-(CH=CH)-R²² steht, umgesetzt.

Alternativ werden Verbindungen der vorstehend angegebenen allgemeinen Formel IV mit Carbonsäuren der allgemeinen Formel OH-C(=O)-R¹⁵, OH-C(=O)-(CH₂)ⱼ-Xₖ-(CH₂)ₘ-(CH₂)ₙ-C(=O)-OR¹⁶, OH-C(=O)-(CHR¹⁹)-NH-C(=O)-OR²⁰, OH-C(=O)-(CH₂)-(CH₂)ₚ-(CH₂)_{q}-Yᵣ-(CH₂)ₛ-R²¹ oder OH-C(=O)-(CH=CH)-R²², worin R¹⁵ R¹⁶, R¹⁹, R²⁰, R²¹, R²², j, k, m, n, p, q, r, s, X und Y die vorstehend angegebene Bedeutung haben, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1 H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, N-Methylmorpholin, 4,4-Dimethylaminopyridin und Diisopropylethylamin vorzugsweise bei Temperaturen von vorzugsweise -70°C bis 100°C zu Verbindungen der allgemeinen Formel I, worin R³ für -C(=O)-R¹⁵, -C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂) -(CH₂)ₚ-(CH₂)_{q}-Yᵣ-(CH₂)ₛ-R²¹ oder -C(=O)-(CH=CH)-R²² steht, umgesetzt.

Ebenfalls können Verbindungen der allgemeinen Formel IV mit Sulfonsäure-Derivaten der allgemeinen Formel LG-S(=O)₂-R²³ oder LG-S(=O)₂-NR²⁴R²⁵, worin R²³, R²⁴ und R²⁵ die vorstehend angegebene Bedeutung haben und LG für eine Abgangsgruppe, bevorzugt für ein Halogen-Atom, besonders bevorzugt für ein Chloratom steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimethylaminopyridin, Pyridin und Diisopropylethylamin, oder einer anorganischen Base, bei Temperaturen von vorzugsweise -70°C bis 100°C zu Verbindungen der allgemeinen Formel I, worin R³ für -S(=O)₂-R²³ oder -S(=O)₂-NR²⁴R²⁵ steht, umgesetzt werden.

Alternativ werden Verbindungen der allgemeinen Formel IV mit einem Isocyanat der allgemeinen Formel R¹⁴-N=C=O, worin R¹⁴ die vorstehend angegebene Bedeutung hat, in einem Reaktionsmedium, vorzugsweise ausgewählt aus der Gruppe bestehend aus Acetonitril, Toluol, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, N-Methylmorpholin, Pyridin, 4,4-Dimethylaminopyridin und Diisopropylethylamin, zu Verbindungen der allgemeinen Formel I umgesetzt, worin R³ für -C(=O)-NR¹³R¹⁴ steht.

Alternativ werden in Stufe 3. Verbindungen der allgemeinen Formel IV mit Isothiocyanaten der allgemeinen Formel S=C=N-(CH₂)ₜ-(CH₂)ᵤ-Zᵥ-R²⁷, S=C=N-(CHR²⁸)-R²⁹ oder S=C=N-C(=O)-R³⁰, worin R²⁷, R²⁸, R²⁹, R³⁰, Z, t, u und v die vorstehend angegebene Bedeutung haben, in einem Reaktionsmedium, vorzugsweise ausgewählt aus der Gruppe bestehend aus Acetonitril, Toluol, Benzol, Ethanol, Methanol, Wasser und entsprechenden Mischungen, ggf. in Gegenwart wenigstens einer Base, bevorzugt in Gegenwart wenigstens einer Base ausgewählt aus der Gruppe bestehend aus Triethylamin, 4,4-Dimethylaminopyridin, Pyridin, N-Methylmorpholin und Diisopropylethylamin, zu Verbindungen der allgemeinen Formel I, worin R³ für -C(=S)-NR²⁶-(CH₂)ₜ-(CH₂)ᵤ-Zᵥ-R²⁷, -C(=S)-NR²⁶-(CHR²⁸)-R²⁹ oder-C(=S)-NR²⁶-C(=O)-R³⁰ steht, umgesetzt.

Ebenfalls werden in Stufe 3. Verbindungen der allgemeinen Formel IV mit Verbindungen der allgemeinen Formel LG-(CH₂)-R³¹, worin R³¹ die vorstehend angegebene Bedeutung hat und LG für eine Abgangsgruppe, bevorzugt für ein Halogen-Atom, besonders bevorzugt für ein Chloratom steht, in einem Reaktionsmedium, vorzugsweise ausgewählt aus der Gruppe bestehend aus Dichlormethan, Toluol, Tetrahydrofuran, Acetonitril, Diethylether, Dioxan und entsprechenden Mischungen ggf. in Gegenwart wenigstens einer Base, bevorzugt in Gegenwart wenigstens eines Metallhydridsalzes, besonders bevorzugt in Gegenwart von Natrium- und/oder Kaliumhydrid, zu Verbindungen der allgemeinen Formel I, worin R³ für -(CH₂)-R³¹ steht, umgesetzt.

Alternativ werden in Stufe 3. Verbindungen der allgemeinen Formel IV mit Verbindungen der allgemeinen Formel R³¹-C(=O)-H, worin R³¹ die vorstehend genannte Bedeutung hat, in einem Reaktionsmedium, vorzugsweise ausgewählt aus der Gruppe bestehend aus Diethylether, Tetrahydrofuran, Methanol, Ethanol, Dichlormethan, Toluol und entsprechenden Mischungen, unter Zusatz wenigstens eines Reduktionsmittels, vorzugsweise unter Zusatz wenigstens eines Reduktionsmittels ausgewählt aus der Gruppe bestehend aus Natriumborhydrid, Natriacetoxyborhydrid, Natriumcyanoborhydrid und Boran-Pyridin-Komplex (Pyridin-Boran, BH₃●C₅H₅N), besonders bevorzugt in Gegenwart von Boran-Pyridin-Komplex, zu Verbindungen der allgemeinen Formel I, worin R³ für -(CH₂)-R³¹ steht, umgesetzt.

Die Verbindungen der allgemeinen Formel II lassen sich bevorzugt wie in Schema 2 beschrieben erhalten.

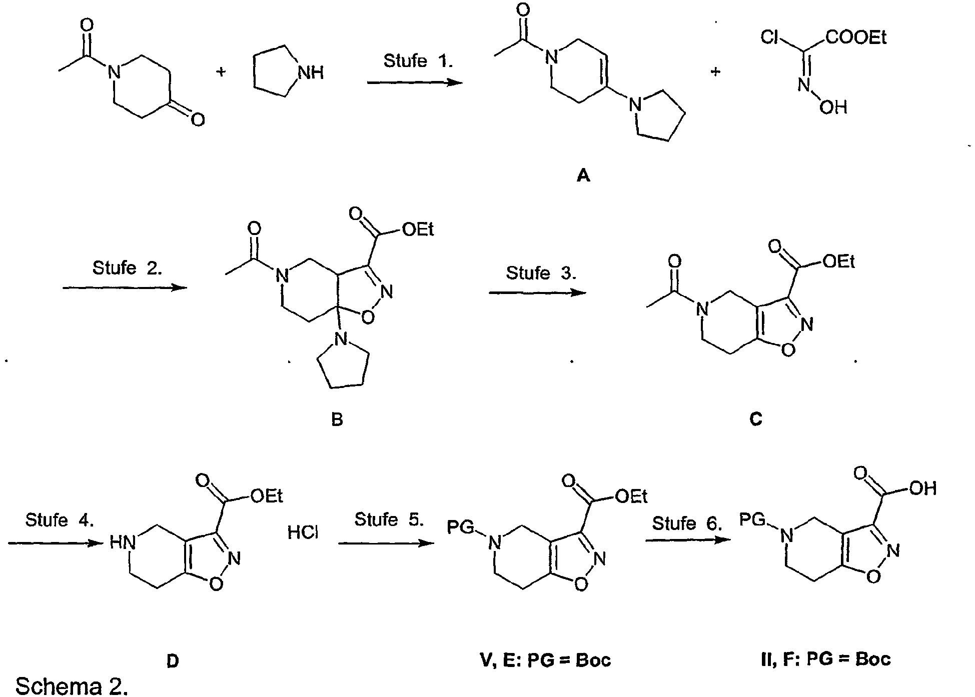

In Stufe 1. wird die Verbindung 1-Acetyl-4-piperidinon in einem Reaktionsmedium, vorzugsweise ausgewählt aus der Gruppe bestehend aus Toluol und Benzol, mit Pyrrolidin in Gegenwart einer katalytischen Menge einer Säure, vorzugsweise in Gegenwart von p-Toluolsulfonsäure, unter Rückfluß am Wasserabscheider zur Verbindung 1-(4-Pyrrolidin-1-yl-3,6-dihydro-2H-pyridin-1-yl)-ethanon (A) umgesetzt.

In Stufe 2, wird 1-(4-Pyrrolidin-1-yl-3,6-dihydro-2H-pyridin-1-yl)-ethanon (A) mit 2-Chlorhydroxyiminoessigsäureethylester in einem Reaktionsmedium, vorzugsweise ausgewählt aus der Gruppe bestehend aus Acetonitril, Dichlormethan, Chloroform, Dimethylformamid, Dimethylacetamid, Dimethylsulfoxid und entsprechenden Mischungen, in Gegenwart wenigstens einer Base, vorzugsweise ausgewählt aus der Gruppe bestehend aus Triethylamin, Pyridin, 4,4-Dimethylaminopyridin, N-Methylmorpholin und Diisopropylethylamin, bei einer Temperatur von vorzugsweise 0 bis 30 °C zur Verbindung 5-Acetyl-7a-pyrrolidin-1-yl-3a,4,5,6,7,7a-hexahydro-isoxazolo[4,5-c]pyridin-3-carbonsäureethylester (B) umgesetzt.

In Stufe 3. wird 5-Acetyl-7a-pyrrolidin-1-yl-3a,4,5,6,7,7a-hexahydro-isoxazolo[4,5-c]pyridin-3-carbonsäureethylester (B) in einem Reaktionsmedium, vorzugsweise ausgewählt aus der Gruppe bestehend aus Acetonitril, Dichlormethan, Chloroform, Dimethylformamid, Dimethylacetamid, Dimethylsulfoxid und entsprechenden Mischungen, in Gegenwart einer organischen Säure, vorzugsweise in Gegenwart vonTifluoressigsäure, unter Rückfluß zur Verbindung 5-Acetyl-4,5,6,7,-tetrahydro-isoxazolo[4,5-c]pyridin-3-carbonsäure-ethylester (C) umgesetzt.

In Stufe 4. wird die Verbindung 5-Acetyl-4,5,6,7,-tetrahydro-isoxazolo[4,5-c]pyridin-3-carbonsäure-ethylester(C) in einem Reaktionsmedium, vorzugsweise ausgewählt aus der Gruppe bestehend aus Methanol, Ethanol, Isopropanol, Tetrahydrofuran, Wasser und entsprechenden Mischungen, in Gegenwart einer anorganischen Säure, vorzugsweise in Gegenwart von Salzsäure und/oder Schwefelsäure unter Rückfluß zur Verbindung 4,5,6,7-Tetrahydro-isoxazolo[4,5-c]pyridin-3-carbonsäure-ethylester Hydrochlorid (D) in Form des entsprechenden Hydrochlorids umgesetzt.

In Stufe 5. wird die Verbindung 4,5,6,7-Tetrahydro-isoxazolo[4,5-c]pyridin-3-carbonsäure-ethylester Hydrochlorid (D) in einem Reaktionsmedium, vorzugsweise ausgewählt aus der Gruppe bestehend aus Dioxan, Ethanol, Methanol, Isopropanol, Wasser und entsprechenden Mischungen, in Gegenwart wenigstens einer organischen Base, vorzugsweise ausgewählt aus der Gruppe bestehend aus Triethylamin, Diisopropylethylamin, Pyridin, 4,4-Dimethylaminopyridin und N-Methylmorpholin, mit einem schutzgruppenübertragenden Reagenz, vorzugsweise mit einem Reagenz ausgewählt aus der Gruppe bestehend aus Di-tert-butyldicarbonat [(Boc)₂O] und Benzylchloroformiat, bei 0 °C und anschließend bei 20 bis 30°C zu einer Verbindung der allgemeinen Formel V (mit PG = Boc: 6,7-Dihydro-4H-isoxazolo[4,5-c]pyridin-3,5-dicarbonsäure-3-ethylester-5-tert-butylester (E)) umgesetzt. Vorzugsweise wird als Reaktionsmedium eine Dioxan/Wasser-Mischung (2:1) verwendet.

In Stufe 6. wird eine Verbindung der allgemeinen Formel V in einem Reaktionsmedium, vorzugsweise ausgewählt aus der Gruppe bestehend aus Methanol, Ethanol, Isopropanol, Wasser und entsprechenden Mischungen, mit einer anorganischen Base, vorzugsweise mit einer Base ausgewählt aus der Gruppe bestehend aus Lithiumhydroxid, Kaliumhydroxid und Natriumhydroxid bei einer Temperatur von vorzugsweise 20 bis 30 °C zu einer Verbindung der allgemeinen Formel II (PG = Boc: 6,7-Dihydro-4H-isoxazolo[4,5-c]pyridin-3,5-dicarbonsäure-5-tert-butylester (F)) umgesetzt.

Die Verbindungen der vorstehend angegebenen allgemeinen Formeln LG-C(=O)-R¹⁵, LG-C(=O)-(CH₂)ⱼ-Xₖ(CH₂)ₘ-(CH₂)ₙ-C(=O)-OR¹⁶, LG-C(=O)-(CHR¹⁹)-NH-C(=O)-OR²⁰, LG-C(=O)-(CH₂)-(CH₂)ₚ-(CH₂)_{q}-Yᵣ-(CH₂)ₛ-R²¹, LG-C(=O)-(CH=CH)-R²²,)-NH-C(=O)-OR²⁰, -C(=O)-(CH₂)-(CH₂)ₚ-(CH₂)_{q}-Yᵣ-(CH₂)ₛ-R²¹, -C(=O)-(CH=CH)-R²², OH-C(=O)-R¹⁵, OH-C(=O)-(CH₂)ⱼ-Xₖ-(CH₂)ₘ-(CH₂)ₙ-C(=O)-OR¹⁶, OH-C(=O)-(CHR¹⁹)-NH-C(=O)-OR²⁰, OH-C(=O)-(CH₂)-(CH₂)ₚ-(CH₂)_{q}-Yᵣ-(CH₂)ₛ-R²¹, OH-C(=O)-(CH=CH)-R²², LG-R¹³, LG-R²⁶, LG-S(=O)₂-R²³, LG-S(=O)₂-NR²⁴R²⁵, R¹⁴-N=C=O, S=C=N-(CH₂)ₜ-(CH₂)ᵤ-Zᵥ-R²⁷, S=C=N-(CHR²⁸)-R²⁹, S=C=N-C(=O)-R³⁰ und LG-(CH₂)-R³¹ sind jeweils am Markt käuflich erhältlich und/oder können nach den üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten 4,5,6,7-Tetrahydro-isoxazolo[4,5-c]pyridin-Verbindungen der vorstehend genannten allgemeinen Formel I sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten 4,5,6,7-Tetrahydro-isoxazolo[4,5-c]pyridin-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden.

Die freien Basen der jeweiligen substituierten 4,5,6,7-Tetrahydro-isoxazolo[4,5-c]pyridin-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten 4,5,6,7-Tetrahydro-isoxazolo[4,5-c]pyridin-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-AlkylRest steht, genannt.

Die erfindungsgemäßen substituierten 4,5,6,7-Tetrahydro-isoxazoio[4,5-c]pyridin-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten 4,5,6,7-Tetrahydro-isoxazolo[4,5-c]pyridin-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 4,5,6,7-Tetrahydro-isoxazolo[4,5-c]pyridin-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße 4,5,6,7-Tetrahydro-isoxazolo[4,5-c]pyridin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Die erfindungsgemäßen Arzneimittel eignen sich insbesondere zur Noradrenalin-Rezeptor-Regulation, insbesondere zur Hemmung der Noradrenalin-Wiederaufnahme (NA-Uptake), zur 5-HT-Rezeptor-Regulation, insbesondere zur Hemmung der 5-Hydroxy-Tryptophan-Wiederaufnahme (5-HT-Uptake) und/oder zur Batrachotoxin-(BTX)-Rezeptor-Regulation.

Ebenfalls bevorzugt eignen sich die erfindungsgemäßen Arzneimittel zur Prophylaxe und/oder Behandlung von Störungen oder Krankheiten, die zumindest teilweise durch Noradrenalin-Rezeptoren, 5-HT-Rezeptoren und/oder Batrachotoxin-Rezeptoren vermittelt werden.

Bevorzugt eignet sich das erfindungsgemäße Arzneimittel daher zur Prophylaxe und/oder Behandlung von Schmerz, vorzugsweise von Schmerz ausgewählt aus der Gruppe bestehend aus akutem Schmerz, chronischem Schmerz und neuropathischem Schmerz, zur Prophylaxe und/oder Behandlung von einer oder mehreren Erkrankungen ausgewählt aus der Gruppe bestehend aus Migräne; Depressionen; Entzündungen; Antriebslosigkeit; Harninkontinenz; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Katalepsie; Diarrhoe und Pruritus; zur Anxiolyse, zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese; zur Antinatriurese; zur Verwendung als Lokalanästhetikum und/oder Antiarrhythmikum und/oder Antiemetikum und/oder Nootropikum (Neurotropikum).

Ganz besonders bevorzugt eignet sich das erfindungsgemäße Arzneimittel zur Behandlung und/oder Prophylaxe von einer oder mehrerer Erkrankungen ausgewählt aus der Gruppe bestehend aus Schmerz, vorzugsweise von Schmerz ausgewählt aus der Gruppe bestehend aus akutem Schmerz, chronischem Schmerz und neuropathischem Schmerz, Depressionen und Angstzuständen.

Ein weiterer Gegenstand der vorliegenden Erfindung ist die Verwendung wenigstens eines erfindungsgemäßen 4,5,6,7-Tetrahydro-isoxazolo[4,5-c]pyridin-Derivats der vorstehend angegebenen allgemeinen Formel I, jeweils ggf. in Form eines seiner reinen Stereoisomeren, insbesondere Enantiomeren oder Diastereomeren; seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Noradrenalin-Rezeptor-Regulation, insbesondere zur Hemmung der Noradrenalin-Wiederaufnahme (NA-Uptake), zur 5-HT-Rezeptor-Regulation, insbesondere zur Hemmung der 5-Hydroxy-Tryptophan-Wiederaufnahme (5-HT-Uptake) und/oder zur Batrachotoxin-(BTX)-Rezeptor-Regulation.

Bevorzugt ist die Verwendung wenigstens eines substituierten 4,5,6,7-Tetrahydroisoxazolo[4,5-c]pyridin-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Noradrenalin-Rezeptoren, 5-HT-Rezeptoren und/oder Batrachotoxin-Rezeptoren vermittelt werden.

Besonders bevorzugt ist die Verwendung wenigstens eines substituierten 4,5,6,7-Tetrahydro-isoxazolo[4,5-c]pyridin-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz und neuropathischem Schmerz, zur Prophylaxe und/oder Behandlung von einer oder mehreren Erkrankungen ausgewählt aus der Gruppe bestehend aus Migräne; Depressionen; Entzündungen; Antriebslosigkeit; Harninkontinenz; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Katalepsie; Diarrhoe und Pruritus; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese; zur Antinatriurese; zur Verwendung als Lokalanästhetikum und/oder Antiarrhythmikum und/oder Antiemetikum und/oder Nootropikum (Neurotropikum).

Ganz besonders bevorzugt ist die Verwendung wenigstens eines substituierten 4,5,6,7-Tetrahydro-isoxazolo[4,5-c]pyridin-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz und neuropathischem Schmerz, zur Prophylaxe und/oder Behandlung von einer oder mehreren Erkrankungen ausgewählt aus der Gruppe bestehend aus Migräne; Depressionen; Entzündungen; Antriebslosigkeit; Harninkontinenz; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie und zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit.

Noch weiter bevorzugt ist die Verwendung wenigstens eines substituierten 4,5,6,7-Tetrahydro-isoxazoio[4,5-c]pyridin-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehrerer Erkrankungen ausgewählt aus der Gruppe bestehend aus Schmerz, vorzugsweise von Schmerz ausgewählt aus der Gruppe bestehend aus akutem Schmerz, chronischem Schmerz und neuropathischem Schmerz, Depressionen und Angstzuständen.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem substituierten 4,5,6,7-Tetrahydro-isoxazolo[4,5-c]pyridin-Derivat der vorstehend angegebenen allgemeinen Formel I, ggf. in Form eines seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten 4,5,6,7-Tetrahydro-isoxazolo[4,5-c]pyridin-Verbindungen können in einem Depot in gelöster Form oder in einem Pflaster, gegebenenfalls unter Zusatz von die Hautpenetration fördernden Mitteln, als geeignete perkutane Applikationszubereitungen vorliegen.

Oral oder perkutan anwendbare Zubereitungsformen können die jeweiligen erfindungsgemäßen substituierten 4,5,6,7-Tetrahydro-isoxazofo[4,5-c]pyridin-Verbindungen auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten 4,5,6,7-Tetrahydro-isoxazolo[4,5-c]pyridin-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

### Pharmakologische Methoden:

### a) Methode zur Bestimmung der Noradrenalin- und der 5-HT-Uptake-Inhibierung:

Für in vitro Studien werden Synaptosomen aus Rattenhirnarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1ml) in einem Glas-Homogenisierer mit Teflonstößel homogenisiert, indem fünf volle Auf und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen (P₂-Fraktion), die in 0,32 M Glucose (0,5 mU 100 mg des ursprünglichen Gewichts) noch einmal suspendiert werden.
Der jeweilige Uptake wird in einer 96-well Mikrotiterplatte gemessen. Das Volumen beträgt 250 µl und die Inkubation erfolgt bei Raumtemperatur (ca. 20-25 °C) unter O₂ Atmosphäre.

Die Inkubationszeit beträgt 7,5 Minuten für [³H]-NA und 5 Minuten für [³H]-5-HT. Anschließend werden die 96 Proben durch eine Unifilter GF/B^{®} Mikrotiterplatte (Packard) filtriert und mit 200 ml inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal^{®} (Packard) verschlossen und mit 35 µl Szintilationsflüssigkeit pro Well (Ultima Gold^{®}, Packard) versetzt. Nach dem Verschließen mit einem top seal^{®} (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193, 265-275, 1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden.

Die entsprechenden Literaturbeschreibungen werden hiermit jeweils als Referenz eingeführt und gelten als Teil der vorliegenden Offenbarung.

Folgende Kenndaten wurden für den NA- bzw. 5-HT-Transporter ermittelt:
NA-Uptake : Km = 0,32 ± 0,11 µM
5HT-Uptake : Km = 0,084 ± 0,011 µM

### b) Methode zur Bestimmung der Affinität zur Batrachotoxin-(BTX)-Bindungsstelle des Natriumkanals

Die Bindungsstelle 2 des Natriumkanals ist die sogenannte Batrachotoxin-(BTX) Bindungsstelle. Als Ligand wird [³H]-Batrachotoxinin A20 α-Benzoat (10 nM im Ansatz) eingesetzt. Die Ionenkanal-Partikel (Synaptosomen) werden aus dem Ratten Cerebrocortex angereichert, wie in der Veröffentlichung von Gray und Whittaker, 1962, J. Anat. 76, 79-88 beschrieben. Die entsprechende Beschreibung wird hiermit als Referenz eingeführt und gilt als Teil der vorliegenden Offenbarung. Als unspezifische Bindung ist die Radioaktivität definiert, die in Gegenwart von Veratridin (3 x 10⁻⁴ M im Ansatz) gemessen wird.

Die Assaybedingungen werden entsprechend der Veröffentlichung von Pauwels, Leysen und Laduron durchgeführt, wie in Eur. J. Pharmacol. 124, 291-298 beschrieben. Die entsprechende Beschreibung wird hiermit als Referenz eingeführt und gilt als Teil der vorliegenden Offenbarung.

Abweichend von dieser Vorschrift wird der Gesamtansatz auf 250 µl verkleinert, so daß der Assay auf 96-well Mikrotiterplatten durchgeführt werden kann. Die Inkubationszeit in diesen Mikrotiterplatten beträgt zwei Stunden bei Raumtemperatur (ca. 20-25°C).

Folgende Kenndaten wurden für den K_{D}-Wert der Bindungsstelle vermittelt:
K_{D}: 24,63 ± 1,56 nM

Im folgenden wird die Erfindung anhand von Beispielen erläutert. Diese Erläuterungen sind lediglich beispielhaft und schränken den allgemeinen Erfindungsgedanken nicht ein.

### Beispiele:

Die Ausbeuten der hergestellten Verbindungen wurden nicht optimiert.

Alle Temperaturen sind unkorrigiert.

Die Angabe "Ether" bedeutet Diethylether, "EtOAc" Ethylacetat, "DCM" Dichlormethan, "DMF" N,N-Dimethylformamid, "EtOH" Ethanol, "MeOH" Methanol Die Angabe "Äquivalente" bedeutet Stoffmengenäquivalente, "Smp." Schmelzpunkt bzw. Schmelzbereich, "RT" Raumtemperatur, d.h. ca 20 °C, "min" Minuten, "h" Stunden, "ges." gesättigt und aq. "wäßrig".

### Weitere Abkürzungen:

- Boc: tert-Butoxy-carbonyl
- BOP: Benzotriazol-1-yloxy-tris-dimethylaminophosphoniumhexafluorphosphat
- DMAP: 4,4-Dimethylaminopyridin

Die eingesetzten Chemikalien und Lösungsmittel wurden kommerziell bei den herkömmlichen Anbietern (Acros, Acocado, Aldrich, Bachem, Fluka, Lancaster, Maybridge, Merck, Sigma, TCi etc.) erworben oder nach üblichen, dem Fachmann geläufigen Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben. Die Analytik erfolgte über NMR und HPLC-MS.

### Synthese von 6,7-Dihydro-4H-isoxazolo[4,5-c]pyridin-3,5-dicarbonsäure-5-tert-butylester (F)

Die Synthese der Ausgangssubstanz 6,7-Dihydro-4H-isoxazolo[4,5-c]pyridin-3,5-dicarbonsäure-5-tert-butylester (F) zur Darstellung der erfindungsgemäßen substituierten 4,5,6,7- Tetrahydro-isoxazolo[4,5-c]pyridin-Verbindungen der allgemeinen Formel I ist in Schema 1. wiedergegeben.

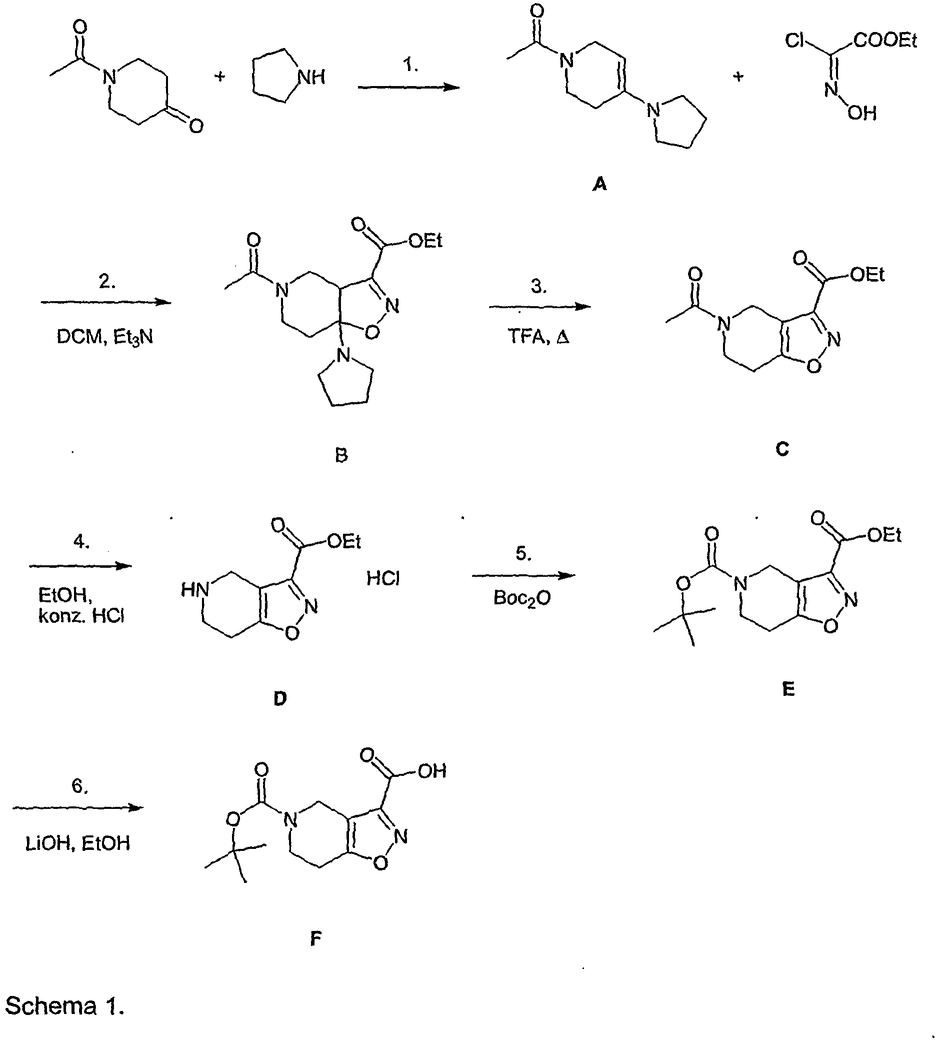

### Stufe 1.

### Synthese von 1-(4-Pyrrolidin-1-yl-3,6-dihydro-2H-pyridin-1-yl)-ethanon (A)

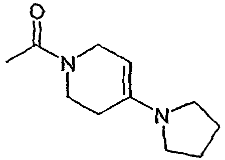

Ein Gemisch aus 4,95 mL (40 mmol) 1-Acetyl-4-piperidinon, 3,64 mL (44 mmol) Pyrrolidin, 15 mL Toluol und einer katalytischen Menge p-Toluolsulfonsäure wurde unter Rückfluß am Wasserabscheider 12 h erwärmt. Nach Entfernen des Lösungsmittels im Vakuum erhielt man 7,7 g (99 % der Theorie) des gewünschten Produktes 1-(4-Pyrrolidin-1-yl-3,6-dihydro-2H-pyridin-1-yl)-ethanon (A) in Form eines braun-roten Öls.

### Stufe 2.

### Synthese von 5-Acetyl-7a-pyrrolidin-1-yl-3a,4,5,6,7,7a-hexahydro-isoxazolo[4,5-c]pyridin-3-carbonsäureethylester (B)

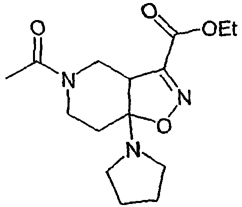

Zu einer Lösung von 16,3 g (84 mmol) 1-(4-Pyrrolidin-1-y(-3,6-dihydro-2H-pyridin-1-yl)-ethanon (A) in 150 mL DCM wurde eine Lösung von 17,9 g (0,118 mol) 2-Chlorhydroxyiminoessigsäureethylester in 20 mL DCM gegeben. Die Lösung erwärmte sich dabei und wurde rot. Nach Kühlen auf 0 °C (Eisbad) wurden unter Rühren langsam 16,4 g (0,118 mol) Triethylamin zugetropft. Das Gemisch wurde bei RT 12 h gerührt und dann zunächst mit 10%-iger (Gewichtsprozent) ZitronensäureLösung in Wasser und anschließend mit ges. aq. Kochsalzlösung gewaschen. Nach Entfernen des Lösungsmittels im Vakuum wurde der Rückstand säulenchromatographisch (SiO₂, EtOAc/EtOH 9:1) gereinigt. Es wurden 18,5 g (71 % der Theorie) des gewünschten Produkts 5-Acetyl-7a-pyrrolidin-1-yl-3a,4,5,6,7,7a-hexahydro-isoxazolo[4,5-c]pyridin-3-carbonsäureethylester (B) in Form eines braunen Öls erhalten.

### Stufe 3.

### Synthese von 5-Acetyl-4,5,6,7,-tetrahydro-isoxazolo[4,5-c]pyridin-3-carbonsäure-ethylester (C)

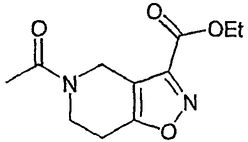

Ein Gemisch aus 18,0 g (58 mmol) 5-Acetyl-7a-pyrrolidin-1-yl-3a,4,5,6,7,7a-hexahydro-isoxazoto[4,5-c]pyridin-3-carbonsäureethylester (B) in 150 mL DCM und 6,47 mL (87 mmol) Tifluoressigsäure wurde 8 h zum Rückfluß erhitzt. Nach Zugabe von 100 mL Wasser wurde die organische Phase über MgSO₄ getrocknet und das Lösungsmittel im Vakuum entfernt. Das zurückbleibende braune Öl wurde säulenchromatographisch (SiO₂, EtOAc/EtOH 10: 1) gereinigt. Es wurden 12,5 g (90 % der Theorie) des gewünschten Produkts 5-Acetyl-4,5,6,7,-tetrahydro-isoxazolo[4,5-c]pyridin-3-carbonsäure-ethylester (C) in Form eines braungelben Öls erhalten.

### Stufe 4.

### Synthese von 4,5,6,7-Tetrahydro-isoxazolo[4,5-c]pyridin-3-carbonsäure-ethylester Hydrochlorid (D)

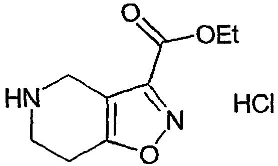

31,1 g (130 mmol) 5-Acetyl-4,5,6,7,-tetrahydro-isoxazolo[4,5-c]pyridin-3-carbonsäure-ethylester (C) wurden in 30 mL EtOH gelöst und bei RT mit 30 mL (260 mmol) 32%-iger (Gewichtsprozent) Salzsäure-Lösung in Wasser versetzt. Nach zweistündigem Erhitzen unter Rückfluß wurde die Lösung eingeengt und in 500 mL gekühlten EtOAc eingetragen (Eisbad). Der dabei entstehende Niederschlag wurde abgetrennt und im Vakuum getrocknet. Es wurden 8,13 g (27 % der Theorie) des gewünschten Produkts 4,5,6,7-Tetrahydro-isoxazolo[4,5-c]pyridin-3-carbonsäure-ethylester Hydrochlorids (D) erhalten.
Smp.:74°C

### Stufe 5.

### Synthese von 6,7-Dihydro-4H-isoxazolo[4,5-c]pyridin-3,5-dicarbonsäure-3-ethylester-5-tert-butylester (E)

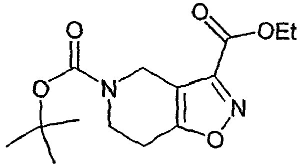

300 mg (1,21 mmol) 4,5,6,7-Tetrahydro-isoxazolo[4,5-c]pyridin-3-carbonsäure-ethylester Hydrochlorid (D) wurden in 20 mL einer Dioxan/Wasser-Mischung (2:1) gelöst und mit 505 µl (3,63 mmol) Triethylamin sowie anschließend unter Eiskühlung mit 290 mg (1,33 mmol) Di-tert-butyl-dicarbonat [(Boc)₂O] versetzt. Das Gemisch wurde bei RT 12 h gerührt und das Lösungsmittel im Vakuum entfernt. Der Rückstand wurde säulenchromatographisch (SiO₂, EtOAc/EtOH 20:1) gereinigt. Es wurden 90 mg (25 % der Theorie) des gewünschten Produkts 6,7-Dihydro-4H-isoxazolo[4,5-c]pyridin-3,5-dicarbonsäure-3-ethylester-5-tert-butylester (E) in Form eines farblosen Öls erhalten.

### Stufe 6.

### Synthese von 6,7-Dihydro-4H-isoxazolo[4,5-c]pyridin-3,5-dicarbonsäure-5-tert-butylester (F)

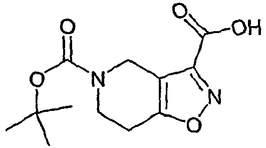

Ein Gemisch aus 7,31 g (25 mmol) 6,7-Dihydro-4H-isoxazolo[4,5-c]pyridin-3,5-dicarbonsäure-3-ethylester-5-tert-butylester (E), 43 g (30 mmol) Lithiumhydroxid und 75 mL EtOH wurde 12 h bei RT gerührt. Das Lösungsmittel wurde im Vakuum entfernt und der Rückstand in einer Mischung aus Wasser/Zitronensäure (pH = 4) und Ether aufgenommen. Die organische Phase wurde abgetrennt, über Magnesiumsulfat getrocknet und das Lösungsmittel wurde im Vakuum entfernt. Es wurden 6,65 g (99 % der Theorie) des gewünschten Produkts 6,7-Dihydro-4H-isoxazolo[4,5-c]pyridin-3,5-dicarbonsäure-5-tert-butylester (F) in Form eines gelben Öls erhalten, welches bei RT kristallisierte.

### Allgemeine Vorschrift 1.

### Umsetzung von 6,7-Dihydro-4H-isoxazolo[4,5-c]pyridin-3,5-dicarbonsäure-5-tert-butylester (F) mit primären oder sekundären Aminen

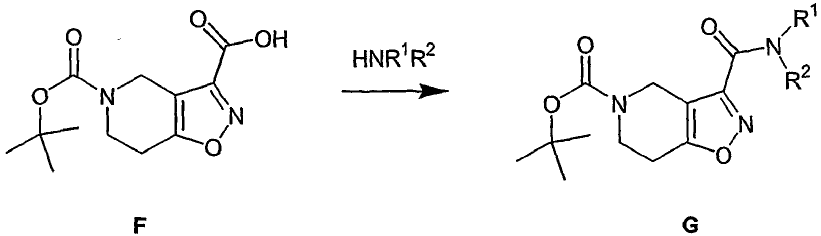

Eine Mischung aus 1.0 Äquivalenten der Verbindung 6,7-Dihydro-4H-isoxazolo[4,5-c]pyridin-3,5-dicarbonsäure-5-tert-butylester (F), 1.0 Äquivalenten des jeweiligen primären oder sekundären Amins HNR¹R², 2.7 Äquivalenten N-Methylmorpholin und 1.8 Äquivalenten BOP in DMF wurde 12 h bei RT gerührt. Nach Entfernen des Lösungsmittels im Vakuum wurde der Rückstand mit Wasser und EtOAc versetzt. Die organische Phase wurde mit Wasser, 10%-iger (Gewichtsprozent) ZitronensäureLösung in Wasser, ges. aq. Na₂CO₃- und ges. aq. NaCl-Lösung gewaschen, über MgSO₄ getrocknet und das Lösungsmittel wurde im Vakuum entfernt. Nach säulenchromatographischer Reinigung an Kieselgel (Ether/Hexan 10:1) wurde das gewünschte Kupplungsprodukt der allgemeinen Formel G erhalten.

### Allgemeine Vorschrift 2.

### Abspaltung der Boc-Gruppe aus Verbindungen der allgemeinen Formel G

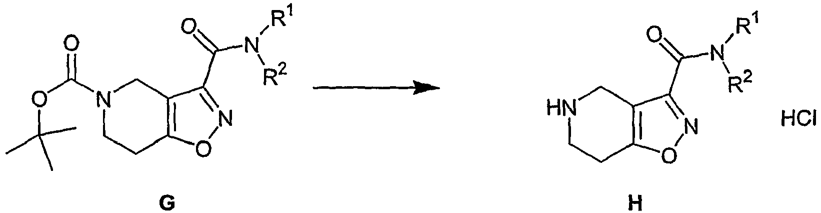

Verbindung G wurde in einer 4 M Salzsäure-Lösung in MeOH gelöst und bei RT bis zur vollständigen Umsetzung der Ausgangsverbindung gerührt. Das Lösungsmittel wurde im Vakuum bis zu einer Trübung der Lösung entfernt und das Reaktionsgemisch wurde über Nacht bei 4 °C stehen gelassen. Der Niederschlag wurde abfiltriert, mit wenig Ether gewaschen und im Vakuum getrocknet, um das gewünschte Produkt der allgemeinen Formel H, ggf. in Form des entsprechenden Hydrochlorids, zu erhalten.

### Allgemeine Vorschrift 3.

### Umsetzung von Verbindungen der allgemeinen Formel H mit Carbonsäurehalogeniden oder Sulfonsäurehalogeniden

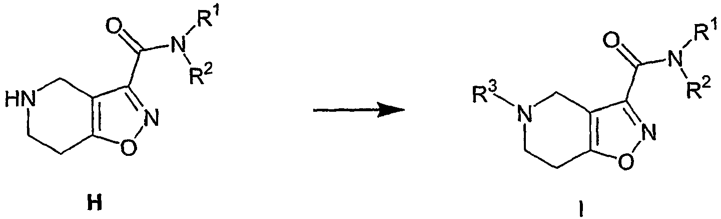

Zu einer Lösung aus dem jeweiligen Säurehalogenid (1.5 Äquivalente), Triethylamin (2.0 Äquivalente) und einer katalytischen Menge DMAP in DCM wurde bei 0 °C die Verbindung der allgemeinen Formel H (1.0 Äquivalente) gegeben. Die Reaktionslösung wurde auf RT erwärmt und über Nacht gerührt. Nach Zugabe von 10%-iger (Gewichtsprozent) aq. NH₄Cl-Lösung wurde die organische Phase abgetrennt und über MgSO₄ getrocknet. Das Lösungsmittel wurde im Vakuum entfernt und der Rückstand säulenchromatographisch an Kieselgel mit EtOAc/Hexan-Gemischen als Eluent gereinigt, um das gewünschte Produkt der allgemeinen Formel I zu erhalten.

### Beispiel 66. 5-Pent-4-enoyl-4,5,6,7-tetrahydro-isoxazolo[4,5-c]pyridin-3-carbonsäure cyclopropylmethyl-amid

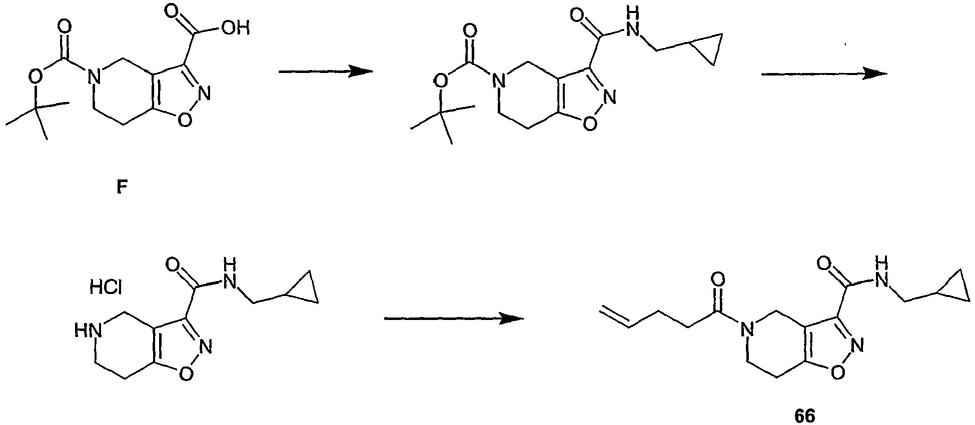

### Synthese von 3-(Cyclopropylmethyl-carbamoyl)-6,7-dihydro-4H-isoxazolo[4,5-c]pyridin-5-carbonsäure-tert-butylester

Analog zu der allgemeinen Vorschrift 1 wurden 280 mg (1.0 mmol) an 6,7-Dihydro-4H-isoxazolo[4,5-c]pyridin-3,5-dicarbonsäure-5-tert-butylester mit 90 µL Cyclopropylmethylamin zu 260 mg (78 % der Theorie) 3-(Cyclopropylmethyl-carbamoyl)-6,7-dihydro-4H-isoxazolo[4,5-c]pyridin-5-carbonsäure-tert-butylester umgesetzt, der in Form eines gelben Öls erhalten wurde.

### Synthese von 4,5,6,7-Tetrahydro-isoxazolo[4,5-c]pyridin-3-carbonsäure-cyclopropylmethylamid Hydrochlorid

Analog zu der allgemeinen Vorschrift 2 wurden 260 mg 3-(Cyclopropylmethyl-carbamoyl)-6,7-dihydro-4H-isoxazolo[4,5-c]pyridin-5-carbonsäure-tert-butylester zu 160 mg (77 % der Theorie) 4,5,6,7-Tetrahydro-isoxazolo[4,5-c]pyridin-3-carbonsäure-cyclopropylmethylamid Hydrochlorid umgesetzt, das als ein farbloser Feststoff erhalten wurde.
Smp. 227,6 °C

### Synthese von 5-Pent-4-enoyl-4,5,6,7-tetrahydro-isoxazolo[4,5-c]pyridin-3-carbonsäure cyclopropylmethyl-amid

Aus 4,5,6,7-Tetrahydro-isoxazolo[4,5-c]pyridin-3-carbonsäure-cyclopropylmethylamid Hydrochlorid wurde die entsprechende Base freigesetzt. Analog zu der allgemeinen Vorschrift 3 wurden 170 mg (0.42 mmol) 4,5,6,7-Tetrahydro-isoxazolo[4,5-c]pyridin-3-carbonsäure-cyclopropylmethylamid mit 89 µL (0.89 mmol) 4-Pentenoylchlorid zu 150 mg (84 % der Theorie) 5-Pent-4-enoyl-4,5,6,7-tetrahydro-isoxazolo[4,5-c]pyridin-3-carbonsäure-cyclopropylmethyl-amid (66) umgesetzt, das in Form eines braunen Öls erhalten wurde.

### Beispiel 86. 1-(4-{4-[5-(Toluol-4-sulfonyl)-4,5,6,7-tetrahydro-isoxazolo[4,5-c]pyridin-3-carbonyl]-piperazin-1-yl}-phenyl)-ethanon

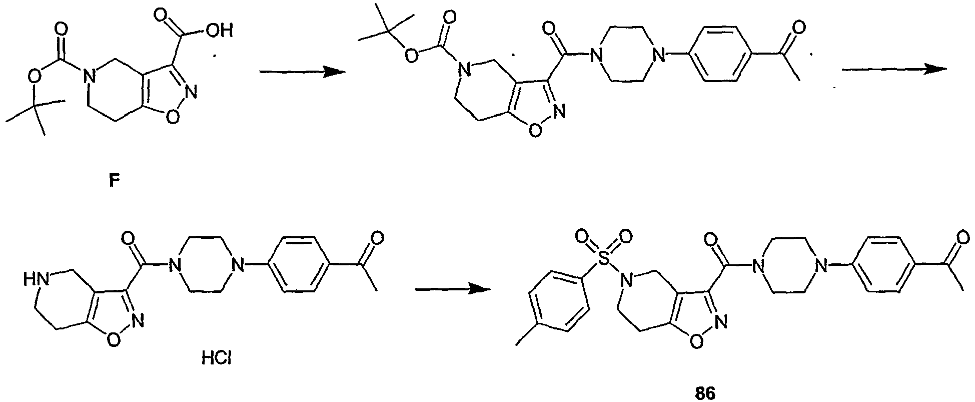

### Synthese von 3-[4-(4-Acetyl-phenyl)-piperazin-1-carbonyl]-6,7-dihydro-4H-isoxazolo[4,5-c]pyridin-5-carbonsäure-tert-butylester

Analog zu der allgemeinen Vorschrift 1 wurden 1.1 g (4.1 mmol) 6,7-Dihydro-4H-isoxazolo[4,5-c]pyridin-3,5-dicarbonsäure-5-tert-butylester mit 838 mg (4.1 mmol) 4-Piperazinactophenon zu 790 mg (42 % der Theorie) 3-[4-(4-Acetyl-phenyl)-piperazin-1-carbonyl]-6,7-dihydro-4H-isoxazolo[4,5-c]pyridin-5-carbonsäure-tert-butylester umgesetzt, der in Form eines gelb-orangen Öls erhalten wurde.

### Synthese von 1-[4-[4-(4,5,6,7-Tetrahydro-isoxazolo[4,5-c]pyridin-3-carbonyl)-piperazin-1-yl]-phenyl]-ethanon Hydrochlorid

Analog zu der allgemeinen Vorschrift 2 wurden 160 mg (0.35 mmol) 3-[4-(4-Acetyl-phenyl)-piperazin-1-carbonyl]-6,7-dihydro-4H-isoxazolo[4,5-c]pyridin-5-carbonsäure-tert-butylester zu 90 mg (63 % der Theorie) 1-[4-[4-(4,5,6,7-Tetrahydro-isoxazolo[4,5-c]pyridin-3-carbonyl)-piperazin-1-yl]-phenyl]-ethanon Hydrochlorid umgesetzt, das in Form eines weißen Feststoffs erhalten wurde.
Smp. 189,5 °C

### Synthese von 1-(4-{4-[5-(Toluol-4-sulfonyl)-4,5,6,7-tetrahydro-isoxazolo[4,5-c]pyridin-3-carbonyl]-piperazin-1-yl}-phenyl)-ethanon

Aus 1-[4-[4-(4,5,6,7-Tetrahydro-isoxazolo[4,5-c]pyridin-3-carbonyl)-piperazin-1-yl]-phenyl]-ethanon Hydrochlorid wurde die entsprechend Base freigesetzt. p-Toluolsulfonsäurechlorid wurde mittels HPLC an Multospher 120 RP18 AQ 5 µm (CS-Chromatographie, Langerwehe, Deutschland) mit dem Laufmittel MeOH/Wasser 7:3 mit 0.1 Volumen-% Triethylamin gereinigt.
Analog zu der allgemeinen Vorschrift wurden 170 mg (0.42 mmol) 1-[4-[4-(4,5,6,7-Tetrahydro-isoxazolo[4,5-c]pyridin-3-carbonyl)-piperazin-1-yl]-phenyl]-ethanon mit 120 mg (0.63 mmol) p-Toluolsulfonsäurechlorid zu 60 mg (28 % der Theorie) 1-(4-{4-[5-(Toluol-4-sulfonyl)-4,5,6,7-tetrahydro-isoxazolo[4,5-c]pyridin-3-carbonyl]-piperazin-1-yl}-phenyl)-ethanon (86) umgesetzt, das in Form eines farblosen Feststoffs erhalten wurde.
Smp. 187,8 °C

### Allgemeine Vorschrift 4.

### Umsetzung von Verbindungen der allgemeinen Formel H mit Isocyanaten bzw. Thioisocyanaten

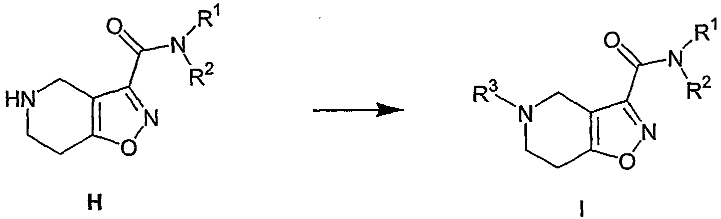

Zu einer Lösung der Verbindung der allgemeinen Formel H (100 µmol in 1 mL Toluol) in Toulol (3 mL) wurde bei RT das jeweilige Isocyanat bzw. Thioisocyanat (100 µmol in 1 mL Toluol, 1.0 Äquivalent) gegeben. Die Reaktionsmischung wurde für mindestens 8 Stunden bei 50 °C gerührt. Das Lösungsmittel wurde im Vakuum entfernt und der Rückstand mittels präparativer HPLC gereinigt, um das gewünschte Produkt der allgemeinen Formel I zu erhalten.

### Allgemeine Vorschrift 5.

### Umsetzung von Verbindungen der allgemeinen Formel H mit Aldehyden

Zu einer Lösung der Verbindung der allgemeinen Formel H (120 µmol in 0.5 mL MeOH) wurde unter Rühren bei RT der jeweilige Aldehyd (120 µmol in 0.5 mL MeOH) und anschließend Boran-Pyridin-Komplex (BH₃•C₅H₅N, 100 µmol in 0.5 mL MeOH) gegeben. Die Reaktionsmischung wurde wenigstens 16 Stunden bei 64 °C gerührt und anschließend unter Rühren mit 5%-iger (Gewichtsprozent) Salzsäure-Lösung in Wasser (0.5 mL) versetzt. Zu der Reaktionsmischung wurde 10%-ige (Gewichtsprozent) Natriumhydroxid-Lösung in Wasser (1 mL) gegeben und die Mischung wurde dreimal mit DCM (jeweils 2 mL) extrahiert. Die vereinigten organischen Phasen wurden über MgSO₄-Kartuschen getrocknet, das Lösungsmittel im Vakuum entfernt und der Rückstand mittels präparativer HPLC gereinigt, um das gewünschte Produkt der allgemeinen Formel I zu erhalten.

Die zuvor nicht detailliert beschriebene Herstellung der übrigen Verbindungen gemäß den nachstehend angegebenen Beispielen erfolgte ebenfalls analog den vorstehend angegebenen Herstellungsvorschriften, wobei dem Fachmann aufgrund dieser Vorschriften die jeweils eingesetzten Edukte bekannt sind.

| **Beispiel** | **Name** |
|---|---|
| 1 | 6,7-Dihydro-4H-isoxazolo[4,5-c]pyridin-3,5-dicarbonsäure 3-[(2-methoxy-ethyl)-amid] 5-[(3-methoxy-phenyl)-amid] |
| 2 | 6,7-Dihydro-4H-isoxazolo[4,5-c]pyridin-3,5-dicarbonsäure 3-cyclopentylamid 5-(4-fluor-benzylamid) |
| 3 | 6,7-Dihydro-4H-isoxazolo[4,5-c]pyridin-3,5-dicarbonsäure 3-phenylamid 5-[(4-trifluormethoxy-phenyl)-amid] |
| 4 | 6,7-Dihydro-4H-isoxazolo[4,5-c]pyridin-3,5-dicarbonsäure 5-[(4-methyl-3-nitro-phenyl)-amid] 3-(phenethyl-amid) |
| 5 | 6,7-Dihydro-4H-isoxazofo[4,5-c]pyridin-3,5-dicarbonsäure 3-[(2-methoxy-ethyl)-amid] 5-[(4-methyl-3-nitro-phenyl)-amid] |
| 6 | [3-{[5-(2,5-Difluor-phenylcarbamoyl)-4,5,6,7-tetrahydro-isoxazolo[4,5-c]pyridin-3-carbonyl]-amino}-propionsäure ethyl ester |
| 7 | 6,7-Dihydro-4H-isoxazolo[4,5-c]pyridin-3,5-dicarbonsäure 5-[(4-butoxy-phenyl)-amid] 3-[(2-methoxy-ethyl)-amid] |
| 8 | 6,7-Dihydro-4H-isoxazolo[4,5-c]pyridin-3,5-dicarbonsäure 5-[(3-fluor-phenyl)-amid] 3-(phenethyl-amid) |
| 9 | 6,7-Dihydro-4H-isoxazolo[4,5-c]pyridin-3,5-dicarbonsäure 5-[(4-methyl-3-nitro-phenyl)-amid] 3-[(thiophen-2-ylmethyl)-amid] |
| 10 | 6,7-Dihydro-4H-isoxazolo[4,5-c]pyridin-3,5-dicarbonsäure 3-benzylamid 5-(phenethyl-amid) |
| 11 | 6,7-Dihydro-4H-isoxazolo[4,5-c]pyridin-3,5-dicarbonsäure 3-[(2-ethylsulfanyl-ethyl)-amid] 5-[(3-methoxy-phenyl)-amid] |
| 12 | 6,7-Dihydro-4H-isoxazolo[4,5-c]pyridin-3,5-dicarbonsäure 5-[(3-cyano-phenyl)-amid] 3-[(thiophen-2-ylmethyl)-amid] |
| 13 | 6,7-Dihydro-4H-isoxazolo[4,5-c]pyridin-3,5-dicarbonsäure 5-[(4-ethoxy-phenyl)-amid] 3-phenylamid |
| 14 | 6,7-Dihydro-4H-isoxazolo[4,5-c]pyridin-3,5-dicarbonsäure 3-(4-fluor-benzylamid) 5-[(4-methyl-3-nitro-phenyl)-amid] |
| 15 | 6,7-Dihydro-4H-isoxazolo[4,5-c]pyridin-3,5-dicarbonsäure 5-[(3-acetyl-phenyl)-amid] 3-[(5-methyl-furan-2-ylmethyl)-amid] |
| 16 | 6,7-Dihydro-4H-isoxazolo[4,5-c]pyridin-3,5-dicarbonsäure 5-[(4-methyl-3-nitro-phenyl)-amid] 3-prop-2-ynylamid |
| 17 | 6,7-Dihydro-4H-isoxazolo[4,5-c]pyridin-3,5-dicarbonsäure 3-benzylamid 5-phenylamid |
| 18 | 6,7-Dihydro-4H-isoxazolo[4,5-c]pyridin-3,5-dicarbonsäure 5-[(1-naphthalen-1-yl-ethyl)-amid] 3-(phenethyl-amid) |
| 19 | 6,7-Dihydro-4H-isoxazolo[4,5-c]pyridin-3,5-dicarbonsäure 5-[(3-fluorphenyl)-amid] 3-prop-2-ynylamid |
| 20 | 3-{[5-(2,5-Dimethoxy-phenylcarbamoyl)-4,5,6,7-tetrahydro-isoxazolo[4,5-c]pyridin-3-carbonyl]-amino}-propionsäure ethyl ester |
| 21 | 6,7-Dihydro-4H-isoxazolo[4,5-c]pyridin-3,5-dicarbonsäure 5-[(4-ethoxy-phenyl)-amid] 3-(4-fluor-benzylamid) |
| 22 | 6,7-Dihydro-4H-isoxazolo[4,5-c]pyridin-3,5-dicarbonsäure 5-[(5-chlor-2-methoxy-phenyl)-amid] 3-(phenethyl-amid) |
| 23 | 3-({3-[(5-Methyl-furan-2-ylmethyl)-carbamoyl]-6,7-dihydro-4H-isoxazolo[4,5-c]pyridin-5-carbonyl}-amino)-benzoesäure ethyl ester |
| 24 | 6,7-Dihydro-4H-isoxazolo[4,5-c]pyridin-3,5-dicarbonsäure 5-[(5-chlor-2-methoxy-phenyl)-amid] 3-(isobutyl-amid) |
| 25 | [2-Oxo-2-(3-prop-2-ynylcarbamoyl-6,7-dihydro-4H-isoxazolo[4,5-c]pyridin-5-yl)-ethoxy]-essigsäure |
| 26 | 3-Ethyl-5-[3-(2-methoxy-ethylcarbamoyl)-6,7-dihydro-4H-isoxazolo[4,5-c]pyridin-5-yl]-3-methyl-5-oxo-pentansäure |
| 27 | (1-Benzyloxymethyl-2-oxo-2-{3-[(pyridin-3-ylmethyl)-carbamoyl]-6,7-dihydro-4H-isoxazolo[4,5-c]pyridin-5-yl}-ethyl)-carbaminsäure tert-butyl ester |
| 28 | {1-[2-Oxo-2-(3-phenylcarbamoyl-6,7-dihydro-4H-isoxazolo[4,5-c]pyridin-5-yl)-ethyl]-cyclopentyl}-essigsäure |
| 29 | 3-Ethyl-3-methyl-5-oxo-5-(3-phenylcarbamoyl-6,7-dihydro-4H-isoxazolo[4,5-c]pyridin-5-yl)-pentansäure |
| 30 | (2-Oxo-2-{3-[(thiophen-2-ylmethyl)-carbamoyl]-6,7-dihydro-4H-isoxazolo[4,5-c]pyridin-5-yl}-ethoxy)-essigsäure |
| 31 | 3,3-Dimethyl-4-{3-[(5-methyl-furan-2-ylmethyl)-carbamoyl]-6,7-dihydro-4H-isoxazolo[4,5-c]pyridin-5-yl}-4-oxo-butansäure |
| 32 | 5-(4-Trifluormethoxy-benzolsulfonyl)-4,5,6,7-tetrahydro-isoxazolo[4,5-c]pyridin-3-carbonsäure benzyl-phenethyl-amid |
| 33 | 5-Benzolsulfonyl-4,5,6,7-tetrahydro-isoxazolo[4,5-c]pyridin-3-carbonsäure (1-naphthalen-2-ylmethyl-pyrrolidin-3-yl)-amid |
| 34 | 5-(4-Fluor-benzolsulfonyl)-4,5,6,7-tetrahydro-isoxazolo[4,5-c]pyridin-3-yl]-(4-thieno[2,3-d]pyrimidin-4-yl-piperazin-1-yl)-methanon |
| 35 | 5-(4-Fluor-benzolsulfonyl)-4,5,6,7-tetrahydro-isoxazolo[4,5-c]pyridin-3-carbonsäure (3-phenyl-propyl)-amid |
| 36 | 5-(3-Phenyl-acryloyl)-4,5,6,7-tetrahydro-isoxazolo[4,5-c]pyridin-3-carbonsäure (2-dimethylamino-ethyl)-amid |
| 37 | 5-(2-Phenoxy-acetyl)-4,5,6,7-tetrahydro-isoxazolo[4,5-c]pyridin-3-carbonsäure (2-dimethylamino-ethyl)-amid |
| 38 | 5-(4-Fluor-benzolsulfonyl)-4,5,6,7-tetrahydro-isoxazolo[4,5-c]pyridin-3-carbonsäure (1-methyl-3-phenyl-propyl)-amid |
| 39 | 5-(4-Fluor-benzolsulfonyl)-4,5,6,7-tetrahydro-isoxazolo[4,5-c]pyridin-3-carbonsäure [1-(4-trifluormethyl-benzyl)-pyrrolidin-3-yl]-amid |
| 40 | 5-(2,5-Dichlor-benzolsulfonyl)-4,5,6,7-tetrahydro-isoxazolo[4,5-c]pyridin-3-carbonsäure (2-benzyloxy-cyclopentyl)-amid |
| 41 | 5-(5-tert-Butyl-2-methyl-2H-pyrazole-3-carbonyl)-4,5,6,7-tetrahydro-isoxazolo[4,5-c]pyridin-3-carbonsäure (2-dimethylamino-ethyl)-amid |
| 42 | [5-(4-Fluor-benzolsulfonyl)-4,5,6,7-tetrahydro-isoxazolo[4,5-c]pyridin-3-yl]-[4-(4-fluor-phenyl)-3,6-dihydro-2H-pyridin-1-yl]-methanon |
| 43 | 4-{[5-(4-Fluor-benzolsulfonyl)-4,5,6,7-tetrahydro-isoxazolo[4,5-c]pyridin-3-carbonyl]-amino}-piperidin-1-carbonsäure ethyl ester |
| 44 | 5-(3-Phenyl-acryloyl)-4,5,6,7-tetrahydro-isoxazolo[4,5-c]pyridin-3-carbonsäure cyclopentylamid |
| 45 | 5-(4-Acetylamino-benzoyl)-4,5,6,7-tetrahydro-isoxazolo[4,5-c]pyridin-3-carbonsäure cyclopropylmethyl-amid |
| 46 | 5-(5-Methyl-2-phenyl-2H-[1,2,3]triazol-4-carbonyl)-4,5,6,7-tetrahydro-isoxazolo[4,5-c]pyridin-3-carbonsäure (pyridin-3-ylmethyl)-amid |
| 47 | 5-Benzolsulfonyl-4,5,6,7-tetrahydro-isoxazolo[4,5-c]pyridin-3-carbonsäure 3,4-dimethoxy-benzylamid |
| 48 | 2-(3,4-Difluor-phenyl)-1-{4-[5-(4-fluor-benzolsulfonyl)-4,5,6,7-tetrahydro-isoxazolo[4,5-c]pyridin-3-carbonyl]-piperazin-1-yl}-ethanon |
| 49 | 3-(Morpholin-4-carbonyl)-6,7-dihydro-4H-isoxazolo[4,5-c]pyridin-5-sulfonsäure dimethylamid |
| 50 | 5-(Furan-2-carbonyl)-4,5,6,7-tetrahydro-isoxazolo[4,5-c]pyridin-3-carbonsäure (thiophen-2-ylmethyl)-amid |
| 51 | 5-(4,7,7-Trimethyl-3-oxo-2-oxa-bicyclo[2.2.1]heptan-1-carbonyl)-4,5,6,7-tetrahydro-isoxazolo[4,5-c]pyridin-3-carbonsäure (2-pyrrolidin-1-yl-ethyl)-amid |
| 52 | 5-(2,5-Dichlor-benzolsulfonyl)-4,5,6,7-tetrahydro-isoxazolo[4,5-c]pyridin-3-carbonsäure [2-(2-chlor-phenyl)-ethyl]-amid |
| 53 | 5-(4-Methoxy-benzolsulfonyl)-4,5,6,7-tetrahydro-isoxazolo[4,5-c]pyridin-3-carbonsäure p-tolyl-amid |
| 54 | 5-(2-Phenoxy-acetyl)-4,5,6,7-tetrahydro-isoxazolo[4,5-c]pyridin-3-carbonsäure 4-fluor-benzylamid |
| 55 | 1-{4-[5-(4-Methoxy-benzolsulfonyl)-4,5,6,7-tetrahydro-isoxazolo[4,5-c]pyridin-3-carbonyl]-piperazin-1-yl}-ethanon |
| 56 | 5-(Toluol-4-sulfonyl)-4,5,6,7-tetrahydro-isoxazolo[4,5-c]pyridin-3-carbonsäure 4-sulfamoyl-benzylamid |
| 57 | 5-(Thiophen-2-sulfonyl)-4,5,6,7-tetrahydro-isoxazolo[4,5-c]pyridin-3-carbonsäure (2-morpholin-4-yl-ethyl)-amid |
| 58 | 5-(4-Methoxy-benzoyl)-4,5,6,7-tetrahydro-isoxazolo[4,5-c]pyridin-3-carbonsäure (2-dimethylamino-ethyl)-amid |
| 59 | 5-(2,5-Dichlor-benzolsulfonyl)-4,5,6,7-tetrahydro-isoxazolo[4,5-c]pyridin-3-carbonsäure benzyl-phenethyl-amid |
| 60 | 5-Dimethylsulfamoyl-4,5,6,7-tetrahydro-isoxazolo[4,5-c]pyridin-3-carbonsäure (2,6,6-trimethyl-bicyclo[3.1.1]hept-3-yl)-amid |
| 61 | 5-Benzolsulfonyl-4,5,6,7-tetrahydro-isoxazolo[4,5-c]pyridin-3-carbonsäure indan-1-ylamid |
| 62 | 5-(4-Methoxy-benzolsulfonyl)-4,5,6,7-tetrahydro-isoxazolo[4,5-c]pyridin-3-carbonsäure (3-methoxypropyl)-amid |
| 63 | 5-(4,5-Dichlor-thiophen-2-sulfonyl)-4,5,6,7-tetrahydro-isoxazolo[4,5-c]pyridin-3-carbonsäure (2-morpholin-4-yl-ethyl)-amid |
| 64 | 5-(2-Benzyloxy-acetyl)-4,5,6,7-tetrahydro-isoxazolo[4,5-c]pyridin-3-carbonsäure (2-pyrrolidin-1-yl-ethyl)-amid |
| 65 | [5-(2,5-Dichlor-benzolsulfonyl)-4,5,6,7-tetrahydro-isoxazolo[4,5-c]pyridin-3-yl]-[4-(2,4-dimethyl-phenyl)-piperazin-1-yl]-methanon |
| 67 | 3-Methyl-2-{[5-(3-trifluormethyl-benzolsulfonyl)-4,5,6,7-tetrahydro-isoxazolo[4,5-c]pyridin-3-carbonyl]-amino}-butansäure tert-butyl ester |
| 68 | 5-(3-Trifluormethyl-benzolsulfonyl)-4,5,6,7-tetrahydro-isoxazolo[4,5-c]pyridin-3-carbonsäure benzyl-phenethyl-amid |
| 69 | 5-(3-Trifluormethyl-benzolsulfonyl)-4,5,6,7-tetrahydro-isoxazolo[4,5-c]pyridin-3-carbonsäure 2,4-dichlor-benzylamid |
| 70 | 5-Dimethylsulfamoyl-4,5,6,7-tetrahydro-isoxazolo[4,5-c]pyridin-3-carbonsäure (2-cyano-ethyl)-pyridin-3-ylmethyl-amid |
| 71 | 5-(2-Cyclopentyl-acetyl)-4,5,6,7-tetrahydro-isoxazolo[4,5-c]pyridin-3-carbonsäure (2-pyrrolidin-1-yl-ethyl)-amid |
| 72 | 5-(5-Methyl-2-phenyl-2H-[1,2,3]triazol-4-carbonyl)-4,5,6,7-tetrahydro-isoxazolo[4,5-c]pyridin-3-carbonsäure 4-fluor-benzylamid |
| 73 | 5-(3-Trifluormethyl-benzolsulfonyl)-4,5,6,7-tetrahydro-isoxazolo[4,5-c]pyridin-3-carbonsäure (2-ethyl-hexyl)-amid |
| 74 | 5-(2,3-Difluor-4-methyl-benzoyl)-4,5,6,7-tetrahydro-isoxazolo[4,5-c]pyridin-3-carbonsäure (2-dimethylamino-ethyl)-amid |
| 75 | 5-(4-Trifluormethoxy-benzolsulfonyl)-4,5,6,7-tetrahydro-isoxazolo[4,5-c]pyridin-3-carbonsäure 2,3-dichlor-benzylamid |
| 76 | 5-Dimethylsulfamoyl-4,5,6,7-tetrahydro-isoxazolo[4,5-c]pyridin-3-carbonsäure [2-(4-chlor-phenyl)-ethyl]-amid |
| 77 | 5-(6-Chlor-pyridin-3-carbonyl)-4,5,6,7-tetrahydro-isoxazolo[4,5-c]pyridin-3-carbonsäure (2-pyrrolidin-1-yl-ethyl)-amid |
| 78 | 5-[3-(4-Fluor-benzylcarbamoyl)-6,7-dihydro-4H-isoxazolo[4,5-c]pyridin-5-yl]-5-oxo-pentansäure methyl ester |
| 79 | 5-[2-(4-Methoxy-phenyl)-acetyl]-4,5,6,7-tetrahydro-isoxazolo[4,5-c]pyridin-3-carbonsäure benzylamid |
| 80 | 5-(4-Trifluormethoxy-benzolsulfonyl)-4,5,6,7-tetrahydro-isoxazolo[4,5-c]pyridin-3-carbonsäure [3-(methyl-phenyl-amino)-propyl]-amid |
| 81 | 5-[2-(4-Chlor-phenoxy)-acetyl]-4,5,6,7-tetrahydro-isoxazolo[4,5-c]pyridin-3-carbonsäure cyclopentylamid |
| 82 | [4-(2-Cyclohexyl-ethyl)-piperadin-1-yl]-[5-(toluol-4-sulfonyl)-4,5,6,7-tetrahydro-isoxazolo[4,5-c]pyridin-3-yl]-methanon |
| 83 | N-{1-[5-(2,5-Dichlor-benzolsulfonyl)-4,5,6,7-tetrahydro-isoxazolo[4,5-c]pyridin-3-carbonyl]-piperidin-4-ylmethyl}-2,2,2-trifluor-acetamid |
| 84 | 5-(4-Methoxy-benzolsulfonyl)-4,5,6,7-tetrahydro-isoxazolo[4,5-c]pyridin-3-carbonsäure [1-(4-methoxy-phenyl)-ethyl]-amid |
| 85 | 2-{[5-(Thiophen-2-sulfonyl)-4,5,6,7-tetrahydro-isoxazolo[4,5-c]pyridin-3-carbonyl]-amino}-propionsäure ethyl ester |
| 87 | 5-(3-Trifiuormethyl-benzolsulfonyl)-4,5,6,7-tetrahydro-isoxazolo[4,5-c]pyridin-3-carbonsäure (4-allyloxy-benzyl)-furan-2-ylmethyl-amid |
| 88 | 4-Carbamoyl-4-{[5-(4-methoxy-benzolsulfonyl)-4,5,6,7-tetrahydro-isoxazolo[4,5-c]pyridin-3-carbonyl]-amino}-butansäure tert-butyl ester |
| 89 | 5-Dimethylsulfamoyl-4,5,6,7-tetrahydro-isoxazolo[4,5-c]pyridin-3-carbonsäure furan-2-ylmethyl-(4-methylsulfanyl-benzyl)-amid |
| 90 | 5-(4-Bromo-3-methyl-benzoyl)-4,5,6,7-tetrahydro-isoxazolo[4,5-c]pyridin-3-carbonsäure (2-morpholin-4-yl-ethyl)-amid |
| 91 | 5-(Toluol-4-sulfonyl)-4,5,6,7-tetrahydro-isoxazolo[4,5-c]pyridin-3-carbonsäure [1-(2,6-dichlor-benzyl)-pyrrolidin-3-yl]-amid |
| 92 | 5-Benzolsulfonyl-4,5,6,7-tetrahydro-isoxazolo[4,5-c]pyridin-3-carbonsäure [2-(3,4-dichlor-phenyl)-ethyl]-amid |
| 93 | 5-(3-Difluormethylsulfanyl-benzoyl)-4,5,6,7-tetrahydro-isoxazolo[4,5-c]pyridin-3-carbonsäure (2-dimethy)amino-ethyl)-amid |
| 94 | 5-[2-(3-Chlor-phenoxy)-acetyl]-4,5,6,7-tetrahydro-isoxazolo[4,5-c]pyridin-3-carbonsäure (2-pyrrolidin-1-yl-ethyl)-amid |
| 95 | 5-(4-Methoxy-benzolsulfonyl)-4,5,6,7-tetrahydro-isoxazolo[4,5-c]pyridin-3-carbonsäure 2,3-dimethoxy-benzylamid |
| 96 | 5-(4-Trifluormethoxy-benzolsulfonyl)-4,5,6,7-tetrahydro-isoxazoio[4,5-c]pyridin-3-carbonsäure (1-methyl-3-phenyl-propyl)-amid |
| 97 | (5-Benzolsulfonyl-4,5,6,7-tetrahydro-isoxazolo[4,5-c]pyridin-3-yl)-[4-(3-phenyl-allyl)-piperazin-1-yl]-methanon |
| 98 | 3-{[5-(4-Methoxy-benzoyl)-4,5,6,7-tetrahydro-isoxazolo[4,5-c]pyridin-3-carbonyl]-amino}-propionsäure ethyl ester |
| 99 | 5-(4-Phenoxy-butyryl)-4,5,6,7-tetrahydro-isoxazolo[4,5-c]pyridin-3-carbonsäure (2-dimethylamino-ethyl)-amid |
| 100 | 5-(4-Trifluormethoxy-benzolsulfonyl)-4,5,6,7-tetrahydro-isoxazolo[4,5-c]pyridin-3-carbonsäure [2-(7-methyl-1H-indol-3-yl)-ethyl]-amid |
| 101 | [4-(3-Phenyl-allyl)-piperazin-1-yl]-[5-(thiophen-2-sulfonyl)-4,5,6,7-tetrahydro-isoxazolo[4,5-c]pyridin-3-yl]-methanon |
| 102 | 5-(4-Bromo-3-methyl-benzoyl)-4,5,6,7-tetrahydro-isoxazolo[4,5-c]pyridin-3-carbonsäure (2-pyrrolidin-1-yl-ethyl)-amid |
| 103 | 5-Benzolsulfonyl-4,5,6,7-tetrahydro-isoxazolo[4,5-c]pyridin-3-carbonsäure [1-(4-trifluormethyl-benzyl)-pyrrolidin-3-yl]-amid |
| 104 | 5-(Thiophen-2-sulfonyl)-4,5,6,7-tetrahydro-isoxazolo[4,5-c]pyridin-3-carbonsäure (2-phenyl-propyl)-amid |
| 105 | 6-(3-Chlor-4-fluor-benzoyl)-4,5,6,7-tetrahydro-isoxazolo[4,5-c]pyridin-3-carbonsäure phenethyl-amid |
| 106 | 5-Benzolsulfonyl-4,5,6,7-tetrahydro-isoxazolo[4,5-c]pyridin-3-carbonsäure [2-(4-methoxy-phenoxy)-ethyl]-amid |
| 107 | 5-Dimethylsulfamoyl,4,5,6,7-tetrahydro-isoxazolo[4,5-c]pyridin-3-carbonsäure 3-fluor-4-trifluormethyl-benzylamid |
| 108 | 5-(3-Methyl-benzoyl)-4,5,6,7-tetrahydro-isoxazolo[4,5-c]pyridin-3-carbonsäure prop-2-ynylamid |
| 109 | 5-(Toluol-4-sulfonyl)-4,5,6,7-tetrahydro-isoxazolo[4,5-c]pyridin-3-carbonsäure [2-(4-methoxy-phenoxy)-ethyl]-amid |
| 110 | [4-(2-Chlor-phenyl)-piperazin-1-yl]-[5-(2,5-dichlor-benzolsulfonyl)-4,5,6,7-tetrahydro-isoxazolo[4,5-c]pyridin-3-yl]-methanon |
| 111 | 3-{[5-(2,6-Difluor-3-methyl-benzoyl)-4,5,6,7-tetrahydro-isoxazolo[4,5-c]pyridin-3-carbonyl]-amino}-propionsäure ethyl ester |
| 112 | 5-(2,5-Dichlor-benzolsulfonyl)-4,5,6,7-tetrahydro-isoxazolo[4,5-c]pyridin-3-carbonsäure 2,4-difluor-benzylamid |
| 113 | 5-Benzolsulfonyl-4,5,6,7-tetrahydro-isoxazolo[4,5-c]pyridin-3-carbonsäure 3-fluor-4-trifluormethyl-benzylamid |
| 114 | 5-(4-Butoxy-benzolsulfonyl)-4,5,6,7-tetrahydro-isoxazolo[4,5-c]pyridin-3-carbonsäure isobutyl-amid |
| 115 | 5-(3-Fluor-4-methyl-benzoyl)-4,5,6,7-tetrahydro-isoxazolo[4,5-c]pyridin-3-carbonsäure (2-pyrrolidin-1-yl-ethyl)-amid |
| 116 | 5-(2-Chlor-5-trifluormethyl-benzoyl)-4,5,6,7-tetrahydro-isoxazolo[4,5-c]pyridin-3-carbonsäure isobutyl-amid |
| 117 | 5-(4-Trifluormethoxy-benzolsulfonyl)-4,5,6,7-tetrahydro-isoxazolo[4,5-c]pyridin-3-carbonsäure (1-benzyl-pyrrolidin-3-yl)-amid |
| 118 | 5-(3-Chlor-benzolsulfonyl)-4,5,6,7-tetrahydro-isoxazolo[4,5-c]pyridin-3-carbonsäure (2-pyrrolidin-1-yl-ethyl)-amid |
| 119 | 5-(3-Trifluormethoxy-benzoyl)-4,5,6,7-tetrahydro-isoxazolo[4,5-c]pyridin-3-carbonsäure (2-dimethylamino-ethyl)-amid |
| 120 | 2-{[5-(Toluol-4-sulfonyl)-4,5,6,7-tetrahydro-isoxazolo[4,5-c]pyridin-3-carbonyl]-amino}-propionsäure tert-butyl ester |
| 121 | 3-{[5-(4-Chlor-benzolsulfonyl)-4,5,6,7-tetrahydro-isoxazolo[4,5-c]pyridin-3-carbonyl]-amino}-propionsäure ethyl ester |
| 122 | 5-Dimethylsulfamoyl-4,5,6,7-tetrahydro-isoxazolo[4,5-c]pyridin-3-carbonsäure [2-(3,4-dichlor-phenyl)-ethyl]-amid |
| 123 | 5-(Thiophen-2-sulfonyl)-4,5,6,7-tetrahydro-isoxazolo[4,5-c]pyridin-3-carbonsäure cyclopropylmethyl-amid |
| 124 | 5-(Chinolin-6-carbonyl)-4,5,6,7-tetrahydro-isoxazolo[4,5-c]pyridin-3-carbonsäure 4-fluor-benzylamid |
| 125 | 3-{[5-(2-Naphthalen-2-yl-acetyl)-4,5,6-7-tetrahydro-isoxazolo[4,5-c]pyridin-3-carbonyl]-amino}-propionsäure ethyl ester |
| 126 | [2-(3-Isobutylcarbamoyl-6,7-dihydro-4H-isoxazolo[4,5-c]pyridin-5-yl)-2-oxo-1-phenyl-ethyl]-carbaminsäure benzyl ester |
| 127 | 5-(4-Methoxy-benzylthiocarbamoyl)-4,5,6,7-tetrahydro-isoxazolo[4,5-c]pyridin-3-carbonsäure cyclopentylamid |
| 128 | 5-Benzoylaminocarbothioyl-4,5,6,7-tetrahydro-isoxazolo[4,5-c]pyridin-3-carbonsäure (2-pyrrolidin-1-yl-ethyl)-amid |
| 129 | 5-(4-Chlor-benzylthiocarbamoyl)-4,5,6,7-tetrahydro-isoxazolo[4,6-c]pyridin-3-carbonsäure-cyclopropylmethyl-amid |
| 130 | 6-(2-Methoxy-ethylthiocarbarmoyl)-4,5,8,7-tetrahydro-isoxazolo[4,5-c]pyridin-3-carbonsäure phenethyl-amid |
| 131 | 5-Pentafluorphenylthiocarbamoyl-4,5,6,7-tetrahydro-isoxazolo[4,5-c]pyridin-3-carbonsäure phenethyl-amid |
| 132 | 5-(1-Phenyl-ethylthiocarbamoyl)-4,5,6,7-tetrahydro-isoxazolo[4,5-c]pyridln-3-carbonsäure phenethyl-amid |
| 133 | 5-Pentafluorphenylthiocarbamoyl-4,5,6,7-tetrahydro-isoxazolo[4,5-c]pyridin-3-carbonsäure (5-methyl-furan-2-ylmethyl)-amid |
| 134 | 3-{[5-(Cyclohexylmethyl-thiocarbamoyl)-4,5,6,7-tetrahydro-isoxazolo[4.5-c]pyridin-3-carbonyl]-amino}-propionsäure ethyl ester |
| 135 | 5-(1-Brom-naphthalen-2-ylmethyl)-4,5,6,7-tetrahydro-isoxazolo[4,5-c]pyridin-3-carbonsäurephenylamid |

### Pharmakologische Daten:

Die 5-HT-Uptake-Inhibierung und Noradrenalin-Uptake-Inhibierung der erfindungsgemäßen substituierten 4,5,6,7-Tetrahydro-isoxazolo[4,5-c]pyridin-Verbindungen der allgemeinen Formel I wurde wie vorstehend beschrieben bestimmt.

Die untersuchten 4,5,6,7-Tetrahydro-isoxazolo[4,5-c]pyridin-Verbindungen der allgemeinen Formel I zeigen eine ausgezeichnete Hemmung der 5-HT- und Noradrenalin-Wiederaufnahme.

Ebenfalls wurde die Affinität der erfindungsgemäßen substituierten 4,5,6,7-Tetrahydro-isoxazolo[4,5-c]pyridin-Verbindungen für die Batrachotoxin-(BTX)-Bindungsstelle wie vorstehend beschrieben bestimmt.

Die erfindungsgemäßen Verbindungen zeigen auch eine ausgezeichnete Affinität für die Batrachotoxin-(BTX)-Bindungsstelle des Natriumkanals.

| **Verbindung gemäß Beispiel** | **5-HT-Uptake (Ratte), 10 µM, % Hemmung** | **NA-Uptake (Ratte), 10 µM, % Hemmung** | **BTX Inhibierung (Ratte) (% Hemmung** |
|---|---|---|---|
| 1 | 47 | | |
| 2 | 41 | | 83 |
| 3 | 45 | | 73 |
| 4 | 45 | 57 | 80 |
| 5 | 47 | | |
| 6 | 43 | | |
| 7 | 44 | | |
| 8 | 54 | 66 | 72 |
| 9 | 45 | | 43 |
| 10 | 51 | 42 | 64 |
| 11 | 54 | | |
| 12 | 51 | 46 | 47 |
| 13 | 48 | 51 | 58 |
| 14 | 53 | | 70 |
| 15 | 58 | | |
| 16 | 55 | | |
| 17 | 64 | | |
| 18 | 42 | 47 | 75 |
| 19 | 50 | | |
| 20 | 51 | | |
| 21 | 56 | | 68 |
| 22 | 60 | | 56 |
| 23 | 52 | | 46 |
| 24 | | 51 | |
| 25 | | | |
| 26 | 54 | | |
| 27 | 41 | | |
| 28 | 55 | 44 | 60 |
| 29 | | 44 | |
| 30 | 57 | | |
| 31 | 57 | | |
| 32 | | 82 | 59 |
| 33 | 74 | 88 | 93 |
| 34 | 74 | | 42 |
| 35 | | 73 | 59 |
| 36 | 72 | | |
| 37 | 61 | | |
| 38 | | 66 | |
| 39 | | 61 | 79 |
| 40 | | 71 | 65 |
| 41 | 76 | | |
| 42 | 69 | | 54 |
| 43 | | 63 | |
| 44 | | 76 | |
| 45 | 85 | | |
| 46 | 79 | | |
| 47 | 68 | 67 | |
| 48 | | 61 | |
| 49 | | 73 | |
| 50 | | 71 | |
| 51 | | 73 | 42 |
| 52 | 83 | | 44 |
| 53 | 89 | | |
| 54 | 84 | | |
| 55 | 68 | | |
| 56 | 96 | | |
| 57 | 63 | | |
| 58 | 81 | | |
| 59 | 71 | 79 | 58 |
| 60 | 81 | | |
| 61 | 64 | | |
| 62 | 67 | | |
| 63 | | 77 | 55 |
| 64 | 77 | | |
| 65 | 88 | | 49 |
| 66 | 62 | | |
| 67 | 73 | | |
| 68 | 63 | 84 | 51 |
| 69 | 64 | | |
| 70 | 63 | | |
| 71 | 69 | | |
| 72 | 70 | | |
| 73 | 63 | | |
| 74 | 62 | | |
| 75 | 65 | | |
| 76 | 63 | | 41 |
| 77 | 62 | | |
| 78 | 70 | | |
| 79 | 71 | | |
| 80 | 65 | | |
| 81 | | 68 | 65 |
| 82 | 62 | | 69 |
| 83 | 61 | | |
| 84 | 69 | | |
| 85 | 67 | | |
| 86 | 86 | | |
| 87 | 66 | | 41 |
| 88 | 62 | | |
| 89 | 64 | | |
| 90 | 65 | | 42 |
| 91 | 67 | | 61 |
| 92 | 61 | | |
| 93 | 88 | | |
| 94 | 94 | | 60 |
| 95 | 68 | | |
| 96 | | 63 | |
| 97 | 67 | 75 | 70 |
| 98 | 75 | | |
| 99 | 69 | | |
| 100 | 83 | | |
| 101 | 66 | 75 | 78 |
| 102 | 70 | | 73 |
| 103 | | 91 | 92 |
| 104 | | 77 | 50 |
| 105 | | 62 | |
| 106 | 76 | | |
| 107 | 61 | | |
| 108 | 88 | | |
| 109 | | 64 | 56 |
| 110 | | 86 | 55 |
| 111 | | 66 | |
| 112 | 71 | | |
| 113 | 83 | | 60 |
| 114 | 85 | | 58 |
| 115 | 65 | | |
| 116 | 77 | | |
| 117 | 68 | 75 | |
| 118 | | 63 | 76 |
| 119 | 73 | | |
| 120 | | 62 | |
| 121 | | 61 | |
| 122 | | 63 | |
| 123 | | 70 | |
| 124 | 65 | | |
| 125 | | 63 | |
| 126 | 80 | | 88 |
| 127 | | 63 | 74 |
| 128 | 80 | | 74 |
| 129 | 75 | | 53 |
| 130 | | 67 | 68 |
| 131 | | 73 | 58 |
| 132 | | 76 | 90 |
| 133 | 63 | | |
| 134 | 61 | | 55 |
| 135 | | 60 | 61 |

## Patentansprüche

1. Substituierte 4,5,6,7-Tetrahydro-isoxazolo[4,5-c]pyridin-Verbindungen der allgemeinen Formel I 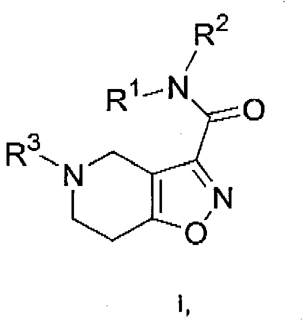 worin
R¹ für -(CHR⁴)-(CH₂)_{c}-(CH₂)_{d}-C(=O)-OR⁵ mit c = 0 oder 1 und d = 0 oder 1;
für -(CHR⁶)-(CHR⁷)ₑ-V_{f}-(CH₂)_{g}-Wₕ-(CH₂)ᵢ-R⁸ mit e = 0 oder 1, f = 0 oder 1, g = 0 oder 1, h = 0 oder 1 und i = 0 oder 1, worin V und W unabhängig voneinander jeweils für O, S, NH, N(CH₃) oder N(C₂H₅) stehen;
für einen linearen oder verzweigten, gesättigten oder ungesättigten, ggf. substituierten C₁₋₁₀ aliphatischen Rest,
für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. aromatischen, ggf. substituierten mono- oder polyzyklischen Ringsystem kondensiert und/oder mit einer linearen oder verzweigten, ggf. substituierten C₁₋₅-Alkylen-Gruppe überbrückt sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -GF₃, -SF₅, -OH, -O-C₁₋₅-Alkyl, -NO₂, -O-CF₃, -S-CF₃, -SH, -S-C₁₋₅-Alkyl, -C₁₋₅-Alkyl,-C(=O)-H, -C(=O)-C₁₋₅-Alkyl, -C(=O)-OH, -C(=O)-O-C₁₋₅-Alkyl, -(CH₂)-C(=O)-OH, -(CH₂)-C(=O)-O-C₁₋₅-Alkyl, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
R² für einen Wasserstoff-Rest;
für -(CH₂)-R⁹
oder für einen linearen oder verzweigten, gesättigten oder ungesättigten, ggf. substituierten C₁₋₁₀ aliphatischen Rest steht;
oder
R¹ und R² zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden,
wobei der heterocycloaliphatische Rest jeweils mit 1, 2, 3, 4 oder 5 Substituenten unabhängig voneinander ausgewählt aus der Gruppe bestehend aus R¹⁰, -C(=O)-R¹¹ und -(CH₂)-NH-C(=O)-R¹² substituiert und/oder jeweils weitere 1, 2, 3, 4 oder 5 Heteroatom(e) unabhängig voneinander ausgewählt aus der Gruppe bestehend aus Sauerstoff, Stickstoff und Schwefel als Ringglied(er) aufweisen kann;
R³ für -C(=O)-NR¹³R¹⁴;
für -C(=O)-R¹⁵;
für -C(=O)-(CH₂)ⱼ-Xₖ-(CH₂)ₘ-(CH₂)ₙ-C(=O)-OR¹⁶ mit j = 0 oder 1, k = 0 oder 1, m = 0 oder 1 und n = 0 oder 1, worin X für O, S, NH, N(CH₃), N(C₂H₅) oder CR¹⁷R¹⁸ steht;
für -C(=O)-(CHR¹⁹)-NH-C(=O)-OR²⁰;
für -C(=O)-(CH₂)-(CH₂)ₚ-(CH₂)_{q}-Yᵣ-(CH₂)ₛ-R²¹ mit p = 0 oder 1, q = 0 oder 1, r = 0 oder 1 und s = 0 oder 1, worin Y für O, S, NH, N(CH₃) oder N(C₂H₅) steht;
für -C(=O)-(CH=CH)-R²²;
für -S(=O)₂-R²³;
für -S(=O)₂-NR²⁴R²⁵;
für -C(=S)-NR²⁶-(CH₂)ₜ-(CH₂)ᵤ-Zᵥ-R²⁷ mit t = 0 oder 1, u = 0 oder 1 und v = 0 oder 1, worin Z für O, S, NH, N(CH₃) oder N(C₂H₅) steht;
für -C(=S)-NR²⁶-(CHR²⁸)-R²⁹;
für -C(=S)-NR²⁶-C(=O)-R³⁰;
oder für -(CH₂)-R³¹ steht;
R⁴ für -C(=O)-NH₂;
für einen Wasserstoff-Rest
oder für einen linearen oder verzweigten, gesättigten oder ungesättigten, ggf. substituierten C₁₋₁₀ aliphatischen Rest steht;
R⁵, R¹⁷, R¹⁸, R²⁴ und R²⁵, unabhängig voneinander, jeweils
für einen linearen oder verzweigten, gesättigten oder ungesättigten, ggf. substituierten C₁₋₁₀ aliphatischen Rest stehen;
R⁶, R⁷, R¹³, R¹⁶ und R²⁶, unabhängig voneinander, jeweils
für einen Wasserstoff-Rest
oder für einen linearen oder verzweigten, gesättigten oder ungesättigten, ggf. substituierten C₁₋₁₀ aliphatischen Rest stehen;
R⁸, R⁹, R²¹, R²², R²³, R³⁰ und R³¹, unabhängig voneinander, jeweils
für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. aromatischen, ggf. substituierten mono- oder polyzyklischen Ringsystem kondensiert sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH, -C(=O)-O-C₁₋₅-Alkyl, -(CH₂)-C(=O)-OH, -(CH₂)-C(=O)-O-C₁₋₅-Alkyl, - NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -O-C₁₋₅-Alkyl, -C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
R¹⁰, R¹¹, R¹², R¹⁴ und R²⁰, unabhängig voneinander, jeweils
für einen linearen oder verzweigten, gesättigten oder ungesättigten, ggf. substituierten C₁₋₁₀ aliphatischen Rest,
für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. aromatischen, ggf. substituierten mono- oder polyzyklischen Ringsystem kondensiert und/oder über eine lineare oder verzweigte, ggf. substituierte C₁₋₅-Alkylen-, C₂₋₅-Alkenylen-oder C₂₋₅-Alkinylen-Gruppe gebunden sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, - SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH, -C(=O)-O-C₁₋₅-Alkyl, -(CH₂)-C(=O)-OH, -(CH₂)-C(=O)-O-C₁₋₅-Alkyl, -NH-C₁₋₅-Alkyl, - N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein können, wobei jeweils der zyklische Teil der Reste -O-Phenyl, -O-Benzyl, Phenyl, - (CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der über eine lineare oder verzweigte, ggf. substituierte C₁₋₅-Alkylen-, C₂₋₅-Alkenylen- oder C₂₋₅-Alkinylen-Gruppe gebunden sein kann, stehen;
R¹⁵ und R²⁷, unabhängig voneinander, jeweils
für einen linearen oder verzweigten, gesättigten oder ungesättigten, ggf. substituierten C₁₋₁₀ aliphatischen Rest,
für einen ungesättigten oder gesättigten, ggf. substituierten 3-, 4-, 5-, 6-, 7-, 8- oder 9-gliedrigen cycloaliphatischen Rest, der mit einer linearen oder verzweigten, ggf. substituierten C₁₋₅-Alkylen-Gruppe überbrückt sein kann, wobei die vorstehend genannten ggf. substituierten cycloaliphatischen Reste jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, - NH₂, -NO₂, -O-CF₃, -S-CF₃, -SH, -S-C₁₋₅-Alkyl, -C₁₋₅-Alkyl, -C(=O)-H, - C(=O)-C₁₋₅-Alkyl, -C(=O)-OH, -C(=O)-O-C₁₋₅-Alkyl, -(CH₂)-C(=O)-OH, - (CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und-(CH₂)-Naphthyl substituiert sein können, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁-₅-Alkyl, -O-CF₃, -S-CF₃, Phenyl und -O-Benzyl substituiert sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
R¹⁹ für einen ggf. substituierten 5- bis 14-gliedrigen Aryl- oder Heteroaryl-Rest, der über eine lineare oder verzweigte, ggf. substituierte, ggf. 1 oder 2 Heteroatom(e) als Kettenglied(er) aufweisende C₁₋₅-Alkylen-, C₂₅-Alkenylen- oder C₂₋₅-Alkinylen-Gruppe gebunden sein kann, steht;
R²⁸ und R²⁹, unabhängig voneinander, jeweils
für einen linearen oder verzweigten, gesättigten oder ungesättigten, ggf. substituierten C₁₋₁₀ aliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
wobei
die vorstehend genannten ggf. substituierten C₁₋₁₀ aliphatischen Reste jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein können;
die vorstehend genannten cycloaliphatischen Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten ggf. substituierten C₁₋₅-Alkylen-, C₂₋₅-Alkenylen- oder C₂₋₅-Alkinylen-Gruppen jeweils mit 1, 2, 3, 4 oder 5 Substituenten unabhängig voneinander ausgewählt aus der Gruppe bestehend aus F, Cl, Br, -OH, -SH, -NH₂, -CN, NO₂ und Phenyl substituiert sein können,
und die vorstehend genannten C₁₋₅-Alkylen-, C₂₋₅-Alkenylen- oder C₂₋₅-Alkinylen-Gruppen ggf. jeweils 1 oder 2 Heteroatom(e) ausgewählt aus der Gruppe bestehend aus Sauerstoff, Stickstoff und Schwefel als Kettenglied(er) aufweisen können;
die Ringe der vorstehend genannten ggf. substituierten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, - SH, -S-C₁₋₅-Alkyl, -C₁₋₅-Alkyl, -C(=O)-OH, -C(=O)-O-C₁₋₅-Alkyl, -NH-C₁₋₅-Alkyl, - N(C₁₋₅-Alkyl)₂, -NH-C(=O)-O-C₁₋₅-Alkyl, -NH-C(=O)-C₁₋₅-Alkyl, -C(=O)-H, - C(=O)-C₁₋₅-Alkyl, -C(=O)-NH₂, -C(=O)-NH-C₁₋₅-Alkyl, -C(=O)-N-(C₁₋₅-Alkyl)₂, - S(=O)₂-NH₂, -S(=O)₂-NH-C₁₋₅-Alkyl, -(CH₂)-Benzo[b]furanyl, -O-Phenyl, -0-Benzyl, Phenyl und Benzyl substituiert sein können, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -O-C₁₋₅-Alkyl, -C₁₋₅-Alkyl, -O-CF₃, -S-CF₃, Phenyl und -O-benzyl substituiert sein kann,
und die Ringe der vorstehend genannten mono- oder polyzyklischen Ringsysteme jeweils 5-, 6- oder 7-gliedrig sind und jeweils 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten ggf. substituierten Aryl- oder Heteroaryl-Reste jeweils mit 1, 2, 3, 4 oder 5 Substituenten unabhängig voneinander ausgewählt aus der Gruppe bestehend aus F, Cl, Br, I, -CN, -CF₃, -SF₅, -OH, - O-C₁₋₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-O-C₁₋₅-Alkyl, -NH-C₁₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, -(CH₂)-Benzo[b]furanyl, -0-Phenyl, -O-Benzyl, Phenyl und Benzyl substituiert sein können, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, - SF₅, -CN, -NO₂, -O-C₁₋₅-Alkyl, -C₁₋₅-Alkyl, -O-CF₃, -S-CF₃, Phenyl und -0-Benzyl substituiert sein kann,
und
die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, daß**
R¹ für -(CHR⁴)-(CH₂)_{c}-(CH₂)_{d}-C(=O)-OR⁵ mit c = 0 oder 1 und d = 0 oder 1 steht;
für -(CHR⁶)-(CHR⁷)ₑ-V_{f}-(CH₂)_{g}-Wₕ-(CH₂)ᵢ-R⁸ mit e = 0 oder 1, f = 0 oder 1, g = 0 oder 1, h = 0 oder 1 und i = 0 oder 1 steht, worin V und W unabhängig voneinander jeweils für O, S, NH, N(CH₃) oder N(C₂H₅) stehen;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
für einen Alkenyl-Rest ausgewählt aus der Gruppe bestehend aus Vinyl, 1-Propenyl, 2-Propenyl, 1-Butenyl, 2-Butenyl und 3-Butenyl steht, wobei der Alkenyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
für einen Alkinyl-Rest ausgewählt aus der Gruppe bestehend aus Ethinyl, 1-Propinyl, 2-Propinyl, 1-Butinyl, 2-Butinyl und 3-Butinyl steht, wobei der Alkinyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, (6,6)-Dimethyl-[3.1.1]-bicycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, - SF₅, -OH, -O-C₁₋₅-Alkyl, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH, C(=O)-O-C₁₋₅-Alkyl, - (CH₂)-C(=O)-OH, -(CH₂)-C(=O)-O-C₁₋₅-Alkyl, -(CH₂)-Benzo[b]furanyl, - O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-O-C₁₋₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, - (CH₂)-Benzo[b]furanyl, -O-Phenyl, -O-Benzyl, Phenyl und Benzyl substituiert sein kann, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
R² für einen Wasserstoff-Rest;
für -(CH₂)-R⁹
oder für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, - N(CH₃)₂, -N(C₂H₅)₂ und -N(CH₃)(C₂H₅) substituiert sein kann;
oder
R¹ und R² zusammen mit dem sie verbindenden Stickstoffatom als Ringglied einen heterocycloaliphatischen Rest bilden ausgewählt aus der Gruppe bestehend aus Imidazolidinyl, (1,3)-Thiazolidinyl, Piperazinyl, Morpholinyl, Thiomorpholinyl, Pyrrolidinyl; Piperidinyl; (1,2,3,6)-Tetrahydropyridinyl und (1,2,3,4)-Tetrahydropyridinyl, wobei der heterocycloaliphatische Rest jeweils mit 1, 2 oder 3 Substituenten unabhängig voneinander ausgewählt aus der Gruppe bestehend aus R¹⁰, -C(=O)-R¹¹ und -(CH₂)-NH-C(=O)-R¹² substituiert sein kann;
R³ für -C(=O)-NR¹³R¹⁴;
für -C(=O)-R¹⁵;
für -C(=O)-(CH₂)-X-(CH₂)-C(=O)-OR¹⁶, -C(=O)-X-(CH₂)-C(=O)-OR¹⁶ oder -C(=O)-(CH₂)-(CH₂)-(CH₂)-C(=O)-OR¹⁶, worin X für O, S, NH, N(CH₃), N(C₂H₅) oder CR¹⁷R¹⁸ steht;
für -C(=O)-(CHR¹⁹)-NH-C(=O)-OR²⁰;
für -C(=O)-(CH₂)-R²¹, -C(=O)-(CH₂)-Y-R²¹, -C(=O)-(CH₂)-Y-(CH₂)-R²¹ oder -C(=O)-(CH₂)-(CH₂)-(CH₂)-Y-R²¹, worin Y für O, S, NH, N(CH₃) oder N(C₂H₅) steht;
für -C(=O)-(CH=CH)-R²²;
für -S(=O)₂-R²³;
für -S(=O)₂-NR²⁴R²⁵;
für -C(=S)-NR²⁶-R²⁷, -C(=S)-NR²⁶-(CH₂)-R²⁷, -C(=S)-NR²⁶-(CH₂)-(CH₂)-R²⁷ oder -C(=S)-NR²⁶-(CH₂)-(CH₂)-Z-R²⁷, worin Z für O, S, NH, N(CH₃) oder N(C₂H₅) steht;
für -C(=S)-NR²⁶-(CHR²⁸)-R²⁹;
für -C(=S)-NR²⁶-C(=O)-R³⁰;
oder für -(CH₂)-R³¹ steht;
R⁴ für -C(=O)-NH₂;
für einen Wasserstoff-Rest;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
oder für einen Alkenyl-Rest ausgewählt aus der Gruppe bestehend aus Vinyl, 1-Propenyl, 2-Propenyl, 1-Butenyl, 2-Butenyl und 3-Butenyl steht, wobei der Alkenyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-C₁₋₅-Alkyl, -SH, -S-C₁₋₅-Alkyl, -NH₂, -NH-C₁₋₅-Alkyl und -N(C₁₋₅-Alkyl)(C₁₋₅-Alkyl) substituiert sein kann;
R⁵, R¹⁷, R¹⁸, R²⁴ und R²⁵, unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann,
oder für einen Alkenyl-Rest ausgewählt aus der Gruppe bestehend aus Vinyl, 1-Propenyl, 2-Propenyl, 1-Butenyl, 2-Butenyl und 3-Butenyl stehen, wobei der Alkenyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und-N(CH₃)(C₂H₅) substituiert sein kann;
R⁶ R⁷, R¹³, R¹⁶ und R²⁶, unabhängig voneinander, jeweils
für einen Wasserstoff-Rest
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann,
oder für einen Alkenyl-Rest ausgewählt aus der Gruppe bestehend aus Vinyl, 1-Propenyl, 2-Propenyl, 1-Butenyl, 2-Butenyl und 3-Butenyl stehen, wobei der Alkenyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und - N(CH₃)(C₂H₅) substituiert sein kann;
R⁸, R⁹, R²¹, R²², R²³, R³⁰ und R³¹, unabhängig voneinander, jeweils
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (-S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH, -C(=O)-O-C₁₋₅-Alkyl, - (CH₂)-C(=O)-OH, -(CH₂)-C(=O)-O-C₁₋₅-Alkyl, -NH-C₁₋₅-Alkyl, -N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Reste -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-O-C₁₋₅-Alkyl, -NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, - (CH₂)-Benzo[b]furanyl, -O-Phenyl, -O-Benzyl, Phenyl und Benzyl substituiert sein kann, wobei jeweils der zyklische Teil der Reste -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₋₅-Alkyl, -O-CF₃, -S-CF₃, Phenyl und -O-Benzyl substituiert sein kann;
R¹⁰, R¹¹, R¹², R¹⁴ und R²⁰, unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-C₁₋₅-Alkyl, -SH, -S-C₁₋₅-Alkyl, -NH₂, -NN-C₁₋₅-Alkyl und -N(C₁₋₅-Alkyl)(C₁₋₅-Alkyl) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl und Indenyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, - CF₃, -SF₅, -OH, -O-C₁₋₅-Alkyl, -NH₂, -NO₂, -O-CF₃, -S-CF_{3,} -SH, -S-C₁₋₅-Alkyl, -C₁₋₅-Alkyl, -C(=O)-H, -C(=O)-C₁₋₅-Alkyl, -C(=O)-OH, -C(=O)-O-C₁₅-Alkyl, -(CH₂)-C(=O)-OH, -(CH₂)-C(=O)-O-C₁₋₅-Alkyl, -NH-C₁₋₅-Alkyl, - N(C₁₋₅-Alkyl)₂, -(CH₂)-Benzo[b]furanyl, -O-Phenyl, -O-Benzyl, Phenyl, Benzyl, Naphthyl und -(CH₂)-Naphthyl substituiert sein kann, wobei jeweils der zyklische Teil der Substituenten -O-Phenyl, -O-Benzyl, Phenyl, -(CH₂)-Benzo[b]furanyl, Benzyl, Naphthyl und -(CH₂)-Naphthyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, - O-C₁₋₅-Alkyl, -C₁₋₅-Alkyl, -O-CF₃, -C₁₋₅-Alkyl, -S-CF₃, Phenyl und -O-Benzyl substituiert und/oder wobei der (hetero)cycloaliphatische Rest über eine -(CH₂)-, -CH(CH₃)-, -(CH₂)-(CH₂)-, -(CH₂)-(CH₂)-(CH₂)- oder -(CH₂)-(CH=CH)-Gruppe gebunden sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl, Isochinolinyl und Thieno[2,3-d]pyrimidinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, - O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-O-C₁₋₅-Alkyl, - NH-C₁₋₅-Alkyl, -N(C₁₋₅-Alkyl)₂, -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -S(=O)₂-NH₂, -S(=O)₂-NH-C₁₋₅-Alkyl, -(CH₂)-Benzo[b]furanyl, -O-Phenyl, -O-Benzyl, Phenyl und Benzyl substituiert und/oder über eine -(CH₂)-, -CH(CH₃)-, -(CH₂)-(CH₂)-, -(CH₂)-(CH₂)-(CH₂)- oder -(CH₂)-(CH=CH)-Gruppe gebunden sein kann, wobei jeweils der zyklische Teil der Substituenten -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁₅-Alkyl, -O-CF₃, -S-CF₃, Phenyl und -O-Benzyl substituiert sein kann;
R¹⁵ und R²⁷, unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
für einen Alkenyl-Rest ausgewählt aus der Gruppe bestehend aus Vinyl, 1-Propenyl, 2-Propenyl, 1-Butenyl, 2-Butenyl und 3-Butenyl stehen, wobei der Alkenyl-Rest jeweils mit 1, 2, 3, 4 oder 5 Substituenten unabhängig voneinander ausgewählt aus der Gruppe bestehend aus F, Cl, Br, I, -CN, -NO₂, -OH, -O-CH₃, -O-C₂H₅, -SH, -S-CH₃, -S-C₂H₅, - NH₂, -NH-CH₃, -NH-C₂H₅, -N(CH₃)₂, -N(C₂H₅)₂ und -N(CH₃)(C₂H₅) substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydrapyranyl, Azepanyl, Diazepanyl, Dithiolanyl und 7,7-Dimethyl-2-oxa-bicyclo[2.2.1]heptyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-H, -C(=O)-C₁₋₅-Alkyl, -C(=O)-OH. -C(=O)-O-C₁₋₅-Alkyl, -(CH₂)-C(=O)-OH, -(CH₂)-C(=O)-O-C₁₋₅-Alkyl, - NH-C₁₋₅-Alkyl, -N(C₁₋₅-Alkyl)₂, -O-Phenyl, -O-Benzyl, Phenyl und Benzyl, substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-C₁₋₅-Alkyl, -C₁-₅-Alkyl, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzadioxolyl, (1,4)-Benzodioxanyl), Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁-₅-Alkyl, -O-C₂₋₅-Alkenyl, -NH₂, -NO₂, -O-CF₃, -O-CHF₂, -O-CH₂F, -S-CF₃, -S-CHF₂, -S-CH₂F, -SH, -S-C₁₋₅-Alkyl, -C₁₋₅-Alkyl, -C(=O)-O-C₁₋₅-Alkyl, -NH-C₁₋₅-Alkyl, -N(C₁₋₅-Alkyl)₂. -NH-C(=O)-O-C₁₋₅-Alkyl, -NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-NH₂, -S(=O)₂-NH-C₁₋₅-Alkyl, - O-Phenyl, -O-Benzyl, Phenyl und Benzyl substituiert sein kann, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -O-C₁₋₅-Alkyl, -C₁₋₅-Alkyl, -O-CF₃, -S-CF₃, Phenyl und -O-Benzyl substituiert sein kann;
R¹⁹ für einen Rest ausgewählt aus der Gruppe bestehend aus Phenyl, Naphthyl, Thiophenyl, Furanyl und Pyridinyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -NH₂, - NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -N(CH₃)₂, -N(C₂H₅)₂, -N(H)(CH₃), -N(H)(C₂H₅), -NH-C(=O)-CH₃, -NH-C(=O)-C₂H₅, -C(=O)-H, - C(=O)-CH₃, -C(=O)-C₂H₅, -C(=O)-NH₂, -C(=O)-NH-CH₃, -C(=O)-N-(CH₃)₂, Phenyl und Benzyl substituiert und/oder über eine -(CH₂)-, - (CH₂)-(CH₂)- oder -(CH₂)-O-(CH₂)-Gruppe gebunden sein kann;
und
R²⁸ und R²⁹, unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) stehen, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, - CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, - C(=O)-O-C₂H₅, -N(CH₃)₂, -N(C₂H₅)₂, -N(H)(CH₃), -N(H)(C₂H₆), -NH-C(=O)-CH₃, -NH-C(=O)-C₂H₅, -C(=O)-H, -C(=O)-CH₃, -C(=O)-C₂H₅, - C(=O)-NH₂, -C(=O)-NH-CH₃, -C(=O)-N-(CH₃)₂, -S(=O)₂-NH₂, -S(=O)₂-CH₃, Phenyl und Benzyl substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, daß**
R¹ für -(CHR⁴)-C(=O)-OR⁵, -(CHR⁴)-(CH₂)-C(=O)-OR⁵ oder -(CHR⁴)-(CH₂)-(CH₂)-C(=O)-OR⁵ steht;
für-(CHR⁶)-R⁸, -(CHR⁶)-(CHR⁷)-R⁸, -(CHR⁶)-(CHR⁷)-V-R⁸, -(CHR⁶)-(CHR⁷)-(CH₂)-R⁸, -(CHR⁶)-(CHR⁷)-(CH₂)-W-R⁸ oder-(CHR⁶)-(CHR⁷)-(CH₂)-W-(CH₂)-R⁸ steht, worin V und W unabhängig voneinander jeweils für O, S, NH, N(CH₃) oder N(C₂H₅) stehen;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
für einen Alkinyl-Rest ausgewählt aus der Gruppe bestehend aus Ethinyl, 1-Propinyl, 2-Propinyl, 1-Butinyl, 2-Butinyl und 3-Butinyl steht;
für einen (hetero)cycloaliphatischen Rest ausgewählt aus der Gruppe bestehend aus Cyclopropyl, Cyclobutyl, Cyclopentyl, Cyclohexyl, Cycloheptyl, (6,6)-Dimethyl-[3.1.1]-bicycloheptyl, Pyrrolidinyl, Piperidinyl, Morpholinyl, Thiomorpholinyl, Indanyl und Indenyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, - OH, -O-CH₃, -O-C₂H₅, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-C(CH₃)₃, -C(=O)-OH, - C(-O)-O-CH₃, -C(=O)-O-C₂H₅, -(CH₂)-C(=O)-OH, -(CH₂)-C(=O)-O-CH₃, -(CH₂)-C(=O)-O-C₂H₅, -(CH₂)-Benzo[b]furanyl, -O-Phenyl, -O-Benzyl, Phenyl, Benzyl, Naphthyl und -(CH₂)-Naphthyl substituiert sein kann, wobei der zyklische Teil der Benzyl-Reste mit 1, 2, 3, 4 oder 5 Substituenten unabhängig voneinander ausgewählt aus der Gruppe bestehend aus F, Cl, Br, -OH, -CF₃, -SF₅, -CN, -NO₂, -O-CH₃, -O-C₂H₅ und -O-CF₃ substituiert sein kann,
oder für einen Rest ausgewählt aus der Gruppe bestehend aus Phenyl, Naphthyl, (1,3)-Benzadioxalyl, (1,4)-Benzodioxanyl, Thiophenyl, Furanyl, Pyridinyl, Imidazolyl und Indolyl steht, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, Methyl, Ethyl, n-Propyl, Isopropyl, n-Butyl, sec-Butyl, Isobutyl und tert-Butyl substituiert sein kann;
R² für einen Wasserstoff-Rest oder
für -(CH₂)-R⁹ steht.
oder
R¹ und R² zusammen mit dem sie verbindenden Stickstoffatom als Ringglied einen Rest bilden, der ausgewählt ist aus der Gruppe bestehend aus 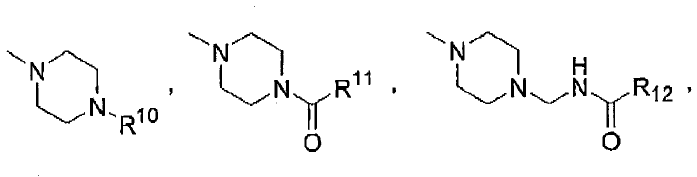 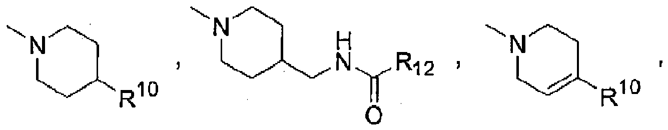 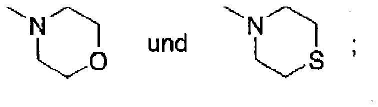
R³ für -C(=O)-NR¹³R¹⁴;
für -(=O)-R¹⁵_{;}
für -C(=O)-(CH₂)-O-(CH₂)-C(=O)-OR¹⁶, -C(=O)-(CH₂)-CR¹⁷R¹⁸-(CH₂)-C(-O)-OR¹⁶, -C(=O)-CR¹⁷R¹⁸-(CH₂)-C(-O)-OR¹⁶ oder -C(=O)-(CH₂)-(CH₂)-(CH₂)-C(=O)-OR¹⁶;
für -C(=O)-(CHR¹⁹)-NH-C(=O)-OR²⁰;
für -C(=O)-(CH₂)-R²¹, -C(=O)-(CH₂)-O-R²¹, -C(=OHCH₂)-O-(CH₂)-R²¹ oder -C(=O)-(CH₂)-(CH₂)-(CH₂)-O-R¹¹;
für -C(=O)-(CH=CH)-R²²;
für -S(=O)₂-R²³;
für -S(=O)₂-NR²⁴R²⁵_{;}
für -C(=S)-NR²-R²⁷, -C(=S)-NR²⁶-(CH₂)-R²⁷, -C(=S)-NR²⁶-(CH₂)-(CH₂)-R²⁷ oder -C(=S)-NR²⁶-(CH₂)-(CH₂)-O-R²⁷;
für -C(=S)-NR²⁶-(CHR²⁸)-R²⁹;
für -C(=S)-NR²⁶-C(=O)-R³⁰;
oder für -(CH₂)-R³¹ steht;
R⁴ für -C(=O)-NH₂;
für einen Wasserstoff-Rest oder
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl(CH₂)-(CH₂)-(C(CH₃)₃), n-Hexyl, n-Heptyl, n-Octyl und -(CH₂)-(CH)(C₂H₅)-H₂)-(CH₂)-(CH₂)-(CH₃) steht, wobei der Alkyl-Rest jeweils mit 1, 2, 3, 4 oder 5 Substituenten unabhängig voneinander ausgewählt aus der Gruppe bestehend aus F, Cl, Br, I, - CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ und -N(CH₃)(C₂H₅) substituiert sein kann;
R⁵ für einen Alkyl-Rest ausgewählt aus der Gruppe bestehend aus Methyl, Ethyl, n-Propyl, Isopropyl, n-Butyl, sec-Butyl, Isobutyl und tert-Butyl steht;
R⁶, R⁷ und R¹⁶, unabhängig voneinander, jeweils für
einen Wasserstoff-Rest oder für einen Alkyl-Rest ausgewählt aus der Gruppe bestehend aus Methyl, Ethyl, n-Propyl und Isopropyl stehen;
R⁸, R⁹, R²¹, R²², R²³, R³⁰ und R³¹, unabhängig voneinander, jeweils
für einen (hetero)cycloaliphatischen Rest ausgewählt aus der Gruppe bestehend aus Cyclopropyl, Cyclobutyl, Cyclopentyl, Cyclohexyl, Cycloheptyl, Pyrrolidinyl, Piperidinyl, Morpholinyl, Thiomorpholinyl, Indanyl und Indenyl stehen, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NN₂, -NO₂, -O-CF₃,-S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, - C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -N(CH₃)₂, - N(C₂H₅)₂, -N(H)(CH₃), -N(H)(C₂H₅), Phenyl und Benzyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyridinyl, Imidazolyl, Indolyl und Isoindolyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -C(=O)-H, -C(=O)-CH₃, -C(=O)-C₂H₅, -C(=O)-NH₂, -S(=O)₂-NH₂, -S(=O)₂-NH-CH₃, -O-Phenyl, -O-Benzyl, Phenyl und Benzyl substituiert sein kann;
R¹⁰, R¹¹, R¹², R¹⁴ und R²⁰, unabhängig voneinander, jeweils
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -(CH₂)-(CH₂)-(C(CH₃)₃), n-Hexyl, n-Heptyl und n-Octyl stehen, wobei der Alkyl-Rest jeweils mit 1, 2, 3, 4 oder 5 Substituenten unabhängig voneinander ausgewählt aus der Gruppe bestehend aus -N(CH₃)₂, -N(C₂H₅)₂, F, Cl und Br substituiert sein kann;
für einen cycloaliphatischen Rest ausgewählt aus der Gruppe bestehend aus Pyrrolidinyl, Piperidinyl, Piperazinyl, Morpholinyl, Cyclopropyl, Cyclobutyl, Cyclopentyl und Cyclohexyl stehen, wobei der cycloaliphatische Rest jeweils mit 1, 2 oder 3 Substituenten unabhängig voneinander ausgewählt aus der Gruppe bestehend aus F, Cl, Br, I, - CN, -CF₃, -SF₅, -OH, -O-CH₃, -O-C₂H₅, -NH₂, -NO₂, -O-CF₃, -S-CF₃, - SH, -S-CH₃, -S-C₂H₅, Methyl, Ethyl, n-Propyl, Isopropyl, n-Butyl, sec-Butyl, Isobutyl, tert-Butyl, -C(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, - N(H)(CH₃), -N(H)(C₂H₅), Phenyl und Benzyl substituiert und/oder über eine -(CH₂)- oder -(CH₂)-(CH₂)-Gruppe gebunden sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl, Isochinolinyl und Thieno[2,3-d]pyrimidinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, - NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -N(CH₃)₂, - N(C₂H₅)₂, -N(H)(CH₃), -N(H)(C₂H₅), -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, - S(=O)₂-CH₃, Phenyl und Benzyl substituiert und/oder über eine -(CH₂)-, -CH(CH₃)-, -(CH₂)-(CH₂)-, -(CH₂)-(CH₂)-(CH₂)- oder -(CH₂)-(CH=CH)-Gruppe gebunden sein kann;
R¹³ und R²⁶ für einen Wasserstoff-Rest stehen;
R¹⁵ und R²⁷, unabhängig voneinander, jeweils
für einen Rest ausgewählt aus der Gruppe bestehend aus Methyl, Ethyl, n-Propyl, Isopropyl, n-Butyl, sec-Butyl, Isobutyl, tert-Butyl, Vinyl, 1-Propenyl, 2-Propenyl, 1-Butenyl, 2-Butenyl und butenyl stehen;
für einen (hetero)cycloaliphatischen Rest ausgewählt aus der Gruppe bestehend aus Cyclopropyl, Cyclobutyl, Cyclopentyl, Cyclohexyl und 7,7-Dimethyl-2-oxa-bicycla[2.2.1]heptyl stehen, wobei der (hetero)cycloaliphatische Rest jeweils mit 1, 2 oder 3 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-H,-C(=O)-CH₃, -C(=O)-C₂H₅, -C(=O)-C(CH₃)₃, -C(=O)-OH, -C(=O)-O-CH₃,-C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), Phenyl und Benzyl substituiert sein kann;
oder für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benza[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -N(CH₃)₂, -N(C₂H₅)₂, - N(H)(CH₃), -N(H)(C₂H₅), -NH-C(=O)-CH₃, -NH-C(=O)-C₂H₅, -C(=O)-H, - C(=O)-CH₃, -C(=O)-C₂H₅, -C(=O)-NH_{2,} -C(=O)-NH-CH₃, -C(=O)-N-(CH₃)₂, -S(=O)₂-NH₂, -S(=O)₂-CH₃, Phenyl und Benzyl substituiert sein kann;
R¹⁷ und R¹⁸, unabhängig voneinander, jeweils für einen Methyl- oder Ethyl-Rest stehen;
R¹⁹ für einen Phenyl-Rest steht, der über eine -(CH₂)-, -(CH₂)-(CH₂)- oder - (CH₂)-O-(CH₂)-Gruppe gebunden sein kann;
R²⁴ und R²⁵, unabhängig voneinander, jeweils für einen Methyl- oder Ethyl-Rest stehen; R²⁸ für einen Phenyl-Rest steht, der mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, - O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl substituiert sein kann;
und
R²⁹ für einen Alkyl-Rest ausgewählt aus der Gruppe bestehend aus Methyl, Ethyl, n-Propyl, Isopropyl, n-Butyl, Isobutyl und tert-Butyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

4. Verbindungen gemäß einem oder mehreren der Ansprüche 1 bis 3, **dadurch gekennzeichnet, daß**
R¹ für -(CHR⁴)-C(=O)-OR⁵, -(CHR⁴)-(CH₂)-C(=O)-OR⁵ oder -(CHR⁴)-(CH₂)-(CH₂)-C(=O)-OR⁵ steht;
für -(CHR⁶)-R⁸, -(CHR⁶)-(CHR⁷)-R⁸, -(CHR⁶)-(CHR⁷)-O-R⁸, -(CHR⁶)-(CHR⁷)-(CH₂)-R⁸ oder -(CHR⁶)-(CHR⁷)-(CH₂)-N(CH₃)-R⁸ steht;
für einen ggf. substituierten Alkyl-Rest ausgewählt aus der Gruppe bestehend aus Methyl, Ethyl, n-Propyl, Isopropyl, -(CH₂)-(CH₂)-CN, -
(CH₂)-(CH₂)-N(CH₃)₂, -(CH₂)-(CH₂)-O-CH₃, -(CH₂)-(CH₂)-S-C₂H₅, -n-Butyl, sec-Butyl, Isobutyl, tert-Butyl, -(CH₂)-(CH₂)-(CH₂)-O-CH₃, n-Pentyl, sec-Pentyl,-(CH₂)-(CH₂)-(C(CH₃)₃), n-Hexyl, n-Heptyl, n-Octyl und -(CH₂)-(CH)(C₂H₅)-(CH₂)-(CH₂)-(CH₂)-(CH₃) steht;
für einen Alkinyl-Rest ausgewählt aus der Gruppe bestehend aus 1-Propinyl und 2-Proplnyl steht;
für einen cycloaliphatischen Rest ausgewählt aus der Gruppe bestehend aus Cyclopropyl, Cyclobutyl, Cyclopentyl, Cyclohexyl, Cycloheptyl, (6,6)-Dimethyl-[3.1.1]-bicycloheptyl, Indanyl und Indenyl, wobei der cycloaliphatische Rest jeweils mit einem -O-Benzyl-Rest oder einem Methyl-Rest substituiert sein kann,
für einen Pyrrolidinyl- oder Piperidinyl-Rest steht, der jeweils am Stickstoffatom mit einem Substituenten ausgewählt aus der Gruppe bestehend aus -C(=O)-CH₃, -C(=O)-C₂H₅, -C(=O)-C(CH₃)₃, -C(=O)-O-C₂H₅, Benzyl und -(CH₂)-Naphthyl substituiert sein kann, wobei der zyklische Teil des Benzyl-Rests mit 1, 2 oder 3 Substituenten unabhängig voneinander ausgewählt aus der Gruppe bestehend aus F, Cl, Br, -CF₃ und -O-CF₃ substituiert sein kann,
oder für einen Phenyl-Rest steht, der mit 1, 2 oder 3 Substituenten unabhängig voneinander ausgewählt aus der Gruppe bestehend aus - O-CH₃, -O-C₂H₅, Methyl, Ethyl, n-Propyl, Isopropyl, n-Butyl, sec-Butyl, Isobutyl und tert-Butyl substituiert sein kann;
R² für einen Wasserstoff-Rest oder
für-(CH₂)-R⁹ steht;
oder
R¹ und R² zusammen mit dem sie verbindenden Stickstoffatom als Ringglied einen Reste bilden, der ausgewählt ist aus der Gruppe bestehend aus 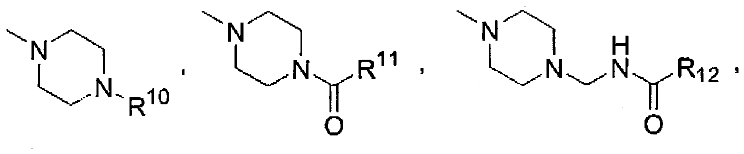 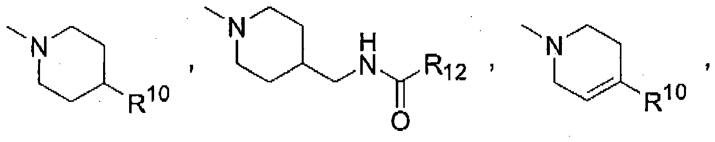 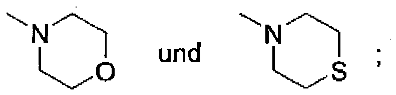
R³ für -C(=O)-NR¹³R¹⁴;
für -C(=O)-R¹⁵;
für -C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-CR¹⁷R¹⁸-(CH₂)-C(=O)-OR¹⁶ oder-C(=O)-(CH₂)-(CH₂)-(CH₂)-C(=O)-OR¹⁶;
für -C(=O)-(CHR¹⁹)-NH-C(=O)-OR²⁰;
für -C(=O)-(CH₂)-R²¹, -C(=O)-(CH₂)-O-R²¹, -C(=O)-(CH₂)-O-(CH₂)-R²¹ oder -C(=O)-(CH₂)-(CH₂)-(CH₂)-O-R²¹;
für -C(=O)-(CH=CH)-R²²;
für -S(=O)₂-R²³_{;}
für -S(=O)₂-NR²⁴R²⁵_{;}
für -C(=S)-NR²⁶-R²⁷, -C(=S)-NR²⁶-(CH₂)-R²⁷, -C(=S)-NR²⁶-(CH₂)-(CH₂)-R²⁷ oder -C(=S)-NR²⁶-(CH₂)-(CH₂)-O-R²⁷;
für -C(=S)-NR²⁶-(CHR²⁸)-R²⁹;
für -C(=S)-NR²⁶-C(=O)-R³⁰;
oder für -(CH₂)-R³¹ steht;
R⁴ für -C(=O)-NH₂;
für einen Wasserstoff-Rest oder
für einen Alkyl-Rest ausgewählt aus der Gruppe bestehend aus Methyl, Ethyl, n-Propyl, Isopropyl und n-Butyl steht;
R⁵ für einen Alkyl-Rest ausgewählt aus der Gruppe bestehend aus Methyl, Ethyl, n-Propyl, Isopropyl, n-Butyl, sec-Butyl, Isobutyl und tert-Butyl steht;
R⁶, R⁷ und R¹⁶, unabhängig voneinander, jeweils für einen Wasserstoff-Rest oder für einen Alkyl-Rest ausgewählt aus der Gruppe bestehend aus Methyl, Ethyl, n-Propyl und Isopropyl stehen;
R⁸ für einen (hetero)cycloaliphatischen Rest ausgewählt aus der Gruppe bestehend aus Cyclopropyl, Cyclobutyl, Cyclopentyl, Cyclohexyl, Pyrrolidinyl, Piperidinyl, Morpholinyl und Thiomorpholinyl steht
oder für einen Rest ausgewählt aus der Gruppe bestehend aus Phenyl, Naphthyl, Thiophenyl, Furanyl, Pyridinyl und Indolyl steht, wobei der Rest jeweils mit 1, 2 oder 3 Substituenten unabhängig voneinander ausgewählt aus der Gruppe bestehend aus F, Cl, Br, -CF₃, -O-CH₃, -O-C₂H₅, -O-CH₂-CH=CH₂, Methyl, Ethyl, n-Propyl, Isopropyl, n-Butyl, tert-Butyl, -S-CH₃, -S-C₂H₅, -S(=O)₂-NH₂ und -S(=O)₂-NH-CH₃ substituiert sein kann;
R⁹ für einen Rest ausgewählt aus der Gruppe bestehend aus Phenyl, Naphthyl, Thiophenyl, Furanyl und Pyridinyl steht;
R¹⁰ für einen ggf. substituierten Alkyl-Rest ausgewählt aus der Gruppe bestehend aus -(CH₂)-N(CH₃)₂, -(CH₂)-(CH₂)-N(CH₃)₂, -(CH₂)-(CH₂)-(CH₂)-N(CH₃)₂, -(CH₂)-N(C₂H₅)₂, -(CH₂)-(CH₂)-N(C₂H₅)₂ und-(CH₂)-(CH₂)-(CH₂)-N(C₂H₅)₂;
für einen cycloaliphatischen Rest ausgewählt aus der Gruppe bestehend aus Piperazinyl, Pyrrolidinyl, Piperidinyl, Morpholinyl, Cyclopropyl, Cyclobutyl, Cyclopentyl und Cyclohexyl steht, wobei der cycloaliphatische Rest über eine -(CH₂)- oder -(CH₂)-(CH₂)- oder-(CH₂)-(CH₂)-(CH₂)-Gruppe gebunden sein kann;
oder für einen Rest ausgewählt aus der Gruppe bestehend aus Thiazolyl, Phenyl, Naphthyl und Thieno[2,3-d]pyrimidinyl steht, wobei der Rest jeweils mit 1, 2 oder 3 Substituenten unabhängig voneinander ausgewählt aus der Gruppe bestehend aus F, Cl, Br, Methyl, Ethyl, n-Propyl, -C(=O)-CH3 und -C(=O)-C₂H₅ substituiert und/oder über eine-(CH₂)-, -CH(CH₃)-, -(CH₂)-(CH₂)-, -(CH₂)-(CH₂)-(CH₂)- oder -(CH₂)-(CH=CH)-Gruppe gebunden sein kann;
R¹¹ für einen ggf. substituierten Alkyl-Rest ausgewählt aus der Gruppe bestehend aus Methyl, Ethyl, n-Propyl, -CF₃ und -CF₂-CF₃ steht,
oder für einen Rest ausgewählt aus der Gruppe bestehend aus Phenyl, und Naphthyl steht, wobei der Rest jeweils mit 1, 2 oder 3 Substituenten unabhängig voneinander ausgewählt aus der Gruppe bestehend aus F, Cl und Br substituiert und/oder über eine -(CH₂)- oder -(CH₂)-(CH₂)-Gruppe gebunden sein kann;
R¹² für einen ggf. substituierten Alkyl-Rest ausgewählt aus der Gruppe bestehend aus Methyl, Ethyl, n-Propyl, -CF₃ und -CF₂-CF₃ steht;
R¹³ für einen Wasserstoff-Rest steht;
R¹⁴ für einen Rest ausgewählt aus der Gruppe bestehend aus Phenyl und Naphthyl steht, wobei der Rest jeweils mit 1, 2 oder 3 Substituenten unabhängig voneinander ausgewählt aus der Gruppe bestehend aus F, Cl, Br, -CN, -CF₃, -O-CH₃, -O-C₂H₅, -O-CH(CH₃)₂, -O-CH₂-CH₂-CH₂-CH₃, -NO₂, -O-CF₃, -O-CHF₂, -O-CH₂F, -S-CF₃, -S-CHF₂, -S-CH₂F, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-CH₃ und -C(=O)-C₂H₅ substituiert und/oder über eine -(CH₂)-, -CH(CH₃)- oder -(CH₂)-(CH₂)-Gruppe gebunden sein kann;
R¹⁵ für einen Rest ausgewählt aus der Gruppe bestehend aus 1-Butenyl, 2-Butenyl und 3-Butenyl steht;
für einen 4,7,7-Trimethyl-3-oxo-2-oxa-bicyclo[2,2,1]heptyl-Rest steht
oder für einen Rest ausgewählt aus der Gruppe bestehend aus Phenyl, Naphthyl, Thiophenyl, Furanyl, Triazolyl, Pyridinyl, Pyrazolyl, Chinolinyl und Isochinolinyl steht, wobei der Rest jeweils mit 1, 2 oder 3 Substituenten unabhängig voneinander ausgewählt aus der Gruppe bestehend aus F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, -S-CF₃, -S-CHF₂, -S-CH₂F, Methyl, Ethyl, n-Propyl, Isopropyl, n-Butyl, sec-Butyl, Isobutyl, tert-Butyl, Phenyl, -NH-C(=O)-CH₃ und -NH-C(=O)-C₂H₅ substituiert sein kann;
R¹⁷ und R¹⁸, unabhängig voneinander, jeweils für einen Methyl- oder Ethyl-Rest stehen;
R¹⁹ für einen Phenyl-Rest steht, der über eine -(CH₂)-, -(CH₂)-(CH₂)- oder-(CH₂)-O-(CH₂)-Gruppe gebunden sein kann;
R²⁰ für einen Alkyl-Rest ausgewählt aus der Gruppe bestehend aus Methyl, Ethyl, n-Propyl, Isopropyl, n-Butyl, sec-Butyl, Isobutyl und tert-Butyl steht
oder für einen Benzyl-Rest steht;
R²¹ für einen cycloaliphatischen Rest ausgewählt aus der Gruppe bestehend aus Cyclopropyl, Cyclobutyl, Cyclopentyl, Cyclohexyl, Cycloheptyl steht, wobei der cycloaliphatische Rest jeweils mit 1, 2 oder 3 Substituenten unabhängig voneinander ausgewählt aus der Gruppe bestehend aus -C(=O)-OH und -(CH₂)-C(=O)-OH substituiert sein kann,
oder für einen Rest ausgewählt aus der Gruppe bestehend aus Phenyl, Naphthyl, (1,3)-Benzodioxolyl und (1,4)-Benzodioxanyl steht, wobei der Rest jeweils mit 1, 2 oder 3 Substituenten unabhängig voneinander ausgewählt aus der Gruppe bestehend aus F, Cl, Br, -O-CH₃ und -O-C₂H₅ substituiert sein kann;
R²² für einen Phenyl-Rest steht;
R²³ für einen Rest ausgewählt aus der Gruppe bestehend aus Phenyl, Naphthyl, Thiophenyl, Furanyl und Pyridinyl steht, wobei der Rest jeweils mit 1, 2 oder 3 Substituenten unabhängig voneinander ausgewählt aus der Gruppe bestehend aus F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, Methyl, Ethyl, n-Propyl, Isopropyl, n-Butyl, sec-Butyl, Isobutyl und tert-Butyl substituiert sein kann;
R²⁴ und R²⁵, unabhängig voneinander, jeweils für einen Methyl- oder Ethyl-Rest stehen;
R²⁶ für einen Wasserstoff-Rest steht;
R²⁷ für einen Alkyl-Rest ausgewählt aus der Gruppe bestehend aus Methyl, Ethyl, n-Propyl, lsopropyl und n-Butyl steht;
für einen Cyclohexyl-Rest steht
oder für einen Phenyl-Rest steht, der mit 1, 2, 3, 4 oder 5 Substituenten unabhängig voneinander ausgewählt aus der Gruppe bestehend aus F, Cl, Br, -O-CH₃ und -O-C₂H₅ substituiert sein kann;
R²⁸ für einen Phenyl-Rest steht, der mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃,-O-CHF₂, -O-CH₂F, -S-CF₃, -S-CHF₂, -S-CH₂F, -SH, -S-CH₃, -S-C₂H₅, Methyl, Ethyl, n-Propyl, Isopropyl, n-Butyl, sec-Butyl, Isobutyl, tert-Butyl substituiert sein kann;
R²⁹ für einen Alkyl-Rest ausgewählt aus der Gruppe bestehend aus Methyl, Ethyl, n-Propyl, Isopropyl, n-Butyl, Isobutyl und tert-Butyl steht; R³⁰ für einen Phenyl-Rest steht
und
R³¹ für einen Rest ausgewählt aus der Gruppe bestehend aus Phenyl und Naphthyl steht, wobei der Rest jeweils mit 1, 2 oder 3 Substituenten unabhängig voneinander ausgewählt aus der Gruppe bestehend aus F, Cl und Br substituiert sein kann;
Jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

5. Verbindungen gemäß einem oder mehreren der Ansprüche 1 bis 4 ausgewählt aus der Gruppe bestehend aus
[1] 6,7-Dihydro-4H-isoxazolo[4,5-c]pyridin-3,5-dicarbonsäure 3-[(2-methoxy-ethyl)-amid] 5-[(3-methoxy-phenyl)-amid],
[2] 6,7-Dihydro-4H-isoxazolo[4,5-c]pyridin-3,5-dicarbonsäure 3-cyclopentylamid 5-(4-fluor-benzylamid),
[3] 6,7-Dihydro-4H-isoxazolo[4,5-c]pyridin-3,5-dicarbonsäure 3-phenylamid 5-[(4-trifluormethoxy-phenyl)-amid],
[4] 6,7-Dihydro-4H-isoxazolo[4,5-c]pyridin-3,5-dicarbonsäure 5-[(4-methyl-3-nitro-phenyl)-amid] 3-(phenethyl-amid),
[5] 6,7-Dihydro-4H-isoxazolo[4,5-c]pyridin-3,5-dicarbonsäure 3-[(2-methoxy-ethyl)-amid] 5-[(4-methyl-3-nitro-phenyl)-amid],
[6] 3-{[5-(2,5-Difluor-phenylcarbamoyl)-4,5,6,7-tetrahydro-isoxazolo[4,5-c]pyridin-3-carbonyl]-amino}-propionsäure ethyl ester,
[7] 6,7-Dihydro-4H-isoxazolo[4,5-c]pyridin-3,5-dicarbonsäure 5-[(4-butoxy-phenyl)-amid] 3-[(2-methoxy-ethyl)-amid],
[8] 6,7-Dihydro-4H-isoxazolo[4,5-c]pyridin-3,5-dicarbonsäure 5-[(3-fluor-phenyl)-amid] 3-(phenethyl-amid),
[9] 6,7-Dihydro-4H-isoxazolo[4,5-c]pyridin-3,5-dicarbonsäure 5-[(4-methyl-3-nitro-phenyl)-amid] 3-[(thiophen-2-ylmethyl)-amid],
[10] 6,7-Dihydro-4H-isoxazolo[4,5-c]pyridin-3,5-dicarbonsäure 3-benzylamid 5-(phenethyl-amid),
[11] 6,7-Dihydro-4H-isoxazolo[4,5-c]pyridin-3,5-dicarbonsäure 3-[(2-ethylsulfanyl-ethyl)-amid] 5-[(3-methoxy-phenyl)-amid],
[12] 6,7-Dihydro-4H-isoxazolo[4,5-c]pyridin-3,5-dicarbonsäure 5-[(3-cyano-phenyl)-amid] 3-[(thiophen-2-ylmethyl)-amid],
[13] 6,7-Dihydro-4H-isoxazolo[4,5-c]pyridin-3,5-dicarbonsäure 5-[(4-ethoxy-phenyl)-amid] 3-phenylamid,
[14] 6,7-Dihydro-4H-isoxazolo[4,5-c]pyridin-3,5-dicarbonsäure 3-(4-fluor-benzylamid) 5-[(4-methyl-3-nitro-phenyl)-amid],
[15] 6,7-Dihydro-4H-isoxazolo[4,5-c]pyridin-3,5-dicarbonsäure 5-[(3-acetyl-phenyl)-amid] 3-[(5-methyl-furan-2-ylmethyl)-amid],
[16] 6,7-Dihydro-4H-isoxazolo[4,5-c]pyridin-3,5-dicarbonsäure 5-[(4-methyl-3-nitro-phenyl)-amid] 3-prop-2-ynylamid,
[17] 6,7-Dihydro-4H-isoxazolo[4,5-c]pyridin-3,5-dicarbonsäure 3-benzylamid 5-phenylamid,
[18] 6,7-Dihydro-4H-isoxazolo[4,5-c]pyridin-3,5-dicarbonsäure 5-[(1-naphthalen-1-yl-ethyl)-amid] 3-(phenethyl-amid),
[19] 6,7-Dihydro-4H-isoxazolo[4,5-c]pyridin-3,5-dicarbonsäure 5-[(3-fluor-phenyl)-amid] 3-prop-2-ynylamid,
[20] 3-{[5-(2,5-Dimethoxy-phenylcarbamoyl)-4,5,6,7-tetrahydro-isoxazolo[4,5-c]pyridin-3-carbonyl]-amino}-propionsäure ethyl ester,
[21] 6,7-Dihydro-4H-isoxazolo[4,5-c]pyridin-3,5-dicarbonsäure 5-[(4-ethoxy-phenyl)-amid] 3-(4-fluor-benzylamid),
[22] 6,7-Dihydro-4H-isoxazolo[4,5-c]pyridin-3,5-dicarbonsäure 5-[(5-chlor-2-methoxy-phenyl)-amid] 3-(phenethyl-amid),
[23] 3-({3-[(5-Methyl-furan-2-ylmethyl)-carbamoyl]-6,7-dihydro-4H-isoxazolo[4,5-c]pyridin-5-carbonyl}-amino)-benzoesäure ethyl ester,
[24] 6,7-Dihydro-4H-isoxazolo[4,5-c]pyridin-3,5-dicarbonsäure 5-[(5-chlor-2-methoxy-phenyl)-amid] 3-(isobutyl-amid),
[25] [2-Oxo-2-(3-prop-2-ynylcarbamoyl-6,7-dihydro-4H-isoxazolo[4,5-c]pyridin-5-yl)-ethoxy]-essigsäure,
[26] 3-Ethyl-5-[3-(2-methoxy-ethylcarbamoyl)-6,7-dihydro-4H-isoxazolo[4,5-c]pyridin-5-yl]-3-methyl-5-oxo-pentansäure,
[27] (1-Benzyloxymethyl-2-oxo-2-{3-[(pyridin-3-ylmethyl)-carbamoyl]-6,7-dihydro-4H-isoxazolo[4,5-c]pyridin-5-yl}-ethyl)-carbaminsäure tert-butyl ester,
[28] {1-[2-Oxo-2-(3-phenylcarbamoyl-6,7-dihydro-4H-isoxazolo[4,5-c]pyridin-5-yl)-ethyl]-cyclopentyl}-essigsäure,
[29] 3-Ethyl-3-methyl-5-oxo-5-(3-phenylcarbamoyl-6,7-dihydro-4H-isoxazolo[4,5-c]pyridin-5-yl)-pentansäure,
[30] (2-Oxo-2-{3-[(thiophen-2-ylmethyl)-carbamoyl]-6,7-dihydro-4h-isoxazolo[4,5-c]pyridin-5-yl}ethoxy)-essigsäure,
[31] 3,3-Dimethyl-4-{3-[(5-methyl-furan-2-ylmethyl)-carbamoyl]-6,7-dihydro-4H-isoxazolo[4,5-c]pyridin-5-yl}-4-oxo-butansäure,
[32] 5-(4-Trifluormethoxy-benzolsulfonyl)-4,5,6,7-tetrahydro-isoxazolo[4,5-c]pyridin-3-carbonsäure benzyl-phenethyl-amid,
[33] 5-Benzolsulfonyl-4,5,6,7-tetrahydro-isoxazolo[4,5-c]pyridin-3-carbonsäure (1-naphthalen-2-ylmethyl-pyrrolidin-3-yl)-amid,
[34] [5-(4-Fluor-benzolsulfonyl)-4,5,6,7-tetrahydro-isoxazolo[4,5-c]pyridin-3-yl]-(4-thieno[2,3-d]pyrimidin-4-yl-piperazin-1-yl)methanon,
[35] 6-(4-Fluor-benzolsulfonyl)-4,5,6,7-tetrahydro-isoxazolo[4,5-c]pyridin-3-carbonsäure (3-phenyl-propyl)-amid,
[36] 5-(3-Phenyl-acryloyl)-4,5,6,7-tetrahydro-isoxazolo[4,5-c]pyridin-3-carbonsäure (2-dimethylamino-ethyl)-amid,
[37] 5-(2-Phenoxy-acetyl)-4,5,6,7-tetrahydro-isoxazolo[4,5-c]pyridin-3-carbonsäure (2-dimethylamino-ethyl)-amid,
[38] 5-(4-Fluor-benzolsulfonyl)-4,5,6,7-tetrahydro-isoxazolo[4,5-c]pyridin-3-carbonsäure (1-methyl-3-phenyl-propyl)-amid,
[39] 5-(4-Fluor-benzolsulfonyl)-4,5,6,7-tetrahydro-isoxazolo[4,5-c]pyridin-3-carbonsäure [1-(4-trifluormethyl-benzyl)-pyrrolidin-3-yl]-amid,
[40] 5-(2,5-Dichlor-benzolsulfonyl)-4,5,6,7-tetrahydro-isoxazolo[4,5-c]pyridin-3-carbonsäure (2-benzyloxy-cyclopentyl)-amid,
[41] 5-(5-tert-Butyl-2-methyl-2H-pyrazole-3-carbonyl)-4,5,6,7-tetrahydro-isoxazolo[4,5-c]pyridin-3-carbonsäure (2-dimethylamino-ethyl)-amid,
[42] [5-(4-Fluor-benzolsulfonyl)-4,5,6,7-tetrahydro-isoxazolo[4,5-c]pyridin-3-yl]-[4-(4-fluor-phenyl)-3,6-dihydro-2H-pyridin-1-yl]-methanon,
[43] 4-{[5-(4-Fluor-benzolsulfonyl)-4,5,6,7-tetrahydro-isoxazolo[4,5-c]pyridin-3-carbonyl]-amino}-piperidin-1-carbonsäure ethyl ester,
[44] 5-(3-Phenyl-acryloyl)-4,5,6,7-tetrahydro-isoxazolo[4,5-c]pyridin-3-carbonsäure cyclopentylamid,
[45] 5-(4-Acetylamino-benzoyl)-4,5,6,7-tetrahydro-isoxazolo[4,5-c]pyridin-3-carbonsäure cyclopropylmethyl-amid,
[46] 5-(5-Methyl-2-phenyl-2H-[1,2,3]triazol-4-carbonyl)-4,5,6,7-tetrahydro-isoxazolo[4,5-c]pyridin-3-carbonsäure (pyridin-3-ylmethyl)-amid,
[47] 5-Benzolsulfonyl-4,5,6,7-tetrahydro-isoxazolo[4,5-c]pyridin-3-carbonsäure 3,4-dimethoxy-benzylamid,
[48] 2-(3,4-Difluor-phenyl)-1-{4-[5-(4-fluor-benzolsulfonyl)-4,5,6,7-tetrahydro-isoxazolo[4,5-c]pyridin-3-carbonyl]-piperazin-1-yl}-ethanon,
[49] 3-(Morpholin-4-carbonyl)-6,7-dihydro-4H-isoxazolo[4,5-c]pyridin-5-sulfonsäure dimethylamid,
[50] 5-(Furan-2-carbonyl)-4,5,6,7-tetrahydro-isoxazolo[4,5-c]pyridin-3-carbonsäure (thiophen-2-ylmethyl)-amid,
[51] 5-(4,7,7-Trimethyl-3-oxo-2-oxa-bicyclo[2.2.1]heptan-1-carbonyl)-4,5,6,7-tetrahydro-isoxazolo[4,5-c]pyridin-3-carbonsäure (2-pyrrolidin-1-yl-ethyl)-amid,
[52] 5-(2,5-Dichlor-benzolsulfonyl)-4,5,6,7-tetrahydro-isoxazolo[4,5-c]pyridin-3-carbonsäure [2-(2-chlor-phenyl)-ethyl]-amid,
[53] 5-(4-Methoxy-benzolsulfonyl)-4,5,6,7-tetrahydro-isoxazolo[4,5-c]pyridin-3-carbonsäure p-tolyl-amid,
[54] 5-(2-Phenoxy-acetyl)-4,5,6,7-tetrahydro-isoxazolo[4,5-c]pyridin-3-carbonsäure 4-fluor-benzylamid,
[55] 1-{A-[5-(4-Methoxy-benzolsulfonyl)-4,5,6,7-tetrahydro-isoxazolo[4,5-c]pyridin-3-carbonyl]-piperazin-1-yl}-ethanon,
[56] 5-(Toluol-4-sulfonyl)-4,5,6,7-tetrahydro-isoxazolo[4,5-c]pyridin-3-carbonsäure 4-sulfamoyl-benzylamid,
[57] 5-(Thiophen-2-sulfonyl)-4,5,6,7-tetrahydro-isoxazolo[4,5-c]pyridin-3-carbonsäure (2-morpholin-4-yl-ethyl)-amid,
[58] 5-(4-Methoxy-benzoyl)-4,5,6,7-tetrahydro-isoxazolo[4,5-c]pyridin-3-carbonsäure (2-dimethylamino-ethyl)-amid,
[59] 5-(2,5-Dichlor-benzolsulfonyl)-4,5,6,7-tetrahydro-isoxazolo[4,5-c]pyridin-3-carbonsäure benzyl-phenethyl-amid,
[60] 5-Dimethylsulfamoyl-4,5,6,7-tetrahydro-isoxazolo[4,5-c]pyridin-3-carbonsäure (2,6,6-trimethyl-bicyclo[3.1.1]hept-3-yl)-amid,
[61] 5-Benzolsulfonyl-4,5,6,7-tetrahydro-isoxazolo[4,5-c]pyridin-3-carbonsäure indan-1-ylamid,
[62] 5-(4-Methoxy-benzolsulfonyl)-4,5,6,7-tetrahydro-isoxazolo[4,5-c]pyridin-3-carbonsäure (3-methoxy-propyl)-amid,
[63] 5-(4,5-Dichlor-thiophen-2-sulfonyl)-4,5,6,7-tetrahydro-isoxazolo[4,5-c]pyridin-3-carbonsäure (2-morpholin-4-yl-ethyl)-amid,
[64] 5-(2-Benzyloxy-acetyl)-4,5,6,7-tetrahydro-isoxazolo[4,5-c]pyridin-3-carbonsäure (2-pyrrolidin-1-yl-ethyl)-amid,
[65] [5-(2,5-Dichlor-benzolsulfonyl)-4,5,6,7-tetrahydro-isoxazolo[4,5-c]pyridin-3-yl]-[4-(2,4-dimethyl-phenyl)-piperazin-1-yl]-methanon,
[66] 5-Pent-4-enoyl-4,5,6,7-tetrahydro-isoxazolo[4,5-c]pyridin-3-carbonsäure cyclopropylmethyl-amid,
[67] 3-Methyl-2-{[5-(3-trifluormethyl-benzolsulfonyl)-4,5,6,7-tetrahydro-isoxazolo[4,5-c]pyridin-3-carbonyl]-amino}-butansäure tert-butyl ester,
[68] 5-(3-Trifluormethyl-benzolsulfonyl)-4,5,6,7-tetrahydro-isoxazolo[4,5-c]pyridin-3-carbonsäure benzyl-phenethyl-amid,
[69] 5-(3-Trifluormethyl-benzolsulfonyl)-4,5,6,7-tetrahydro-isoxazolo[4,5-c]pyridin-3-carbonsäure 2,4-dichlor-benzylamid,
[70] 5-Dimethylsulfamoyl-4,5,6,7-tetrahydro-isoxazolo[4,5-c]pyridin-3-carbonsäure (2-cyano-ethyl)-pyridin-3-ylmethyl-amid,
[71] 5-(2-Cyclopentyl-acetyl)-4,5,6,7-tetrahydro-isoxazolo[4,5-c]pyridin-3-carbonsäure (2-pyrrolidin-1-yl-ethyl)-amid,
[72] 5-(5-Methyl-2-phenyl-2H-[1,2,3]triazol-4-carbonyl)-4,5,6,7-tetrahydro-isoxazolo[4,5-c]pyridin-3-carbonsäure 4-fluor-benzylamid,
[73] 5-(3-Trifluormethyl-benzolsulfonyl)-4,5,6,7-tetrahydro-isoxazolo[4,5-c]pyridin-3-carbonsäure (2-ethyl-hexyl)-amid,
[74] 5-(2,3-Difluor-4-methyl-benzoyl)-4,5,6,7-tetrahydro-isoxazolo[4,5-c]pyridin-3-carbonsäure (2-dimethylamino-ethyl)-amid,
[75] 5-(4-Trifluormethoxy-benzolsulfonyl)-4,5,6,7-tetrahydro-isoxazolo[4,5-c]pyridin-3-carbonsäure 2,3-dichlor-benzylamid,
[76] 5-Dimethylsulfamoyl-4,5,6,7-tetrahydro-isoxazolo[4,5-c]pyridin-3-carbonsäure [2-(4-chlor-phenyl)-ethyl]-amid,
[77] 5-(6-Chlor-pyridin-3-carbonyl)-4,5,6,7-tetrahydro-isoxazolo[4,5-c]pyridin-3-carbonsäure (2-pyrrolidin-1-yl-ethyl)-amid,
[78] 5-[3-(4-Fluor-benzylcarbamoyl)-6,7-dihydro-4H-isoxazolo[4,5-c]pyridin-5-yl]-5-oxo-pentansäure methyl ester,
[79] 5-[2-(4-Methoxy-phenyl)-acetyl]-4,5,6,7-tetrahydro-isoxazolo[4,5-c]pyridin-3-carbonsäure benzylamid,
[80] 5-(4-Trifluormethoxy-benzolsulfonyl)-4,5,6,7-tetrahydro-isoxazolo[4,5-c]pyridin-3-carbonsäure [3-(methyl-phenyl-amino)-propyl]-amid,
[81] 5-[2-(4-Chlor-phenoxy)-acetyl]-4,5,6,7-tetrahydro-isoxazolo[4,5-c]pyridin-3-carbonsäure cyclopentylamid,
[82] [4-(2-Cyclohexyl-ethyl)-piperazin-1-yl]-[5-(toluol-4-sulfonyl)-4,5,6,7-tetrahydro-isoxazolo[4,5-c]pyridin-3-yl]-methanon,
[83] N-{1-[5-(2,5-Dichlor-benzolsulfonyl)-4,5,6,7-tetrahydro-isoxazolo[4,5-c]pyridin-3-carbonyl]-piperidin-4-ylmethyl}-2,2,2-trifluor-acetamid,
[84] 5-(4-Methoxy-benzolsulfonyl)-4,5,6,7-tetrahydro-isoxazolo[4,5-c]pyridin-3-carbonsäure [1-(4-methoxy-phenyl)-ethyl]-amid,
[85] 2-{[5-(Thiophen-2-sulfonyl)-4,5,6,7-tetrahydro-isoxazolo[4,5-c]pyridin-3-carbonyl]-amino}-propionsäure ethyl ester,
[86] 1-(4-{4-[5-(Toluol-4-sulfonyl)-4,5,6,7-tetrahydro-isoxazolo[4,5-c]pyridin-3-carbonyl]-piperazin-1-yl}-phenyl)-ethanon,
[87] 5-(3-Trifluormethyl-benzolsulfonyl)-4,5,6,7-tetrahydro-isoxazolo[4,5-c]pyridin-3-carbonsäure (4-allyloxy-benzyl)-furan-2-ylmethyl-amid,
[88] 4-Carbamoyl-4-{[5-(4-methoxy-benzolsulfonyl)-4,5,6,7-tetrahydro-isoxazolo[4,5-c]pyridin-3-carbonyl]-amino}-butansäure tert-butyl ester,
[89] 5-Dimethylsulfamoyl-4,5,6,7-tetrahydro-isoxazolo[4,5-c]pyridin-3-carbonsäure furan-2-ylmethyl-(4-methylsulfanyl-benzyl)-amid,
[90] 5-(4-Bromo-3-methyl-benzoyl)-4,5,6,7-tetrahydro-isoxazolo[4,5-c]pyridin-3-carbonsäure (2-morpholin-4-yl-ethyl)-amid,
[91] 5-(Toluol-4-sulfonyl)-4,5,6,7-tetrahydro-isoxazolo[4,5-c]pyridin-3-carbonsäure [1-(2,6-dichlor-benzyl)-pyrrolidin-3-yl]-amid,
[92] 5-Benzolsulfonyl-4,5,6,7-tetrahydro-isoxazolo[4,5-c]pyridin-3-carbonsäure [2-(3,4-dlchlor-phenyl)-ethyl]-amid,
[93] 5-(3-Difluormethylsulfanyl-benzoyl)-4,5,6,7-tetrahydro-isoxazolo[4,5-c]pyridin-3-carbonsäure (2-dimethylamino-ethyl)-amid,
[94] 5-[2-(3-Chlor-phenoxy)-acetyl]-4,5,6,7-tetrahydro-isoxazolo[4,5-c]pyridin-3-carbonsäure (2-pyrrolidin-1-yl-ethyl)-amid,
[95] 5-(4-Methoxy-benzolsulfonyl)-4,5,6,7-tetrahydro-isoxazolo[4,5-c]pyridin-3-carbonsäure 2,3-dimethoxy-benzylamid,
[96] 5-(4-Trifluormethoxy-benzolsulfonyl)-4,5,6,7-tetrahydro-isoxazolo[4,5-c]pyridin-3-carbonsäure (1-methyl-3-phenyl-propyl)-amid,
[97] (5-Benzolsulfonyl-4,5,6,7-tetrahydro-isoxazolo[4,5-c]pyridin-3-yl)-[4-(3-phenyl-allyl)-piperazin-1-yl]-methanon,
[98] 3-{[5-(4-Methoxy-benzoyl)-4,5,6,7-tetrahydro-isoxazolo[4,5-c]pyridin-3-carbonyl]-amino}-propionsäure ethyl ester,
[99] 5-(4-Phenoxy-butyryl)-4,5,6,7-tetrahydro-isoxazolo[4,5-c]pyridin-3-carbonsäure (2-dimethylamino-ethyl)-amid,
[100] 5-(4-Trifluormethoxy-benzolsulfonyl)-4,5,6,7-tetrahydro-isoxazolo[4,5-c]pyridin-3-carbonsäure [2-(7-methyl-1H-indol-3-yl)-ethyl]-amid,
[101] [4-(3-Phenyl-allyl)-piperazin-1-yl]-[5-(thiophen-2-sulfonyl)-4,5,6,7-tetrahydro-isoxazolo[4,5-c]pyridin-3-yl]-methanon,
[102] 5-(4-Bromo-3-methyl-benzoyl)-4,5,6,7-tetrahydro-isoxazolo[4,5-c]pyridin-3-carbonsäure (2-pyrrolidin-1-yl-ethyl)-amid,
[103] 5-Benzolsulfonyl-4,5,6,7-tetrahydro-isoxazolo[4,5-c]pyridin-3-carbonsäure [1-(4-trifluormethyl-benzyl)-pyrrolidin-3-yl]-amid,
[104] 5-(Thiophen-2-sulfonyl)-4,5,6,7-tetrahydro-isoxazolo[4,5-c]pyridin-3-carbonsäure (2-phenyl-propyl)-amid,
[105] 5-(3-Chlor-4-fluor-benzoyl)-4,5,6,7-tetrahydro-isoxazolo[4,5-c]pyridin-3-carbonsäure phenethyl-amid,
[106] 5-Benzolsulfonyl-4,5,6,7-tetrahydro-isoxazolo[4,5-c]pyridin-3-carbonsäure [2-(4-methoxy-phenoxy)-ethyl]-amid,
[107] 5-Dimethylsulfamoyl-4,5,6,7-tetrahydro-isoxazolo[4,5-c]pyridin-3-carbonsäure 3-fluor-4-trifluormethyl-benzylamid,
[108] 5-(3-Methyl-benzoyl)-4,5,6,7-tetrahydro-isoxazolo[4,5-c]pyridin-3-carbonsäure prop-2-ynylamid,
[109] 5-(Toluol-4-sulfonyl)-4,5,6,7-tetrahydro-isoxazolo[4,5-c]pyridin-3-carbonsäure [2-(4-methoxy-phenoxy)-ethyl]-amid,
[110] [4-(2-Chlor-phenyl)-piperazin-1-yl]-[5-(2,5-dichlor-benzolsulfonyl)-4,5,6,7-tetrahydro-isoxazolo[4,5-c]pyridin-3-yl]-methanon,
[111] 3-{[5-(2,6-Difluor-3-methyl-benzoyl)-4,5,6,7-tetrahydro-isoxazolo[4,5-c]pyridin-3-carbonyl]-amino}-propionsäure ethyl ester,
[112] 5-(2,5-Dichror-benzolsulfonyl)-4,5,6,7-tetrahydro-isoxazolo[4,5-c]pyridin-3-carbonsäure 2,4-difluor-benzylamid,
[113] 5-Benzolsulfonyl-4,5,6,7-tetrahydro-isoxazolo[4,5-c]pyrldin-3-carbonsäure 3-fluor-4-trifluormethyl-benzylamid,
[114] 5-(4-Butoxy-benzolsulfonyl)-4,5,6,7-tetrahydro-isoxazolo[4,5-c]pyridin-3-carbonsäure isobutyl-amid,
[115] 5-(3-Fluor-4-methyl-benzoyl)-4,5,6,7-tetrahydro-isoxazolo[4,5-c]pyridin-3-carbonsäure (2-pyrrolidin-1-yl-ethyl)-amid,
[116] 5-(2-Chlor-5-trifluormethyl-benzoyl)-4,5,6,7-tetrahydro-isoxazolo[4,5-c]pyridin-3-carbonsäure isobutyl-amid,
[117] 5-(4-Trifluormethoxy-benzolsulfonyl)-4,5,6,7-tetrahydro-isoxazolo[4,5-c]pyridin-3-carbonsäure (1-benzyl-pyrrolidin-3-yl)-amid,
[118] 5-(3-Chlor-benzolsulfonyl)-4,5,6,7-tetrahydro-isoxazolo[4,5-c]pyridin-3-carbonsäure (2-pyrrolidin-1-yl-ethyl)-amid,
[119] 5-(3-Trifluormethoxy-benzoyl)-4,5,6,7-tetrahydro-isoxazolo[4,5-c]pyridin-3-carbonsäure (2-dimethylamino-ethyl)-amid,
[120] 2-{[5-(Toluol-4-sulfonyl)-4,5,6,7-tetrahydro-isoxazolo[4,5-c]pyridin-3-carbonyl]-amino}-propionsäure tert-butyl ester,
[121] 3-{[5-(4-Chlor-benzolsulfonyl)-4,5,6,7-tetrahydro-isoxazolo[4,5-c]pyridin-3-carbonyl]-amino}-propionsäure ethyl ester,
[122] 5-Dimethylsulfamoyl-4,5,6,7-tetrahydro-isoxazolo[4,5-c]pyridin-3-carbonsäure [2-(3,4-dichlor-phenyl)-ethyl]-amid,
[123] 5-(Thiophen-2-sulfonyl)-4,5,6,7-tetrahydro-isoxazolo[4,5-c]pyridin-3-carbonsäure cyclopropylmethyl-amid,
[124] 5-(Chinolin-6-carbonyl)-4,5,6,7-tetrahydro-isoxazolo[4,5-c]pyridin-3-carbonsäure 4-fluor-benzylamid,
[125] 3-{[5-(2-Naphthalen-2-yl-acetyl)-4,5,6,7-tetrahydro-isoxazolo[4,5-c]pyridin-3-carbonyl]-amino}-propionsäure ethyl ester,
[126] [2-(3-Isobutylcarbamoyl-6,7-dihydro-4H-isoxazolo[4,5-c]pyridin-5-yl)-2-oxo-1-phenyl-ethyl]-carbaminsäure benzyl ester,
[127] 5-(4-Methoxy-benzylthiocarbamoyl)-4,5,6,7-tetrahydro-isoxazolo[4,5-c]pyridin-3-carbonsäure cyclopentylamid,
[128] 5-Benzoylaminocarbothioyl-4,5,6,7-tetrahydro-isoxazolo[4,5-c]pyridin-3-carbonsäure (2-pyrrolidin-1-yl-ethyl)-amid,
[129] 5-(4-Chlor-benzylthiocarbamoyl)- 4,5,6,7-tetrahydro-isoxazolo[4,5-c]pyridin-3-carbonsäure-cyclopropylmethyl-amid,
[130] 5-(2-Methoxy-ethylthiocarbamoyl)-4,5,6,7-tetrahydro-isoxazolo[4,5-c]pyridin-3-carbonsäure phenethyl-amid,
[131] 9-Pentafluorphenylthiocarbamoyl-4,5,6,7-tetrahydro-isoxazolo[4,5-c]pyridin-3-carbonsäure phenethyl-amid,
[132] 5-(1-Phenyl-ethylthiocarbamoyl)-4,5,6,7-tetrahydro-isoxazolo[4,5-c]pyridin-3-carbonsäure phenethyl-amid,
[133] 5-Pentafluorphenylthiocarbamoyl-4,5,6,7-tetrahydro-isoxazolo[4,5-c]pyridin-3-carbonsäure (5-methyl-furan-2-ylmethyl)-amid,
[134] 3-{[5-(Cyclohexylmethyl-thiocarbamoyl)-4,5,6,7-tetrahydro-isoxazolo[4,5-c]pyridin-3-carbonyl]-amino}-propionsäure ethyl ester,
und
[135] 5-(1-Brom-naphthalen-2-ylmethyl)-4,5,6,7-tetrahydro-isoxazolo[4,5-c]pyridin-3-carbonsäurephenylamid,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

6. Verfahren zur Herstellung substituierter 4,5,6,7-Tetrahydro-isoxazolo[4,5-c]pyridin-Verbindungen der allgemeinen Formel I gemäß einem oder mehreren der Ansprüche 1 bis 5, **dadurch gekennzeichnet, daß** wenigstens eine Verbindung der allgemeinen Formel II, 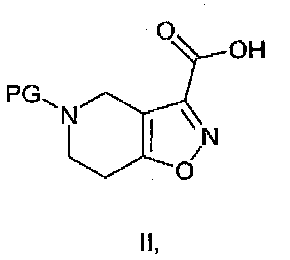 worin PG für eine Schutzgruppe, bevorzugt eine tert-Butyloxy-carbonyl- oder Benzyloxycarbonyl-Gruppe steht, in einem Reaktionsmedium, in Gegenwart wenigstens eines Kupplungsreagenzes, ggf. in Gegenwart wenigstens einer Base, bevorzugt in Gegenwart wenigstens einer Base ausgewählt aus der Gruppe bestehend aus Pyridin, N-Methylmorpholin, Diisopropylethylamin, Triethylamin und 4,4-Dimethylaminopyridin, mit wenigstens einem Amin der allgemeinen Formel HNR¹R², worin die Reste R¹ und R² die Bedeutung gemäß einem oder mehreren der Ansprüche 1 bis 5 haben, zu wenigstens einer Verbindung der allgemeinen Formel III, 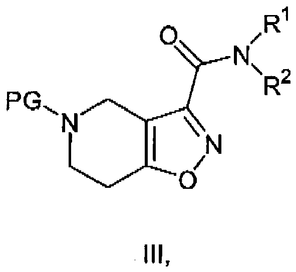 worin R¹, R² und PG die vorstehend genannte Bedeutung haben, umgesetzt wird, und diese ggf. gereinigt und/oder isoliert wird,
und wenigstens eine Verbindung der allgemeinen Formel III, durch Abspaltung in einem Reaktionsmedium, vorzugsweise in Gegenwart wenigstens einer Säure oder in Gegenwart wenigstens einer Base für die tert-Butyloxy-carbonylGruppe oder in Gegenwart von Wasserstoff und Katalysator, bevorzugt Palladium auf Kohle, für die Benzyloxycarbonyl-Gruppe zu wenigstens einer Verbindung der allgemeinen Formel IV, ggf. in Form eines entsprechenden Salzes, bevorzugt in Form eines entsprechenden Hydrochlorids, 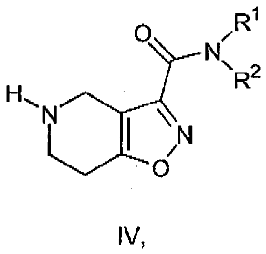 worin R¹ und R² die vorstehend genannte Bedeutung haben, umgesetzt wird, und diese ggf. gereinigt und/oder isoliert wird,
und
ggf. wenigstens eine Verbindung der allgemeinen Formel IV in Form eines entsprechenden Salzes, bevorzugt in Form eines entsprechenden Hydrochlorids, in einem Reaktionsmedium, in Gegenwart wenigstens einer Base, bevorzugt in Gegenwart wenigstens eines Metallhydroxids, besonders bevorzugt in Gegenwart von Kalium- und/oder Natriumhydroxid, zu wenigstens einer Verbindung der allgemeinen Formel IV umgesetzt wird, und diese ggf. gereinigt und/oder isoliert wird,
und
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Pyridin, Trimethylamin, 4,4-Dimethylaminopyridin, Diisopropylethylamin und Diisopropylamin, mit wenigstens einem Carbonsäure-Derivat der allgemeinen Formel LG-C(=O)-R¹⁵, LG-C(=O)-(CH₂)ⱼ-Xₖ-(CH₂)ₘ-(CH₂)ₙ-C(=O)-OR¹⁶, LG-C(=O)-(CHR¹⁹)-NH-C(=O)-OR²⁰, LG-C(=O)-(CH₂)-(CH₂)ₚ-(CH₂)_{q}-Yᵣ(CH₂)ₛ-R²¹ oder LG-C(=O)-(CH=CH)-R²², worin R¹⁵, R¹⁶ , R¹⁹, R²⁰, R²¹, R²², j, k, m, n, p, q, r, s, X und Y die Bedeutung gemäß einem oder mehreren der Ansprüche 1 bis 5 haben und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ und R² die vorstehend genannte Bedeutung haben und R³ für -C(=O)-R¹⁵, -C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂) -(CH₂)ₚ-(CH₂)_{q}-Yᵣ-(CH₂)ₛ-R²¹ oder -C(=O)-(CH=CH)-R²² steht, wobei R¹⁵, R¹⁶, R¹⁹, R²⁰, R²¹ R²², j, k, m, n, p, q, r, s, X und Y die vorstehend genannte Bedeutung haben, und diese ggf. gereinigt und/oder isoliert wird
oder
wenigstens eine Verbindung der allgemeinen Formel IV in einem Reaktionsmedium, in Gegenwart eines Kupplungsreagenzes, ggf. in Gegenwart wenigstens einer Base, bevorzugt in Gegenwart wenigstens einer Base ausgewählt aus der Gruppe bestehend aus Pyridin, N-Methylmorpholin, Diisopropylethylamin, Triethylamin und 4,4-Dimethylaminopyridin, mit wenigstens einer Carbonsäure der allgemeinen Formel HO-C(=O)-R¹⁵, HO-C(=O)-(CH₂)ⱼ-Xₖ-(CH₂)ₘ-(CH₂)ₙ-C(=O)-OR¹⁶, HO-C(=O)-(CHR¹⁹)-NH-C(=O)-OR²⁰, HO-C(=O)-(CH₂)-(CH₂)ₚ-(CH₂)_{q}-Yᵣ-(CH₂)ₛ-R²¹ oder HO-C(=O)-(CH=CH)-R²², worin R¹⁵, R¹⁶, R¹⁹, R²⁰, R²¹, R²², j, k, m, n, p, q, r, s, X und Y die Bedeutung gemäß einem oder mehreren der Ansprüche 1 bis 5 haben, zu wenigstens einer Verbindung der allgemeinen Formel I umgesetzt wird, worin R¹ und R² die vorstehend genannte Bedeutung haben und R³ für -C(=O)-R¹⁵,-C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂)-(CH₂)ₚ-(CH₂)_{q}-Yᵣ-(CH₂)ₛ-R²¹ oder -C(=O)-(CH=CH)-R²² steht, wobei R¹⁵, R¹⁶, R¹⁹, R²⁰, R²¹ , R²², j, k, m, n, p, q, r, s, X und Y die vorstehend genannte Bedeutung haben, und diese ggf. gereinigt und/oder isoliert wird
oder
wenigstens eine Verbindung der allgemeinen Formel IV in einem Reaktionsmedium, ggf. in Gegenwart wenigstens einer Base, bevorzugt in Gegenwart wenigstens einer Base ausgewählt aus der Gruppe bestehend aus Pyridin, Triethylamin, 4,4-Dimethylaminopyridin, Diisopropylethylamin und Diisopropylamin, mit wenigstens einem Sulfonsäure-Derivat der allgemeinen Formel LG-S(=O)₂-R²³ oder LG-S(=O)₂-NR²⁴R²⁵, worin R²³, R²⁴ und R²⁵ die Bedeutung gemäß einem oder mehreren der Ansprüche 1 bis 5 haben und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ und R² die vorstehend genannte Bedeutung haben und R³ für -S(=O)₂-R²³ oder -S(=O)₂-NR²⁴R²⁵ steht, wobei R²³, R²⁴ und R²⁵ die vorstehend genannte Bedeutung haben, und diese ggf. gereinigt und/oder isoliert wird
oder
wenigstens eine Verbindung der allgemeinen Formel IV in einem Reaktionsmedium mit wenigstens einem Isocyanat der allgemeinen Formel R¹⁴-N=C=O, worin R¹⁴ die Bedeutung gemäß einem oder mehreren der Ansprüche 1 bis 5 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹ und R² die vorstehend genannte Bedeutung haben und R³ für -C(=O)-NR¹³R¹⁴ steht, wobei R¹⁴ die vorstehend genannte Bedeutung hat und R¹³ für Wasserstoff steht, und diese ggf. gereinigt und/oder isoliert wird
oder
wenigstens eine Verbindung der allgemeinen Formel IV in einem Reaktionsmedium mit wenigstens einem Isothiocyanat der allgemeinen Formel S=C=N-(CH₂)ₜ-(CH₂)ᵤ-Zᵥ-R²⁷, S=C=N-(CHR²⁸)-R²⁹ oder S=C=N-C(=O)-R³⁰, worin R²⁷, R²⁸, R²⁹, R³⁰, Z, t, u und v die Bedeutung gemäß einem oder mehreren der Ansprüche 1 bis 5 haben, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹ und R² die vorstehend genannte Bedeutung haben und R³ für -C(=S)-NR²⁶-(CH₂)ₜ-(CH₂)ᵤ-Zᵥ-R²⁷, -C(=S)-NR²⁶-(CHR²⁸)-R²⁹ oder-C(=S)-NR²⁶-C(=O)-R³⁰ steht,
wobei R²⁷, R²⁸, R²⁹, R³⁰, Z, t, u und v die vorstehend genannte Bedeutung haben und R²⁶ für Wasserstoff steht, und diese ggf. gereinigt und/oder isoliert wird
und ggf. wenigstens eine Verbindung der allgemeinen Formel I, worin R¹ und R² die vorstehend genannte Bedeutung haben und R³ für -C(=S)-NR²⁶-(CH₂)ₜ-(CH₂)ᵤ-Zᵥ-R²⁷, -C(=S)-NR²⁶-(CHR²⁸)-R²⁹, -C(=S)-NR²⁶-C(=O)-R³⁰ oder-C(=O)-NR¹³R¹⁴ steht, wobei R¹⁴, R²⁷, R²⁸, R²⁹, R³⁰, Z, t, u und v die vorstehend genannte Bedeutung haben und R¹³ und R²⁶ für Wasserstoff stehen, in einem Reaktionsmedium, ggf. in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-R¹³ oder LG-R²⁶, worin R¹³ und R²⁶ die Bedeutung gemäß einem oder mehreren der Ansprüche 1 bis 5 mit Ausnahme eines Wasserstoff-Restes haben und LG für eine Abgangsgruppe, bevorzugt für ein Halogen-Atom, besonders bevorzugt für ein Chloratom steht, zu wenigstens einer Verbindung der allgemeinen Formel ungesetzt wird, worin R¹ und R² die vorstehend genannte Bedeutung haben und R³ für-C(=S)-NR²⁶-(CH₂)ₜ-(CH₂)ᵤ-Zᵥ-R²⁷, -C(=S)-NR²⁶-(CHR²⁸)-R²⁹, -C(=S)-NR²⁶-C(=O)-R³⁰ oder -C(=O)-NR¹³R¹⁴ steht, wobei R¹³, R²⁶, R¹⁴, R²⁷, R²⁸, R²⁹, R³⁰, Z, t, u und v die vorstehend genannte Bedeutung haben, und diese ggf. gereinigt und/oder isoliert wird
oder
wenigstens eine Verbindung der allgemeinen Formel IV in einem Reaktionsmedium, ggf. in Gegenwart wenigstens einer Base, bevorzugt in Gegenwart wenigstens eines Metallhydridsalzes, besonders bevorzugt in Gegenwart von Natrium- und/oder Kaliumhydrid, mit wenigstens einer Verbindung der allgemeinen Formel LG-(CH₂)-R³¹, worin R³¹ die Bedeutung gemäß einem oder mehreren der Ansprüche 1 bis 5 hat und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ und R² die vorstehend genannte Bedeutung haben und R³ für -(CH₂)-R³¹ steht, wobei R³¹ die vorstehend genannte Bedeutung hat, und diese ggf. gereinigt und/oder isoliert wird
oder
wenigstens eine Verbindung der allgemeinen Formel IV in einem Reaktionsmedium, in Gegenwart wenigstens eines Reduktionsmittels, mit wenigstens einer Verbindung der allgemeinen Formel R³¹-C(=O)-H, worin R³¹ die Bedeutung gemäß einem oder mehreren der Ansprüche 1 bis 15 hat, zu wenigstens einer Verbindung der allgemeinen Formel I umgesetzt wird, worin R¹ und R² die vorstehend genannte Bedeutung haben und R³ für -(CH₂)-R³¹ steht, wobei R³¹ die vorstehend genannte Bedeutung hat, und diese ggf. gereinigt und/oder isoliert wird.

7. Arzneimittel enthaltend wenigstens eine Verbindung gemäß einem oder mehreren der Ansprüche 1 bis 5 und ggf. einen oder mehrere physiologisch verträgliche Hilfsstoffe.

8. Arzneimittel gemäß Anspruch 7 zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

9. Arzneimittel gemäß Anspruch 7 zur Prophylaxe und/oder Behandlung von einer oder mehreren Erkrankungen ausgewählt aus der Gruppe bestehend aus Migräne; Depressionen; Entzündungen; Antriebslosigkeit; Harninkontinenz; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Katalepsie; Diarrhoe und Pruritus;
zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit;
zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese; zur Antinatriurese; zur Verwendung als Lokalanästhetikum und/oder Antiarrhythmikum und/oder Antiemetikum und/oder Nootropikum (Neurotropikum).

10. Verwendung wenigstens einer Verbindung gemäß einem oder mehreren der Ansprüche 1 bis 5 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

11. Verwendung wenigstens einer Verbindung gemäß einem oder mehreren der Ansprüche 1 bis 5 zur Herstellung eines Arzneimittels
zur Prophylaxe und/oder Behandlung von einer oder mehreren Erkrankungen ausgewählt aus der Gruppe bestehend aus Migräne; Depressionen; Entzündungen; Antriebslosigkeit; Harninkontinenz; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Katalepsie; Diarrhoe und Pruritus;
zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit;
zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese; zur Antinatriurese; zur Verwendung als Lokalanästhetikum und/oder Antiarrhythmikum und/oder Antiemetikum und/oder Nootropikum (Neurotropikum).

## Claims

1. Substituted 4,5,6,7-tetrahydro-isoxazolo[4,5-c]pyridine compounds of the general formula I 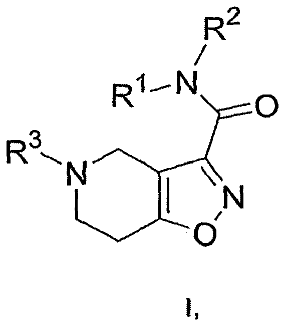 in which
R¹ denotes -(CHR⁴)-(CH₂)_{c}-(CH₂)_{d}-C(=O)-OR⁵ with c = 0 or 1 and d = 0 or 1;
-(CHR⁶)-(CHR⁷)ₑ-V_{f}-(CH₂)_{g}-Wₕ-(CH₂)ᵢ-R⁸ with e = 0 or 1, f = 0 or 1, g = 0 or 1, h = 0 or 1 and i = 0 or 1, in which V and W respectively independently of one another denote O, S, NH, N(CH₃) or N(C₂H₅);
a linear or branched, saturated or unsaturated, possibly substituted C₁₋₁₀ aliphatic residue;
an unsaturated or saturated, possibly substituted 3-, 4-, 5-, 6-, 7-, 8- or 9-membered cycloaliphatic residue, which may be fused with a saturated or unsaturated, possibly aromatic, possibly substituted mono- or polycyclic ring system and/or be bridged with a linear or branched, possibly substituted C₁₋₅ alkylene group, wherein the above-stated possibly substituted cycloaliphatic residues may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, -(CH₂)-benzo[b]furanyl, -O-phenyl, -O-benzyl, phenyl, benzyl, naphthyl and -(CH₂)-naphthyl, wherein in each case the cyclic moiety of the residues -O-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl,-C₁₋₅ alkyl, -O-CF₃, -S-CF₃, phenyl and -O-benzyl,
or a possibly substituted 5- to 14-membered aryl or heteroaryl residue, which may be fused with a saturated or unsaturated, possibly substituted mono- or polycyclic ring system;
R² denotes a hydrogen residue;
-(CH₂)-R⁹
or a linear or branched, saturated or unsaturated, possibly substituted C₁₋₁₀ aliphatic residue;
or
R¹ and R², together with the nitrogen atom joining them together as a ring member, form a saturated or unsaturated, possibly substituted 4-, 5-, 6-, 7-, 8- or 9-membered heterocycloaliphatic residue,
wherein the heterocycloaliphatic residue may in each case be substituted with 1, 2, 3, 4 or 5 substituents selected independently of one another from the group consisting of R¹⁰, -C(=O)-R¹¹ and -(CH₂)-NH-C(=O)-R¹² and/or may in each case comprise a further 1, 2, 3, 4 or 5 heteroatom(s) selected independently of one another from the group consisting of oxygen, nitrogen and sulphur as ring member(s);
R³ denotes -C(=O)-NR¹³R¹⁴;
-C(=O)-R¹⁵;
-C(=O)-(CH₂)ⱼ-Xₖ-(CH₂)ₘ-(CH₂)ₙ-C(=O)-OR¹⁶ withj = 0 or 1, k = 0 or 1, m = 0 or 1 and n = 0 or 1, in which X denotes O, S, NH, N(CH₃), N(C₂H₅) or CR¹⁷R¹⁸;
-C(=O)-(CHR¹⁹)-NH-C(=O)-OR²⁰;
-C(=O)-(CH₂)-(CH₂)ₚ-(CH₂)_{q}Yᵣ(CH₂)ₛ-R²¹ with p = 0 or 1, q = 0 or 1, r = 0 or 1 and s = 0 or 1, in which Y denotes O, S, NH, N(CH₃) or N(C₂H₅);
-C(=O)-(CH=CH)-R²²;
_S(=O)₂-R²³;
-S(=O)₂-NR²⁴R²⁵;
-C(=S)-NR²⁶-(CH₂)ᵣ(CH₂)ᵤ-Zᵥ-R²⁷ with t = 0 or 1, u = 0 or 1 and v = 0 or 1, in which Z denotes O, S, NH, N(CH₃) or N(C₂H₅);
-C(=S)-NR²⁶-(CHR²⁸)-R²⁹;
-C(=S)-NR²⁶-C(=O)-R³⁰;
or -(CH₂)-R³¹;
R⁴ denotes -C(=O)-NH₂;
a hydrogen residue
or a linear or branched, saturated or unsaturated, possibly substituted C₁₋₁₀ aliphatic residue;
R⁵, R¹⁷, R¹⁸, R²⁴ and R²⁵, independently of one another, respectively denote
a linear or branched, saturated or unsaturated, possibly substituted C₁₋₁₀ aliphatic residue,
R⁶, R⁷, R¹³, R¹⁶ and R²⁶, independently of one another, respectively denote
a hydrogen residue
or a linear or branched, saturated or unsaturated, possibly substituted C₁₋₁₀ aliphatic residue;
R⁸, R⁹, R²¹, R²², R²³, R³⁰ and R³¹, independently of one another, respectively denote
an unsaturated or saturated, possibly substituted 3-, 4-, 5-, 6-, 7-, 8- or 9-membered cycloaliphatic residue, which may be fused with a saturated or unsaturated, possibly aromatic, possibly substituted mono- or polycyclic ring system, wherein the above-stated possibly substituted cycloaliphatic residues may in each case be substituted with 1, 2, 3, 4 or 5 substituents selected independently of one another from the group consisting of oxo (=O), thioxo (=S), F, Cl, Br, I, -CN, -CF₃, -SF₅, -O_{H}, -O-C₁₋₅ alkyl,- NH₂, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, NH-C₁₋₅ alkyl, -N(C₁₋₅ alkyl)₂, -(CH₂)-benzo[b]furanyl, -O-phenyl, -O-benzyl, phenyl, benzyl, naphthyl and -(CH₂)-naphthyl, wherein in each case the cyclic moiety of the residues -O-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl,
or a possibly substituted 5- to 14-membered aryl or heteroaryl residue, which may be fused with a saturated or unsaturated, possibly substituted mono- or polycyclic ring system;
R¹⁰, R¹¹, R¹² R¹⁴ and R²⁰, independently of one another, respectively denote
a linear or branched, saturated or unsaturated, possibly substituted C₁₋₁₀ aliphatic residue,
an unsaturated or saturated, possibly substituted 3-, 4-, 5-, 6-, 7-, 8- or 9-membered cycloaliphatic residue, which may be fused with a saturated or unsaturated, possibly aromatic, possibly substituted mono- or polycyclic ring system and/or be attached via a linear or branched, possibly substituted C₁₋₅ alkylene, C₂₋₅ alkenylene or C₂₋₅ alkynylene group, wherein the above-stated, possibly substituted cycloaliphatic residues may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, NH-C₁₋₅ alkyl, -N(C₁₋₅ alkyl)₂, -(CH₂)-benzo[b]furanyl, -O-phenyl, -O-benzyl, phenyl, benzyl, naphthyl and -(CH₂)-naphthyl, wherein in each case the cyclic moiety of the residues -O-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl,
or a possibly substituted 5- to 14-membered aryl or heteroaryl residue, which may be attached via a linear or branched, possibly substituted C₁₋₅ alkylene, C₂₋₅ alkenylene or C₂₋₅ alkynylene group;
R¹⁵ and R²⁷, independently of one another, respectively denote
a linear or branched, saturated or unsaturated, possibly substituted C₁₋₁₀ aliphatic residue,
an unsaturated or saturated, possibly substituted 3-, 4-, 5-, 6-, 7-, 8- or 9-membered cycloaliphatic residue, which may be bridged with a linear or branched, possibly substituted C₁₋₅ alkylene group, wherein the above-stated possibly substituted cycloaliphatic residues may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-H,-C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, NH-C₁₋₅ alkyl, -N(C₁₋₅ alkyl)₂, -(CH₂)-benzo[b]furanyl, -O-phenyl, -O-benzyl, phenyl, benzyl, naphthyl and -(CH₂)-naphthyl, wherein in each case the cyclic moiety of the residues -O-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl,
or a possibly substituted 5- to 14-membered aryl or heteroaryl residue, which may be fused with a saturated or unsaturated, possibly substituted mono- or polycyclic ring system;
R¹⁹ denotes a possibly substituted 5- to 14-membered aryl or heteroaryl residue, which may be attached via a linear or branched, possibly substituted C₁₋₅ alkylene, C₂₋₅ alkenylene or C₂₋₅ alkynylene group possibly comprising 1 or 2 heteroatom(s) as chain link(s);
R²⁸ and R²⁹, independently of one another, respectively denote
a linear or branched, saturated or unsaturated, possibly substituted C₁₋₁₀ aliphatic residue
or a possibly substituted 5- to 14-membered aryl or heteroaryl residue, which may be fused with a saturated or unsaturated, possibly substituted mono- or polycyclic ring system;
wherein
the above-stated C₁₋₁₀ aliphatic residues may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
and the above-stated cycloaliphatic residues may in each case possibly comprise 1, 2, 3, 4 or 5 heteroatom(s) selected independently of one another from the group consisting of oxygen, nitrogen and sulphur as ring member(s);
the above-stated possibly substituted C₁₋₅ alkylene, C₂₋₅ alkenylene or C₂₋₅ alkynylene groups may in each case be substituted with 1, 2, 3, 4 or 5 substituents selected independently of one another from the group consisting of F, Cl, Br, -OH, -SH, -NH₂, -CN, NO₂ and phenyl,
and the above-stated C₁₋₅ alkylene, C₂₋₅ alkenylene or C₂₋₅ alkynylene groups may possibly respectively comprise 1 or 2 heteroatom(s) selected from the group consisting of oxygen, nitrogen and sulphur as chain link(s);
the rings of the above-stated possibly substituted mono- or polycyclic ring systems may possibly be respectively substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -0-C₂₋₅ alkenyl, -NH₂, -NO₂, -O-CF₃, -O-CHF₂, -O-CH₂F, -S-CF₃, -S-CHF₂, -S-CH₂F, -SH, -S-C₁₋₅ alkyl, -C₁₋₅ alkyl, -C(=O)-OH, -C(=O)-O-C₁₋₅ alkyl, -NH-C₁₋₅ alkyl, -N(C₁₋₅ alkyl)₂, -NH-C(=O)-O-C₁₋₅ alkyl, -NH-C(=O)-C₁₋₅ alkyl, -C(=O)-H, -C(=O)-C₁₋₅ alkyl, -C(=O)-NH₂, -C(=O)-NE-C₁₋₅ alkyl, -C(=O)-N-(C₁₋₅ alkyl)₂, -S(=O)₂-NH₂, -S(=O)₂-NH-C₁₋₅ alkyl, -(CH₂)-benzo[b]furanyl, -O-phenyl, -O-benzyl, phenyl and benzyl, wherein in each case the cyclic moiety of the residues -O-phenyl, -O-benzyl, phenyl, -(CH₂)-benzo[b]furanyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl,
and the rings of the above-stated mono- or polycyclic ring systems are in each case 5-, 6- or 7-membered and may in each case comprise 1, 2, 3, 4 or 5 heteroatom(s) as ring member(s) which are selected independently of one another from the group consisting of oxygen, nitrogen and sulphur;
and the above-stated possibly substituted aryl or heteroaryl residues may in each case be substituted with 1, 2, 3, 4 or 5 substituents selected independently of one another from the group consisting of F, Cl, Br, I, -CN, -CF₃, -SF₅, -OH, -O-C₁₋₅ alkyl, -O-C₂₋₅ alkenyl, -NH₂, -NO₂, -O-CF₃, -O-CHF₂, -O-CH₂F, -S-CF₃, -S-CHF₂, -S-CH₂F, -SH, -S-C₁₋₅ alkyl, -C₁₋₅ alkyl, -C(=O)-O-C₁₋₅ alkyl, -NH-C₁₋₅ alkyl, -N(C₁₋₅ alkyl)₂, -NH-C(=O)-O-C₁₋₅ alkyl, -NH-C(=O)-C₁₋₅ alkyl, -C(=O)-H, -C(=O)-C₁₋₅ alkyl, -C(=O)-NH₂, -C(=O)-NE-C₁₋₅ alkyl, -C(=O)-N-(C₁₋₅ alkyl)₂, -S(=O)₂-NH₂, -S(=O)₂-NH-C₁₋₅ alkyl, -(CH₂)-benzo[b]furanyl, -O-phenyl, -O-benzyl, phenyl and benzyl, wherein in each case the cyclic moiety of the residues -O-phenyl, -O-benzyl, phenyl, -(CH₂)-benzo[b]furanyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CHF₃, -S-CF₃, phenyl and -O-benzyl,
and
the above-stated heteroaryl residues may in each case comprise 1, 2, 3, 4 or 5 heteroatom(s) selected independently of one another from the group consisting of oxygen, nitrogen and sulphur as ring member(s);
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

2. Compounds according to claim 1, **characterised in that**
R¹ denotes -(CHR⁴)-(CH₂)_{c}-(CH₂)_{d}-C(=O)-OR⁵ with c = 0 or 1 and d = 0 or 1;
-(CHR⁶)-(CHR⁷)ₑ-V_{f-}(CH₂)_{g}-Wₕ-(CH₂)ⱼR⁸ with e = 0 or 1, f = 0 or 1, g = 0 or 1, h = 0 or 1 and i = 0 or 1, in which V and W respectively independently of one another denote O, S, NH, N(CH₃) or N(C₂H₅);
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
an alkenyl residue selected from the group consisting of vinyl, 1-propenyl, 2-propenyl, 1-butenyl, 2-butenyl and 3-butenyl, wherein the alkenyl-residue may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
an alkynyl residue selected from the group consisting of ethynyl, 1-propynyl, 2-propynyl, 1-butynyl, 2-butynyl and 3-butynyl, wherein the alkynyl residue may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
a (hetero)cycloaliphatic residue selected from the group consisting of cyclopropyl, cyclobutyl, cyclopentyl, cyclohexyl, cycloheptyl, (6,6)-dimethyl-[3.1.1]-bicycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, wherein the (hetero)cycloaliphatic residue may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, -(CH₂)-benzo[b]furanyl, -O-phenyl, -O-benzyl, phenyl, benzyl, naphthyl and -(CH₂)-naphthyl, wherein in each case the cyclic moiety of the residues -O-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl;
or a residue selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl and isoquinolinyl, wherein the residue may in each case be substituted with 1, 2, 3, 4 or 5 substituents selected independently of one another from the group consisting of F, Cl, Br, I, -CN, -CF₃, -SF₅, -OH, -O-C₁₋₅ alkyl, -O-C₂₋₅ alkenyl, -NH₂, -NO₂, -O-CF₃, -O-CHF₂, -O-CH₂F, -S-CF₃, -S-CHF₂, -S-CH₂F, -SH, -S-C₁₋₅ alkyl, -C₁₋₅ alkyl, -C(=O)-O-C₁₋₅ alkyl, -NH-C₁₋₅ alkyl, -N(C₁₋₅ alkyl)₂, -NH-C(=O)-O-C₁₋₅ alkyl, -NH-C(=O)-C₁₋₅ alkyl, -C(=O)-H, -C(=O)-C₁₋₅ alkyl, -C(=O)-NH₂, -C(=O)-NH-C₁₋₅ alkyl, -C(=O)-N-(C₁₋₅ alkyl)₂, -S(=O)₂-NH₂, -S(=O)₂-NH-C₁₋₅ alkyl, -(CH₂)-benzo[b]furanyl, -O-phenyl, -O-benzyl, phenyl and benzyl, wherein in each case the cyclic moiety of the residues -O-phenyl, -O-benzyl, phenyl, -(CH₂)-benzo[b]furanyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl;
R² denotes a hydrogen residue;
-(CH₂)-R⁹
or an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and - (CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅);
or
R¹ and R², together with the nitrogen atom joining them together as a ring member, form a heterocycloaliphatic residue selected from the group consisting of imidazolidinyl, (1,3)-thiazolidinyl, piperazinyl, morpholinyl, thiomorpholinyl, pyrrolidinyl; piperidinyl; (1,2,3,6)-tetrahydropyridinyl and (1,2,3,4)-tetrahydropyridinyl, wherein the heterocycloaliphatic residue may in each case be substituted with 1, 2 or 3 substituents selected independently of one another from the group consisting of R¹⁰, -C(=O)-R¹¹ and -(CH₂)-NH-C(=O)-R¹²;
R³ denotes -C(=O)-NR¹³R¹⁴;
-C(=O)-R¹⁵;
-C(=O)-(CH₂)-X-(CH₂)-C(=O)-OR¹⁶, -C(=O)-X-(CH₂)-C(=O)-OR¹⁶ or -C(=O)-(CH₂)-(CH₂)-(CH₂)-C(=O)-OR¹⁶, in which X denotes O, S, NH, N(CH₃), N(C₂H₅) or CR¹⁷R¹⁸;
-C(=O)-(CHR¹⁹)-NH-C(=O)-OR²⁰;
-C(=O)-(CH₂)-R²¹, -C(=O)-(CH₂)-Y-R²¹, -C(=O)-(CH₂)-Y-(CH₂)-R²¹ or -C(=O)-(CH₂)-(CH₂)-(CH₂)-Y-R²¹, in which Y denotes O, S, NH, N(CH₃) or N(C₂H₅);
-C(=O)-(CH=CH)-R²²;
-S(=O)₂-R²³;
-S(=O)₂-NR²⁴R²⁵;
-C(=S)-NR²⁶-R²⁷, -C(=S)-NR²⁶-(CH₂)-R²⁷, -C(=S)-NR²⁶-(CH₂)-(CH₂)-R²⁷ or -C(=S)-NR²⁶-(CH₂)-(CH₂)-Z-R²⁷, in which Z denotes O, S, NH, N(CH₃) or N(C₂H₅) ;
-C(=S)-NR²⁶-(CHR2²⁸)-R²⁹;
-C(=S)-NR²⁶-C(=O)-R³⁰;
or -(CH₂)-R³¹;
R⁴ denotes -C(=O)-NH₂;
a hydrogen residue;
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
or an alkenyl residue selected from the group consisting of vinyl, 1-propenyl, 2-propenyl, 1-butenyl, 2-butenyl and 3-butenyl, wherein the alkenyl residue may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
R⁵, R¹⁷, R¹⁸, R²⁴ and R²⁵, independently of one another, respectively denote
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅),
or an alkenyl residue selected from the group consisting of vinyl, 1-propenyl, 2-propenyl, 1-butenyl, 2-butenyl and 3-butenyl, wherein the alkenyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅);
R⁶, R⁷, R¹³, R¹⁶ and R²⁶, independently of one another, respectively denote
a hydrogen residue
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and - (CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅),
or an alkenyl residue selected from the group consisting of vinyl, 1-propenyl, 2-propenyl, 1-butenyl, 2-butenyl and 3-butenyl, wherein the alkenyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅);
R⁸, R⁹, R²¹, R²², R²³, R³⁰ and R³¹, independently of one another, respectively denote
a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, wherein the (hetero)cycloaliphatic residue may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, -NH-C₁₋₅ alkyl, -N(C₁₋₅ alkyl)₂, -(CH₂)-benzo[b]furanyl, -O-phenyl, -O-benzyl, phenyl, benzyl, naphthyl and -(CH₂)-naphthyl, wherein in each case the cyclic moiety of the residues -O-phenyl, -O-benzyl, phenyl, -(CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl;
or a residue selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl and isoquinolinyl, wherein the residue may in each case be substituted with 1, 2, 3, 4 or 5 substituents selected independently of one another from the group consisting of F, Cl, Br, I, -CN, -CF₃, -SF₅, -OH, -O-C₁₋₅ alkyl, -O-C₂₋₅ alkenyl, -NH₂, -NO₂, -O-CF₃, -O-CHF₂, -O-CH₂F, -S-CF₃, -S-CHF₂, -S-CH₂F, -SH, -S-C₁₋₅ alkyl, -C₁₋₅ alkyl, -C(=O)-O-C₁₋₅ alkyl, -NH-C₁₋₅ alkyl, -N(C₁₋₅ alkyl)₂, -NH-C(=O)-O-C₁₋₅ alkyl, -NH-C(=O)-C₁₋₅ alkyl, -C(=O)-H, -C(=O)-C₁₋₅ alkyl, -C(=O)-NH₂, -C(=O)-NH-C₁₋₅ alkyl, -C(=O)-N-(C₁₋₅ alkyl)₂, -S(=O)₂-NH₂, -S(=O)₂-NH-C₁₋₅ alkyl, -(CH₂)-benzo[b]furanyl, -O-phenyl, -O-benzyl, phenyl and benzyl, wherein in each case the cyclic moiety of the residues -O-phenyl, -O-benzyl, phenyl, -(CH₂)-benzo[b]furanyl and benzyl maybe substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl;
R¹⁰, R¹¹, R¹², R¹⁴ and R²⁰, independently of one another, respectively denote
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-C₁₋₅ alkyl, -SH, -S-C₁₋₅ alkyl, -NH₂, -NH-C₁₋₅ alkyl and -N(C₁₋₅ alkyl)(C₁₋₅ alkyl);
a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, indanyl and indenyl, wherein the (hetero)cycloaliphatic residue may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, -NH-C₁₋₅ alkyl, -N(C₁₋₅ alkyl)₂, -(CH₂)-benzo[b]furanyl, -O-phenyl, -O-benzyl, phenyl, benzyl, naphthyl and -(CH₂)-naphthyl, wherein in each case the cyclic moiety of the substituents -O-phenyl, -O-benzyl, phenyl, - (CH₂)-benzo[b]furanyl, benzyl, naphthyl and -(CH₂)-naphthyl may be substituted with 1, 2, 3, 4 or 5 substituents selected independently of one another from the group consisting of F, Cl, Br, -OH, -CF₃, -SF₅, - CN, -NO₂, -O-C₁₋₅ alkyl, -C₁₋₅ alkyl, -O-CF₃, -C₁₋₅ alkyl, -S-CF₃, phenyl and -O-benzyl and/or wherein the (hetero)cycloaliphatic residue may be attached via a -(CH₂)-, -CH(CH₃)-, -(CH₂)-(CH₂)-, -(CH₂)-(CH₂)-(CH₂)- or -(CH₂)-(CH=CH)- group;
or a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl, isoquinolinyl and thieno[2,3-d]pyrimidinyl, wherein the residue may in each case be substituted with 1, 2, 3, 4 or 5 substituents selected independently of one another from the group consisting of F, Cl, Br, I, -CN, -CF₃, -SF₅, -OH, -O-C-₁₋₅ alkyl, -O-C₂₋₅ alkenyl, -NH₂, -NO₂, -O-CF₃, -O-CHF₂, -O-CH₂F, -S-CF₃, -S-CHF₂, -S-CH₂F, -SH, -S-C₁₋₅ alkyl, -C₁₋₅ alkyl, -C(=O)-O-C₁₋₅ alkyl, -NH-C₁₋₅ alkyl, -N(C₁₋₅ alkyl)₂, -NH-C(=O)-O-C₁₋₅ alkyl, -NH-C(=O)-C₁₋₅ alkyl, -C(=O)-H, -C(=O)-C₁₋₅ alkyl, -C(=O)-NH₂, -C(=O)-NH-C₁₋₅ alkyl, -C(=O)-N-(C₁₋₅ alkyl)₂, -S(=O)₂-NH₂, -S(=O)₂-NH-C₁₋₅ alkyl, -(CH₂)-benzo[b]furanyl, -O-phenyl, -O-benzyl, phenyl and benzyl and/or be attached via a -(CH₂)-, -CH(CH₃)-, -(CH₂)-(CH₂)-, -(CH₂)-(CH₂)-(CH₂)- or -(CH₂)-(CH=CH)-group, wherein in each case the cyclic moiety of the substituents -O-phenyl, -O-benzyl, phenyl, -(CH₂)-benzo[b]furanyl and benzyl may be substituted with 1, 2, 3, 4 or 5 substituents selected independently of one another from the group consisting of F, Cl, Br, -OH, -CF₃, -SF₅, - CN, -NO₂, -O-C₁₋₅ alkyl, -C₁₋₅ alkyl, -O-CF₃, -S-CF₃, phenyl and -O-benzyl;
R¹⁵ and R²⁷, independently of one another, respectively denote
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, - NH₂, -NH-CH₃, -NH-C₂H₅, -N(CH₃)₂, -N(C₂H₅)₂ and -N(CH₃)(C₂H₅);
an alkenyl residue selected from the group consisting of vinyl, 1-propenyl, 2-propenyl, 1-butenyl, 2-butenyl and 3-butenyl, wherein the alkenyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₅)₂, -N(C₂H₅)₂ and - N(CH₃)(C₂H₅);
a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl and 7,7-dimethyl-2-oxa-bicyclo[2.2.1]heptyl, wherein the (hetero)cycloaliphatic residue may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-H, -C(=O)-C₁₋₅ alkyl, -C(=O)-OH, -C(=O)-O-C₁₋₅ alkyl, -(CH₂)-C(=O)-OH, -(CH₂)-C(=O)-O-C₁₋₅ alkyl, -NH-C₁₋₅ alkyl, -N(C₁₋₅ alkyl)₂, -O-phenyl, -O-benzyl, phenyl and benzyl, wherein in each case the cyclic moiety of the residues -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -O-C₁₋₅ alkyl, -C₁₋₅ alkyl, -O-CF₃, -S-CF₃, phenyl and -O-benzyl;
or a residue selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl and isoquinolinyl, wherein the residue may in each case be substituted with 1, 2, 3, 4 or 5 substituents selected independently of one another from the group consisting of F, Cl, Br, I, -CN, -CF₃, -SF₅, -OH, -O-C₁₋₅ alkyl, -O-C₂₋₅ alkenyl, -NH₂, -NO₂, -O-CF₃, -O-CHF₂, -O-CH₂F, -S-CF₃, -S-CHF₂, -S-CH₂F, -SH, -S-C₁₋₅ alkyl, -C₁₋₅ alkyl, -C(=O)-O-C₁₋₅ alkyl, -NH-C₁₋₅ alkyl, -N(C₁₋₅ alkyl)₂, -NH-C(=O)-O-C₁₋₅ alkyl, -NH-C(=O)-C₁₋₅ alkyl, -C(=O)-H, -C(=O)-C₁₋₅ alkyl, - C(=O)-NH₂, -C(=O)-NH-C₁₋₅ alkyl, -C(=O)-N-(C₁₋₅ alkyl)₂, -S(=O)₂-NH₂, -S(=O)₂-NH-C₁₋₅ alkyl, -O-phenyl, -O-benzyl, phenyl and benzyl, wherein in each case the cyclic moiety of the residues -O-phenyl, -O-benzyl, phenyl and benzyl may be substituted with 1, 2, 3, 4 or 5 substituents selected independently of one another from the group consisting of F, Cl, Br, -OH, -CF₃, -SF₅, -CN, -NO₂, -0-C₁₋₅ alkyl, -C₁₋₅ alkyl, -O-CF₃, -S-CF₃, phenyl and -O-benzyl;
R¹⁹ denotes a residue selected from the group consisting of phenyl, naphthyl, thiophenyl, furanyl and pyridinyl, wherein the residue may in each case be substituted with 1, 2, 3, 4 or 5 substituents selected independently of one another from the group consisting of F, Cl, Br, I, -CN, -CF₃, -SF₅, -OH, -O-CH₃, -O-C₂H₅, -O-CH(CH₃)₂, -O-CH₂-CH₂-CH₂-CH₃, -NH₂-, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, phenyl and benzyl and/or be attached via a -(CH₂)-, -(CH₂)-(CH₂)- or -(CH₂)-O-(CH₂)- group;
and
R²⁸ and R²⁹, independently of one another, respectively denote
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and - (CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅);
or a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl and isoquinolinyl, wherein the residue may in each case be substituted with 1, 2, 3, 4 or 5 substituents selected independently of one another from the group consisting of F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, -S(=O)₂-CH₃, phenyl and benzyl;
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

3. Compounds according to claim 1 or 2, **characterised in that**
R¹ denotes -(CHR⁴)-C(=O)-OR⁵, -(CHR⁴)-(CH₂)-C(=O)-OR⁵ or -(CHR⁴)-(CH₂)-(CH₂)-C(=O)-OR⁵;
-(CHR⁶)-R⁸, -(CHR⁶)-(CHR⁷)-R⁸, -(CHR⁶)-(CHR⁷)-V-R⁸, -(CHR⁶)-(CHR⁷)-(CH₂)-R⁸, -(CHR⁶)-(CHR⁷)-(CH₂)-W-R⁸ or -(CHR⁶)-(CHR⁷)-(CH₂)-W-(CH₂)-R⁸, in which V and W respectively independently of one another denote O, S, NH, N(CH₃) or N(C₂H₅);
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and - (CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, - NH₂, -NH-CH₃, -NH-C₂H₅, -N(CH₃)₂, -N(C₂H₅)₂ and -N(CH₃)(C₂H₅);
an alkynyl residue selected from the group consisting of ethynyl, 1-propynyl, 2-propynyl, 1-butynyl, 2-butynyl and 3-butynyl;
a (hetero)cycloaliphatic residue selected from the group consisting of cyclopropyl, cyclobutyl, cyclopentyl, cyclohexyl, cycloheptyl, (6,6)-dimethyl-[3.1.1]-bicycloheptyl, pyrrolidinyl, piperidinyl, morpholinyl, thiomorpholinyl, indanyl and indenyl, wherein the (hetero)cycloaliphatic residue may in each case be substituted with 1, 2, 3, 4 or 5 substituents selected independently of one another from the group consisting of oxo (=O), thioxo (=S), F, Cl, Br, I, -CN, -CF₃, - SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -CO(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -(CH₂)-benzo[b]furanyl, -O-phenyl, -O-benzyl, phenyl, benzyl, naphthyl and -(CH₂)-naphthyl, wherein the cyclic moiety of the benzyl residue may be substituted with 1, 2, 3, 4 or 5 substituents selected independently of one another from the group consisting of F, Cl, Br, -OH, -CF₃, -SF₅, -CN, -NO₂, -O-CH₃, -O-C₂H₅ and -O-CF₃,
or a residue selected from the group consisting of phenyl, naphthyl, (1,3)-benzodioxolyl, (1,4)-benzodioxanyl, thiophenyl, furanyl, pyridinyl, imidazolyl and indolyl, wherein the residue may in each case be substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, methyl, ethyl, n-propyl, isopropyl, n-butyl, sec-butyl, isobutyl and tert-butyl;
R² denotes a hydrogen residue or
-(CH₂)-R⁹,
or
R¹ and R², together with the nitrogen atom joining them together as a ring member, form a residue which is selected from the group consisting of 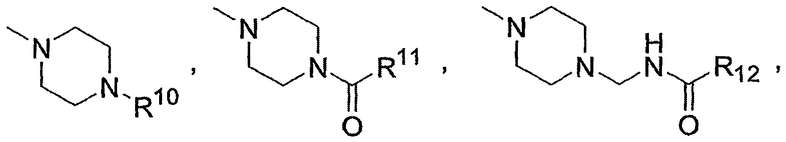 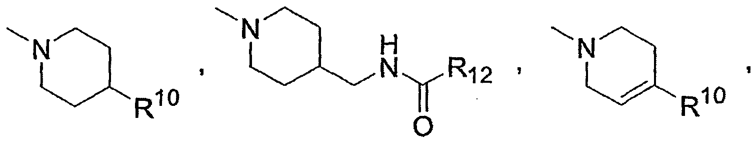 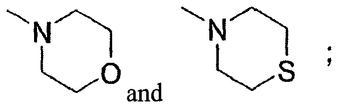
R³ denotes -C(=O)-NR¹³R¹⁴;
-C(=O)-R¹⁵;
-C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-CR¹⁷R¹⁸-(CH₂)-C(=O)-OR¹⁶ or -C(=O)-(CH₂)-(CH₂)-(CH₂)-C(=O)-OR¹⁶;
-C(=O)-(CHR¹⁹)-NH-C(=O)-OR²⁰;
-C(=O)-(CH₂)-R²¹, -C(=O)-(CH₂)-O-R²¹, -C(=O)-(CH₂)-O-(CH₂)-R²¹ or -C(=O)-(CH₂)-(CH₂)-(CH₂)-O-R²¹;
-C(=O)-(CH=CH)-R²²;
-S(=O)₂-R²³;
-S(=O)₂-NR²⁴R²⁵;
-C(=S)-NR²⁶-R²⁷, -C(=S)-NR²⁶-(CH₂)-R²⁷, -C(=S)-NR²⁶-(CH₂)-(CH₂)-R²⁷ or -C(=S)-NR²⁶-(CH₂)-(CH₂)-O-R²⁷;
-C(=S)-NR²⁶-(CHR²⁸)-R²⁹;
-C(=S)-NR²⁶-C(=O)-R³⁰;
or -(CH₂)-R³¹;
R⁴ denotes -C(=O)-NH₂;
a hydrogen residue or
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)-(C(CH₃)₃), n-hexyl, n-heptyl, n-octyl and -(CH₂)-(CH)(C₂H₅)-(CH₂)-(CH₂)-(CH₂)-(CH₃), wherein the alkyl residue may in each case be substituted with 1, 2, 3, 4 or 5 substituents selected independently of one another from the group consisting of F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ and -N(CH₃)(C₂H₅);
R⁵ denotes an alkyl residue selected from the group consisting of methyl, ethyl, n-propyl, isopropyl, n-butyl, sec-butyl, isobutyl and tert-butyl;
R⁶, R⁷ and R¹⁶, independently of one another, respectively denote
a hydrogen residue or an alkyl residue selected from the group consisting of methyl, ethyl, n-propyl and isopropyl;
R⁸, R⁹, R²¹, R²², R²³, R³⁰ and R³¹, independently of one another, respectively denote
a (hetero)cycloaliphatic residue selected from the group consisting of cyclopropyl, cyclobutyl, cyclopentyl, cyclohexyl, cycloheptyl, pyrrolidinyl, piperidinyl, morpholinyl, thiomorpholinyl, indanyl and indenyl, wherein the (hetero)cycloaliphatic residue may in each case be substituted with 1, 2, 3, 4 or 5 substituents selected independently of one another from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, - isopropyl, n-butyl, sec-butyl, isobutyl, tert-butyl, -C(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), phenyl and benzyl;
or a residue selected from the group consisting of phenyl, naphthyl, (1,3)-benzodioxolyl, (1,4)-benzodioxanyl, thiophenyl, furanyl, pyridinyl, imidazolyl, indolyl and isoindolyl, wherein the residue may in each case be substituted with 1, 2, 3, 4 or 5 substituents selected independently of one another from the group consisting of F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), -C(=O)-H, -C(=O)-CH₃, -C(=O)-C₂H₅, -C(=O)-NH₂, -S(=O)₂-NH₂, -S(=O)₂-NH-CH₃, -O-phenyl, -O-benzyl, phenyl and benzyl;
R¹⁰, R¹¹, R¹², R¹⁴ and R²⁰, independently of one another, respectively denote
an alkyl residue selected from the group consisting of methyl, ethyl, n-propyl, isopropyl, n-butyl, sec-butyl, isobutyl, tert-butyl, n-pentyl, sec-pentyl, -(CH₂)-(CH₂)(C(CH₃)₃), n-hexyl, n-heptyl and n-octyl, wherein the alkyl residue may in each case be substituted with 1, 2, 3, 4 or 5 substituents selected independently of one another from the group consisting of -N(CH₃)₂, -N(C₂H₅)₂, F, Cl and Br;
a cycloaliphatic residue selected from the group consisting of pyrrolidinyl, piperidinyl, piperazinyl, morpholinyl, cyclopropyl, cyclobutyl, cyclopentyl and cyclohexyl, wherein the cycloaliphatic residue may in each case be substituted with 1, 2 or 3 substituents selected independently of one another from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), phenyl and benzyl substituted and/or be attached via a -(CH₂)- or -(CH₂)-(CH₂)-group;
or a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl, isoquinolinyl and thieno[2,3-d]pyrimidinyl, wherein the residue may in each case be substituted with 1, 2, 3, 4 or 5 substituents selected independently of one another from the group consisting of F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -N(CH₃)₂, -N(C₂H₅)₂, -N(H)(CH₃), -N(H)(C₂H₅), -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, -S(=O)₂-CH₃, phenyl and benzyl and/or be attached via a -(CH₂)-, -CH(CH₃)-, -(CH₂)-(CH₂)-, -(CH₂)-(CH₂)-(CH₂)- or -(CH₂)-(CH=CH)- group;
R¹³ and R²⁶ denote a hydrogen residue;
R¹⁵ and R²⁷, independently of one another, respectively denote
a residue selected from the group consisting of methyl, ethyl, n-propyl, isopropyl, n-butyl, sec-butyl, isobutyl, tert-butyl, vinyl, 1-propenyl, 2-propenyl, 1-butenyl, 2-butenyl and 3-butenyl;
a (hetero)cycloaliphatic residue selected from the group consisting of cyclopropyl, cyclobutyl, cyclopentyl, cyclohexyl and 7,7-dimethyl-2-oxa-bicyclo[2.2.1]heptyl, wherein the (hetero)cycloaliphatic residue may in each case be substituted with 1, 2 or 3 substituents selected independently of one another from the group consisting of oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -N(CH₃)₂, -N(C₂H₅)₂, -N(H)(CH₃), -N(H)(C₂H₅), phenyl and benzyl;
or a residue selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl and isoquinolinyl, wherein the residue may in each case be substituted with 1, 2, 3, 4 or 5 substituents selected independently of one another from the group consisting of F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, -S(=O)₂-CH₃, phenyl and benzyl;
R¹⁷ and R¹⁸, independently of one another, respectively denote a methyl or ethyl residue;
R¹⁹ denotes a phenyl residue, which may be attached via a -(CH₂)-, -(CH₂)-(CH₂)- or -(CH₂)-O-(CH₂)- group;
R²⁴ and R²⁵, independently of one another, respectively denote a methyl or ethyl residue; R²⁸ denotes a phenyl residue, which may be substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl;
and
R²⁹ denotes an alkyl residue selected from the group consisting of methyl, ethyl, n-propyl, isopropyl, n-butyl, isobutyl and tert-butyl;
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

4. Compounds according to one or more of claims 1 to 3, **characterised in that**
R¹ denotes -(CHR⁴)-C(=O)-OR⁵, -(CHR⁴)-(CH₂)-C(=O)-OR⁵ or -(CHR⁴)-(CH₂)-(CH₂)-C(=O)-OR⁵;
-(CHR⁶)-R⁸, -(CHR⁶)-(CHR⁷)-R⁸, -(CHR⁶)-(CHR⁷)-O-R⁸, -(CHR⁶)-(CHR⁷)-(CH₂)-R⁸ or -(CHR⁶)-(CHR⁷)-(CH₂)-N(CH₃)-R⁸;
a possibly substituted alkyl residue selected from the group consisting of methyl, ethyl, n-propyl, isopropyl, -(CH₂)-(CH₂)-CN, -(CH₂)-(CH₂)-N(CH₃)₂, -(CH₂)-(CH₂)-O-CH₃, -(CH₂)-(CH₂)-S-C₂H₅, -n- butyl, sec-butyl, isobutyl, tert-butyl, -(CH₂)-(CH₂)-(CH₂)-O-CH₃, n-pentyl, sec-pentyl -(CH₂)-(CH₂)(C(CH₃)₃), n-hexyl, n-heptyl, n-octyl and -(CH₂)-(CH)(C₂H₅)-(CH₂)-(CH₂)-(CH₂)-(CH₃);
an alkynyl residue selected from the group consisting of 1-propynyl and 2-propynyl;
a cycloaliphatic residue selected from the group consisting of cyclopropyl, cyclobutyl, cyclopentyl, cyclohexyl, cycloheptyl, (6,6)-dimethyl-[3.1.1]-bicycloheptyl, indanyl and indenyl, wherein the cycloaliphatic residue may in each case be substituted with an -O-benzyl residue or a methyl residue,
a pyrrolidinyl or piperidinyl residue, which may in each case be substituted on the nitrogen atom with a substituent selected from the group consisting of -C(=O)-CH₃, -C(=O)-C₂H₅, -C(=O)-C(CH₃)₃, -C(=O)-O-C₂H₅, benzyl and -(CH₂)-naphthyl, wherein the cyclic moiety of the benzyl residue may be substituted with 1, 2 or 3 substituents selected independently of one another from the group consisting of F, Cl, Br, -CF₃ and -O-CF₃,
or a phenyl residue, which may be substituted with 1, 2 or 3 substituents selected independently of one another from the group consisting of -O-CH₃, -O-C₂H₅, methyl, ethyl, n-propyl, isopropyl, n-butyl, sec-butyl, isobutyl and tert-butyl;
R² denotes a hydrogen residue or -(CH₃)-R⁹;
or
R¹ and R², together with the nitrogen atom joining them together as a ring member, form a residue which is selected from the group consisting of 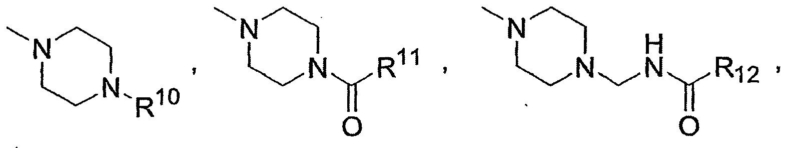 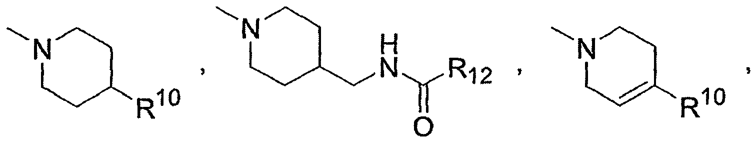 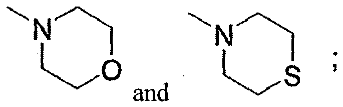
R³ denotes -C(=O)-NR¹³R¹⁴;
-C(=O)-R¹⁵;
-C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-C¹⁷R18-(CH₂)-C(=O)-OR¹⁶ or -C(=O)-(CH₂)-(CH₂)-(CH₂)-C(=O)-OR¹⁶;
-C(=O)-(CHR¹⁹)-NH-C(=O)-OR²⁰;
-C(=O)-(CH₂)-R²¹, -C(=O)-(CH₂)-O-R²¹, -C(=O)-(CH₂)-O-(CH₂)-R²¹ or -C(=O)-(CH₂)-(CH₂)-(CH₂)-O-R²¹;
-C(=O)-(CH=CH)-R²²;
-S(=O)₂-R²³;
-S(=O)₂-NR²⁴R²⁵;
-C(=S)-NR²⁶-R²⁷, -C(=S)-NR²⁶-(CH₂)-R²⁷, -C(=S)-NR²⁶-(CH₂)-(CH₂)-R²⁷ or -C(=S)-NR²⁶-(CH₂)-(CH₂)-O-R²⁷;
-C(=S)-NR²⁶-(CHR²⁸)-R²⁹;
-C(=S)-NR²⁶-C(=O)-R³⁰;
or -(CH₂)-R³¹;
R⁴ denotes -C(=O)-NH₂;
a hydrogen residue or
an alkyl residue selected from the group consisting of methyl, ethyl, n-propyl, isopropyl and n-butyl;
R⁵ denotes an alkyl residue selected from the group consisting of methyl, ethyl, n-propyl, isopropyl, n-butyl, sec-butyl, isobutyl and tert-butyl;
R⁶, R⁷ and R¹⁶, independently of one another, respectively denote a hydrogen residue or an alkyl residue selected from the group consisting of methyl, ethyl, n-propyl and isopropyl;
R⁸ denotes a (hetero)cycloaliphatic residue selected from the group consisting of cyclopropyl, cyclobutyl, cyclopentyl, cyclohexyl, pyrrolidinyl, piperidinyl, morpholinyl and thiomorpholinyl
or a residue selected from the group consisting of phenyl, naphthyl, thiophenyl, furanyl, pyridinyl and indolyl, wherein the residue may in each case be substituted with 1, 2 or 3 substituents selected independently of one another from the group consisting of F, Cl, Br, -CF₃, -O-CH₃, -O-C₂H₅, -O-CH₂-CH=CH₂, methyl, ethyl, n-propyl, isopropyl, n-butyl, tert-butyl, -S-CH₃, -S-C₂H₅, -S(=O)₂-NH₂ and -S(=O)₂-NH-CH₃;
R⁹ denotes a residue selected from the group consisting of phenyl, naphthyl, thiophenyl, furanyl and pyridinyl;
R¹⁰ denotes a possibly substituted alkyl residue selected from the group consisting of -(CH₂)-N(CH₃)₂, -(CH₂)-(CH₂)-N(CH₃)₂, -(CH₂)-(CH₂)-(CH₂)-N(CH₃)₂, -(CH₂)-N(C₂H₅)₂, -(CH₂)-(CH₂)-N(C₂H₅)₂ and -(CH₂)-(CH₂)-(CH₂)-N(C₂H₅)₂;
a cycloaliphatic residue selected from the group consisting of piperazinyl, pyrrolidinyl, piperidinyl, morpholinyl, cyclopropyl, cyclobutyl, cyclopentyl and cyclohexyl, wherein the cycloaliphatic residue may be attached via a -(CH₂)- or -(CH₂)-(CH₂)- or -(CH₂)-(CH₂)-(CH₂)- group;
or a residue selected from the group consisting of thiazolyl, phenyl, naphthyl and thieno[2,3-d]pyrimidinyl, wherein the residue may in each case be substituted with 1, 2 or 3 substituents selected independently of one another from the group consisting of F, Cl, Br, methyl, ethyl, n-propyl, -C(=O)-CH₃ and -C(=O)-C₂H₅ and/or be attached via a -(CH₂)-, -CH(CH₃)-, -(CH₂)-(CH₂)-, -(CH₂)-(CH₂)-(CH₂)- or -(CH₂)-(CH=CH)- group;
R¹¹ denotes a possibly substituted alkyl residue selected from the group consisting of methyl, ethyl, n-propyl, -CF₃ and -CF₂-CF₃,
or a residue selected from the group consisting of phenyl, and naphthyl, wherein the residue may in each case be substituted with 1, 2 or 3 substituents selected independently of one another from the group consisting of F, Cl and Br and/or be attached via a -(CH₂)- or -(CH₂)-(CH₂)- group;
R¹² denotes a possibly substituted alkyl residue selected from the group consisting of methyl, ethyl, n-propyl, -CF₃ and -CF₂-CF₃;
R¹³ denotes a hydrogen residue;
R¹⁴ denotes a residue selected from the group consisting of phenyl and naphthyl, wherein the residue may in each case be substituted with 1, 2 or 3 substituents selected independently of one another from the group consisting of F, Cl, Br, -CN, -CF₃, -O-CH₃, -O-C₂H₅, -O-CH(CH₃)₂, -O-CH₂-CH₂-CH₂-CH₃, -NO₂, -O-CF₃, -O-CHF₂, -O-CH₂F, -S-CF₃, -S-CHF₂, -S-CH₂F, methyl, ethyl, n-propyl, isopropyl, n-butyl, sec-butyl, isobutyl, tert-butyl, -C(=O)-O-CH₃, -C(=O)-O-C₂H₅, -C(=O)-CH₃ and -C(=O)-C₂H₅ and/or be attached via a -(CH₂)-, -CH(CH₃)- or -(CH₂)-(CH₂)- group;
R¹⁵ denotes a residue selected from the group consisting of 1-butenyl, 2-butenyl and 3-butenyl;
a 4,7,7-trimethyl-3-oxo-2-oxa-bicyclo[2.2.1]heptyl residue
or a residue selected from the group consisting of phenyl, naphthyl, thiophenyl, furanyl, triazolyl, pyridinyl, pyrazolyl, quinolinyl and isoquinolinyl, wherein the residue may in each case be substituted with 1, 2 or 3 substituents selected independently of one another from the group consisting of F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, -S-CF₃, -S-CHF₂, -S-CH₂F, methyl, ethyl, n-propyl, isopropyl, n-butyl, sec-butyl, isobutyl, tert-butyl, phenyl, -NH-C(=O)-CH₃ and -NH-C(=O)-C₂H₅;
R¹⁷ and R¹⁸, independently of one another, respectively denote a methyl or ethyl residue;
R¹⁹ denotes a phenyl residue, which may be attached via a -(CH₂)-, -(CH₂)-(CH₂)- or -(CH₂)-O-(CH₂)- group;
R²⁰ denotes an alkyl residue selected from the group consisting of methyl, ethyl, n-propyl, isopropyl, n-butyl, sec-butyl, isobutyl and tert-butyl
or a benzyl residue;
R²¹ denotes a cycloaliphatic residue selected from the group consisting of cyclopropyl, cyclobutyl, cyclopentyl, cyclohexyl, cycloheptyl, wherein the cycloaliphatic residue may in each case be substituted with 1, 2 or 3 substituents selected independently of one another from the group consisting of -C(=O)-OH and -(CH₂)-C(=O)-OH,
or a residue selected from the group consisting of phenyl, naphthyl, (1,3)-benzodioxolyl and (1,4)-benzodioxanyl, wherein the residue may in each case be substituted with 1, 2 or 3 substituents selected independently of one another from the group consisting of F, Cl, Br, - O-CH₃ and -O-C₂H₅;
R²² denotes a phenyl residue;
R²³ denotes a residue selected from the group consisting of phenyl, naphthyl, thiophenyl, furanyl and pyridinyl, wherein the residue may in each case be substituted with 1, 2 or 3 substituents selected independently of one another from the group consisting of F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, methyl, ethyl, n-propyl, isopropyl, n-butyl, sec-butyl, isobutyl and tert-butyl;
R²⁴ and R²⁵, independently of one another, respectively denote a methyl or ethyl residue;
R²⁶ denotes a hydrogen residue;
R²⁷ denotes an alkyl residue selected from the group consisting of methyl, ethyl, n-propyl, isopropyl and n-butyl;
a cyclohexyl residue
or a phenyl residue, which may be substituted with 1, 2, 3, 4 or 5 substituents selected independently of one another from the group consisting of F, Cl, Br, -O-CH₃ and -O-C₂H₅;
R²⁸ denotes a phenyl residue, which may be substituted with 1, 2, 3, 4 or 5 substituents selected independently of one another from the group consisting of F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, -S-CH₃, -S-C₂H₅, methyl, ethyl, n-propyl, isopropyl, n-butyl, sec-butyl, isobutyl, tert-butyl;
R²⁹ denotes an alkyl residue selected from the group consisting of methyl, ethyl, n-propyl, isopropyl, n-butyl, isobutyl and tert-butyl; R³⁰ denotes a phenyl residue
and
R³¹ denotes a residue selected from the group consisting of phenyl and naphthyl, wherein the residue may in each case be substituted with 1, 2 or 3 substituents selected independently of one another from the group consisting of F, Cl and Br;
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

5. Compounds according to one or more of claims 1 to 4 selected from the group consisting of
[1] 6,7-dihydro-4H-isoxazolo[4,5-c]pyridine-3,5-dicarboxylic acid 3-[(2-methoxy-ethyl) amide] 5-[(3-methoxyphenyl) amide],
[2] 6,7-dihydro-4H-isoxazolo[4,5-c]pyridine-3,5-dicarboxylic acid 3-cyclopentyl amide 5-(4-fluorobenzyl amide),
[3] 6,7-dihydro-4H-isoxazolo[4,5-c]pyridine-3,5-dicarboxylic acid 3-phenyl amide 5-[(4-trifluoromethoxy-phenyl) amide],
[4] 6,7-dihydro-4H-isoxazolo[4,5-c]pyridine-3,5-dicarboxylic acid 5-[(4-methyl-3-nitro-phenyl) amide] 3-(phenethyl amide),
[5] 6,7-dihydro-4H-isoxazolo[4,5-c]pyridine-3,5-dicarboxylic acid 3-[(2-methoxy-ethyl) amide] 5-[(4-methyl-3-nitro-phenyl) amide],
[6] 3- {[5-(2,5-difluoro-phenylcarbamoyl)-4,5,6,7-tetrahydro-isoxazolo[4,5-c]pyridine-3-carbonyl]-amino}-propionic acid ethyl ester,
[7] 6,7-dihydro-4H-isoxazolo[4,5-c]pyridine-3,5-dicarboxylic acid 5-[(4-butoxy-phenyl) amide] 3-[(2-methoxy-ethyl) amide],
[8] 6,7-dihydro-4H-isoxazolo[4,5-c]pyridine-3,5-dicarboxylic acid 5-[(3-fluorophenyl) amide] 3-(phenethyl amide),
[9] 6,7-dihydro-4H-isoxazolo[4,5-c]pyridine-3,5-dicarboxylic acid 5-[(4-methyl-3-nitro-phenyl) amide] 3-[(thiophen-2-ylmethyl) amide],
[10] 6,7-dihydro-4H-isoxazolo[4,5-c]pyridine-3,5-dicarboxylic acid 3-benzyl amide 5-(phenethyl amide),
[11] 6,7-dihydro-4H-isoxazolo[4,5-c]pyridine-3,5-dicarboxylic acid 3-[(2-ethylsulphanyl-ethyl) amide] 5-[(3-methoxyphenyl) amide],
[12] 6,7-dihydro-4H-isoxazolo[4,5-c]pyridine-3,5-dicarboxylic acid 5-[(3-cyano-phenyl) amide] 3-[(thiophen-2-ylmethyl) amide],
[13] 6,7-dihydro-4H-isoxazolo[4,5-c]pyridine-3,5-dicarboxylic acid 5-[(4-ethoxy-phenyl) amide] 3-phenyl amide,
[14] 6,7-dihydro-4H-isoxazolo[4,5-c]pyridine-3,5-dicarboxylic acid 3-(4-fluorobenzyl amide) 5-[(4-methyl-3-nitro-phenyl) amide],
[15] 6,7-dihydro-4H-isoxazolo[4,5-c]pyridine-3,5-dicarboxylic acid 5-[(3-acetyl-phenyl) amide] 3-[(5-methyl-furan-2-ylmethyl) amide],
[16] 6,7-dihydro-4H-isoxazolo[4,5-c]pyridine-3,5-dicarboxylic acid 5-[(4-methyl-3-nitro-phenyl) amide] 3-prop-2-ynyl amide,
[17] 6,7-dihydro-4H-isoxazolo[4,5-c]pyridine-3,5-dicarboxylic acid 3-benzyl amide 5-phenyl amide,
[18] 6,7-dihydro-4H-isoxazolo[4,5-c]pyridine-3,5-dicarboxylic acid 5-[(1-naphthalen-1-yl-ethyl) amide] 3-(phenethyl amide),
[19] 6,7-dihydro-4H-isoxazolo[4,5-c]pyridine-3,5-dicarboxylic acid 5-[(3-fluorophenyl) amide] 3-prop-2-ynyl amide,
[20] 3- {[5-(2,5-dimethoxy-phenylcarbamoyl)-4,5,6,7-tetrahydro-isoxazolo[4,5-c]pyridine-3-carbonyl]-amino}-propionic acid ethyl ester,
[21] 6,7-dihydro-4H-isoxazolo[4,5-c]pyridine-3,5-dicarboxylic acid 5-[(4-ethoxy-phenyl) amide] 3-(4-fluorobenzyl amide),
[22] 6,7-dihydro-4H-isoxazolo[4,5-c]pyridine-3,5-dicarboxylic acid 5-[(5-chloro-2-methoxyphenyl) amide] 3-(phenethyl amide),
[23] 3-({3-[(5-methyl-furan-2-ylmethyl)-carbamoyl]-6,7-dihydro-4H-isoxazolo[4,5-c]pyridine-5-carbonyl}-amino)-benzoic acid ethyl ester,
[24] 6,7-dihydro-4H-isoxazolo[4,5-c]pyridine-3,5-dicarboxylic acid 5-[(5-chloro-2-methoxyphenyl) amide] 3-(isobutyl-amide),
[25] [2-oxo-2-(3-prop-2-ynylcarbamoyl-6,7-dihydro-4H-isoxazolo[4,5-c]pyridin-5-yl)-ethoxy]acetic acid,
[26] 3-ethyl-5-[3-(2-methoxy-ethylcarbamoyl)-6,7-dihydro-4H-isoxazolo[4,5-c]pyridin-5-yl]-3-methyl-5-oxo-pentanoic acid,
[27] (1-benzyloxymethyl-2-oxo-2-{3-[(pyridin-3-ylmethyl)-carbamoyl]-6,7-dihydro-4H-isoxazolo[4,5-c]pyridin-5-yl}-ethyl)-carbamic acid tert-butyl ester,
[28] {1-[2-oxo-2-(3-phenylcarbamoyl-6,7-dihydro-4H-isoxazolo[4,5-c]pyridin-5-yl)-ethyl]-cyclopentyl} acetic acid,
[29] 3-ethyl-3-methyl-5-oxo-5-(3-phenylcarbamoyl-6,7-dihydro-4H-isoxazolo[4,5-c]pyridin-5-yl)-pentanoic acid,
[30] (2-oxo-2-{3-[(thiophen-2-ylmethyl)-carbamoyl]-6,7-dihydro-4H-isoxazolo[4,5-c]pyridin-5-yl}-ethoxy)acetic acid,
[31] 3,3-dimethyl-4-{3-[(5-methyl-furan-2-ylmethyl)-carbamoyl]-6,7-dihydro-4H-isoxazolo[4,5-c]pyridin-5-yl}-4-oxo-butanoic acid,
[32] 5-(4-trifluoromethoxy-benzenesulphonyl)-4,5,6,7-tetrahydro-isoxazolo[4,5-c]pyridine-3-carboxylic acid benzyl-phenethyl amide,
[33] 5-benzenesulphonyl-4,5,6,7-tetrahydro-isoxazolo[4,5-c]pyridine-3-carboxylic acid (1-naphthalen-2-ylmethyl-pyrrolidin-3-yl) amide,
[34] [5-(4-fluoro-benzenesulphonyl)-4,5,6,7-tetrahydro-isoxazolo[4,5-c]pyridin-3-yl]-(4-thieno[2,3-d]pyrimidine-4-yl-piperazin-1-yl)-methanone,
[35] 5-(4-fluoro-benzenesulphonyl)-4,5,6,7-tetrahydro-isoxazolo[4,5-c]pyridine-3-carboxylic acid (3-phenyl-propyl) amide,
[36] 5-(3-phenyl-acryloyl)-4,5,6,7-tetrahydro-isoxazolo[4,5-c]pyridine-3-carboxylic acid (2-dimethylamino-ethyl) amide,
[37] 5-(2-phenoxy-acetyl)-4,5,6,7-tetrahydro-isoxazolo[4,5-c]pyridine-3-carboxylic acid (2-dimethylamino-ethyl) amide,
[38] 5-(4-fluoro-benzenesulphonyl)-4,5,6,7-tetrahydro-isoxazolo[4,5-c]pyridine-3-carboxylic acid (1-methyl-3-phenyl-propyl) amide,
[39] 5-(4-fluoro-benzenesulphonyl)-4,5,6,7-tetrahydro-isoxazolo[4,5-c]pyridine-3-carboxylic acid [1-(4-trifluoromethyl(-benzyl)-pyrrolidin-3-yl] amide,
[40] 5-(2,5-dichloro-benzenesulphonyl)-4,5,6,7-tetrahydio-isoxazolo[4,5-c]pyridine-3-carboxylic acid (2-benzyloxy-cyclopentyl) amide,
[41] 5-(5-tert-butyl-2-methyl-2H-pyrazole-3-carbonyl)-4,5,6,7-tetrahydro-isoxazolo[4,5-c]pyridine-3-carboxylic acid (2-dimethylamino-ethyl) amide,
[42] [5-(4-fluoro-benzenesulphonyl)-4,5,6,7-tetrahydro-isoxazolo[4,5-c]pyridin-3-yl]-[4-(4-fluorophenyl)-3,6-dihydro-2H-pyridin-1-yl]-methanone,
[43] 4- {[5-(4-fluoro-benzenesulphonyl)-4,5,6,7-tetrahydro-isoxazolo[4,5-c]pyridine-3-carbonyl]-amino}-piperidine-1-carboxylic acid ethyl ester,
[44] 5-(3-phenyl-acryloyl)-4,5,6,7-tetrahydro-isoxazolo[4,5-c]pyridine-3-carboxylic acid cyclopentyl amide,
[45] 5-(4-acetylamino-benzoyl)-4,5,6,7-tetrahydro-isoxazolo[4,5-c]pyridine-3-carboxylic acid cyclopropylmethyl amide,
[46] 5-(5-methyl-2-phenyl-2H-[1,2,3]triazole-4-carbonyl)-4,5,6,7-tetrahydro-isoxazolo[4,5-c]pyridine-3-carboxylic acid (pyridin-3-ylmethyl) amide,
[47] 5-benzenesulphonyl-4,5,6,7-tetrahydro-isoxazolo[4,5-c]pyridine-3-carboxylic acid 3,4-dimethoxy-benzyl amide,
[48] 2-(3,4-difluoro-phenyl)-1-{4-[5-(4-fluoro-benzenesulphonyl)-4,5,6,7-tetrahydro-isoxazolo[4,5-c]pyridine-3-carbonyl]-piperazin-1-yl}-ethanone,
[49] 3-(morpholine-4-carbonyl)-6,7-dihydro-4H-isoxazolo[4,5-c]pyridine-5-sulphonic acid dimethyl amide,
[50] 5-(furan-2-carbonyl)-4,5,6,7-tetrahydro-isoxazolo[4,5-c]pyridine-3-carboxylic acid (thiophen-2-ylmethyl) amide,
[51] 5-(4,7,7-trimethyl-3-oxo-2-oxa-bicyclo[2.2.1]heptane-1-carbonyl)-4,5,6,7-tetrahydro-isoxazolo[4,5-c]pyridine-3-carboxylic acid (2-pyrrolidin-1-yl-ethyl) amide,
[52] 5-(2,5-dichloro-benzenesulphonyl)-4,5,6,7-tetrahydro-isoxazolo[4,5-c]pyridine-3-carboxylic acid [2-(2-chlorophenyl)-ethyl] amide,
[53] 5-(4-methoxy-benzenesulphonyl)-4,5,6,7-tetrahydro-isoxazolo[4,5-c]pyridine-3-carboxylic acid p-tolyl amide,
[54] 5-(2-phenoxy-acetyl)-4,5,6,7-tetrahydro-isoxazolo[4,5-c]pyridine-3-carboxylic acid 4-fluorobenzyl amide,
[55] 1-{4-[5-(4-methoxy-benzenesulphonyl)-4,5,6,7-tetrahydro-isoxazolo[4,5-c]pyridine-3-carbonyl]-piperazin-1-yl}-ethanone,
[56] 5-(toluene-4-sulphonyl)-4,5,6,7-tetrahydro-isoxazolo[4,5-c]pyridine-3-carboxylic acid 4-sulphamoyl-benzyl amide,
[57] 5-(thiophene-2-sulphonyl)-4,5,6,7-tetrahydro-isoxazolo[4,5-c]pyridine-3-carboxylic acid (2-morpholin-4-yl-ethyl) amide,
[58] 5-(4-methoxy-benzoyl)-4,5,6,7-tetrahydro-isoxazolo[4,5-c]pyridine-3-carboxylic acid (2-dimethylamino-ethyl) amide,
[59] 5-(2,5-dichloro-benzenesulphonyl)-4,5,6,7-tetrahydro-isoxazolo[4,5-c]pyridine-3-carboxylic acid benzyl-phenethyl amide,
[60] 5-dimethylsulphamoyl-4,5,6,7-tetrahydro-isoxazolo[4,5-c]pyridine-3-carboxylic acid (2,6,6-trimethyl-bicyclo[3.1.1]hept-3-yl) amide,
[61] 5-benzenesulphonyl-4,5,6,7-tetrahydro-isoxazolo[4,5-c]pyridine-3-carboxylic acid indan-1-yl amide,
[62] 5-(4-methoxy-benzenesulphonyl)-4,5,6,7-tetrahydro-isoxazolo[4,5-c]pyridine-3-carboxylic acid (3-methoxy-propyl) amide,
[63] 5-(4,5-dichloro-thiophene-2-sulphonyl)-4,5,6,7-tetrahydro-isoxazolo[4,5-c]pyridine-3-carboxylic acid (2-morpholin-4-yl-ethyl) amide,
[64] 5-(2-benzyloxy-acetyl)-4,5,6,7-tetrahydro-isoxazolo[4,5-c]pyridine-3-carboxylic acid (2-pyrrolidin-1-yl-ethyl) amide,
[65] [5-(2,5-dichloro-benzenesulphonyl)-4,5,6,7-tetrahydro-isoxazolo[4,5-c]pyridin-3-yl]-[4-(2,4-dimethyl-phenyl)-piperazin-1-yl]-methanone,
[66] 5-pent-4-enoyl-4,5,6,7-tetrahydro-isoxazolo[4,5-c]pyridine-3-carboxylic acid cyclopropylmethyl amide,
[67] 3-methyl-2- {[5-(3-trifluoromethyl-benzenesulphonyl)-4,5,6,7-tetrahydro-isoxazolo[4,5-c]pyridine-3-carbonyl]-amino}-butanoic acid tert-butyl ester,
[68] 5-(3-trifluoromethyl-benzenesulphonyl)-4,5,6,7-tetrahydro-isoxazolo[4,5-c]pyridine-3-carboxylic acid benzyl-phenethyl amide,
[69] 5-(3-trifluoromethyl-benzenesulphonyl)-4,5,6,7-tetrahydro-isoxazolo[4,5-c]pyridine-3-carboxylic acid 2,4-dichloro-benzyl amide,
[70] 5-dimethylsulphamoyl-4,5,6,7-tetrahydro-isoxazolo[4,5-c]pyridine-3-carboxylic acid (2-cyano-ethyl)-pyridin-3-ylmethyl-amide,
[71] 5-(2-cyclopentyl-acetyl)-4,5,6,7-tetrahydro-isoxazolo[4,5-c]pyridine-3-carboxylic acid (2-pyrrolidin-1-yl-ethyl) amide,
[72] 5-(5-methyl-2-phenyl-2H-[1,2,3]triazole-4-carbonyl)-4,5,6,7-tetrahydro-isoxazolo[4,5-c]pyridine-3-carboxylic acid 4-fluorobenzyl amide,
[73] 5-(3-trifluoromethyl-benzenesulphonyl)-4,5,6,7-tetrahydro-isoxazolo[4,5-c]pyridine-3-carboxylic acid (2-ethyl-hexyl)-amide,
[74] 5-(2,3-difluoro-4-methyl-benzoyl)-4,5,6,7-tetrahydro-isoxazolo[4,5-c]pyridine-3-carboxylic acid (2-dimethylamino-ethyl) amide,
[75] 5-(4-trifluoromethoxy-benzenesulphonyl)-4,5,6,7-tetrahydro-isoxazolo[4,5-c]pyridine-3-carboxylic acid 2,3-dichloro-benzyl amide,
[76] 5-dimethylsulphamoyl-4,5,6,7-tetrahydro-isoxazolo[4,5-c]pyridine-3-carboxylic acid [2-(4-chlorophenyl)-ethyl] amide,
[77] 5-(6-chloro-pyridine-3-carbonyl)-4,5,6,7-tetrahydro-isoxazolo[4,5-c]pyridine-3-carboxylic acid (2-pyrrolidin-1-yl-ethyl) amide,
[78] 5-[3-(4-fluoro-benzylcarbamoyl)-6,7-dihydro-4H-isoxazolo[4,5-c]pyridin-5-yl]-5-oxo-pentanoic acid methyl ester,
[79] 5-[2-(4-methoxyphenyl)-acetyl]-4,5,6,7-tetrahydro-isoxazolo[4,5-c]pyridine-3-carboxylic acid benzyl amide,
[80] 5-(4-trifluoromethoxy-benzenesulphonyl)-4,5,6,7-tetrahydro-isoxazolo[4,5-c]pyridine-3-carboxylic acid [3-(methylphenyl-amino)-propyl] amide,
[81] 5-[2-(4-chlorophenoxy)-acetyl]-4,5,6,7-tetrahydro-isoxazolo[4,5-c]pyridine-3-carboxylic acid cyclopentyl amide,
[82] [4-(2-cyclohexyl-ethyl)-piperazin-1-yl]-[5-(toluene-4-sulphonyl)-4,5,6,7-tetrahydro-isoxazolo[4,5-c]pyridin-3-yl]-methanone,
[83] N- {1-[5-(2,5-dichloro-benzenesulphonyl)-4,5,6,7-tetrahydro-isoxazolo[4,5-c]pyridine-3-carbonyl]-piperidin-4-ylmethyl)-2,2,2-trifluoroacetamide,
[84] 5-(4-methoxy-benzenesulphonyl)-4,5,6,7-tetrahydro-isoxazolo[4,5-c]pyridine-3-carboxylic acid [1-(4-methoxyphenyl)-ethyl] amide,
[85] 2- {[5-(thiophene-2-sulphonyl)-4,5,6,7-tetrahydro-isoxazolo[4,5-c]pyridine-3-carbonyl]-amino}-propionic acid ethyl ester,
[86] 1-(4-{4-[5-(toluene-4-sulphonyl)-4,5,6,7-tetrahydro-isoxazolo[4,5-c]pyridine-3-carbonyl]-piperazin-1-yl}-phenyl)-ethanone,
[87] 5-(3-trifluoromethyl-benzenesulphonyl)-4,5,6,7-tetrahydro-isoxazolo[4,5-c]pyridine-3-carboxylic acid (4-allyloxy-benzyl)-furan-2-ylmethyl amide,
[88] 4-carbamoyl-4- {[5-(4-methoxy-benzenesulphonyl)-4,5,6,7-tetrahydro-isoxazolo[4,5-c]pyridine-3-carbonyl]-amino}-butanoic acid tert-butyl ester,
[89] 5-dimethylsulphamoyl-4,5,6,7-tetrahydro-isoxazolo[4,5-c]pyridine-3-carboxylic acid furan-2-ylmethyl-(4-methylsulphanyl-benzyl) amide,
[90] 5-(4-bromo-3-methyl-benzoyl)-4,5,6,7-tetrahydro-isoxazolo[4,5-c]pyridine-3-carboxylic acid (2-morpholin-4-yl-ethyl) amide,
[91] 5-(toluene-4-sulphonyl)-4,5,6,7-tetrahydro-isoxazolo[4,5-c]pyridine-3-carboxylic acid [1-(2,6-dichloro-benzyl)-pyrrolidin-3-yl] amide,
[92] 5-benzenesulphonyl-4,5,6,7-tetrahydro-isoxazolo[4,5-c]pyridine-3-carboxylic acid [2-(3,4-dichloro-phenyl)-ethyl] amide,
[93] 5-(3-difluoromethylsulphanyl-benzoyl)-4,5,6,7-tetrahydro-isoxazolo[4,5-c]pyridine-3-carboxylic acid (2-dimethylamino-ethyl) amide,
[94] 5-[2-(3-chlorophenoxy)-acetyl]-4,5,6,7-tetrahydro-isoxazolo[4,5-c]pyridine-3-carboxylic acid (2-pyrrolidin-1-yl-ethyl) amide,
[95] 5-(4-methoxy-benzenesulphonyl)-4,5,6,7-tetrahydro-isoxazolo[4,5-c]pyridine-3-carboxylic acid 2,3-dimethoxy-benzyl amide,
[96] 5-(4-trifluoromethoxy-benzenesulphonyl)-4,5,6,7-tetrahydro-isoxazolo[4,5-c]pyridine-3-carboxylic acid (1-methyl-3-phenyl-propyl) amide,
[97] (5-benzenesulphonyl-4,5,6,7-tetrahydro-isoxazolo[4,5-c]pyridin-3-yl)-[4-(3-phenyl-allyl)-piperazin-1-yl]-methanone,
[98] 3- {[5-(4-methoxy-benzoyl)-4,5,6,7-tetrahydro-isoxazolo[4,5-c]pyridine-3-carbonyl]-amino}-propionic acid ethyl ester,
[99] 5-(4-phenoxy-butyryl)-4,5,6,7-tetrahydro-isoxazolo[4,5-c]pyridine-3-carboxylic acid (2-dimethylamino-ethyl) amide,
[100] 5-(4-trifluoromethoxy-benzenesulphonyl)-4,5,6,7-tetrahydro-isoxazolo[4,5-c]pyridine-3-carboxylic acid [2-(7-methyl-1H-indol-3-yl)-ethyl] amide,
[101] [4-(3-phenyl-allyl)-piperazin-1-yl]-[5-(thiophene-2-sulphonyl)-4,5,6,7-tetrahydro-isoxazolo[4,5-c]pyridin-3-yl]-methanone,
[102] 5-(4-bromo-3-methyl-benzoyl)-4,5,6,7-tetrahydro-isoxazolo[4,5-c]pyridine-3-carboxylic acid (2-pyrrolidin-1-yl-ethyl) amide,
[103] 5-benzenesulphonyl-4,5,6,7-tetrahydro-isoxazolo[4,5-c]pyridine-3-carboxylic acid [1-(4-trifluoromethyl-benzyl)-pyrrolidin-3-yl] amide,
[104] 5-(thiophene-2-sulphonyl)-4,5,6,7-tetrahydro-isoxazolo[4,5-c]pyridine-3-carboxylic acid (2-phenyl-propyl) amide,
[105] 5-(3-chloro-4-fluoro-benzoyl)-4,5,6,7-tetrahydro-isoxazolo[4,5-c]pyridine-3-carboxylic acid phenethyl amide,
[106] 5-benzenesulphonyl-4,5,6,7-tetrahydro-isoxazolo[4,5-c]pyridine-3-carboxylic acid [2-(4-methoxy-phenoxy)-ethyl]amide,
[107] 5-dimethylsulphamoyl-4,5,6,7-tetrahydro-isoxazolo[4,5-c]pyridine-3-carboxylic acid 3-fluoro-4-trifluoromethyl-benzyl amide,
[108] 5-(3-methyl-benzoyl)-4,5,6,7-tetrahydro-isoxazolo[4,5-c]pyridine-3-carboxylic acid prop-2-ynyl amide,
[109] 5-(toluene-4-sulphonyl)-4,5,6,7-tetrahydro-isoxazolo[4,5-c]pyridine-3-carboxylic acid [2-(4-methoxy-phenoxy)-ethyl] amide,
[110] [4-(2-chlorophenyl)-piperazin-1-yl]-[5-(2,5-dichloro-benzenesulphonyl)-4,5,6,7-tetrahydro-isoxazolo[4,5-c]pyridin-3-yl]-methanone,
[111] 3-{[5-(2,6-difluoro-3-methyl-benzoyl)-4,5,6,7-tetrahydro-isoxazolo[4,5-c]pyridine-3-carbonyl]-amino}-propionic acid ethyl ester,
[112] 5-(2,5-dichloro-benzenesulphonyl)-4,5,6,7-tetrahydro-isoxazolo[4,5-c]pyridine-3-carboxylic acid 2,4-difluoro-benzyl amide,
[113] 5-benzenesulphonyl-4,5,6,7-tetrahydro-isoxazolo[4,5-c]pyridine-3-carboxylic acid 3-fluoro-4-trifluoromethyl-benzyl amide,
[114] 5-(4-butoxy-benzenesulphonyl)-4,5,6,7-tetrahydro-isoxazolo[4,5-c]pyridine-3-carboxylic acid isobutyl amide,
[115] 5-(3-fluoro-4-methyl-benzoyl)-4,5,6,7-tetrahydro-isoxazolo[4,5-c]pyridine-3-carboxylic acid (2-pyrrolidin-1-yl-ethyl) amide,
[116] 5-(2-chloro-5-trifluoromethyl-benzoyl)-4,5,6,7-tetrahydro-isoxazolo[4,5-c]pyridine-3-carboxylic acid isobutyl amide,
[117] 5-(4-trifluoromethoxy-benzenesulphonyl)-4,5,6,7-tetrahydro-isoxazolo[4,5-c]pyridine-3-carboxylic acid (1-benzyl-pyrrolidin-3-yl) amide,
[118] 5-(3-chloro-benzenesulphonyl)-4,5,6,7-tetrahydro-isoxazolo[4,5-c]pyridine-3-carboxylic acid (2-pyrrolidin-1-yl-ethyl) amide,
[119] 5-(3-trifluoromethoxy-benzoyl)-4,5,6,7-tetrahydro-isoxazolo[4,5-c]pyridine-3-carboxylic acid (2-dimethylamino-ethyl) amide,
[120] 2- {[5-(toluene-4-sulphonyl)-4,5,6,7-tetrahydro-isoxazolo[4,5-c]pyridine-3-carbonyl]-amino}-propionic acid tert-butyl ester,
[121] 3-{[5-(4-chloro-benzenesulphonyl)-4,5,6,7-tetrahydro-isoxazolo[4,5-c]pyridine-3-carbonyl]-amino}-propionic acid ethyl ester,
[122] 5-dimethylsulphamoyl-4,5,6,7-tetrahydro-isoxazolo[4,5-c]pyridine-3-carboxylic acid [2-(3,4-dichloro-phenyl)-ethyl] amide,
[123] 5-(thiophene-2-sulphonyl)-4,5,6,7-tetrahydro-isoxazolo[4,5-c]pyridine-3-carboxylic acid cyclopropylmethyl amide,
[124] 5-(quinoline-6-carbonyl)-4,5,6,7-tetrahydro-isoxazolo[4,5-c]pyridine-3-carboxylic acid 4-fluorobenzyl amide,
[125] 3- {[5-(2-naphthalen-2-yl-acetyl)-4,5,6,7-tetrahydro-isoxazolo[4,5-c]pyridine-3-carbonyl]-amino}-propionic acid ethyl ester,
[126] [2-(3-isobutylcarbamoyl-6,7-dihydro-4H-isoxazolo[4,5-c]pyridin-5-yl)-2-oxo-1-phenyl-ethyl]-carbamic acid benzyl ester,
[127] 5-(4-methoxy-benzylthiocarbamoyl)-4,5,6,7-tetrahydro-isoxazolo[4,5-c]pyridine-3-carboxylic acid cyclopentyl amide,
[128] 5-benzoylaminocarbothioyl-4,5,6,7-tetrahydro-isoxazolo[4,5-c]pyridine-3-carboxylic acid (2-pyrrolidin-1-yl-ethyl) amide,
[129] 5-(4-chloro-benzylthiocarbamoyl)-4,5,6,7-tetrahydro-isoxazolo[4,5-c]pyridine-3-carboxylic acid cyclopropylmethyl amide,
[130] 5-(2-methoxy-ethylthiocarbamoyl)-4,5,6,7-tetrahydro-isoxazolo[4,5-c]pyridine-3-carboxylic acid phenethyl amide,
[131] 5-pentafluorophenylthiocarbamoyl-4,5,6,7-tetrahydro-isoxazolo[4,5-c]pyridine-3-carboxylic acid phenethyl amide,
[132] 5-(1-phenyl-ethylthiocarbamoyl)-4,5,6,7-tetrahydro-isoxazolo[4,5-c]pyridine-3-carboxylic acid phenethyl amide,
[133] 5-pentafluorophenylthiocarbamoyl-4,5,6,7-tetrahydro-isoxazolo[4,5-c]pyridine-3-carboxylic acid (5-methyl-furan-2-ylmethyl) amide,
[134] 3-{[5-(cyclohexylmethyl-thiocarbamoyl)-4,5,6,7-tetrahydro-isoxazolo[4,5-c]pyridine-3-carbonyl]-amino}-propionic acid ethyl ester,
and
[135] 5-(1-bromo-naphthalen-2-ylmethyl)-4,5,6,7-tetrahydro-isoxazolo[4,5-c]pyridine-3-carboxylic acid phenyl amide,
in each case possibly in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

6. A method for the production of substituted 4,5,6,7-tetrahydro-isoxazolo[4,5-c]pyridine compounds of the general formula I according to one or more of claims 1 to 5, **characterised in that** at least one compound of the general formula II, 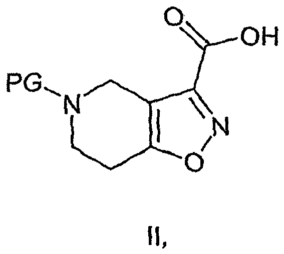 in which PG denotes a protective group, preferably a tert-butyloxy-carbonyl or benzyloxycarbonyl group, is reacted in a reaction medium, in the presence of at least one coupling reagent, possibly in the presence of at least one base, preferably in the presence of at least one base selected from the group consisting of pyridine, N-methylmorpholine, diisopropylethylamine, triethylamine and 4,4-dimethylaminopyridine, with at least one amine of the general formula HNR¹R², in which the residues R¹ and R² have the meaning according to one or more of claims 1 to 5, to yield at least one compound of the general formula III, 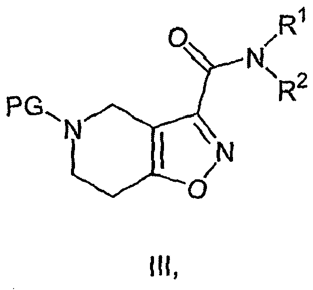 in which R¹, R² and PG have the above-stated meaning and said compound is possibly purified and/or isolated,
and at least one compound of the general formula III is reacted by elimination in a reaction medium, preferably in the presence of at least one acid or in the presence of at least one base for the tert-butyloxy-carbonyl group or in the presence of hydrogen and catalyst, preferably palladium on carbon, for the benzyloxycarbonyl group to yield at least one compound of the general formula IV, possibly in the form of a corresponding salt, preferably in the form of a corresponding hydrochloride, 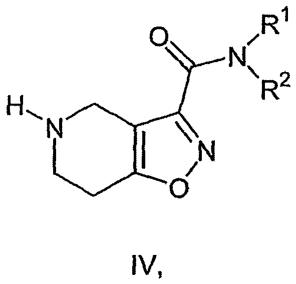 in which R¹ and R² have the above-stated meaning and said compound is possibly purified and/or isolated,
and
possibly at least one compound of the general formula IV in the form of a corresponding salt, preferably in the form of a corresponding hydrochloride, is reacted in a reaction medium in the presence of at least one base, preferably in the presence of at least one metal hydroxide, particularly preferred in the presence of potassium hydroxide and/or sodium hydroxide, to yield at least one compound of the general formula IV, and said compound is possibly purified and/or isolated,
and
at least one compound of the general formula IV is reacted in a reaction medium, possibly in the presence of at least one base, preferably in the presence of at least one base selected from the group consisting of pyridine, triethylamine, 4,4-dimethylaminopyridine, diisopropylethylamine and diisopropylamine, with at least one carboxylic acid derivative of the general formula LG-C(=O)-R¹⁵, LG-C(=O)-(CH₂)ⱼXₖ-(CH₂)ₘ-(CH₂)ₙ-C(=O)-OR¹⁶, LG-C(=O)-(CHR¹⁹)-NH-C(=O)-OR²⁰, LG-C(=O)-(CH₂)-(CH₂)ₚ-(CH₂)_{q}-Yᵣ-(CH₂)ₛ-R²¹ or LG-C(=O)-(CH=CH)-R²², in which R¹⁵, R¹⁶, R¹⁹, R²⁰, R²¹, R²², j, k, m, n, p, q, r, s, X and Y have the meaning according to one or more of claims 1 to 5 and LG denotes a leaving group, preferably a halogen atom, particularly preferred a chlorine atom, to yield at least one compound of the general formula I, in which R¹ and R² have the above-stated meaning and R³ denotes -C(=O)-R¹⁵, -C(=O)-(CH₂)ⱼ-Xₖ-(CH₂)ₘ-(CH₂)ₙ-C(=O)-OR¹⁶, -C(=O)-(CHR¹⁹)-NH-C(=O)-OR²⁰, -C(=O)-(CH₂)-(CH₂)ₚ-(CH₂)_{q}-Yᵣ-(CH₂)ₛ-R²¹ or -C(=O)-(CH=CH)-R²², wherein R¹⁵, R¹⁶, R¹⁹, R²⁰, R²¹, R²², j, k, m, n, p, q, r, s, X and Y have the above-stated meaning, and said compound is possibly purified and/or isolated
or
at least one compound of the general formula IV is reacted in a reaction medium, in the presence of a coupling reagent, possibly in the presence of at least one base, preferably in the presence of at least one base selected from the group consisting of pyridine, N-methylmorpholine, diisopropylethylamine, triethylamine and 4,4-dimethylaminopyridine, with at least one carboxylic acid of the general formula HO-C(=O)-R¹⁵, HO-C(=O)-(CH₂)ⱼXₖ-(CH₂)ₘ-(CH₂)ₙ-C(=O)-OR¹⁶, HO-C(=O)-(CHR¹⁹)-NH-C(=O)-OR²⁰, HO-C(=O)-(CH₂)-(CH₂)ₚ-(CH₂)_{q}-Yᵣ-(CH₂)ₛ-R²¹ or HO-C(=O)-(CH=CH)-R²², in which R¹⁵, R¹⁶, R¹⁹, R²⁰, R²¹, R²², j, k, m, n, p, q, r, s, X and Y have the meaning according to one or more of claims 1 to 5, to yield at least one compound of the general formula I, in which R¹ and R² have the above-stated meaning and R³ denotes -C(=O)-R¹⁵, -C(=O)-(CH₂)ⱼXₖ-(CH₂)ₘ-(CH₂)ₙ-C(=O)-OR¹⁶,-C(=O)-(CHR¹⁹)-NH-C(=O)-OR²⁰, -C(=O)-(CH₂)-(CH₂)ₚ-(CH₂)_{q}-Yᵣ-(CH₂)ₛ-R²¹ or -C(=O)-(CH=CH)-R²², wherein R¹⁵, R¹⁶, R¹⁹, R²⁰, R²¹, R²², j, k, m, n, p, q, r, s, X and Y have the above-stated meaning, and said compound is possibly purified and/or isolated
or
at least one compound of the general formula IV is reacted in a reaction medium, possibly in the presence of at least one base, preferably in the presence of at least one base selected from the group consisting of pyridine, triethylamine, 4,4-dimethylaminopyridine, diisopropylethylamine and diisopropylamine, with at least one sulphonic acid derivative of the general formula LG-S(=O)₂-R²³ or LG-S(=O)₂-NR²⁴R²⁵, in which R²³, R²⁴ and R²⁵ have the meaning according to one or more of claims 1 to 5 and LG denotes a leaving group, preferably for a halogen atom, particularly preferred a chlorine atom, to yield at least one compound of the general formula I, in which R¹ and R² have the above-stated meaning and R³ denotes -S(=O)₂-R²³ or -S(=O)₂-NR²⁴R²⁵, wherein R²³, R²⁴ and R²⁵ have the above-stated meaning, and said compound is possibly purified and/or isolated
or
at least one compound of the general formula IV is reacted in a reaction medium with at least one isocyanate of the general formula R¹⁴-N=C=O, in which R¹⁴ has the meaning according to one or more of claims 1 to 5, possibly in the presence of at least one base, preferably in the presence of at least one base selected from the group consisting of triethylamine, 4,4-dimethylaminopyridine and diisopropylethylamine, to yield at least one compound of the general formula I, in which R¹ and R² have the above-stated meaning and R³ denotes -C(=O)-NR¹³R¹⁴, wherein R¹⁴ has the above-stated meaning and R¹³ denotes hydrogen, and said compound is possibly purified and/or isolated
or
at least one compound of the general formula IV is reacted in a reaction medium with at least one isothiocyanate of the general formula S=C=N-(CH₂)ₜ(CH₂)ᵤ-Zᵥ-R²⁷, S=C=N-(CHR²⁸)-R²⁹ or S=C=N-C(=O)-R³⁰, in which R²⁷, R²⁸, R²⁹, R³⁰, Z, t, u and v have the meaning according to one or more of claims 1 to 5, possibly in the presence of at least one base, preferably in the presence of at least one base selected from the group consisting of triethylamine, 4,4-dimethylaminopyridine and diisopropylethylamine, to yield at least one compound of the general formula I, in which R¹ and R² have the above-stated meaning and R³ denotes -C(=S)-NR²⁶-(CH₂)ₜ(CH₂)ᵤ-Zᵥ-R²⁷, -C(=S)-NR²⁶-(CHR²⁸)-R²⁹ or -C(=S)-NR²⁶-C(=O)-R³⁰,
wherein R²⁷, R²⁸, R²⁹, R³⁰, Z, t, u and v have the above-stated meaning and R²⁶ denotes hydrogen, and said compound is possibly purified and/or isolated
and possibly at least one compound of the general formula I, in which R¹ and R² have the above-stated meaning and R³ denotes -C(=S)-NR²⁶-(CH₂)ₜ(CH₂)ᵤ-Zᵥ-R²⁷, -C(=S)-NR²⁶-(CHR²⁸)-R²⁹, -C(=S)-NR²⁶-C(=O)-R³⁰ or -C(=O)-NR¹³R¹⁴, wherein R¹⁴, R²⁷, R²⁸, R²⁹, R³⁰, Z, t, u and v have the above-stated meaning and R¹³ and R²⁶ denote hydrogen, is reacted in a reaction medium, possibly in the presence of at least one base, preferably in the presence of at least one metal hydride salt, particularly preferred in the presence of sodium hydride and/or potassium hydride, with at least one compound of the general formula LG-R¹³ or LG-R²⁶, in which R¹³ and R²⁶ have the meaning according to one or more of claims 1 to 5 with exception of a hydrogen residue and LG denotes a leaving group, preferably a halogen atom, particularly preferred a chlorine atom, to yield at least one compound of the general formula I, in which R¹ and R² have the above-stated meaning and R³ denotes -C(=S)-NR²⁶-(CH₂)ₜ(CH₂)ᵤ-Zᵥ-R²⁷, -C(=S)-NR²⁶-(CHR²⁸)-R²⁹, -C(=S)-NR²⁶-C(=O)-R³⁰ or -C(=O)-NR¹³R¹⁴, wherein R¹³, R²⁶, R¹⁴, R²⁷, R²⁸, R²⁹, R³⁰, Z, t, u and v have the above-stated meaning and said compound is possibly purified and/or isolated
or
at least one compound of the general formula IV is reacted in a reaction medium, possibly in the presence of at least one base, preferably in the presence of at least one metal hydride salt, particularly preferred in the presence of sodium hydride and/or potassium hydride, with at least one compound of the general formula LG-(CH₂)-R³¹, in which R³¹ has the meaning according to one or more of claims 1 to 5 and LG denotes a leaving group, preferably a halogen atom, particularly preferred a chlorine atom, to yield at least one compound of the general formula I, in which R¹ and R² have the above-stated meaning and R³ denotes -(CH₂)-R³¹, wherein R³¹ has the above-stated meaning, and said compound is possibly purified and/or isolated
or
at least one compound of the general formula IV is reacted in a reaction medium, in the presence of at least one reducing agent, with at least one compound of the general formula R³¹-C(=O)-H, in which R³¹ has the meaning according to one or more of claims 1 to 15, to yield at least one compound of the general formula I, in which R¹ and R² have the above-stated meaning and R³ denotes -(CH₂)-R³¹, wherein R³¹ has the above-stated meaning, and said compound is possibly purified and/or isolated.

7. A medication containing at least one compound according to one or more of claims 1 to 5 and possibly one or more physiologically acceptable auxiliary substances.

8. A medication according to claim 7 for the prevention and/or treatment of pain, preferably of pain selected from the group consisting of acute pain, chronic pain and neuropathic pain.

9. A medication according to claim 7 for the prevention and/or treatment of one or more diseases selected from the group consisting of migraine; depression; inflammation; lack of drive; urinary incontinence; neurodegenerative diseases, preferably selected from the group consisting of Parkinson's disease, Huntington's disease, Alzheimer's disease and multiple sclerosis; eating disorders, preferably selected from the group consisting of bulimia, anorexia, obesity and cachexia; anxiety states; cognitive dysfunction, preferably memory disorders; cognitive deficiency states (attention deficit syndrome, ADS); epilepsy; catalepsy; diarrhoea and pruritus;
for the prevention and/or treatment of the abuse of alcohol and/or drugs and/or medicines and dependency on alcohol and/or drugs and/or medicines, preferably for the prevention and/or reduction of withdrawal symptoms associated with dependency on alcohol and/or drugs and/or medicines;
for regulating food intake; for modulating locomotor activity; for regulating the cardiovascular system; for local anaesthesia; for increasing vigilance; for increasing libido; for diuresis; for antinatriuresis; for use as a local anaesthetic and/or antiarrhythmic and/or antiemetic and/or nootropic (neurotropic).

10. Use of at least one compound according to one or more of claims 1 to 5 for producing a medication for the treatment and/or prevention of pain, preferably of pain selected from the group consisting of acute pain, chronic pain and neuropathic pain.

11. Use of at least one compound according to one or more of claims 1 to 5 for producing a medication for the prevention and/or treatment of one or more diseases selected from the group consisting of migraine; depression; inflammation; lack of drive; urinary incontinence; neurodegenerative diseases, preferably selected from the group consisting of Parkinson's disease, Huntington's disease, Alzheimer's disease and multiple sclerosis; eating disorders, preferably selected from the group consisting of bulimia, anorexia, obesity and cachexia; anxiety states; cognitive dysfunction, preferably memory disorders; cognitive deficiency states (attention deficit syndrome, ADS); epilepsy; catalepsy; diarrhoea and pruritus;
for the prevention and/or treatment of the abuse of alcohol and/or drugs and/or medicines and dependency on alcohol and/or drugs and/or medicines, preferably for the prevention and/or reduction of withdrawal symptoms associated with dependency on alcohol and/or drugs and/or medicines;
for regulating food intake; for modulating locomotor activity; for regulating the cardiovascular system; for local anaesthesia; for increasing vigilance; for increasing libido; for diuresis; for antinatriuresis; for use as a local anaesthetic and/or antiarrhythmic and/or antiemetic and/or nootropic (neurotropic).

## Revendications

1. Composés de 4,5,6,7-tétrahydro-isoxazolo[4,5-c]pyridine substitués de formule générale I 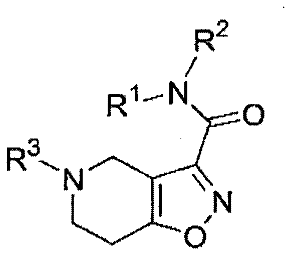 dans laquelle
R¹ représente
-(CHR⁴)-(CH₂)_{c}-(CH₂)_{d}-C(=O)-OR⁵ avec c = 0 ou 1 et d = 0 ou 1 ;
-(CHR⁶)-(CHR⁷)ₑ-V_{f}-(CH₂)_{g}-Wₕ-(CH₂)ᵢ-R⁸ avec e = 0 ou 1, f = 0 ou 1, g = 0 ou 1, h = 0 ou 1 et i = 0 ou 1, V et W représentant à chaque fois indépendamment l'un de l'autre 0, S, NH, N(CH₃) ou N(C₂H₅) ;
un radical aliphatique en C₁₋₁₀ linéaire ou ramifié, saturé ou insaturé, éventuellement substitué ;
un radical cycloaliphatique de 3, 4, 5, 6, 7, 8 ou 9 éléments insaturé ou saturé, éventuellement substitué, qui peut être condensé avec un système cyclique mono- ou polycyclique saturé ou insaturé, éventuellement aromatique, éventuellement substitué, et/ou ponté avec un groupe alkylène en C₁₋₅ linéaire ou ramifié, éventuellement substitué, les radicaux cycloaliphatiques éventuellement substitués mentionnés précédemment pouvant à chaque fois être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NO₂, -O-CF₃, -S-CF₃, -SH, -S-alkyle en C₁₋₅, -alkyle en C₁₋₅, -C(=O)-H, -C(=O)-alkyle en C₁₋₅, - C(=O)-OH, -C(=O)-O-alkyle en C₁₋₅, -(CH₂)-C(=O)-OH, - (CH₂)-C(=O)-O-alkyle en C₁₋₅, -(CH₂)-benzo[b]furanyle, - 0-phényle, -0-benzyle, phényle, benzyle, naphtyle et - (CH₂)-naphtyle, la partie cyclique des radicaux -0-phényle, -O-benzyle, phényle, -(CH₂)-benzo[b]furanyle, benzyle, naphtyle et -(CH₂)-naphtyle pouvant être substituée par 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, alkyle en C₁₋₅, -O-CF₃, S-CF₃, phényle et -O-benzyle,
ou un radical aryle ou hétéroaryle de 5 à 14 éléments éventuellement substitué, qui peut être condensé avec un système cyclique mono- ou polycyclique saturé ou insaturé, éventuellement substitué ;
R² représente
un radical hydrogène ;
-(CH₂)-R⁹
ou un radical aliphatique en C₁₋₁₀ linéaire ou ramifié, saturé ou insaturé, éventuellement substitué ; ou
R¹ et R² forment ensemble avec l'atome d'azote auquel ils sont reliés en tant qu'élément de cycle un radical hétérocycloaliphatique de 4, 5, 6, 7, 8 ou 9 éléments, saturé ou insaturé, éventuellement substitué,
le radical hétérocycloaliphatique pouvant à chaque fois être substitué par 1, 2, 3, 4 ou 5 substituants choisis indépendamment les uns des autres dans le groupe constitué par R¹⁰, -C(=O)-R¹¹ et -(CH₂)-NH-C(=O)-R¹² et/ou comprendre à chaque fois 1, 2, 3, 4 ou 5 hétéroatomes supplémentaires choisis indépendamment les uns des autres dans le groupe constitué par oxygène, azote et soufre en tant qu'éléments de cycle ;
R³ représente
-C(=O)-NR¹³R¹⁴ ;
-C(=O)-R¹⁵ ;
-C(=O)-(CH₂)ⱼ-Xₖ-(CH₂)ₘ-(CH₂)ₙ-C(=O)-OR¹⁶ avec j = 0 ou 1, k = 0 ou 1, m = 0 ou 1 et n = 0 ou 1, X représentant O, S, NH, N(CH₃), N(C₂H₅) ou CR¹⁷R¹⁸ ;
-C(=O)-(CHR¹⁹)-NH-C(=O)-OR²⁰ ;
-C(=O)-(CH₂)-(CH₂)ₚ-(CH₂)_{q}-Yᵣ-(CH₂)ₛ-R²¹ avec p = 0 ou 1, q = 0 ou 1, r = 0 ou 1 et s = 0 ou 1, Y représentant 0, S, NH, N(CH₃) ou N(C₂H₅) ;
-C(=O)-(CH=CH)-R²² ;
-S(=O)₂-R²³ ;
-S(=O)₂-NR²⁹R²⁵ ;
-C(=S)-NR²⁶-(CH₂)ₜ-(CH₂)ᵤ-Zᵥ-R²⁷ avec t = 0 ou 1, u = 0 ou 1 et v = 0 ou 1, Z représentant 0, S, NH, N(CH₃) ou N(C₂H₅) ;
-C(=S)-NR²⁶-(CHR²⁸)-R²⁹ ;
-C(=S)-NR²⁶-C(=O)-R³⁰ ;
ou (CH₂)-R³¹ ;
R⁴ représente
-C(=O)-NH₂ ;
un radical hydrogène
ou un radical aliphatique en C₁₋₁₀ linéaire ou ramifié, saturé ou insaturé, éventuellement substitué ;
R⁵, R¹⁷, R¹⁸, R²⁴ et R²⁵ représentent à chaque fois, indépendamment les uns des autres
un radical aliphatique en C₁₋₁₀ linéaire ou ramifié, saturé ou insaturé, éventuellement substitué ;
R⁶, R⁷, R¹³, R¹⁶ et R²⁶ représentent à chaque fois, indépendamment les uns des autres
un radical hydrogène
ou un radical aliphatique en C₁₋₁₀ linéaire ou ramifié, saturé ou insaturé, éventuellement substitué ; R⁸, R⁹, R²¹, R²², R²³, R³⁰ et R³¹ représentent à chaque fois, indépendamment les uns des autres
un radical cycloaliphatique de 3, 4, 5, 6, 7, 8 ou 9 éléments insaturé ou saturé, éventuellement substitué, qui peut être condensé avec un système cyclique mono- ou polycyclique saturé ou insaturé, éventuellement aromatique, éventuellement substitué, les radicaux cycloaliphatiques éventuellement substitués mentionnés précédemment pouvant à chaque fois être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, - O-CF₃, -S-CF₃, -SH, -S-alkyle en C₁₋₅, -alkyle en C₁₋₅, - C(=O)-H, -C(=O)-alkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N (alkyle en C₁₋₅)₂, -(CH₂)-benzo[b]furanyle, -O-phényle, -0-benzyle, phényle, benzyle, naphtyle et -(CH₂)-naphtyle, la partie cyclique des radicaux -O-phényle, -O-benzyle, phényle, -(CH₂)-benzo[b]furanyle, benzyle, naphtyle et -(CH₂)-naphtyle pouvant à chaque fois être substituée par 1, 2, 3, 4 ou 5 substituants choisis indépendamment les uns des autres dans le groupe constitué par F, Cl, Br, -OH, - CF₃, -SF₅, -CN, -NO₂, -O-alkyle en C₁₋₅, alkyle en C₁₋₅, - O-CF₃, S-CF₃, phényle et -O-benzyle,
ou un radical aryle ou hétéroaryle de 5 à 14 éléments éventuellement substitué, qui peut être condensé avec un système cyclique mono- ou polycyclique saturé ou insaturé, éventuellement substitué ;
R¹⁰, R¹¹, R¹², R¹⁴ et R²⁰ représentent à chaque fois, indépendamment les uns des autres
un radical aliphatique en C₁₋₁₀ linéaire ou ramifié, saturé ou insaturé, éventuellement substitué,
un radical cycloaliphatique de 3, 4, 5, 6, 7, 8 ou 9 éléments, insaturé ou saturé, éventuellement substitué, qui peut être condensé avec un système cyclique mono- ou polycyclique saturé ou insaturé, éventuellement aromatique, éventuellement substitué, et/ou relié par un groupe alkylène en C₁₋₅, alcénylène en C₂₋₅ ou alcynylène en C₂₋₅ linéaire ou ramifié, éventuellement substitué, les radicaux cycloaliphatiques éventuellement substitués mentionnés précédemment pouvant à chaque fois être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, -O-CF₃, -S-CF₃, -SH, -S-alkyle en C₁₋₅, -alkyle en C₁₋₅, -C(=O)-H, -C(=O)-alkyle en C₁₋₅, -C(=O)-OH, -C(=O)-O-alkyle en C₁₋₅, -(CH₂)-C(=O)-OH, - (CH₂) -C (=O) -O-alkyle en C₁₋₅, -NH-alkyle en C₁₋₅, - N (alkyle en C₁₋₅)₂, -(CH₂)-benzo[b]furanyle, -O-phényle, -O-benzyle, phényle, benzyle, naphtyle et -(CH₂)-naphtyle, la partie cyclique des radicaux -0-phényle, - 0-benzyle, phényle, -(CH₂)-benzo[b]furanyle, benzyle, naphtyle et -(CH₂)-naphtyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, alkyle en C₁₋₅, -O-CF₃, S-CF₃, phényle et -O-benzyle,
ou un radical aryle ou hétéroaryle de 5 à 14 éléments éventuellement substitué, qui peut être relié par un groupe alkylène en C₁₋₅, alcénylène en C₂₋₅ ou alcynylène en C₂₋₅ linéaire ou ramifié, éventuellement substitué ;
R¹⁵ et R²⁷ représentent à chaque fois indépendamment l'un de l'autre
un radical aliphatique en C₁₋₁₀ linéaire ou ramifié, saturé ou insaturé, éventuellement substitué,
un radical cycloaliphatique de 3, 4, 5, 6, 7, 8 ou 9 éléments insaturé ou saturé, éventuellement substitué, qui peut être ponté avec un groupe alkylène en C₁₋₅ linéaire ou ramifié, éventuellement substitué, les radicaux cycloaliphatiques éventuellement substitués mentionnés précédemment pouvant à chaque fois être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -0-alkyle en C₁₋₅, -NH₂, -NO₂, - O-CF₃, -S-CF₃, -SH, -S-alkyle en C₁₋₅, -alkyle en C₁₋₅, - C(=O)-H, -C(=O)-alkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -NH-alkyle en C₁₋₅, -N (alkyle en C₁₋₅)₂, -(CH₂)-benzo[b]furanyle, -O-phényle, -0-benzyle, phényle, benzyle, naphtyle et -(CH₂)-naphtyle, la partie cyclique des radicaux -O-phényle, -0-benzyle, phényle, -(CH₂)-benzo[b]furanyle, benzyle, naphtyle et -(CH₂)-naphtyle pouvant à chaque fois être substituée par 1, 2, 3, 4 ou 5 substituants choisis indépendamment les uns des autres dans le groupe constitué par F, Cl, Br, -OH, - CF₃, -SF₅, -CN, -NO₂, -O-alkyle en C₁₋₅, alkyle en C₁₋₅, - O-CF₃, S-CF₃, phényle et -O-benzyle,
ou un radical aryle ou hétéroaryle de 5 à 14 éléments éventuellement substitué, qui peut être condensé avec un système cyclique mono- ou polycyclique saturé ou insaturé, éventuellement substitué ;
R¹⁹ représente
un radical aryle ou hétéroaryle de 5 à 14 éléments éventuellement substitué, qui peut être relié par un groupe alkylène en C₁₋₅, alcénylène en C₂₋₅ ou alcynylène en C₂₋₅ linéaire ou ramifié, éventuellement substitué, comprenant éventuellement 1 ou 2 hétéroatomes en tant qu'éléments de chaîne ;
R²⁸ et R²⁹ représentent à chaque fois indépendamment l'un de l'autre
un radical aliphatique en C₁₋₁₀ linéaire ou ramifié, saturé ou insaturé, éventuellement substitué,
ou un radical aryle ou hétéroaryle de 5 à 14 éléments éventuellement substitué, qui peut être condensé avec un système cyclique mono- ou polycyclique saturé ou insaturé, éventuellement substitué ;
les radicaux aliphatiques en C₁₋₁₀ éventuellement substitués mentionnés précédemment pouvant à chaque fois être substitués par 1, 2, 3, 4 ou 5 substituants choisis indépendamment les uns des autres dans le groupe constitué par F, Cl, Br, I, -CN, -NO₂, -OH, -0-alkyle en C₁₋₅, -SH, -S-alkyle en C₁₋₅, -NH₂, -NH-alkyle en C₁₋₅ et -N (alkyle en C₁₋₅) (alkyle en C₁₋₅) ;
les radicaux cycloaliphatiques mentionnés précédemment pouvant à chaque fois comprendre 1, 2, 3, 4 ou 5 hétéroatomes choisis indépendamment les uns des autres dans le groupe constitué par oxygène, azote et soufre en tant qu'éléments de cycle ;
les groupes alkylène en C₁₋₅, alcénylène en C₂₋₅ ou alcynylène en C₂₋₅ éventuellement substitués mentionnés précédemment pouvant à chaque fois être substitués par 1, 2, 3, 4 ou 5 substituants choisis indépendamment les uns des autres dans le groupe constitué par F, Cl, Br, -OH, -SH, -NH₂, -CN, NO₂ et phényle,
et les groupes alkylène en C₁₋₅, alcénylène en C₂₋₅ ou alcynylène en C₂₋₅ mentionnés précédemment pouvant à chaque fois éventuellement comprendre 1 ou 2 hétéroatomes choisis dans le groupe constitué par oxygène, azote et soufre en tant qu'éléments de chaîne ;
les cycles des systèmes cycliques mono- ou polycycliques éventuellement substitués mentionnés précédemment pouvant à chaque fois éventuellement être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -0-alcényle en C₂₋₅, - NH₂, -NO₂, -O-CF₃, O-CHF₂, -O-CH₂F, -S-CF₃, S-CHF₂, -S-CH₂F, -SH, -S-alkyle en C₁₋₅, -alkyle en C₁₋₅, -C(=O)-OH, -C(=O)-O-alkyle en C₁₋₅, -NH-alkyle en C₁₋₅, -N (alkyle en C₁₋₅)₂, -NH-C(=O)-O-alkyle en C₁₋₅, -NH-C(=O)-alkyle en C₁₋₅, -C(=O)-H, -C(=O)-alkyle en C₁₋₅, -C(=O)-NH₂, -C(=O)-NH-alkyle en C₁₋₅, -C(=O)-N-(alkyle en C₁₋₅)₂, -S(=O)₂-NH₂, -S(=O)₂-NH-alkyle en C₁₋₅, -(CH₂)-benzo[b]furanyle, - O-phényle, -0-benzyle, phényle et benzyle, la partie cyclique des radicaux -0-phényle, -0-benzyle, phényle, -(CH₂)-benzo[b]furanyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, - NO₂, -0-alkyle en C₁₋₅, alkyle en C₁₋₅, -O-CF₃, S-CF₃, phényle et -0-benzyle,
et les cycles des systèmes cycliques mono- ou polycycliques mentionnés précédemment étant à chaque fois à 5, 6 ou 7 éléments et pouvant à chaque fois comprendre à chaque fois 1, 2, 3, 4 ou 5 hétéroatomes en tant qu'éléments de cycle, choisis indépendamment les uns des autres dans le groupe constitué par oxygène, azote et soufre ;
et les radicaux aryle ou hétéroaryle éventuellement substitués mentionnés précédemment pouvant à chaque fois être substitués par 1, 2, 3, 4 ou 5 substituants choisis indépendamment les uns des autres dans le groupe constitué par F, Cl, Br, I, -CN, -CF₃, -SF₅, -OH, -O-alkyle en C₁₋₅, -O-alcényle en C₂₋₅, -NH₂, -NO₂, -O-CF₃, O-CHF₂, -O-CH₂F, -S-CF₃, S-CHF₂, -S-CH₂F, -SH, -S-alkyle en C₁₋₅, -alkyle en C₁₋₅, -C(=O)-O-alkyle en C₁₋₅, -NH-alkyle en C₁₋₅, -N (alkyle en C₁₋₅)₂, -NH-C(=O)-O-alkyle en C₁₋₅, -NH-C(=O)-alkyle en C₁₋₅, -C(=O)-H, - C(=O)-alkyle en C₁₋₅, -C(=O)-NH₂, -C(=O)-NH-alkyle en C₁₅, -C(=O)-N-(alkyle en C₁₋₅)₂, -S(=O)₂-NH₂, -S(=O)2-NH-alkyle en C₁₋₅, -(CH₂)-benzo[b]furanyle, -O-phényle, -0-benzyle, phényle et benzyle, la partie cyclique des radicaux -O-phényle, -O-benzyle, phényle, -(CH₂)-benzo[b]furanyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, alkyle en C₁₋₅, -O-CF₃, S-CF₃, phényle et -O-benzyle,
et les radicaux hétéroaryle mentionnés précédemment pouvant à chaque fois comprendre 1, 2, 3, 4 ou 5 hétéroatomes choisis indépendamment les uns des autres dans le groupe constitué par oxygène, azote et soufre en tant qu'élément de cycle ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme de sels correspondants, ou à chaque fois sous la forme de solvates correspondants.

2. Composés selon la revendication 1, **caractérisés en ce que**
R¹ représente
-(CHR⁴)-(CH₂)_{c}-(CH₂)_{d}-C(=O)-OR⁵ avec c = 0 ou 1 et d = 0 ou 1 ;
-(CHR⁶)-(CHR⁷)ₑ-V_{f}-(CH₂)_{g}-Wₕ-(CH₂)ᵢ-R⁸ avec e = 0 ou 1, f = 0 ou 1, g = 0 ou 1, h = 0 ou 1 et i = 0 ou 1, V et W représentant à chaque fois indépendamment l'un de l'autre 0, S, NH, N(CH₃) ou N(C₂H₅) ;
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -0-alkyle en C₁₋₅, -SH, -S-alkyle en C₁₋₅, -NH₂, -NH-alkyle en C₁₋₅ et -N (alkyle en C₁₋₅) (alkyle en C₁₋₅) ;
un radical alcényle choisi dans le groupe constitué par vinyle, 1-propényle, 2-propényle, 1-butényle, 2-butényle et 3-butényle, le radical alcényle pouvant à chaque fois être substitué par 1, 2, 3, 4 ou 5 substituants choisis indépendamment les uns des autres dans le groupe constitué par F, Cl, Br, I, -CN, -NO₂, -OH, -0-alkyle en C₁₋₅, -SH, -S-alkyle en C₁₋₅, - NH₂, -NH-alkyle en C₁₋₅ et -N (alkyle en C₁₋₅) (alkyle en C₁₋₅) ;
un radical alcynyle choisi dans le groupe constitué par éthynyle, 1-propynyle, 2-propynyle, 1-butynyle, 2-butynyle et 3-butynyle, le radical alcynyle pouvant à chaque fois être substitué par 1, 2, 3, 4 ou 5 substituants choisis indépendamment les uns des autres dans le groupe constitué par F, Cl, Br, I, -CN, -NO₂, -OH, -0-alkyle en C₁₋₅, -SH, -S-alkyle en C₁₋₅, - NH₂, -NH-alkyle en C₁₋₅ et -N (alkyle en C₁₋₅) (alkyle en C₁₋₅) ;
un radical (hétéro)cycloaliphatique choisi dans le groupe constitué par cyclopropyle, cyclobutyle, cyclopentyle, cyclohexyle, cycloheptyle, (6,6)-diméthyl-[3.1.1]-bicycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical (hétéro)cycloaliphatique pouvant à chaque fois être substitué par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -NO₂, -O-CF₃, -S-CF₃, -SH, -S-alkyle en C₁₋₅, -alkyle en C₁₋₅, -C(=O)-H, - C(=O)-alkyle en C₁₋₅, -C(=O)-OH, -C(=O)-O-alkyle en C₁₋₅, -(CH₂)-C(=O)-OH, -(CH₂)-C(=O)-O-alkyle en C₁₋₅, -(CH₂)-benzo[b]furanyle, -O-phényle, -O-benzyle, phényle, benzyle, naphtyle et -(CH₂)-naphtyle, la partie cyclique des radicaux -0-phényle, -0-benzyle, phényle, -(CH₂)-benzo[b]furanyle, benzyle, naphtyle et -(CH₂)-naphtyle pouvant être substituée par 1, 2, 3, 4 ou 5 substituants choisis indépendamment les uns des autres dans le groupe constitué par F, Cl, Br, -OH, - CF₃, -SF₅, -CN, -NO₂, -0-alkyle en C₁₋₅, alkyle en C₁₋₅, - O-CF₃, S-CF₃, phényle et -O-benzyle,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle benzo[b]thiophényle, thiazolyle, oxazolyle, isoxazolyle, pyridazinyle, pyrimidinyle, indazolyle, quinazolinyle, quinolinyle et isoquinolinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -0-alkyle en C₁₋₅, -0-alcényle en C₂-C₅, -NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-O-alkyle en C₁₋₅, -NH-alkyle en C₁₋₅, -N (alkyle en C₁₋₅)₂, -NH-C(=O)-O-alkyle en C₁₋₅, -NH-C(=O)-alkyle en C₁₋₅, -C(=O)-H, - C(=O)-alkyle en C₁₋₅, -C(=O)-NH₂, -C(=O)-NH-alkyle en C₁₅, -C(=O)-N-(alkyle en C₁₋₅)₂, -S(=O)₂-NH₂, -S(=O)₂-NH-alkyle en C₁₋₅, -(CH₂)-benzo[b]furanyle, -O-phényle, -0-benzyle, phényle et benzyle, la partie cyclique des radicaux -0-phényle, -O-benzyle, phényle, -(CH₂)-benzo[b]furanyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -0-alkyle en C₁₋₅, alkyle en C₁₋₅, -O-CF₃, S-CF₃, phényle et -0-benzyle,
R² représente
un radical hydrogène ;
-(CH₂)-R⁹
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ et - N(CH₃)(C₂H₅) ;
ou
R¹ et R² forment ensemble avec l'atome d'azote auquel ils sont reliés en tant qu'élément de cycle un radical hétérocycloaliphatique choisi dans le groupe constitué par imidazolidinyle, (1,3)-thiazolidinyle, pipérazinyle, morpholinyle, thiomorpholinyle, pyrrolidinyle ; pipéridinyle ; (1,2,3,6)-tétrahydropyridinyle et (1,2,3,4)-tétrahydropyridinyle, le radical hétérocycloaliphatique pouvant à chaque fois être substitué par 1, 2 ou 3 substituants choisis indépendamment les uns des autres dans le groupe constitué par R¹⁰, -C(=O)-R¹¹ et -(CH₂)-NH-C(=O)-R¹² ;
R³ représente
-C(=O)-NR¹³R¹⁴ ;
-C(=O)-R¹⁵ ;
-C(=O)-(CH₂)-X-(CH₂)-C(=O)-OR¹⁶, -C(=O)-X-(CH₂)-C(=O)-OR¹⁶ ou -C(=O)-(CH₂)-(CH₂)-(CH₂)-C(=O)-OR¹⁶, X représentant O, S, NH, N(CH₃), N(C₂H₅) ou CR¹⁷R¹⁸ ;
-C(=O)-(CHR¹⁹)-NH-C(=O)-OR²⁰ ;
-C(=O)-(CH₂)-R²¹, -C(=O)-(CH₂)-Y-R²¹, -C(=O)-(CH₂)-Y-(CH₂)-R²¹ ou -C(=O)-(CH₂)-(CH₂)-(CH₂)-Y-R²¹, Y représentant 0, S, NH, N(CH₃) ou N(C₂H₅) ;
-C(=O)-(CH=CH)-R²² ;
-S (=O)₂-R²³ ;
-S(=O)₂-NR²⁴R²⁵ ;
-C(=S)-NR²⁶-R²⁷, -C(=S)-NR²⁶-(CH₂)-R²⁷, -C(=S)-NR²⁶-(CH₂)-(CH₂)-R²⁷ ou -C(=S)-NR²⁶-(CH₂)-(CH₂) -Z-R²⁷, Z représentant 0, S, NH, N(CH₃) ou N(C₂H₅) ;
-C(=S)-NR²⁶-(CHR²⁸)-R²⁹ ;
-C(=S)-NR²⁶-C(=O)-R³⁰ ;
ou (CH₂)-R³¹ ;
R⁴ représente
-C(=O)-NH₂ ;
un radical hydrogène
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -0-alkyle en C₁₋₅, -SH, -S-alkyle en C₁₋₅, -NH₂, -NH-alkyle en C₁₋₅ et -N (alkyle en C₁₋₅) (alkyle en C₁₋₅) ;
ou un radical alcényle choisi dans le groupe constitué par vinyle, 1-propényle, 2-propényle, 1-butényle, 2-butényle et 3-butényle, le radical alcényle pouvant à chaque fois être substitué par 1, 2, 3, 4 ou 5 substituants choisis indépendamment les uns des autres dans le groupe constitué par F, Cl, Br, I, -CN, -NO₂, -OH, -O-alkyle en C₁₋₅, -SH, -S-alkyle en C₁₋₅, - NH₂, -NH-alkyle en C₁₋₅ et -N(alkyle en C₁₋₅) (alkyle en C₁₋₅) ;
R⁵, R¹⁷, R¹⁸, R²⁴ et R²⁵ représentent à chaque fois, indépendamment les uns des autres
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et - (CH₂)-(CH) (C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N (CH₃)₂, -N(C₂H₅)₂ et - N(CH₃)(C₂H₅) ;
ou un radical alcényle choisi dans le groupe constitué par vinyle, 1-propényle, 2-propényle, 1-butényle, 2-butényle et 3-butényle, le radical alcényle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, - NH-CH₃, -NH-C₂H₅, -N(CH₃)₂, -N(C₂H₅)₂ et -N(CH₃) (C₂H₅) ;
R⁶, R⁷, R¹³, R¹⁶ et R²⁶ représentent à chaque fois, indépendamment les uns des autres
un radical hydrogène
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, ₋NH-CH₃, -NH-C₂H₅, -N(CH₃)₂, -N (C₂H₅)₂ et - N(CH₃)(C₂H₅) ;
ou un radical alcényle choisi dans le groupe constitué par vinyle, 1-propényle, 2-propényle, 1-butényle, 2-butényle et 3-butényle, le radical alcényle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, - NH-CH₃, -NH-C₂H₅, -N(CH₃)₂, -N(C₂H₅)₂ et -N(CH₃)(C₂H₅) ;
R⁸, R⁹, R²¹, R²², R²³, R³⁰ et R³¹ représentent à chaque fois, indépendamment les uns des autres
un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical (hétéro)cycloaliphatique pouvant à chaque fois être substitué par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -NH₂, -NO₂, -O-CF₃, - S-CF₃, -SH, -S-alkyle en C₁₋₅, -alkyle en C₁₋₅, -C(=O)-H, -C(=O)-alkyle en C₁₋₅, -C(=O)-OH, -C(=O)-O-alkyle en C₁-₅, -(CH₂)-C(=O)-OH, -(CH₂)-C(=O)-O-alkyle en C₁₋₅, -NH-alkyle en C₁₋₅, -N (alkyle en C₁₋₅)₂, -(CH₂)-benzo[b]furanyle, -O-phényle, -0-benzyle, phényle, benzyle, naphtyle et -(CH₂)-naphtyle, la partie cyclique des radicaux -O-phényle, -0-benzyle, phényle, -(CH₂)- benzo[b]furanyle, benzyle, naphtyle et -(CH₂)-naphtyle pouvant à chaque fois être substituée par 1, 2, 3, 4 ou 5 substituants choisis indépendamment les uns des autres dans le groupe constitué par F, Cl, Br, -OH, - CF₃, -SF₅, -CN, -NO₂, -O-alkyle en C₁₋₅, alkyle en C₁₋₅, - O-CF₃, S-CF₃, phényle et -O-benzyle,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle benzo[b]thiophényle, thiazolyle, oxazolyle, isoxazolyle, pyridazinyle, pyrimidinyle, indazolyle, quinazolinyle, quinolinyle et isoquinolinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-alkyle en C₁₋₅, -O-alcényle en C₂₋₅, -NH₂, -NO₂, -O-CF₃, O-CHF₂, -O-CH₂F, -S-CF₃, -S-CHF₂, -S-CH₂F, -SH, -S-alkyle en C₁₋₅, -alkyle en C₁₋₅, -C(=O)-O-alkyle en C₁₋₅, -NH-alkyle en C₁₋₅, -N(alkyle en C₁₋₅)₂, -NH-C(=O)-O-alkyle en C₁₋₅, -NH-C(=O)-alkyle en C₁₋₅, -C(=O)-H, -C(=O)-alkyle en C₁₋₅, -C(=O)-NH₂, -C(=O)-NH-alkyle en C₁₋₅, -C(=O)-N-(alkyle en C₁₋₅)₂, -S(=O)₂-NH₂, -S(=O)₂-NH-alkyle en C₁₋₅, -(CH₂)-benzo[b]furanyle, -O-phényle, -O-benzyle, phényle et benzyle, la partie cyclique des radicaux O-phényle, -0-benzyle, phényle, -(CH₂)-benzo[b]furanyle et benzyle pouvant à chaque fois être substituée par 1, 2, 3, 4 ou 5 substituants choisis indépendamment les uns des autres dans le groupe constitué par F, Cl, Br, -OH, - CF₃, -SF₅, -CN, -NO₂, -O-alkyle en C₁₋₅, alkyle en C₁₋₅, - O-CF₃, S-CF₃, phényle et -O-benzyle,
R¹⁰, R¹¹, R¹², R¹⁴ et R²⁰ représentent à chaque fois, indépendamment les uns des autres
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-alkyle en C₁₋₅, -SH, -S-alkyle en C₁₋₅, -NH₂, -NH-alkyle en C₁₋₅ et -N (alkyle en C₁₋₅) (alkyle en C₁₋₅) ;
un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, indanyle et indényle, le radical (hétéro)cycloaliphatique pouvant à chaque fois être substitué par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -NH₂, -NO₂, -O-CF₃,-S-CF₃, -SH, -S-alkyle en C₁₋₅, -alkyle en C₁₋₅, -C(=O)-H, -C(=O)-alkyle en C₁₋₅, -C(=O)-OH, -C(=O)-O-alkyle en C₁₅, -(CH₂)-C(=O)-OH, -(CH₂)-C(=O)-O-alkyle en C₁₋₅, -NH-alkyle en C₁₋₅, -N (alkyle en C₁₋₅)₂, - (CH₂) - benzo[b]furanyle, -O-phényle, -O-benzyle, phényle, benzyle, naphtyle et -(CH₂)-naphtyle, la partie cyclique des substituants -O-phényle, -O-benzyle, phényle, - (CH₂)-benzo[b]furanyle, benzyle, naphtyle et -(CH₂)- naphtyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -O-alkyle en C₁₋₅, alkyle en C₁₋₅, -O-CF₃, -alkyle en C₁₋₅, S-CF₃, phényle et -O-benzyle, et/ou le radical (hétéro)cycloaliphatique pouvant être relié par un groupe -(CH₂)-, -CH(CH₃)-, - (CH₂)-(CH₂)-, -(CH₂)-(CH₂)-(CH₂)- ou -(CH₂)-(CH=CH)- ;
ou un radical choisi dans le groupe constitué par phényle, naphtyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrimidinyle, indazolyle, quinazolinyle, quinolinyle, isoquinolinyle et thiéno[2,3-d]pyrimidinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-alkyle en C₁₋₅, -O-alcényle en C₂₋₅, - NH₂, -NO₂, -O-CF₃, -O-CHF₂, -O-CH₂F, -S-CF₃, -S-CHF₂, -S-CH₂F, -SH, -S-alkyle en C₁₋₅, -alkyle en C₁₋₅, -C(=O)-O-alkyle en C₁₋₅, -NH-alkyle en C₁₋₅, -N (alkyle en C₁₋₅)₂, - NH-C(=O)-O-alkyle en C₁₋₅, -NH-C(=O)-alkyle en C₁₋₅, - C(=O)-H, -C(=O)-alkyle en C₁₋₅, -C(=O)-NH₂, -C (=O) -NH-alkyle en C₁₋₅, -C(=O)-N-(alkyle en C₁₋₅)₂, -S(=O)₂-NH₂, - S(-O)₂-NH-alkyle en C₁₋₅, -(CH₂)-benzo[b]furanyle, -O-phényle, -O-benzyle, phényle et benzyle, et/ou relié par un groupe -(CH₂)-, -CH(CH₃)-, -(CH₂)-(CH₂)-, -(CH₂)-(CH₂)-(CH₂) - ou -(CH₂)-(CH=CH), la partie cyclique des substituants -O-phényle, -O-benzyle, phényle, -(CH₂)-benzo[b]furanyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -O-alkyle en C₁₋₅, alkyle en C₁₋₅, -O-CF₃, -S-CF₃, phényle et -O-benzyle ;
R¹⁵ et R²⁷ représentent à chaque fois indépendamment l'un de l'autre
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N (CH₃) ₂, -N(C₂H₅)₂ et - N(CH₃)(C₂H₅) ;
un radical alcényle choisi dans le groupe constitué par vinyle, 1-propényle, 2-propényle, 1-butényle, 2-butényle et 3-butényle, le radical alcényle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, - NH-CH₃, -NH-C₂H₅, -N(CH₃)₂, -N(C₂H₅)₂ et -N(CH₃)(C₂H₅) ;
un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle et 7,7-diméthyl-2-oxa-bicyclo[2.2.1]heptyle, le radical (hétéro)cycloaliphatique pouvant à chaque fois être substitué par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -NH₂, -NO₂, -O-CF₃, - S-CF₃, -SH, -S-alkyle en C₁₋₅, -alkyle en C₁₋₅, -C(=O)-H, -C(=O)-alkyle en C₁₋₅, -C(=O)-OH, -C (=O) -O-alkyle en C₁₅, -(CH₂)-C(=O)-OH, -(CH₂)-C(=O)-O-alkyle en C₁₋₅, -NH-alkyle en C₁₋₅, -N(alkyle en C₁₋₅)₂, -O-phényle, -O-benzyle, phényle et benzyle, la partie cyclique des radicaux -0-phényle, -O-benzyle, phényle et benzyle pouvant à chaque fois être substituée par 1, 2, 3, 4 ou 5 substituants choisis indépendamment les uns des autres dans le groupe constitué par F, Cl, Br, -OH, - CF₃, -SF₅, -CN, -NO₂, -O-alkyle en C₁₋₅, -alkyle en C₁₋₅, -O-CF₃, S-CF₃, phényle et -O-benzyle,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrimidinyle, indazolyle, quinazolinyle, quinolinyle et isoquinolinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-alkyle en C₁₋₅, -O-alcényle en C₂₋₅, -NH₂, -NO₂, -O-CF₃, - O-CHF₂, -O-CH₂F, -S-CF₃, -S-CHF₂, -S-CH₂F, -SH, -S-alkyle en C₁₋₅, -alkyle en C₁₋₅, -C(=O)-O-alkyle en C₁₋₅, -NH-alkyle en C₁₋₅, -N(alkyle en C₁₋₅)₂, -NH-C(=O)-O-alkyle en C₁₋₅, -NH-C(=O)-alkyle en C₁₋₅, -C(=O)-H, -C(=O)-alkyle en C₁₋₅, -C(=O)-NH₂, -C(=O)-NH-alkyle en C₁₋₅, -C(=O)-N-(alkyle en C₁₋₅)₂, -S(=O)₂-NH₂, -S(=O)₂-NH-alkyle en C_{1-5,} -O-phényle, -O-benzyle, phényle et benzyle, la partie cyclique des radicaux -O-phényle, -O-benzyle, phényle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -O-alkyle en C₁₋₅, -alkyle en C₁₋₅, -O-CF₃, -S-CF₃, phényle et -O-benzyle ;
R¹⁹ représente
un radical choisi dans le groupe constitué par phényle, naphtyle, thiophényle, furanyle et pyridinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -O-CH(CH₃)₂, -O-CH₂-CH₂-CH₂-CH₃, -NH₂, -NO₂, -O-CF₃, -O-CHF₂, -O-CH₂F, -S-CF₃, -S-CHF₂, -S-CH₂F, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, - N(CH₃)₂, -N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, - C(=O)-NH₂, -C(=O)-NH-CH₃, -C(=O)-N-(CH₃)₂, phényle et benzyle et/ou relié par un groupe - (CH₂)-, (CH₂)-CH₂)- ou -(CH₂)-O-(CH₂)- ;
et
R²⁸ et R²⁹ représentent à chaque fois indépendamment l'un de l'autre
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ et - N(CH₃)(C₂H₅) ;
ou un radical choisi dans le groupe constitué par phényle, naphtyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrimidinyle, indazolyle, quinazolinyle, quinolinyle, et isoquinolinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, -N(CH₃)₂, - N(C₂H₅)₂, -N(H)(CH₃), -N(H)(C₂H₅), -NH-C(=O)-CH₃, -NH-C(=O)-C₂H₅, -C(=O)-H, -C(=O)-CH₃, -C(=O)-C₂H₅, -C(=O)-NH₂, -C(=O)-NH-CH₃, -C(=O)-N-(CH₃)₂, -S(=O)₂-NH₂, -S(=O)₂-CH₃, phényle et benzyle ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme de sels correspondants, ou à chaque fois sous la forme de solvates correspondants.

3. Composés selon la revendication 1 ou 2, **caractérisés en ce que**
R¹ représente
-(CHR⁴)-C(=O)-OR⁵, -(CHR⁴)-(CH₂)-C(=O)-OR⁵ ou - (CHR⁴)-(CH₂)-(CH₂)-C(=O)-OR⁵ ;
-(CHR⁶)-R⁸, -(CHR⁶)-(CHR⁷)-R⁸, -(CHR⁶)-(CHR⁷)-V-R⁸,-(CHR⁶)-(CHR⁷)-(CH₂)-R⁸, -(CHR⁶)-(CHR⁷)-(CH₂)-W-R⁸ ou - (CHR⁶)-(CHR⁷)-(CH₂)-W-(CH₂)-R⁸, V et W représentant à chaque fois indépendamment l'un de l'autre O, S, NH, N (CH₃) ou N (C₂H₅) ;
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -N(CH₃)₂, -N(C₂H₅)₂ et - N(CH₃)(C₂H₅) ;
un radical alcynyle choisi dans le groupe constitué par éthynyle, 1-propynyle, 2-propynyle, 1-butynyle, 2-butynyle et 3-butynyle ;
un radical (hétéro)cycloaliphatique choisi dans le groupe constitué par cyclopropyle, cyclobutyle, cyclopentyle, cyclohexyle, cycloheptyle, (6,6)-diméthyl-[3.1.1]-bicycloheptyle, pyrrolidinyle, pipéridinyle, morpholinyle, thiomorpholinyle, indanyle et indényle, le radical (hétéro)cycloaliphatique pouvant à chaque fois être substitué par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, -(CH₂)-C(=O)-O-C₂H₅, -(CH₂)-benzo[b]furanyle, -O-phényle, -O-benzyle, phényle, benzyle, naphtyle et -(CH₂)-naphtyle, la partie cyclique des radicaux benzyle pouvant être substituée par 1, 2, 3, 4 ou 5 substituants choisis indépendamment les uns des autres dans le groupe constitué par F, Cl, Br, -OH, -CF₃, -SF₅, -CN, -NO₂, -O-CH₃, -O-C₂H₅ et -O-CF₃,
ou un radical choisi dans le groupe constitué par phényle, naphtyle, (1,3)-benzodioxolyle, (1,4)-benzodioxanyle, thiophényle, furanyle, pyridinyle, imidazolyle et indolyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, méthyle, éthyle, n-propyle, isopropyle, n-butyle, sec-butyle, isobutyle et tert-butyle ;
R² représente
un radical hydrogène ou
-(CH₂)-R⁹
ou
R¹ et R² forment ensemble avec l'atome d'azote auquel ils sont reliés en tant qu'élément de cycle un radical choisi dans le groupe constitué par 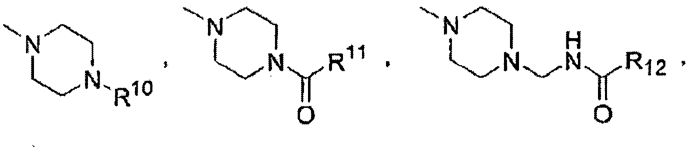 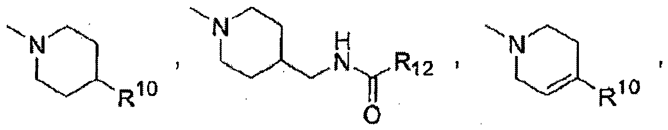 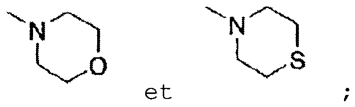 R³ représente
-C(=O)-NR¹³R¹⁴;
-C(=O)-R¹⁵ ;
-C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-CR¹⁷R¹⁸-(CH₂)-C(=O)-OR¹⁶ ou -C(=O)-(CH₂)-(CH₂)-(CH₂)-C(=O)-OR¹⁶ ;
-C(=O)-(CHR¹⁹)-NH-C(=O)-OR²⁰ ;
-C(=O-(CH₂)-R²¹, -C(=O)-(CH₂)-O-R²¹, -C(=O)-(CH₂)-O-(CH₂)-R²¹ ou -C(=O)-(CH₂)-(CH₂)-(CH₂)-O-R²¹ ;
-C(=O)-(CH=CH)-R²² ;
-S(=O)₂-R²³ ;
-S(=O)₂-NR²⁴R²⁵ ;
-C(=S)-NR²⁶-R²⁷, -C(=S)-NR²⁶-(CH₂)-R²⁷, -C(=S)-NR²⁶-(CH₂)-(CH₂)-R²⁷ ou -C(=S)-NR²⁶-(CH₂)-(CH₂)-O-R²⁷ ;
-C(=S)-NR²⁶-(CHR²⁸)-R²⁹ ;
-C(=S)-NR²⁶-C(=O)-R³⁰ ;
ou -(CH₂)-R³¹ ;
R⁴ représente
-C(=O)-NH₂ ;
un radical hydrogène ou
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle, n-octyle et -(CH₂)-(CH)(C₂H₅)-(CH₂)-(CH₂)-(CH₂)-(CH₃), le radical alkyle pouvant à chaque fois être substitué par 1, 2, 3, 4 ou 5 substituants choisis indépendamment les uns des autres dans le groupe constitué par F, Cl, Br, I, -CN, -NO₂, -OH, -O-CH₃, -O-C₂H₅, -SH, -S-CH₃, -S-C₂H₅, -NH₂, -NH-CH₃, -NH-C₂H₅, -NH(CH₃)₂, -NH(C₂H₅)₂ et - N(CH₃)(C₂H₅) ;
R⁵ représente un radical alkyle choisi dans le groupe constitué par méthyle, éthyle, n-propyle, isopropyle, n-butyle, sec-butyle, isobutyle et tert-butyle ;
R⁶, R⁷ et R¹⁶ représentent à chaque fois, indépendamment les uns des autres
un radical hydrogène ou un radical alkyle choisi dans le groupe constitué par méthyle, éthyle, n-propyle et isopropyle ;
R⁸, R⁹, R²¹, R²², R²³, R³⁰ et R³¹ représentent à chaque fois, indépendamment les uns des autres
un radical (hétéro)cycloaliphatique choisi dans le groupe constitué par cyclopropyle, cyclobutyle, cyclopentyle, cyclohexyle, cycloheptyle, pyrrolidinyle, pipéridinyle, morpholinyle, thiomorpholinyle, indanyle et indényle, le radical (hétéro)cycloaliphatique pouvant à chaque fois être substitué par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, - méthyle, éthyle, n-propyle, isopropyle, n-butyle, sec-butyle, isobutyle, tert-butyle, -C(=O)-H, -C(=O)-CH₃, - C(=O)-C₂H₅, C(=O)-C(CH₃)₃, -C (=O) -OH, -C(=O)-O-CH₃,-C(=O)-O-C₂H₅, -(CH₂)-C(=O)-OH, -(CH₂)-C(=O)-O-CH₃, - (CH₂) -C(=O)-O-C₂H₅, -N(CH₃)₂, -N(C₂H₅)₂, -N(H)(CH₃), - N(H)(C₂H₅), phényle et benzyle ;
ou un radical choisi dans le groupe constitué par phényle, naphtyle, (1,3)-benzodioxolyle, (1,4)-benzodioxanyle, thiophényle, furanyle, pyridinyle, imidazolyle, indolyle et isoindolyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -O-CH (CH₃) ₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, - méthyle, éthyle, n-propyle, isopropyle, n-butyle, sec-butyle, isobutyle, tert-butyle, -(CH₂)-C(=O)-O-CH₃,-(CH₂) -C(=O)-O-C₂H₅, -N(CH₃)₂, -N(C₂H₅)₂, -N(H)(CH₃), - N(H)(C₂H₅), -C(=O)-H, -C(=O)-CH₃, -C(=O)-C₂H₅, -C(=O)-NH₂, -S(=O)₂-NH₂, -S(=O)₂-NH-CH₃, -O-phényle, -O-benzyle, phényle et benzyle ;
R¹⁰, R¹¹, R¹², R¹⁴ et R²⁰ représentent à chaque fois, indépendamment les uns des autres
un radical alkyle choisi dans le groupe constitué par méthyle, éthyle, n-propyle, isopropyle, n-butyle, sec-butyle, isobutyle, tert-butyle, n-pentyle, sec-pentyle, -(CH₂)-(CH₂)-(C(CH₃)₃), n-hexyle, n-heptyle et n-octyle, le radical alkyle pouvant à chaque fois être substitué par 1, 2, 3, 4 ou 5 substituants choisis indépendamment les uns des autres dans le groupe constitué par -N(CH₃)₂, -N(C₂H₅)₂, F, Cl et Br ;
un radical cycloaliphatique choisi dans le groupe constitué par pyrrolidinyle, pipéridinyle, pipérazinyle, morpholinyle, cyclopropyle, cyclobutyle, cyclopentyle et cyclohexyle, le radical cycloaliphatique pouvant à chaque fois être substitué par 1, 2 ou 3 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-H, -C(=O)-CH₃, -C(=O)-C₂H₅, -C(=O)-C(CH₃)₃, -C(=O)-OH, -C(=O)-O-CH₃, -C(=O)-O-C₂H₅, -(CH₂)-C(=O)-OH, -(CH₂)-C(=O)-O-CH₃, (CH₂)-C(=O)-O-C₂H₅, - N(CH₃)₂, -N(C₂H₅)₂, -N(H)(CH₃), -N(H)(C₂H₅), phényle et benzyle et/ou relié par un groupe -(CH₂)- ou -(CH₂)-(CH₂) - ;
ou un radical choisi dans le groupe constitué par phényle, naphtyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrimidinyle, indazolyle, quinazolinyle, quinolinyle, isoquinolinyle et thiéno[2,3-d]pyrimidinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -O-CH(CH₃)₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, -N(CH₃)₂, -N(C₂H₅)₂, -N(H)(CH₃), -N(H)(C₂H₅), -C(=O)-H, -C(=O)-CH₃, -C(=O)-C₂H₅, -C(=O)-NH₂, -C(=O)-NH-CH₃, - C(=O)-N-(CH₃)₂, -S(=O)₂-NH₂, -S(=O)₂-CH₃, phényle et benzyle et/ou relié par un groupe -(CH₂)-, -CH(CH₃)-,-(CH₂)-(CH₂)-, -(CH₂)-(CH₂)-(CH₂)- ou -(CH₂)-(CH=CH)- ;
R¹³ et R²⁶ représentent un radical hydrogène ;
R¹⁵ et R²⁷ représentent à chaque fois indépendamment l'un de l'autre
un radical choisi dans le groupe constitué par méthyle, éthyle, n-propyle, isopropyle, n-butyle, sec-butyle, isobutyle, tert-butyle, vinyle, 1-propényle, 2-propényle, 1-butényle, 2-butényle et 3-butényle ;
un radical (hétéro)cycloaliphatique choisi dans le groupe constitué par cyclopropyle, cyclobutyle, cyclopentyle, cyclohexyle et 7,7-diméthyl-2-oxa-bicyclo[2.2.1]heptyle, le radical (hétéro)cycloaliphatique pouvant à chaque fois être substitué par 1, 2 ou 3 substituants choisis indépendamment les uns des autres dans le groupe constitué par oxo (=O), thioxo (=S), F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-H, -C(=O)-CH₃, -C (=O) -C₂H₅, -C(=O)-C(CH₃)₃, -C (=O) -OH, -C (=O) -O-CH₃, -C(=O)-O-C₂H₅, -(CH₂)-C(=O)-OH, - (CH₂) -C (=O) -O-CH₃, -(CH₂)-C(=O)-O-C₂H₅, - N(CH₃)₂, -N(C₂H₅)₂, -N(H)(CH₃), -N(H)(C₂H₅), phényle et benzyle ;
ou un radical choisi dans le groupe constitué par phényle, naphtyle, thiophényle, furanyle, pyrrolyle, pyrazolyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -O-CH (CH₃) ₂, -O-CH₂-CH₂-CH₂-CH₃, -O-CH₂-CH=CH₂, -NH₂, -NO₂, -O-CF₃, -O-CHF₂, -O-CH₂F, -S-CF₃, -S-CHF₂, -S-CH₂F, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, -N(CH₃)₂, - N(C₂H₅)₂, -N(H)(CH₃), -N(H)(C₂H₅), -NH-C(=O)-CH₃, -NH-C (=O) -C₂H₅, -C(=O)-H, -C (=O) -CH₃, -C(=O)-C₂H₅, -C(=O)-NH₂, -C(=O)-NH-CH₃, -C(O)-N-(CH₃)₂, -S(=O)₂-NH₂, -S(=O)₂-CH₃, phényle et benzyle ;
R¹⁷ et R¹⁸ représentent à chaque fois indépendamment l'un de l'autre un radical méthyle ou éthyle ;
R¹⁹ représente un radical phényle qui peut être relié par un groupe -(CH₂)-, -(CH₂)-(CH₂)- ou -(CH₂)-O-(CH₂)- ;
R²⁴ et R²⁵ représentent à chaque fois indépendamment l'un de l'autre un radical méthyle ou éthyle ;
R²⁸ représente un radical phényle qui peut être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, -S-CH₃, -S-C₂H₅, méthyle, éthyle, n-propyle, isopropyle, n-butyle, sec-butyle, isobutyle, tert-butyle ;
et
R²⁹ représente un radical alkyle choisi dans le groupe constitué par méthyle, éthyle, n-propyle, isopropyle, n-butyle, isobutyle et tert-butyle ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme de sels correspondants, ou à chaque fois sous la forme de solvates correspondants.

4. Composés selon une ou plusieurs des revendications 1 à 3, **caractérisés en ce que** R¹ représente
- (CHR⁴)-C(=O)-OR⁵, -(CHR⁴)-(CH₂)-C(=O)-OR⁵ ou - (CHR⁴)-(CH₂)-(CH₂)-C(=O)-OR⁵ ;
-(CHR⁶)-R⁸, -(CHR⁶)-(CHR⁷)-R⁸, -(CHR⁶)-(CHR⁷)-O-R⁸,-(CHR⁶)-(CHR⁷)-(CH₂)-R⁸, -(CHR⁶)-(CHR⁷)-(CH₂)-N(CH₃)-R⁸ ;
un radical alkyle éventuellement substitué choisi dans le groupe constitué par méthyle, éthyle, n-propyle, isopropyle, -(CH₂)-(CH₂)-CN, (CH₂)-(CH₂) - N(CH₃)₂, -(CH₂)-(CH₂)-O-CH₃, -(CH₂)-(CH₂)-S-C₂H₅, -n-butyle, sec-butyle, isobutyle, tert-butyle, -(CH₂)-(CH₂)-(CH₂)-O-CH₃, n-pentyle, sec-pentyle, -(CH₂)-(CH₂) - (C(CH₃)₃), n-hexyle, n-heptyle, n-octyle et -(CH₂)-(CH)(C₂H₅)-(CH₂)-(CH₂)-(CH₂)-(CH₃) ;
un radical alcynyle choisi dans le groupe constitué par 1-propynyle et 2-propynyle ;
un radical cycloaliphatique choisi dans le groupe constitué par cyclopropyle, cyclobutyle, cyclopentyle, cyclohexyle, cycloheptyle, (6,6)-diméthyl-[3.1.1]-bicycloheptyle, indanyle et indényle, le radical cycloaliphatique pouvant à chaque fois être substitué par un radical -O-benzyle ou un radical méthyle,
ou un radical pyrrolidinyle ou pipéridinyle, qui peut à chaque fois être substitué sur l'atome d'azote avec un substituant choisi dans le groupe constitué par -C(=O)-CH₃, -C(=O)-C₂H₅, -C(=O)-C(CH₃)₃, -C(=O)-O-C₂H₅, benzyle et -(CH₂)-naphtyle, la partie cyclique du radical benzyle pouvant être substituée par 1, 2 ou 3 substituants choisis indépendamment les uns des autres dans le groupe constitué par F, Cl, Br, -CF₃ et - O-CF₃,
ou un radical phényle, qui peut être substitué par 1, 2 ou 3 substituants choisis indépendamment les uns des autres dans le groupe constitué par -O-CH₃, -O-C₂H₅, méthyle, éthyle, n-propyle, isopropyle, n-butyle, sec-butyle, isobutyle et tert-butyle ;
R² représente
un radical hydrogène ou
-(CH₂)-R⁹
ou
R¹ et R² forment ensemble avec l'atome d'azote auquel ils sont reliés en tant qu'élément de cycle un radical choisi dans le groupe constitué par 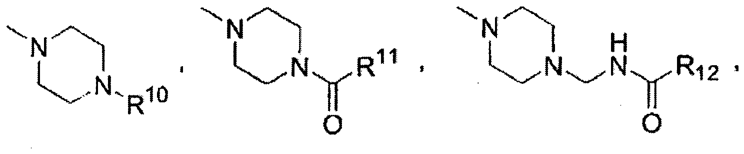 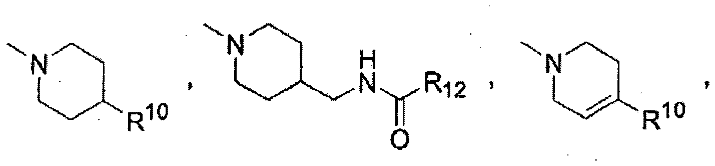 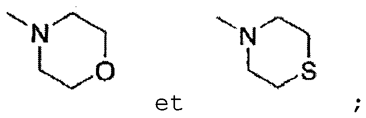
R³ représente
-C(=O)-NR¹³R¹⁴ ;
-C(=O)-R¹⁵ ;
-C(=O)-(CH₂)-O-(CH₂)-C(=O)-OR¹⁶, -C(=O)-(CH₂)-CR¹⁷R¹⁸-(CH₂)-C(=O)-OR¹⁶, -C(=O)-CR¹⁷R¹⁸-(CH₂)-C(=O)-OR¹⁶ ou -C(=O)-(CH₂)-(CH₂)-(CH₂)-C(=O)-OR¹⁶ ;
-C(=O)-(CHR¹⁹)-NH-C(=O)-OR²⁰ ;
-C(=O)-(CH₂)-R²¹, -C(=O)-(CH₂)-O-R²¹, -C(=O)-(CH₂)-O-(CH₂)-R²¹ ou -C(=O)-(CH₂)-(CH₂)-(CH₂)-O-R²¹ ;
-C(=O)-(CH=CH)-R²² ;
-S(=O)₂-R²³ ;
-S(=O)₂-NR²⁴R²⁵ ;
-C(=S)-NR²⁶-R²⁷, -C(=S)-NR²⁶-(CH₂)-R²⁷, -C(=S)-NR²⁶-(CH₂)-(CH₂)-R²⁷ ou -C(=S)-NR²⁶-(CH₂)-(CH₂)-O-R²⁷ ;
-C(=S)-NR²⁶-(CHR²⁸)-R²⁹ ;
-C(=S)-NR²⁶-C(=O)-R³⁰ ;
ou (CH₂)-R³¹ ;
R⁴ représente
-C(=O)-NH₂ ;
un radical hydrogène ou
un radical alkyle choisi dans le groupe constitué par méthyle, éthyle, n-propyle, isopropyle et n-butyle ;
R⁵ représente un radical alkyle choisi dans le groupe constitué par méthyle, éthyle, n-propyle, isopropyle, n-butyle, sec-butyle, isobutyle et tert-butyle ;
R⁶, R⁷ et R¹⁶ représentent à chaque fois, indépendamment les uns des autres un radical hydrogène ou un radical alkyle choisi dans le groupe constitué par méthyle, éthyle, n-propyle et isopropyle ;
R⁸ représente
un radical (hétéro)cycloaliphatique choisi dans le groupe constitué par cyclopropyle, cyclobutyle, cyclopentyle, cyclohexyle, pyrrolidinyle, pipéridinyle, morpholinyle et thiomorpholinyle ;
ou un radical choisi dans le groupe constitué par phényle, naphtyle, thiophényle, furanyle, pyridinyle et indolyle, le radical pouvant à chaque fois être substitué par 1, 2 ou 3 substituants choisis indépendamment les uns des autres dans le groupe constitué par F, Cl, Br, -CF₃, -O-CH₃, -O-C₂H₅, -O-CH₂- CH=CH₂, -méthyle, éthyle, n-propyle, isopropyle, n-butyle, tert-butyle, -S-CH₃, -S-C₂H₅, -S(=O)₂-NH₂ et - S(=O)₂NH-CH₃ ;
R⁹ représente un radical choisi dans le groupe constitué par phényle, naphtyle, thiophényle, furanyle et pyridinyle ;
R¹⁰ représente
un radical alkyle éventuellement substitué choisi dans le groupe constitué par -(CH₂)-N(CH₃)₂, -(CH₂)-(CH₂)-N(CH₃)₂, -(CH₂)-(CH₂)-(CH₂)-N(CH₃)₂, -(CH₂)-N(C₂H₅)₂, -(CH₂)-(CH₂)-N(C₂H₅)₂ et -(CH₂)-(CH₂)-(CH₂)-N(C₂H₅)₂ ;
un radical cycloaliphatique choisi dans le groupe constitué par pipérazinyle, pyrrolidinyle, pipéridinyle, morpholinyle, cyclopropyle, cyclobutyle, cyclopentyle et cyclohexyle, le radical cycloaliphatique pouvant être relié par un groupe - (CH₂) - ou -(CH₂)-(CH₂)- ou -(CH₂)-(CH₂)-(CH₂)- ;
ou un radical choisi dans le groupe constitué par thiazolyle, phényle, naphtyle et thiéno[2,3-d]pyrimidinyle, le radical pouvant à chaque fois être substitué par 1, 2 ou 3 substituants choisis indépendamment les uns des autres dans le groupe constitué par F, Cl, Br, I, méthyle, éthyle, n-propyle, -C(=O)-CH₃ et -C(=O)-C₂H₅, et/ou relié par un groupe - (CH₂) -, -CH(CH₃)-, -(CH₂)-(CH₂)-, -(CH₂)-(CH₂)-(CH₂)- ou - (CH₂)-(CH=CH) ;
R¹¹ représente
un radical alkyle éventuellement substitué choisi dans le groupe constitué par méthyle, éthyle, n-propyle, -CF₃ et -CF₂-CF₃,
ou un radical choisi dans le groupe constitué par phényle et naphtyle, le radical pouvant à chaque fois être substitué par 1, 2 ou 3 substituants choisis indépendamment les uns des autres dans le groupe constitué par F, Cl et Br et/ou relié par un groupe - (CH₂)- ou -(CH₂)-(CH₂)-;
R¹² représente
un radical alkyle éventuellement substitué choisi dans le groupe constitué par méthyle, éthyle, n-propyle, -CF₃ et -CF₂-CF₃ ;
R¹³ représente un radical hydrogène ;
R¹⁴ représente un radical choisi dans le groupe constitué par phényle et naphtyle, le radical pouvant à chaque fois être substitué par 1, 2 ou 3 substituants choisis indépendamment les uns des autres dans le groupe constitué par F, Cl, Br, -CN, -CF₃, -O-CH₃, -O-C₂H₅, -O-CH(CH₃)₂, -O-CH₂-CH₂-CH₂-CH₃, -NO₂, -O-CF₃, -O-CHF₂, -O-CH₂F, -S-CF₃, -S-CHF₂, -S-CH₂F, méthyle, éthyle, n-propyle, isopropyle, n-butyle, sec-butyle, isobutyle, tert-butyle, -C(=O)-O-CH₃, -C(=O)-O-C₂H₅, -C(=O)-CH₃ et - C(=O)-C₂H₅, et/ou relié par un groupe -(CH₂)-, -CH(CH₃)-ou -(CH₂)-(CH₂)-;
R¹⁵ représente
un radical choisi dans le groupe constitué par 1-butényle, 2-butényle et 3-butényle ;
un radical 4,7,7-triméthyl-3-oxo-2-oxabicyclo[2.2.1]heptyle
ou un radical choisi dans le groupe constitué par phényle, naphtyle, thiophényle, furanyle, triazolyle, pyridinyle, pyrazolyle, quinolinyle et isoquinolinyle, le radical pouvant à chaque fois être substitué par 1, 2 ou 3 substituants choisis indépendamment les uns des autres dans le groupe constitué par F, Cl, Br, -CF₃, -O-CH₃, -O-C₂H₅, -O-CH(CH₃)₂, -O-CH₂-CH₂-CH₂-CH₃, -O-CF₃, -O-CHF₂, -O-CH₂F, -S-CF₃, -S-CHF₂, -S-CH₂F, méthyle, éthyle, n-propyle, isopropyle, n-butyle, sec-butyle, isobutyle, tert-butyle, phényle, -NH-C(=O)-CH₃ et -NH-C(=O)-C₂H₅ ;
R¹⁷ et R¹⁸ représentent à chaque fois indépendamment l'un de l'autre un radical méthyle ou éthyle ;
R¹⁹ représente un radical phényle qui peut être relié par un groupe -(CH₂)-, -(CH₂)-(CH₂)- ou -(CH₂)-O-(CH₂)- ; R²⁰ représente
un radical alkyle choisi dans le groupe constitué par méthyle, éthyle, n-propyle, isopropyle, n-butyle, sec-butyle, isobutyle et tert-butyle,
ou un radical benzyle ;
R²¹ représente
un radical cycloaliphatique choisi dans le groupe constitué par cyclopropyle, cyclobutyle, cyclopentyle, cyclohexyle, cycloheptyle, le radical cycloaliphatique pouvant à chaque fois être substitué par 1, 2 ou 3 substituants choisis indépendamment les uns des autres dans le groupe constitué par -C(=O)-OH et -(CH₂)-C(=O)-OH,
ou un radical choisi dans le groupe constitué par phényle, naphtyle, (1,3)-benzodioxolyle et (1,4)-benzodioxanyle, le radical pouvant à chaque fois être substitué par 1, 2 ou 3 substituants choisis indépendamment les uns des autres dans le groupe constitué par F, Cl, Br, -O-CH₃ et -O-C₂H₅ ;
R²² représente un radical phényle ;
R²³ représente un radical choisi dans le groupe constitué par phényle, naphtyle, thiophényle, furanyle et pyridinyle, le radical pouvant à chaque fois être substitué par 1, 2 ou 3 substituants choisis indépendamment les uns des autres dans le groupe constitué par F, Cl, Br, -CF₃, -O-CH₃, -O-C₂H₅, -0-CH(CH₃)₂, -O-CH₂-CH₂-CH₂-CH₃, -O-CF₃, -O-CHF₂, -O-CH₂F, méthyle, éthyle, n-propyle, isopropyle, n-butyle, sec-butyle, isobutyle et tert-butyle ;
R²⁴ et R²⁵ représentent à chaque fois indépendamment l'un de l'autre un radical méthyle ou éthyle ;
R²⁶ représente un radical hydrogène ;
R²⁷ représente
un radical alkyle choisi dans le groupe constitué par méthyle, éthyle, n-propyle, isopropyle et n-butyle ;
un radical cyclohexyle ;
ou un radical phényle, qui peut être substitué par 1, 2, 3, 4 ou 5 substituants choisis indépendamment les uns des autres dans le groupe constitué par F, Cl, Br, -O-CH₃ et -O-C₂H₅ ;
R²⁸ représente un radical phényle qui peut être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -O-CHF₂, -O-CH₂F, -S-CF₃, -S-CHF₂, -S-CH₂F, -SH, -S-CH₃, -S-C₂H₅, méthyle, éthyle, n-propyle, isopropyle, n-butyle, sec-butyle, isobutyle, tert-butyle ;
R²⁹ représente un radical alkyle choisi dans le groupe constitué par méthyle, éthyle, n-propyle, isopropyle, n-butyle, isobutyle et tert-butyle ;
R³⁰ représente un radical phényle
et
R³¹ représente un radical choisi dans le groupe constitué par phényle et naphtyle, le radical pouvant à chaque fois être substitué par 1, 2 ou 3 substituants choisis indépendamment les uns des autres dans le groupe constitué par F, Cl et Br ;
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme de sels correspondants, ou à chaque fois sous la forme de solvates correspondants.

5. Composés selon une ou plusieurs des revendications 1 à 4, choisis dans le groupe constitué par
[1] 3-[(2-méthoxy-éthyl)-amide]-5-[(3-méthoxy-phényl)-amide] de l'acide 6,7-dihydro-4H-isoxazolo[4,5-c]pyridine-3,5-dicarboxylique,
[2] 3-cyclopentylamide-5-(4-fluoro-benzylamide) de l'acide 6,7-dihydro-4H-isoxazolo[4,5-c]pyridine-3,5-dicarboxylique,
[3] 3-phénylamide-5-[(4-trifluorométhoxy-phényl)-amide] de l'acide 6,7-dihydro-4H-isoxazolo[4,5-c]pyridine-3,5-dicarboxylique,
[4] 5-[(4-méthyl-3-nitro-phényl)-amide]-3-(phénéthyl-amide) de l'acide 6,7-dihydro-4H-isoxazolo[4,5-c]pyridine-3,5-dicarboxylique,
[5] 3-[(2-méthoxy-éthyl)-amide]-5-[(4-méthyl-3-nitro-phényl)-amide] de l'acide 6,7-dihydro-4H-isoxazolo[4,5-c]pyridine-3,5-dicarboxylique,
[6] ester éthylique de l'acide 3-{[5-(2,5-difluoro-phénylcarbamoyl)-4,5,6,7-tétrahydro-isoxazolo[4,5-c]pyridine-3-carbonyl]-amino}-propionique,
[7] 5-[(4-butoxy-phényl)-amide]-3-[(2~méthoxy-éthyl)-amide] de l'acide 6,7-dihydro-4H-isoxazolo[4,5-c]pyridine-3,5-dicarboxylique,
[8] 5-[(3-fluoro-phényl)-amide]-3-(phénéthyl-amide) de l'acide 6,7-dihydro-4H-isoxazolo[4,5-c]pyridine-3,5-dicarboxylique,
[9] 5-[(4-méthyl-3-nitro-phényl)-amide]-3-[(thiophén-2-ylméthyl)-amide] de l'acide 6,7-dihydro-4H-isoxazolo[4,5-c]pyridine-3,5-dicarboxylique,
[10] 3-benzylamide-5-(phénéthyl-amide) de l'acide 6,7-dihydro-4H-isoxazolo[4,5-c]pyridine-3,5-dicarboxylique,
[11] 3-[(2-éthylsulfanyl-éthyl)-amide]-5-[(3-méthoxy-phényl)-amide] de l'acide 6,7-dihydro-4H-isoxazolo[4,5-c]pyridine-3,5-dicarboxylique,
[12] 5-[(3-cyano-phényl)-amide]-3-[(thiophén-2-ylméthyl)-amide] de l'acide 6,7-dihydro-4H-isoxazolo[4,5-c]pyridine-3,5-dicarboxylique,
[13] 5-[(4-éthoxy-phényl)-amide]-3-phénylamide de l'acide 6,7-dihydro-4H-isoxazolo[4,5-c]pyridine-3,5-dicarboxylique,
[14] 3-(4-fluoro-benzylamide)-5-[(4-méthyl-3-nitro-phényl)-amide] de l'acide 6,7-dihydro-4H-isoxazolo[4,5-c]pyridine-3,5-dicarboxylique,
[15] 5-[(3-acétyl-phényl)-amide]-3-[(5-méthyl-furan-2-ylméthyl)-amide] de l'acide 6,7-dihydro-4H-isoxazolo[4,5-c]pyridine-3,5-dicarboxylique,
[16] 5-[(4-méthyl-3-nitro-phényl)-amide]-3-prop-2-ynylamide de l'acide 6,7-dihydro-4H-isoxazolo[4,5-c]pyridine-3,5-dicarboxylique,
[17] 3-benzylamide-5-phénylamide de l'acide 6,7-dihydro-4H-isoxazolo[4,5-c]pyridine-3,5-dicarboxylique,
[18] 5-[(1-naphtalén-1-yl-éthyl)-amide]-3-(phénéthyl-amide) de l'acide 6,7-dihydro-4H-isoxazolo[4,5-c]pyridine-3,5-dicarboxylique,
[19] 5-[(3-fluoro-phényl)-amide]-3-prop-2-ynylamide de l'acide 6,7-dihydro-4H-isoxazolo[4,5-c]pyridine-3,5-dicarboxylique,
[20] ester éthylique de l'acide 3-{[5-(2,5-diméthoxy-phénylcarbamoyl)-4,5,6,7-tétrahydroisoxazolo[4,5-c]pyridine-3-carbonyl]-amino}-propionique,
[21] 5-[(4-éthoxy-phényl)-amide]-3-(4-fluoro-benzylamide) de l'acide 6,7-dihydro-4H-isoxazolo[4,5-c]pyridine-3,5-dicarboxylique,
[22] 5-[(5-chloro-2-méthoxy-phényl)-amide]-3-(phénéthyl-amide) de l'acide 6,7-dihydro-4H-isoxazolo[4,5-c]pyridine-3,5-dicarboxylique,
[23] ester éthylique de l'acide 3-({3-[(5-méthyl-furan-2-ylméthyl)-carbamoyl]-6,7-dihydro-4H-isoxazolo[4,5-c]pyridine-5-carbonyl}-amino)-benzoïque,
[24] 5-[(5-chloro-2-méthoxy-phényl)-amide]-3-(isobutyl-amide) de l'acide 6,7-dihydro-4H-isoxazolo[4,5-c]pyridine-3,5-dicarboxylique,
[25] acide [2-oxo-2-(3-prop-2-ynylcarbamoyl-6,7-dihydro-4H-isoxazolo[4,5-c]pyridin-5-yl)-éthoxy]-acétique,
[26] acide 3-éthyl-5-[3-(2-méthoxy-éthylcarbamoyl)-6,7-dihydro-4H-isoxazolo[4,5-c]pyridin-5-yl]-3-méthyl-5-oxo-pentanoïque,
[27] ester tert-butylique de l'acide (1-benzyloxyméthyl-2-oxo-2-{3-[(pyridin-3-ylméthyl)-carbamoyl]-6,7-dihydro-4H-isoxazolo[4,5-c]pyridin-5-yl}-éthyl)-carbamique,
[28] acide {1-[2-oxo-2-(3-phénylcarbamoyl-6,7-dihydro-4H-isoxazolo[4,5-c]pyridin-5-yl)-éthyl]-cyclopentyl}-acétique,
[29] acide 3-éthyl-3-méthyl-5-oxo-5-(3-phénylcarbamoyl-6,7-dihydro-4H-isoxazolo[4,5-c]pyridin-5-yl)-pentanoïque,
[30] acide (2-oxo-2-{3-[(thiophén-2-ylméthyl)-carbamoyl]-6,7-dihydro-4H-isoxazolo[4,5-c]pyridin-5-yl}-éthoxy)-acétique,
[31] acide 3,3-diméthyl-4-{3-[(5-méthyl-furan-2-ylméthyl)-carbamoyl]-6,7-dihydro-4H-isoxazolo[4,5-c]pyridin-5-yl}-4-oxo-butanoïque,
[32] benzyl-phénéthyl-amide de l'acide 5-(4-trifluorométhoxy-benzènesulfonyl)-4,5,6,7-tétrahydro-isoxazolo[4,5-c]pyridine-3-carboxylique,
[33] (1-naphtalén-2-ylméthyl-pyrrolidin-3-yl)-amide de l'acide 5-benzènesulfonyl-4,5,6,7-tétrahydro-isoxazolo[4,5-c]pyridine-3-carboxylique,
[34] [5-(4-fluoro-benzènesulfonyl)-4,5,6,7-tétrahydro-isoxazolo[4,5-c]pyridin-3-yl]-(4-thiéno[2,3-d]pyrimidin-4-yl-pipérazin-1-yl)-méthanone,
[35] (3-phényl-propyl)-amide de l'acide 5-(4-fluoro-benzènesulfonyl)-4,5,6,7-tétrahydro-isoxazolo[4,5-c]pyridine-3-carboxylique,
[36] (2-diméthylamino-éthyl)-amide de l'acide 5-(3-phényl-acryloyl)-4,5,6,7-tétrahydro-isoxazolo[4,5-c]pyridine-3-carboxylique,
[37] (2-diméthylamino-éthyl)-amide de l'acide 5-(2-phénoxy-acétyl)-4,5,6,7-tétrahydro-isoxazolo[4,5-c]pyridine-3-carboxylique,
[38] (1-méthyl-3-phényl-propyl)-amide de l'acide 5-(4-fluoro-benzènesulfonyl)-4,5,6,7-tétrahydro-isoxazolo[4,5-c]pyridine-3-carboxylique,
[39] [1-(4-trifluorométhyl-benzyl)-pyrrolidin-3-yl]-amide de l'acide 5-(4-fluoro-benzènesulfonyl)-4,5,6,7-tétrahydro-isoxazolo[4,5-c]pyridine-3-carboxylique,
[40] (2-benzyloxy-cyclopentyl)-amide de l'acide 5-(2,5-dichloro-benzènesulfonyl)-4,5,6,7-tétrahydro-isoxazolo[4,5-c]pyridine-3-carboxylique,
[41] (2-diméthylamino-éthyl)amide de l'acide 5-(5-tert-butyl-2-méthyl-2H-pyrazole-3-carbonyl)-4,5,6,7-tétrahydro-isoxazolo[4,5-c]pyridine-3-carboxylique,
[42] [5-(4-fluoro-benzènesulfonyl)-4,5,6,7-tétrahydro-isoxazolo[4,5-c]pyridin-3-yl]-[4-(4-fluoro-phényl)-3,6-dihydro-2H-pyridin-1-yl]-méthanone,
[43] ester éthylique de l'acide 4-{[5-(4-fluoro-benzènesulfonyl)-4,5,6,7-tétrahydro-isoxazolo[4,5-c]pyridine-3-carbonyl]-amino}-pipéridin-1-carboxylique,
[44] cyclopentylamide de l'acide 5-(3-phényl-acryloyl)-4,5,6,7-tétrahydro-isoxazolo[4,5-c]pyridine-3-carboxylique,
[45] cyclopropylméthyl-amide de l'acide 5-(4-acétylamino-benzoyl)-4,5,6,7-tétrahydro-isoxazolo[4,5-c]pyridine-3-carboxylique,
[46] (pyridin-3-ylméthyl)-amide de l'acide 5-(5-méthyl-2-phényl-2H-[1,2,3]triazole-4-carbonyl)-4,5,6,7-tétrahydro-isoxazolo[4,5-c]pyridine-3-carboxylique,
[47] 3,4-diméthoxy-benzylamide de l'acide 5-benzènesulfonyl-4,5,6,7-tétrahydro-isoxazolo[4,5-c]pyridine-3-carboxylique,
[48] 2-(3,4-difluoro-phényl)-1-{4-[5-(4-fluoro-benzènesulfonyl)-4,5,6,7-tétrahydro-isoxazolo[4,5-c]pyridine-3-carbonyl]-pipérazin-1-yl}-éthanone,
[49] diméthylamide de l'acide 3-(morpholine-4-carbonyl)-6,7-dihydro-4H-isoxazolo[4,5-c]pyridine-5-sulfonique,
[50] (thiophén-2-ylméthyl)-amide de l'acide 5-(furane-2-carbonyl)-4,5,6,7-tétrahydro-isoxazolo[4,5-c]pyridine-3-carboxylique,
[51] (2-pyrrolidin-1-yl-éthyl)-amide de l'acide 5-(4,7,7-triméthyl-3-oxo-2-oxa-bicyclo[2.2.1]heptane-1-carbonyl)-4,5,6,7-tétrahydro-isoxazolo[4,5-c]pyridine-3-carboxylique,
[52] [2-(2-chloro-phényl)-éthyl]-amide de l'acide 5-(2,5-dichloro-benzènesulfonyl)-4,5,6,7-tétrahydro-isoxazolo[4,5-c]pyridine-3-carboxylique,
[53] p-tolyl-amide de l'acide 5-(4-méthoxy-benzènesulfonyl)-4,5,6,7-tétrahydro-isoxazolo[4,5-c]pyridine-3-carboxylique,
[54] 4-fluoro-benzylamide de l'acide 5-(2-phénoxy-acétyl)-4,5,6,7-tétrahydro-isoxazolo[4,5-c]pyridine-3-carboxylique,
[55] 1-{4-[5-(4-méthoxy-benzènesulfonyl)-4,5,6,7-tétrahydro-isoxazolo[4,5-c]pyridine-3-carbonyl]-pipérazin-1-yl}-éthanone,
[56] 4-sulfamoyl-benzylamide de l'acide 5-(toluène-4-sulfonyl)-4,5,6,7-tétrahydro-isoxazolo[4,5-c]pyridine-3-carboxylique,
[57] (2-morpholin-4-yl-éthyl)-amide de l'acide 5-(thiophène-2-sulfonyl)-4,5,6,7-tétrahydro-isoxazolo[4,5-c]pyridine-3-carboxylique,
[58] (2-diméthylamino-éthyl)-amide de l'acide 5-(4-méthoxy-benzoyl)-4,5,6,7-tétrahydro-isoxazolo[4,5-c]pyridine-3-carboxylique,
[59] benzyl-phénéthyl-amide de l'acide 5-(2,5-dichloro-benzènesulfonyl)-4,5,6,7-tétrahydro-isoxazolo[4,5-c]pyridine-3-carboxylique,
[60] (2,6,6-triméthyl-bicyclo[3.1.1]hept-3-yl)-amide de l'acide 5-diméthylsulfamoyl-4,5,6,7-tétrahydro-isoxazolo[4,5-c]pyridine-3-carboxylique,
[61] indan-1-ylamide de l'acide 5-benzènesulfonyl-4,5,6,7-tétrahydro-isoxazolo[4,5-c]pyridine-3-carboxylique,
[62] (3-méthoxy-propyl)-amide de l'acide 5-(4-méthoxy-benzènesulfonyl)-4,5,6,7-tétrahydro-isoxazolo[4,5-c]pyridine-3-carboxylique,
[63] (2-morpholin-4-yl-éthyl)-amide de l'acide 5-(4,5-dichloro-thiophène-2-sulfonyl)-4,5,6,7-tétrahydro-isoxazolo[4,5-c]pyridine-3-carboxylique,
[64] (2-pyrrolidin-1-yl-éthyl)-amide de l'acide 5-(2-benzyloxy-acétyl)-4,5,6,7-tétrahydro-isoxazolo[4,5-c]pyridine-3-carboxylique,
[65] [5-(2,5-dichloro-benzènesulfonyl)-4,5,6,7-tétrahydro-isoxazolo[4,5-c]pyridin-3-yl]-[4-(2,4-diméthyl-phényl)-pipérazin-1-yl]-méthanone,
[66] cyclopropylméthyl-amide de l'acide 5-pent-4-énoyl-4,5,6,7-tétrahydro-isoxazolo[4,5-c]pyridine-3-carboxylique,
[67] ester tert-butylique de l'acide 3-méthyl-2-{[5-(3-trifluorométhyl-benzènesulfonyl)-4,5,6,7-tétrahydro-isoxazolo[4,5-c]pyridin-3-carbonyl]-amino}-butanoïque,
[68] benzyl-phénéthyl-amide de l'acide 5-(3-trifluorométhyl-benzènesulfonyl)-4,5,6,7-tétrahydro-isoxazolo[4,5-c]pyridine-3-carboxylique,
[69] 2,4-dichloro-benzylamide de l'acide 5-(3-trifluorométhyl-benzènesulfonyl)-4,5,6,7-tétrahydro-isoxazolo[4,5-c]pyridine-3-carboxylique,
[70] (2-cyano-éthyl)-pyridin-3-ylméthyl-amide de l'acide 5-diméthylsulfamoyl-4,5,6,7-tétrahydro-isoxazolo[4,5-c]pyridine-3-carboxylique,
[71] (2-pyrrolidin-1-yl-éthyl)-amide de l'acide 5-(2-cyclopentyl-acétyl)-4,5,6,7-tétrahydro-isoxazolo[4,5-c]pyridine-3-carboxylique,
[72] 4-fluoro-benzylamide de l'acide 5-(5-méthyl-2-phényl-2H-[1,2,3]triazole-4-carbonyl)-4,5,6,7-tétrahydro-isoxazolo[4,5-c]pyridine-3-carboxylique,
[73] (2-éthyl-hexyl)-amide de l'acide 5-(3-trifluorométhyl-benzènesulfonyl)-4,5,6,7-tétrahydro-isoxazolo[4,5-c]pyridine-3-carboxylique,
[74] (2-diméthylamino-éthyl)-amide de l'acide 5-(2,3-difluoro-4-méthyl-benzoyl)-4,5,6,7-tétrahydro-isoxazolo[4,5-c]pyridine-3-carboxylique,
[75] 2,3-dichloro-benzylamide de l'acide 5-(4-trifluorométhoxy-benzènesulfonyl)-4,5,6,7-tétrahydro-isoxazolo[4,5-c]pyridine-3-carboxylique,
[76] [2-(4-chloro-phényl)-éthyl]-amide de l'acide 5-diméthylsulfamoyl-4,5,6,7-tétrahydro-isoxazolo[4,5-c]pyridine-3-carboxylique,
[77] (2-pyrrolidin-1-yl-éthyl)-amide de l'acide 5-(6-chloro-pyridine-3-carbonyl)-4,5,6,7-tétrahydro-isoxazolo[4,5-c]pyridine-3-carboxylique,
[78] ester méthylique de l'acide 5-[3-(4-fluoro-benzylcarbamoyl)-6,7-dihydro-4H-isoxazolo[4,5-c]pyridin-5-yl]-5-oxo-pentanoïque,
[79] benzylamide de l'acide 5-[2-(4-méthoxy-phényl)-acétyl]-4,5,6,7-tétrahydro-isoxazolo[4,5-c]pyridine-3-carboxylique,
[80] [3-(méthyl-phényl-amino)-propyl]-amide de l'acide 5-(4-trifluorométhoxy-benzènesulfonyl)-4,5,6,7-tétrahydro-isoxazolo[4,5-c]pyridine-3-carboxylique,
[81] cyclopentylamide de l'acide 5-[2-(4-chloro-phénoxy)-acétyl]-4,5,6,7-tétrahydro-isoxazolo[4,5-c]pyridine-3-carboxylique,
[82] [4-(2-cyclohexyl-éthyl)-pipérazin-1-yl]-[5-(toluène-4-sulfonyl)-4,5,6,7-tétrahydro-isoxazolo[4,5-c]pyridin-3-yl]-méthanone,
[83] N-{1-[5-(2,5-dichloro-benzènesulfonyl)-4,5,6,7-tétrahydro-isoxazolo[4,5-c]pyridine-3-carbonyl]-pipéridin-4-ylméthyl}-2,2,2-trifluoro-acétamide,
[84] [1-(4-méthoxy-phényl)-éthyl]-amide de l'acide 5-(4-méthoxy-benzènesulfonyl)-4,5,6,7-tétrahydro-isoxazolo[4,5-c]pyridine-3-carboxylique,
[85] ester éthylique de l'acide 2-{[5-(thiophène-2-sulfonyl)-4,5,6,7-tétrahydro-isoxazolo[4,5-c]pyridine-3-carbonyl]-amino-propionique,
[86] 1-(4-{4-[5-(toluène-4-sulfonyl)-4,5,6,7-tétrahydro-isoxazolo[4,5-c]pyridine-3-carbonyl]-pipérazin-1-yl}-phényl)-éthanone,
[87] (4-allyloxy-benzyl)-furan-2-ylméthyl-amide de l'acide 5-(3-trifluorométhyl-benzènesulfonyl)-4,5,6,7-tétrahydro-isoxazolo[4,5-c]pyridine-3-carboxylique,
[88] ester tert-butylique de l'acide 4-carbamoyl-4-{[5-(4-méthoxy-benzènesulfonyl)-4,5,6,7-tétrahydro-isoxazolo[4,5-c]pyridine-3-carbonyl]-amino}-butanoïque,
[89] furan-2-ylméthyl-(4-méthylsulfanyl-benzyl)-amide de l'acide 5-diméthylsulfamoyl-4,5,6,7-tétrahydro-isoxazolo[4(5-c]pyridine-3-carboxylique,
[90] (2-morpholin-4-yl-éthyl)-amide de l'acide 5-(4-bromo-3-méthyl-benzoyl)-4,5,6,7-tétrahydro-isoxazolo[4,5-c]pyridine-3-carboxylique,
[91] [1-(2,6-dichloro-benzyl)-pyrrolidin-3-yl]-amide de l'acide 5-(toluène-4-sulfonyl)-4,5,6,7-tétrahydro-isoxazolo[4,5-c]pyridine-3-carboxylique,
[92] [2-(3,4-dichloro-phényl)-éthyl]-amide de l'acide 5-benzènesulfonyl-4,5,6,7-tétrahydro-isoxazolo[4,5-c]pyridine-3-carboxylique,
[93] (2-diméthylamino-éthyl)-amide de l'acide 5-(3-difluorométhylsulfanyl-benzoyl)-4,5,6,7-tétrahydro-isoxazolo[4,5-c]pyridine-3-carboxylique,
[94] (2-pyrrolidin-1-yl-éthyl)-amide de l'acide 5-[2-(3-chloro-phénoxy)-acétyl]-4,5,6,7-tétrahydro-isoxazolo[4,5-c]pyridine-3-carboxylique,
[95] 2,3-diméthoxy-benzylamide de l'acide 5-(4-méthoxy-benzènesulfonyl)-4,5,6,7-tétrahydro-isoxazolo[4,5-c]pyridine-3-carboxylique,
[96] (1-méthyl-3-phényl-propyl)-amide de l'acide 5-(4-trifluorométhoxy-benzènesulfonyl)-4,5,6,7-tétrahydro-isoxazolo[4,5-c]pyridine-3-carboxylique,
[97] (5-benzènesulfonyl-4,5,6,7-tétrahydro-isoxazolo[4,5-c]pyridin-3-yl)-[4-(3-phényl-allyl)-pipérazin-1-yl]-méthanone,
[98] ester éthylique de l'acide 3-{[5-(4-méthoxy-benzoyl)-4,5,6,7-tétrahydro-isoxazolo[4,5-c]pyridine-3-carbonyl]-amino}-propionique,
[99] (2-diméthylamino-éthyl)-amide de l'acide 5-(4-phénoxy-butyryl)-4,5,6,7-tétrahydro-isoxazolo[4,5-c]pyridine-3-carboxylique,
[100] [2-(7-méthyl-1H-indol-3-yl)-éthyl]-amide de l'acide 5-(4-trifluorométhoxy-benzènesulfonyl)-4,5,6,7-tétrahydro-isoxazolo[4,5-c]pyridine-3-carboxylique,
[101] [4-{3-phényl-allyl)-pipérazin-1-yl]-[5-(thiophène-2-sulfonyl)-4,5,6,7-tétrahydro-isoxazolo[4,5-c]pyridin-3-yl]-méthanone,
[102] (2-pyrrolidin-1-yl-éthyl)-amide de l'acide 5-(4-bromo-3-méthyl-benzoyl)-4,5,6,7-tétrahydro-isoxazolo[4,5-c]pyridine-3-carboxylique,
[103] [1-(4-trifluorométhyl-benzyl)-pyrrolidin-3-yl]-amide de l'acide 5-benzènesulfonyl-4,5,6,7-tétrahydro-isoxazolo[4,5-c]pyridine-3-carboxylique,
[104] (2-phényl-propyl)-amide de l'acide 5-(thiophène-2-sulfonyl)-4,5,6,7-tétrahydro-isoxazolo[4,5-c]pyridine-3-carboxylique,
[105] phénéthyl-amide de l'acide 5-(3-chloro-4-fluoro-benzoyl)-4,5,6,7-tétrahydro-isoxazolo[4,5-c]pyridine-3-carboxylique,
[106] [2-(4-méthoxy-phénoxy)-éthyl]-amide de l'acide 5-benzènesulfonyl-4,5,6,7-tétrahydro-isoxazolo[4,5-c]pyridine-3-carboxylique,
[107] 3-fluoro-4-trifluorométhyl-benzylamide de l'acide 5-diméthylsulfamoyl-4,5,6,7-tétrahydro-isoxazolo[4,5-c]pyridine-3-carboxylique,
[108] prop-2-ynylamide de l'acide 5-(3-méthyl-benzoyl)-4,5,6,7-tétrahydro-isoxazolo[4,5-c]pyridine-3-carboxylique,
[109] [2-(4-méthoxy-phénoxy)-éthyl]-amide de l'acide 5-(toluène-4-sulfonyl)-4,5,6,7-tétrahydro-isoxazolo[4,5-c]pyridine-3-carboxylique,
[110] [4-(2-chloro-phényl)-pipérazin-1-yl]-[5-(2,5-dichloro-benzènesulfonyl)-4,5,6,7-tétrahydro-isoxazolo[4,5-c]pyridin-3-yl]-méthanone,
[111] ester éthylique de l'acide 3-{[5-(2,6-difluoro-3-méthyl-benzoyl)-4,5,6,7-tétrahydro-isoxazolo[4,5-c]pyridine-3-carbonyl]-amino}-propionique,
[112] 2,4-difluoro-benzylamide de l'acide 5-(2,5-dichloro-benzènesulfonyl)-4,5,6,7-tétrahydro-isoxazolo[4,5-c]pyridine-3-carboxylique,
[113] 3-fluoro-4-trifluorométhyl-benzylamide de l'acide 5-benzènesulfonyl-4,5,6,7-tétrahydro-isoxazolo[4,5-c]pyridine-3-carboxylique,
[114] isobutyl-amide de l'acide 5-(4-butoxy-benzènesulfonyl)-4,5,6,7-tétrahydro-isoxazolo[4,5-c]pyridine-3-carboxylique,
[115] (2-pyrrolidin-1-yl-éthyl)-amide de l'acide 5-(3-fluoro-méthyl-benzoyl)-4,5,6,7-tétrahydro-isoxazolo[4,5-c]pyridine-3-carboxylique,
[116] isobutyl-amide de l'acide 5-(2-chloro-5-trifluorométhyl-benzoyl)-4,5,6,7-tétrahydro-isoxazolo[4,5-c]pyridine-3-carboxylique,
[117] (1-benzyl-pyrrolidin-3-yl)-amide de l'acide 5-(4-trifluorométhoxy-benzènesulfonyl)-4,5,6,7-tétrahydro-isoxazolo[4,5-c]pyridine-3-carboxylique,
[118] (2-pyrrolidin-1-yl-éthyl)-amide de l'acide 5-(3-chloro-benzènesulfonyl)-4,5,6,7-tétrahydro-isoxazolo[4,5-c]pyridine-3-carboxylique,
[119] (2-diméthylamino-éthyl)-amide de l'acide 5-(3-trifluorométhoxy-benzoyl)-4,5,6,7-tétrahydro-isoxazolo[4,5-c]pyridine-3-carboxylique,
[120] ester tert-butylique de l'acide 2-{[5-(toluène-4-sulfonyl)-4,5,6,7-tétrahydro-isoxazolo[4,5-c]pyridine-3-carbonyl]-amino}-propionique,
[121] ester éthylique de l'acide 3-{[5-(4-chloro-benzènesulfonyl)-4,5,6,7-tétrahydro-isoxazolo[4,5-c]pyridine-3-carbonyl]-amino}-propionique,
[122] [2-(3,4-dichloro-phényl)-éthyl]-amide de l'acide 5-diméthylsulfamoyl-4,5,6,7-tétrahydro-isoxazolo[4,5-c]pyridine-3-carboxylique,
[123] cyclopropylméthyl-amide de l'acide 5-(thiophène-2-sulfonyl)-4,5,6,7-tétrahydro-isoxazolo[4,5-c]pyridine-3-carboxylique,
[124] 4-fluoro-benzylamide de l'acide 5-(quinoline-6-carbonyl)-4,5,6,7-tétrahydro-isoxazolo[4,5-c]pyridine-3-carboxylique,
[125] ester éthylique de l'acide 3-{[5-(2-naphtalén-2-yl-acétyl)-4,5,6,7-tétrahydro-isoxazolo[4,5-c]pyridine-3-carbonyl]-amino}-propionique,
[126] ester benzylique de l'acide [2-(3-isobutylcarbamoyl-6,7-dihydro-4H-isoxazolo[4,5-c]pyridin-5-yl)-2-oxo-1-phényl-éthyl)-carbamique,
[127] cyclopentylamide de l'acide 5-(4-méthoxy-benzylthiocarbamoyl)-4,5,6,7-tétrahydro-isoxazolo[4,5-c]pyridine-3-carboxylique,
[128] (2-pyrrolidin-1-yl-éthyl)-amide de l'acide 5-benzoylaminocarbothioyl-4,5,6,7-tétrahydro-isoxazolo[4,5-c]pyridine-3-carboxylique,
[129] cyclopropylméthyl-amide de l'acide 5-(4-chloro-benzylthiocarbamoyl)-4,5,6,7-tétrahydro-isoxazolo[4,5-c]pyridine-3-carboxylique,
[130] phénéthyl-amide de l'acide 5-(2-méthoxy-éthylthiocarbamoyl)-4,5,6,7-tétrahydro-isoxazolo[4,5-c]pyridine-3-carboxylique,
[131] phénéthyl-amide de l'acide 5-pentafluorophénylthiocarbamoyl-4,5,6,7-tétrahydro-isoxazolo[4,5-c]pyridine-3-carboxylique,
[132] phénéthyl-amide de l'acide 5-(1-phényl-éthylthiocarbamoyl-4,5,6,7-tétrahydro-isoxazolo[4,5-c]pyridine-3-carboxylique,
[133] (5-méthyl-furan-2-ylméthyl)-amide de l'acide 5-pentafluorophénylthiocarbamoyl-4,5,6,7-tétrahydro-isoxazolo[4,5-c]pyridine-3-carboxylique,
[134] ester éthylique de l'acide 3-{[5-(cyclohexylméthyl-thiocarbamoyl)-4,5,6,7-tétrahydro-isoxazolo[4,5-c]pyridine-3-carbonyl]-amino}-propionique et
[135] phénylamide de l'acide 5-(1-bromo-naphtalén-2-ylméthyl)-4,5,6,7-tétrahydro-isoxazolo[4,5-c]pyridine-3-carboxylique,
à chaque fois éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou à chaque fois sous la forme de sels correspondants, ou à chaque fois sous la forme de solvates correspondants.

6. Procédé de fabrication de composés de 4,5,6,7-tétrahydro-isoxazolo[4,5-c]pyridine de formule générale I selon une ou plusieurs des revendications 1 à 5, **caractérisé en ce qu'**au moins un composé de formule générale II 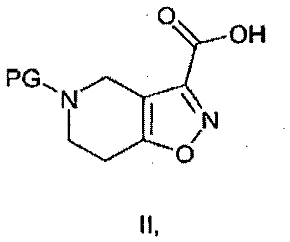 dans laquelle PG représente un groupe protecteur, de préférence un groupe tert-butyloxy-carbonyle ou benzyloxycarbonyle, est mis en réaction dans un milieu réactionnel, en présence d'au moins un réactif de couplage, éventuellement en présence d'au moins une base, de préférence en présence d'au moins d'une base choisie dans le groupe constitué par la pyridine, la N-méthylmorpholine, la diisopropyléthylamine, la triéthylamine et la 4,4-diméthylaminopyridine, avec au moins une amine de formule générale HNR¹R², dans laquelle les radicaux R¹ et R² ont la signification selon une ou plusieurs des revendications 1 à 5, pour former au moins un composé de formule générale III 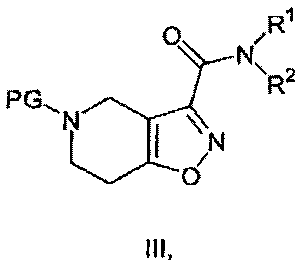 dans laquelle R¹, R² et PG ont la signification indiquée précédemment, et celui-ci est éventuellement purifié et/ou isolé,
et au moins un composé de formule générale III est mis en réaction par clivage dans un milieu réactionnel, de préférence en présence d'au moins un acide ou en présence d'au moins une base pour le groupe tert-butyloxy-carboxyle ou en présence d'hydrogène et d'un catalyseur, de préférence du palladium sur du charbon, pour le groupe benzyloxycarbonyle, pour former au moins un composé de formule générale IV, éventuellement sous la forme d'un sel correspondant, de préférence sous la forme d'un chlorhydrate correspondant, 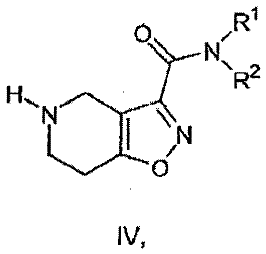 dans laquelle R¹ et R² ont la signification indiquée précédemment, et celui-ci est éventuellement purifié et/ou isolé,
et
éventuellement au moins un composé de formule générale IV sous la forme d'un sel correspondant, de préférence sous la forme d'un chlorhydrate correspondant, est mis en réaction dans un milieu réactionnel, en présence d'au moins une base, de préférence en présence d'au moins un hydroxyde métallique, de manière particulièrement préférée en présence d'hydroxyde de potassium et/ou de sodium, pour former au moins un composé de formule générale IV, et celui-ci est éventuellement purifié et/ou isolé,
et
au moins un composé de formule générale IV est mis en réaction dans un milieu réactionnel, éventuellement en présence d'au moins une base, de préférence en présence d'au moins une base choisie dans le groupe constitué par la pyridine, la triéthylamine, la 4,4-diméthylaminopyridine, la diisopropyléthylamine et la diisopropylamine, avec au moins un dérivé d'acide carboxylique de formule générale LG-C(=O)-R¹⁵, LG-C(=O)-(CH₂)ⱼ-Xₖ- (CH₂)ₘ- (CH₂)ₙ-C (=O) -OR¹⁶, LG-C (=O) - (CHR¹⁹) -NH-C(=O)-OR²¹, LG-C(=O)-(CH₂)-(CH₂)ₚ-(CH₂)_{q}-Yᵣ-(CH₂)ₛ-R²¹ ou LG-C(=O)-(CH=CH)-R²², R¹⁵, R¹⁶, R¹⁹, R²⁰, R²¹, R²², j, k, m, n, p, q, r, s, X et Y ayant la signification selon une ou plusieurs des revendications 1 à 5 et LG représentant un groupe partant, de préférence un atome d'halogène, de manière particulièrement préférée un atome de chlore, pour former au moins un composé de formule générale I, dans laquelle R¹ et R² ont la signification indiquée précédemment et R³ représente - C (=O) -R¹⁵, -C(=O)-(CH₂)ⱼ-Xₖ-(CH₂)ₘ-(CH₂)ₙ-C(=O)-OR¹⁶, - C (=O) - (CHR¹⁹) -NH-C (=O) -OR²⁰, -C (=O) - (CH₂) - (CH₂)ₚ- (CH₂)_{q}-Yᵣ-(CH₂)ₛ-R²¹ ou -C(=O)-(CH=CH)-R²², R¹⁵, R¹⁶, R¹⁹, R²⁰, R²¹, R²², j, k, m, n, p, q, r, s, X et Y ayant la signification indiquée précédemment, et celui-ci est éventuellement purifié et/ou isolé,
ou
au moins un composé de formule générale IV est mis en réaction dans un milieu réactionnel en présence d'un réactif de couplage, éventuellement en présence d'au moins une base, de préférence en présence d'au moins une base choisie dans le groupe constitué par la pyridine, la N-méthylmorpholine, la diisopropyléthylamine, la triéthylamine et la 4,4-diméthylaminopyridine, avec au moins un acide carboxylique de formule générale HO-C(=O)-R¹⁵, HO-C(=O)-(CH₂)ⱼ-Xₖ-(CH₂)ₘ-(CH₂)ₙ-C(=O)-OR¹⁶, HO-C(=O)-(CHR¹⁹) -NH-C(=O)-OR²⁰, HO-C(=O)-(CH₂) - (CH₂)ₚ- (CH₂)_{q}-Yᵣ-(CH₂)ₛ-R²¹ ou HO-C(=O)-(CH=CH)-R²², R¹⁵, R¹⁶, R¹⁹, R²⁰, R²¹, R²², j, k, m, n, p, q, r, s, X et Y ayant la signification selon une ou plusieurs des revendications 1 à 5, pour former au moins un composé de formule générale I, dans laquelle R¹ et R² ont la signification indiquée précédemment et R³ représente -C(=O)-R¹⁵, -C (=O) - (CH₂)ⱼ-Xₖ-(CH₂)ₘ-(CH₂)ₙ-C(=O)-OR¹⁶, -C(=O)-(CHR¹⁹)-NH-C(=O)-OR²⁰, -C (=O) - (CH₂) - (CH₂)ₚ-(CH₂)_{q}-Yᵣ-(CH₂)ₛ-R²¹ ou -C(=O)-(CH=CH)-R²², R¹⁵, R¹⁶, R¹⁹, R²⁰, R²¹, R²², j, k, m, n, p, q, r, s, X et Y ayant la signification indiquée précédemment, et celui-ci est éventuellement purifié et/ou isolé,
ou
au moins un composé de formule générale IV est mis en réaction dans un milieu réactionnel, éventuellement en présence d'au moins une base, de préférence en présence d'au moins une base choisie dans le groupe constitué par la pyridine, la triéthylamine, la 4,4-diméthylaminopyridine, la diisopropyléthylamine et la diisopropylamine, avec au moins un dérivé d'acide sulfonique de formule générale LG-S(=O)₂-R²³ ou LG-S(=O)₂-NR²⁴R²⁵, R²³, R²⁴ et R²⁵ ayant la signification selon une ou plusieurs des revendications 1 à 5 et LG représentant un groupe partant, de préférence un atome d'halogène, de manière particulièrement préférée un atome de chlore, pour former au moins un composé de formule générale I, dans laquelle R¹ et R² ont la signification indiquée précédemment et R³ représente - S(=O)₂-R²³ ou -S(=O)₂-NR²⁴R²⁵, R²³, R²⁴ et R²⁵ ayant la signification indiquée précédemment, et celui-ci est éventuellement purifié et/ou isolé,
ou
au moins un composé de formule générale IV est mis en réaction dans un milieu réactionnel avec au moins un isocyanate de formule générale R¹⁴-N=C=O, R¹⁴ ayant la signification selon une ou plusieurs des revendications 1 à 5, éventuellement en présence d'au moins une base, de préférence en présence d'au moins une base choisie dans le groupe constitué par la triéthylamine, la 4,4-diméthylaminopyridine et la diisopropyléthylamine, pour former au moins un composé de formule générale I, dans laquelle R¹ et R² ont la signification indiquée précédemment et R³ représente - C(=O)-NR¹³R¹⁴, R¹⁴ ayant la signification indiquée précédemment et R¹³ représentant l'hydrogène, et celui-ci est éventuellement purifié et/ou isolé,
ou
au moins un composé de formule générale IV est mis en réaction dans un milieu réactionnel avec au moins un isothiocyanate de formule générale S=C=N-(CH₂)ₜ-(CH₂)ᵤ-Zᵥ-R²⁷, S=C=N-(CHR²⁸)-R²⁹ ou S=C=N-C(=O)-R³⁰, R²⁷ , R²⁸ , R²⁹, R³⁰, Z, t, u et v ayant la signification selon une ou plusieurs des revendications 1 à 5, éventuellement en présence d'au moins une base, de préférence en présence d'au moins une base choisie dans le groupe constitué par la triéthylamine, la 4,4-diméthylaminopyridine et la diisopropyléthylamine, pour former au moins un composé de formule générale I, dans laquelle R¹ et R² ont la signification indiquée précédemment et R³ représente -C(=S)-NR²⁶- (CH₂)ₜ- (CH₂)ᵤ-Zᵥ-R²⁷, -C(=S)-NR²⁶-(CHR²⁸)-R²⁹ ou -C(=S)-NR²⁶-C(=O)-R³⁰, R²⁷, R²⁸, R²⁹, R³⁰, Z, t, u et v ayant la signification indiquée précédemment et R²⁶ représentant l'hydrogène, et celui-ci est éventuellement purifié et/ou isolé,
et éventuellement au moins un composé de formule générale I, dans laquelle R¹ et R² ont la signification indiquée précédemment et R³ représente -C(=S)-NR²⁶-(CH₂)ₜ-(CH₂)ᵤ-Zᵥ-R²⁷, -C(=S)-NR²⁶- (CHR²⁸) -R²⁹, -C(=S) -NR²⁶-C(=O)-R³⁰ ou -C(=O)-NR¹³R¹⁴, R¹⁴, R²⁷, R²⁸, R²⁹, R³⁰, Z, t, u et v ayant la signification indiquée précédemment, et R¹³ et R²⁶ représentant l'hydrogène, est mis en réaction dans un milieu réactionnel, éventuellement en présence d'au moins une base, de préférence en présence d'au moins un sel d'hydrure métallique, de manière particulièrement préférée en présence d'hydrure de sodium et/ou de potassium, avec au moins un composé de formule générale LG-R¹³ ou LG-R²⁶, R¹³ et R²⁶ ayant la signification selon une ou plusieurs des revendications 1 à 5 à l'exception d'un radical hydrogène et LG représentant un groupe partant, de préférence un atome d'halogène, de manière particulièrement préférée un atome de chlore, pour former au moins un composé de formule générale I, dans laquelle R¹ et R² ont la signification indiquée précédemment et R³ représente -C(=S)-NR²⁶-(CH₂)ₜ-(CH₂)ᵤ-Zᵥ-R²⁷, -C(=S)-NR²⁶-(CHR²⁸)-R²⁹, -C(=S)-NR²⁶-C(=O)-R³⁰ ou - C(=O)-NR¹³R¹⁴, R¹³, R²⁶, R¹⁴, R²⁷, R²⁸, R²⁹, R³⁰, Z, t, u et v ayant la signification indiquée précédemment, et celui-ci est éventuellement purifié et/ou isolé,
ou
au moins un composé de formule générale IV est mis en réaction dans un milieu réactionnel, éventuellement en présence d'au moins une base, de préférence en présence d'au moins un sel d'hydrure métallique, de manière particulièrement préférée en présence d'hydrure de sodium et/ou de potassium, avec au moins un composé de formule générale LG-(CH₂)-R³¹, R³¹ ayant la signification selon une ou plusieurs des revendications 1 à 5 et LG représentant un groupe partant, de préférence un atome d'halogène, de manière particulièrement préférée un atome de chlore, pour former au moins un composé de formule générale I, dans laquelle R¹ et R² ont la signification indiquée précédemment et R³ représente - (CH₂)-R³¹, R³¹ ayant la signification indiquée précédemment, et celui-ci est éventuellement purifié et/ou isolé,
ou
au moins un composé de formule générale IV est mis en réaction dans un milieu réactionnel, en présence d'au moins un réducteur, avec au moins un composé de formule générale R³¹-C(=O)-H, R³¹ ayant la signification selon une ou plusieurs des revendications 1 à 5, pour former au moins un composé de formule générale I, dans laquelle R¹ et R² ont la signification indiquée précédemment et R³ représente -(CH₂)-R³¹, R³¹ ayant la signification indiquée précédemment, et celui-ci est éventuellement purifié et/ou isolé.

7. Médicament contenant au moins un composé selon une ou plusieurs des revendications 1 à 5 et éventuellement un ou plusieurs adjuvants physiologiquement compatibles.

8. Médicament selon la revendication 7, pour la prophylaxie et/ou le traitement de la douleur, de préférence de la douleur choisie dans le groupe constitué par la douleur aiguë, la douleur chronique et la douleur neuropathique.

9. Médicament selon la revendication 7, pour la prophylaxie et/ou le traitement d'une ou de plusieurs maladies choisies dans le groupe constitué par la migraine ; les dépressions ; les inflammations ; l'apathie ; l'incontinence urinaire ; les maladies neurodégénératives, de préférence choisies dans le groupe constitué par la maladie de Parkinson, la maladie d'Huntington, la maladie d'Alzheimer et la sclérose en plaques ; les troubles de l'alimentation, de préférence choisis dans le groupe constitué par la boulimie, l'anorexie, l'obésité et la cachexie ; les états d'anxiété ; les dysfonctionnements cognitifs, de préférence les troubles de la mémoire ; les déficiences cognitives (attention deficit syndrom, ADS) ; l'épilepsie ; la catalepsie ; la diarrhée et le prurit ;
pour la prophylaxie et/ou le traitement de l'abus d'alcool et/ou de drogues et/ou de médicaments et de la dépendance à l'alcool et/ou à des drogues et/ou à des médicaments, de préférence pour la prophylaxie et/ou la réduction des phénomènes de sevrage en cas de dépendance à l'alcool et/ou à des drogues et/ou à des médicaments ;
pour la régulation de l'alimentation ; pour la modulation de l'activité physique ; pour la régulation du système cardiovasculaire ; pour l'anesthésie locale ; pour l'augmentation de la vigilance ; pour l'augmentation de la libido ; pour la diurèse ; pour l'antinatriurèse ; pour une utilisation en tant qu'anesthésique local et/ou antiarythmisant et/ou antiémétique et/ou nootropique (neurotropique).

10. Utilisation d'au moins un composé selon une ou plusieurs des revendications 1 à 5 pour la fabrication d'un médicament pour la prophylaxie et/ou le traitement de la douleur, de préférence de la douleur choisie dans le groupe constitué par la douleur aiguë, la douleur chronique et la douleur neuropathique.

11. Utilisation d'au moins un composé selon une ou plusieurs des revendications 1 à 5 pour la fabrication d'un médicament pour la prophylaxie et/ou le traitement d'une ou de plusieurs maladies choisies dans le groupe constitué par la migraine ; les dépressions ; les inflammations ; l'apathie ; l'incontinence urinaire ; les maladies neurodégénératives, de préférence choisies dans le groupe constitué par la maladie de Parkinson, la maladie d'Huntington, la maladie d'Alzheimer et la sclérose en plaques ; les troubles de l'alimentation, de préférence choisis dans le groupe constitué par la boulimie, l'anorexie, l'obésité et la cachexie ; les états d'anxiété ; les dysfonctionnements cognitifs, de préférence les troubles de la mémoire ; les déficiences cognitives (attention deficit syndrom, ADS) ; l'épilepsie ; la catalepsie ; la diarrhée et le prurit ;
pour la prophylaxie et/ou le traitement de l'abus d'alcool et/ou de drogues et/ou de médicaments et de la dépendance à l'alcool et/ou à des drogues et/ou à des médicaments, de préférence pour la prophylaxie et/ou la réduction des phénomènes de sevrage en cas de dépendance à l'alcool et/ou à des drogues et/ou à des médicaments ;
pour la régulation de l'alimentation ; pour la modulation de l'activité physique ; pour la régulation du système cardiovasculaire ; pour l'anesthésie locale ; pour l'augmentation de la vigilance ; pour l'augmentation de la libido ; pour la diurèse ; pour l'antinatriurèse ; pour une utilisation en tant qu'anesthésique local et/ou antiarythmisant et/ou antiémétique et/ou nootropique (neurotropique).
